# EUROPEAN PATENT SPECIFICATION

(11) **EP 1 597 275 B1**
(45) Date of publication and mention of the grant of the patent: **18.08.2010**
(21) Application number: 04713956.3
(22) Date of filing: 24.02.2004
(51) Int. Cl.: C07K 14/295, G01N 33/53

(54) **Use of the Chlamydia pneumoniae CPn0104 polypeptide or antibodies thereto to diagnose Chlamydia infections**
ANWENDUNG DES CPn104 POLYPEPTIDS AUS CHLAMYDIA PNEUMONIAE ODER DESSEN ANTIKÖRPER ZUR DIAGNOSE EINER CHLAMYDIA INFEKTION
Utilisation de la protéine CRn104 de Chlamydia pneumoniae ou d'anticorps contre celle-ci pour le diagnostic d'une infection par Chlamydia

(30) Priority: 24.02.2003 US 448879 P
(43) Date of publication of application: 23.11.2005
(73) Proprietor: INSTITUT PASTEUR, 75724 Paris Cedex 15 (FR); CENTRE NATIONAL DE LA RECHERCHE SCIENTIFIQUE (CNRS), 75794 Paris Cedex 16 (FR)
(72) Inventor: DAUTRY-VARSAT, Alice, F-75014 Paris (FR); SUBTIL-SANDS, Agathe, F-75013 Paris (FR)
(74) Representative: Vialle-Presles, Marie José
(86) International application number: PCT/IB2004/000902
(87) International publication number: WO 2004/074318

(56) References cited:
- WO-A-02/48185
- DATABASE EMBL [Online] 13 September 1999 (1999-09-13), GRIFFAIS R.: "Chlamydia pneumoniae transmembrane protein sequence" XP002287105 Database accession no. AAY34808 & WO 99/27105 A (GENSET) 3 June 1999 (1999-06-03)
- DATABASE EMBL [Online] 13 September 1999 (1999-09-13), GRIFFAIS R.: "Chlamydia pneumoniae transmembrane protein sequence" XP002287106 Database accession no. AAY34704
- STEPHENS R ET AL: "Genome sequence of an obligate intracellular pathogen of humans: Chlamydia trachomatis" SCIENCE, AMERICAN ASSOCIATION FOR THE ADVANCEMENT OF SCIENCE,, US, vol. 282, 23 October 1998 (1998-10-23), pages 754-759, XP002136477 ISSN: 0036-8075 cited in the application
- KALMAN S ET AL: "COMPARATIVE GENOMES OF CHLAMYDIA PNEUMONIAE AND C. TRACHOMATIS" NATURE GENETICS, NEW YORK, NY, US, vol. 21, no. 4, April 1999 (1999-04), pages 385-389, XP000853883 ISSN: 1061-4036 cited in the application
- SUBTIL AGATHE ET AL: "Secretion of predicted Inc proteins of Chlamydia pneumoniae by a heterologous type III machinery" MOLECULAR MICROBIOLOGY, BLACKWELL SCIENTIFIC, OXFORD, GB, vol. 39, no. 3, February 2001 (2001-02), pages 792-800, XP002211783 ISSN: 0950-382X cited in the application
- HSIA R ET AL: "TYPE III SECRETION GENES IDENTIFY A PUTATIVE VIRULENCE LOCUS OF CHLAMYDIA" MOLECULAR MICROBIOLOGY, BLACKWELL SCIENTIFIC, OXFORD, GB, vol. 25, no. 2, 1997, pages 351-359, XP008001280 ISSN: 0950-382X
- FIELDS K A ET AL: "Evidence for the secretion of Chlamydia trachomatis CopN by a type III secretion mechanism" MOLECULAR MICROBIOLOGY, BLACKWELL SCIENTIFIC, OXFORD, GB, vol. 38, no. 5, December 2000 (2000-12), pages 1048-1060, XP002211782 ISSN: 0950-382X

## Description

### Field of the Invention

The present invention relates to secreted *Chlamydia* polypeptides. More particularly, the present invention relates to secreted *Chlamydia* polypeptides expressed by a Gram-negative bacterial strain and secreted by the type III secretion pathway of said bacterial strain. The present invention also relates to the polynucleotides coding for these secreted *Chlamydia* polypeptides, as well as to the therapeutic, including vaccination, and diagnostic uses of these secreted *Chlamydia* polypeptides.

### BACKGROUND OF THE INVENTION

*Chlamydiae* are Gram-negative bacteria that proliferate only within eukaryotic host-cells. The three species pathogenic to humans, *Chlamydia trachomatis, Chlamydia psittaci* and *Chlamydia pneumoniae,* cause a number of diseases, including trachoma, sexually transmitted disease, pelvic inflammatory disease, respiratory diseases, such as bronchitis, pneumonia and their sequelae (Gregory, D.W. and W. Schaffner. (1997). Psittacosis. Seminars in Respiratory Infections. 12: 7-11; Kuo, C.-C., L. Jackson, L. Campbell, and J. Grayston. (1995). Chlamydia pneunoniae (TWAR). Clin. Microbiol. Rev. 8: 451-61; Stamm, W.E. (1999). Chlamydia trachomatis infections: progress and problems. J. Infect. Dis. 179: S380-3). In addition, *Chlamydia psittaci* and *Chlamydia pecorum* have been demonstrated to be pathogenic to animals other than humans, and as such have a significant impact on agricultural economics. In particular, *Chlamydial psittaci* and *Chlamydia pecorum* have been causatively linked to abortion and pneumonitis in cattle, pig, and sheep (Fukushi et al. (1993). Microbiol Immunol. 37(7): 516-522; Tanner et al. (1999). Chlamydia: Intercellular Biology, Pathogenesis, and Immunity. Chapter 1).

During its cycle, *Chlamydia* adopts two distinct morphologies: a small infectious form, the elementary body (EB, 0.3 µm in diameter), and a larger replicative form, the reticulate body (RB, 1 µm in diameter) (Moulder, J.W. (1991). Interaction of chlamydiae and host cells in vitro. Microbiol. Rev. 55: 143-90.). EBs are adapted for survival in the extracellular space: their outer membrane is made rigid by a network of disulfide bonds and the bacteria are metabolically inactive. They initiate infection by attaching to a suitable host cell, principally epithelial cells of the mucosa, and they are internalized by a mechanism resembling phagocytosis (Boleti, H., A. Benmerah, D. Ojcius, N. Cerf-Bensussan, and A. Dautry-Varsat. (1999). Chlamydia infection of epithelial cells expressing dynamin and Eps15 mutants: clathrin-independent entry into cells and dynamin-dependent productive growth. J. Cell. Sci. 112: 1487-1496). Within a few hours after internalization, EBs differentiate into replicative RBs, which proliferate in a growing vacuole and produce up to about a thousand progeny. After 2 to 3 days of infection RBs differentiate back into EBs, which are delivered to the extracellular space, and a new infectious cycle can begin.

Throughout their cycle in the host cell, *Chlamydiae* remain in a membrane-bound compartment called an inclusion. Bacteria escape from host defense mechanisms (i.e., cytosolic proteases or lysosomal enzymes) by preventing fusion of the inclusion with acidic degradative compartments of host cells. In addition, most bacterial antigens avoid detection by the host immune system, since these antigens do not access the infected cell plasma membrane. Inhibition of phagolysosome fusion is limited to *Chlamydia psittaci*-laden vacuoles. (Eissenberg, L.G., and P.B. Wyrick. (1981) Infect. Immun. 32: 889-896; Friis, R.R. (1972). Interaction of L cells and Chlamydia psittaci: entry of the parasite and host responses to its development. J. Bacterial. 110: 706-721; Heinzen, R.A., M.A. Scidmore, D.D. Rockey, and T. Hackstadt. (1996). Differential interaction with endocytic and exocytic pathways distinguish parasitophorous vacuoles of Coxiella burnetii and Chlamydia trachomatis. Infect. Immun. 64: 769-809; Schramm, N., C.R. Bagnell, and P.B. Wyrick. (1996). Vesicles containing Chlamydia trachomatis serovar L2 remain above pH 6 within HEC-1B cells. Infect. Immun. 64:1208-1214). In fact, *Chlamydiae* seem to exchange very little with the endocytic compartments (Taraska, T., D.M. Ward, R.S. Ajioka, P.B. Wyrick, S.R. Davis-Kaplan, C.H. Davis, and J. Kaplan. (1996). The late chlamydial inclusion membrane is not derived from the endocytic pathway and is relatively deficient in host proteins. Infect. Immun. 64: 3713-372; Van Ooij, C., G. Apodaca, and J. Engel. (1997). Characterization of the Chlamydia trachomatis vacuole and its interaction with the host endocytic pathway in HeLa cells. Infect. Immun. 65: 758-766).

Yet, during their development cycle, the volume of the inclusions increases considerably, until they occupy a large portion of the cytosol. Part of the lipids necessary for this growth come from the host cell (Hatch, G.M., and G. McClarty. (1998). Phospholipid composition of purified Chlamydia trachomatis mimics that of the eucaryotic host cell. Infection & Immunity. 66: 3727-35). Sphingomyelin, synthesized in the Golgi apparatus, is transported to the inclusion membrane and incorporated into bacteria (Hackstadt, T., M.A. Scidmore, and D.D. Rockey. (1995). Lipid metabolism in Chlamydia trachomatis- infected cells: directed trafficking of Golgi-derived sphingolipids to the chlamydial inclusion. Proc. Natl. Acad. Sci, USA. 92: 4877-4881).

Surprisingly, no proteins of eukaryotic origin have been found associated with the inclusion. However, a number of prokaryotic proteins have been localized on the membrane of the inclusion, by fluorescence microscopy using antibodies generated against a variety of chlamydial proteins ( Bannantine, J.P., R.S. Griffiths, W. Viratyosin, W.J. Brown, and D.D. Rockey. (2000). A secondary structure motif predictive of protein localization to the chlamydial inclusion membrane. Cell. Microbiol. 2: 35-47; Bannantine, J.P., D.D. Rockey, and T. Hackstadt. (1998). Tandem genes of Chlamydia psittaci that encode proteins located to the inclusion membrane. Mol. Microbiol. 28: 1017-26; Rockey, D.D., R.A. Heinzen, and T. Hackstadt. (1995). Cloning and characterization of a Chlamydia psittaci gene coding for a protein localized in the inclusion membrane of infected cells. Mol. Microbiol. 15: 617-626; Scidmore-Carlson, M.A., E.I. Shaw, C.A. Dooley, E.R. Fischer, and T. Hackstadt. (1999). Identification and characterization of a Chlamydia trachomatis early operon encoding four novel inclusion membrane proteins. Mol. Microbial. 33: 753-765). These proteins show no sequence similarities but have in common the presence of a large (40 to 70 amino acids) hydrophobic region and have therefore been grouped into a structural family called the Inc family.

The three first Inc proteins identified, IncA, IncB and IncC, are probably major components of the inclusion since they are recognized by antisera from convalescent guinea pigs infected with *C*. *psittaci* (Bannantine, J., W. Stamm, R. Suchland, and D. Rockey. (1998). Chlamydia trachomatis IncA is localized to the inclusion membrane and is recognized by antisera from infected humans and primates. Infect. Immun. 66: 6017-6021; Rockey, D.D., R.A. Heinzen, and T. Hackstadt. (1995). Cloning and characterization of a Chlamydia psittaci gene coding for a protein localized in the inclusion membrane of infected cells. Mol. Microbiol. 15: 617-626). They have homologs encoded by *C*. *trachomatis and C. pneumoniae* genomes, exhibiting about 20% global sequence identity and up to 50% sequence identity in the regions of highest similarity.

Inc proteins have been grouped into a family on two criteria: they have a large (larger than 40 residues) hydrophobic domain and they localize to the membrane of the inclusion in the host cell. The demonstration that several proteins encoded by the *C. trachomatis* genome and one protein from *C*. *pneumoniae* belong to this family was based on their localization to the membrane of the inclusion by immunofluorescence using specific antibodies (Bannantine, J., W. Stamm, R. Suchland, and D. Rockey. (1998). Chlamydia trachomatis IncA is localized to the inclusion membrane and is recognized by antisera from infected humans and primates. Infect. Immun. 66: 6017-6021;Bannantine, J.P., R.S. Griffiths, W. Viratyosin, W.J. Brown, and D.D. Rockey. (2000). A secondary structure motif predictive of protein localization to the chlamydial inclusion membrane. Cell. Microbiol. 2: 35-47; Bannantine, J.P., D.D. Rockey, and T. Hackstadt. (1998). Tandem genes of Chlamydia psittaci that encode proteins located to the inclusion membrane. Mol. Microbiol. 28: 1017-26; Rockey, D.D., R.A. Heinzen, and T. Hackstadt. (1995). Cloning and characterization of a Chlamydia psittaci gene coding for a protein localized in the inclusion membrane of infected cells. Mol. Microbiol. 15: 617-626.; Scidmore-Carlson, M.A., E.I. Shaw, C.A. Dooley, E.R. Fischer, and T. Hackstadt. (1999). Identification and characterization of a Chlamydia trachomatis early operon encoding four novel inclusion membrane proteins. Mol. Microbiol. 33: 753-765). However, the mechanism of their secretion and insertion into the membrane of the inclusion had not been investigated yet, due to the lack of genetic tools for members of *Chlamydia* spp.

How do chlamydial proteins reach the membrane of the inclusion? Four pathways of protein secretion (types I to IV) have been described in Gram-negative bacteria. Type III secretion machines allow for the translocation of bacterial effectors into the eukaryotic cell cytosol during bacterial contact with the cell surface (Hueck, C. (1998). Type III protein secretion systems in bacterial pathogens of animals and plants. Microbiol. Mol. Biol. Rev. 62: 379-433). The inventors hypothesized that *Chlamydiae* use type III secretion to insert proteins into the membrane of the inclusion (Subtil A., A. Blocker, and A. Dautry-Varsat. (2000). Type III secretion system in Chlamydia: identified members and candidates. Microbes Infect. 2: 367-369). Presence of genes encoding putative components of a type III secretion machinery in the *Chlamydia* genome suggested that Inc proteins might be secreted by a type III secretion pathway since type III secretion is used by a large number of Gram-negative pathogens for the insertion or the translocation of bacterial proteins into or through eukaryotic cell membranes. In the absence of genetic tools to manipulate the *Chlamydia* genome, it was difficult to test this hypothesis directly. *Shigella* uses a type III secretion system for entry into epithelial cells and for dissemination (Nhieu, G.T., and P.J. Sansonetti. (1999). Mechanism of Shigella entry into epithelial cells. Curr. Opin. Microbiol. 2: 51-5; Page, A.-L., H. Ohayon, P.J. Sansonetti, and C. Parsot. (1999). The secreted IpaB and IpaC invasins and their cytoplasmic chaperone IpgC are required for intercellular dissemination of Shigella flexneri. Cell. Microbiol. 1: 183-193; Schuch, R., R.C. Sandlin, and A.T. Maurelli. (1999). A system for identifying post-invasion functions of invasion genes: requirements for the Mxi-Spa type III secretion pathway of Shigella flexneri in intercellular dissemination, Mol. Microbiol. 34:675-689). The secretion signal involved in secretion of proteins by the type III secretion pathway is not known. However, it is often located either within the first fifteen codons of the mRNA coding for the secreted proteins, or within the N-terminal part of the secreted protein (Anderson, D.M., and O. Schneewind. (1999). Type III machines of Gram-negative pathogens: injecting virulence factors into host cells and more. Curr. Opin. Microbiol. 2: 18-24). Although the genomes of several *Chlamydia* species are now available, it is not possible to identify secreted proteins on the basis of their amino acid sequence.

Genes coding for the type III secretion system in *Shigella* are known (Hueck, C. (1998). Type III protein secretion systems in bacterial pathogens of animals and plants. Microbiol. Mol. Biol. Rev. 62: 379-433) and it has been hypothesized that Inc proteins may be secreted by a similar machinery.

Expression of heterologous proteins by use of type III machinery of *Shigella* has already been obtained (see "Functional conservation of the Salmonella and Shigella effectors of entry into epithelial cells" published in Molecular Microbiology (1995) 17 (4), 781-789, Hermant, Ménard, Arricau, Parsot and Popoff). However, as indicated in the title, this document relates to *Salmonella* proteins, which are different from the *Chlamydia* proteins due to the phylogenic distance of *Chlamydia* from other organisms (see page 369, first column of Subtil, et al.; Microbes and Infection 2, 2000; Subtil A., A. Blocker, and A. Dautry-Varsat. (2000). Type III secretion system in Chlamydia: identified members and candidates. Microbes Infect. 2: 367-369).

Furthermore, methods for screening inhibitors and activators of type III secretion machinery in Gram-negative bacteria, in *Shigella,* have already been disclosed in WO 99/58714.

Several Gram-negative pathogens, including *Chlamydiae,* possess a type III secretion machinery for the insertion or the translocation of bacterial proteins into or through eukaryotic membranes. In U.S. Patent Application 10/014,670 (the entire disclosure of which is incorporated herein by reference), the present inventors constructed chimeras by fusing the N-terminal part of these proteins with a reporter gene, the Cya protein of *Bordetella pertussis*, and expressed these chimeras in various strains of *Shigella flexneri.* The use of a reporter gene is well known by those of ordinary skill in the art. The reporter sequence, which codes for an easily detectable protein, is linked to a fragment of the DNA of interest. The use of the *B. pertussis cya* gene as a reporter gene, chosen in the present invention, is reported by: Jones *et al*., 1998 and Sory and Cornelis, 1994 (Jones, M.A., M.W. Wood, P.B. Mullan, P.R. Watson, T.S. Wallis, and E.E. Galyov. (1998). Secreted effector proteins of Salmonella dublin act in concert to induce enteritis. Infection & Immunity. 66: 5799-804; Sory, M.P., and G.R. Cornelis. (1994). Translocation of a hybrid YopE-adenylate cyclase from Yersinia enterocolitica into HeLa cells. Mol. Microbiol. 14: 583-94). Moreover, the inventors have shown that several chlamydial proteins, including members of the Inc family and proteins selected for a hydropathic profile similar to that of Inc proteins, are secreted by the type III secretion machinery of S. *flexneri.* Identification of such proteins is very important since these proteins may be involved in functions essential for the intracellular survival of *Chlamydia.* They may serve as a basis for the development of a *Chlamydiae* vaccine or a treatment against *Chlamydia* infection, by suggesting non-immune therapeutic interventions capable of inhibiting bacterial development as well as for the development of *Chlamydia* infection diagnosis.

A need continues to exist, however, for further elucidation of chlamydial polypeptides that are secreted, as well as the diagnostic and therapeutic uses of the same.

The inventors describe herein the identification of several new proteins secreted by *Chlamydia spp.* during infection. Most of these proteins are from the human pathogens *C*. *trachomatis* and *C*. *pneumoniae,* and a few proteins are from one strain of the human and animal pathogen *C*. *psittaci.* In several cases the inventors have shown that if a protein is secreted in one species, homologous proteins from other species are also secreted (see below, category 1). Therefore knowledge that a protein is secreted in one species gives a good indication that it is also true in another species and this can be checked with the method described herein. Thus, the results presented here can also be extended to different *Chlamydia* species, to be used as a basis for the development of a *Chlamydiae* vaccine or a treatment against *Chlamydia* infection as well as for the development of *Chlamydia* infection diagnosis, in humans or in animals.

It is an object of the present invention to provide a method for *in vitro* diagnosing a *Chlamydia* infection in an animal, including a human. A method for *in vitro* diagnosing a *Chlamydia* infection in an animal, comprises (a) providing a purified CPn0104 polypeptide from *Chlamydia pneumoniae,* or an immunogenic fragment thereof, optionally labeled; (b) bringing said polypeptide or immunogenic fragment thereof into contact with a serum sample of said animal; and (c) detecting complexes formed between said polypeptide or immunogenic fragment thereof and antibodies contained in the serum sample; wherein said complexes are indicative of a *Chlamydia* infection in said animal.

Alternatively, a method for *in vitro* diagnosing a *Chlamydia* infection in an animal, including a human, comprises (a) adding an antibody, optionally labelled, directed against the CPn0104 polypeptide from *Chlamydia pneumoniae,* under conditions suitable for antigen-antibody complex formation, to an animal sample of a tissue suspected to be infected by *Chlamydia;* and (b) detecting antigen-antibody complex formation ; wherein said complex formation is indicative of a *Chlamydia* infection in said animal.

The Inventors disclose a purified secreted polypeptide from *Chlamydia* spp., e.g., *Chlamydia pneumoniae, Chlamydia trachomatis, Chlamydia pecorum,* or *Chlamydia psittaci.* The purified polypeptide may be selected by a method comprising (a) providing a recombinant expression vector containing at least a polynucleotide coding for the polypeptide of interest; (b) transforming a Gram-negative strain containing a type III secretion pathway with said recombinant vector; (c) expressing this vector in the Gram-negative strain transformed in (b); and (d) detecting the secretion of said polynucleotide expression product; wherein the secretion of said expression product indicates that it corresponds to a secreted *Chlamydia* polypeptide. The detection of the secretion of expression product is made by detecting the presence of said product outside the bacteria.

Alternatively, the purified polypeptide may be selected by a method comprising (a) providing a recombinant expression vector comprising at least a DNA coding for the polypeptide of interest fused to a reporter gene (i.e., the recombinant expression vector comprises a fusion construct in which one component of the construct is the polynucleotide of interest and a second component of the construct is the polynucleotide sequence of a reporter gene); (b) transforming a Gram-negative strain containing a type III secretion pathway with said recombinant vector; (c) expressing this vector in the Gram-negative strain transformed in (b); and (d) detecting the secretion of said reporter gene expression product; wherein the secretion of said expression product indicates that the fused DNA (i.e., the fusion construct of (a), which contains the polynucleotide of interest and a polynucleotide sequence of a reporter gene) contains at least a polynucleotide corresponding to a secreted *Chlamydia* polypeptide. The detection of the secretion of expression product is performed by detecting the presence of said product outside the bacteria.

The Inventors disclose a purified polynucleotide coding for at least one polypeptide that is secreted from *Chlamydia.*

The Inventors disclose an immunogenic composition comprising a secreted *Chlamydia* polypeptide, or an immunogenic fragment thereof.

The Inventors disclose a vaccinating composition against *Chlamydia* infection comprising a secreted *Chlamydia* polypeptide, or an immunogenic fragment thereof.
Said composition is a human and/or a veterinary vaccinating composition. For example, said infection is a sexually transmitted disease, respiratory disease, such as pneumonia or bronchitis, or contributes to atherosclerosis.

The Inventors disclose a therapeutic composition active against *Chlamydia* infection comprising a molecule, which inhibits the secretion of a secreted *Chlamydia* polypeptide.

The Inventors disclose a complex comprising a secreted *Chlamydia* polypeptide and an antibody directed against said polypeptide.

The Inventors disclose an antibody against *Chlamydia* wherein the antibody is directed against a secreted *Chlamydia* polypeptide, or an immunogenic fragment thereof, wherein said antibody is a monoclonal antibody or a polyclonal antibody.

Said purified polypeptides of *Chlamydia* may be identified by their expression and secretion in a Gram-negative bacterial strain containing a type III secretion pathway.

The Inventors disclose a method of detecting *Chlamydia* in an animal, including a human, using a probe or primer pair selected from Table I or II. The method for detecting *Chlamydia* in an animal comprises (a) providing an animal sample of a tissue suspected to be infected by *Chlamydia;* (b) adding a primer pair selected from the list presented in Tables I and II to the tissue sample; (c) amplifying a polynucleotide that encodes for the protein which corresponds to the primer pair selected; and (d) detecting the presence of *Chlamydia* by the presence or absence of said polynucleotide.

The Inventors disclose a method of detecting *Chlamydia* in an animal, including a human, using a probe that hybridizes under stringent conditions with a polynucleotide that encodes a polypeptide of the present invention. The method for detecting *Chlamydia* in an animal comprises (a) providing an animal sample of a tissue suspected to be infected by *Chlamydia;* (b) adding a hybridization probe that hybridizes with a polynucleotide that encodes a polypeptide as defined above under stringent conditions; and (c) detecting the presence of *Chlamydia* by the presence or absence of said polynucleotide.

The Inventors disclose a method of detecting *Chlamydia* in an animal, including a human, using an antibody, optionally labeled, that forms a complex with a polypeptide as defined above. The method for detecting *Chlamydia* in an animal comprises (a) providing an animal sample of a tissue suspected to be infected by *Chlamydia;* (b) adding an antibody, optionally labeled, that forms a complex with said polypeptide of the present invention under conditions suitable for complex formation; and (c) detecting the presence of *Chlamydia* by the presence or absence of said polypeptide.

The Inventors disclose a recombinant plasmid for the expression of a secreted *Chlamydia* polypeptide.

The Inventors disclose a recombinant prokaryotic cell, for example a recombinant Gram-negative bacterial strain, transformed by a vector comprising at least a polynucleotide encoding a secreted *Chlamydia* polypeptide.

The Inventors disclose a method of preventing or treating a *Chlamydia* infection in an animal, including a human, which comprises administering an effective amount of a purified secreted polypeptide, or immunogenic fragment thereof, of *Chlamydia* as defined above or immunogenic fragment thereof.

The Inventors disclose a method of preventing or treating a *Chlamydia* infection in an animal, which comprises administering an effective amount of an antibody directed against a secreted *Chlamydia* polypeptide according as defined above, or an immunogenic fragment thereof, to an animal in need thereof.

The Inventors disclose a method of screening for an active molecule inhibiting the secretion of a secreted *Chlamydia* polypeptide, comprising (a) supplying an active molecule to a culture of *Chlamydia;* (b) growing or incubating said culture for a time and under conditions suitable for said active molecule to exert an activity upon said culture; (c) adding a primer pair for a *Chlamydia* polypeptide to the culture; (d) amplifying a polynucleotide that encodes for the polypeptide which corresponds to the primer pair selected; and (e) detecting the presence of the secreted *Chlamydia* polypeptide by the presence or absence of said polynucleotide.

The Inventors disclose a method of detecting the presence of a *Chlamydia* polypeptide by using a probe that hybridizes under stringent conditions with a polynucleotide that encodes the polypeptide or by using antibodies, optionally labeled, that form a complex with the polypeptide.

The Inventors disclose a method of screening for an active molecule inhibiting the secretion of a secreted *Chlamydia* polypeptide, comprising (a) supplying an active molecule to a culture of *Chlamydia;* (b) growing or incubating said culture for a time and under conditions suitable for said active molecule to exert an activity upon said culture; and (c) evaluating the presence of a polypeptide according as defined above.

The above objects highlight certain aspects of the invention. Additional objects, aspects and embodiments of the invention are found in the following detailed description of the invention.

### BRIEF DESCRIPTION OF THE DRAWING

A more complete appreciation of the invention and many of the attendant advantages thereof will be readily obtained as the same becomes better understood by reference to the following Figures in conjunction with the detailed description below.

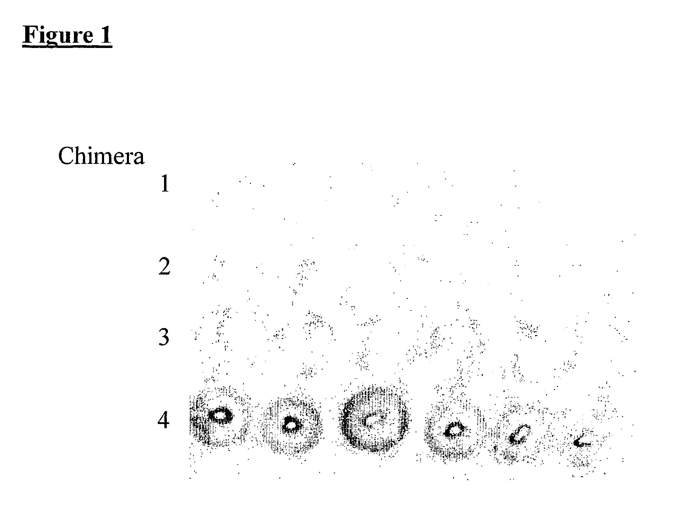

Figure 1: Illustration of the secretion assay in *Shigella.* The *ipaB* mutant strain of *Shigella* constitutively secretes high level of effectors by the type III pathway. This strain was transformed with different chimeric constructs and secretion of the chimeras was probed on colonies. Colonies expressing the different constructs were picked on a fresh LB plate in the morning and incubated for 8 hours at 37°C. The colonies were then covered with a PVDF membrane and grown overnight at 37°C. Secretion of the chimera was detected on this membrane by Western blot using anti-cyclase antibody. Secreted proteins formed a halo around the colonies (2 lower rows) while non-secreted proteins were detected only on the spot where the colonies had grown (2 upper rows).

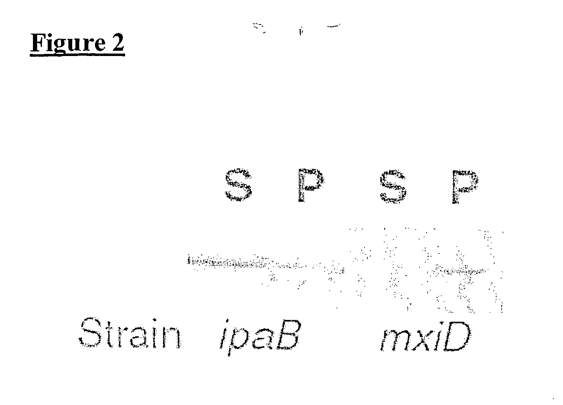

Figure 2: Full length chlamydial proteins are secreted by *Shigella.* The ipaB mutant strain of *Shigella* constitutively secretes a high level of effectors by the type III pathway, while the *mxiD* mutant strain is totally deficient for type III secretion. In this experiment, the strains were transformed with Psi1058 and the presence of the protein in the culture supernatant was probed by Western blot. Psi1058 was found in the supernatant (S) fraction of the *ipaB* stain culture. In the *mxiD* strain it was found only associated with the pellet (P) fraction. This result shows that Psi1058 is secreted by a type III mechanism in *S*. *flexneri.* The same result was found with *S*. *flexneri* transformed with CPn0175, Psi705, Psi725, Psi774 (SEQ ID NO:124), and Psi1005 (SEQ ID NO: 128).

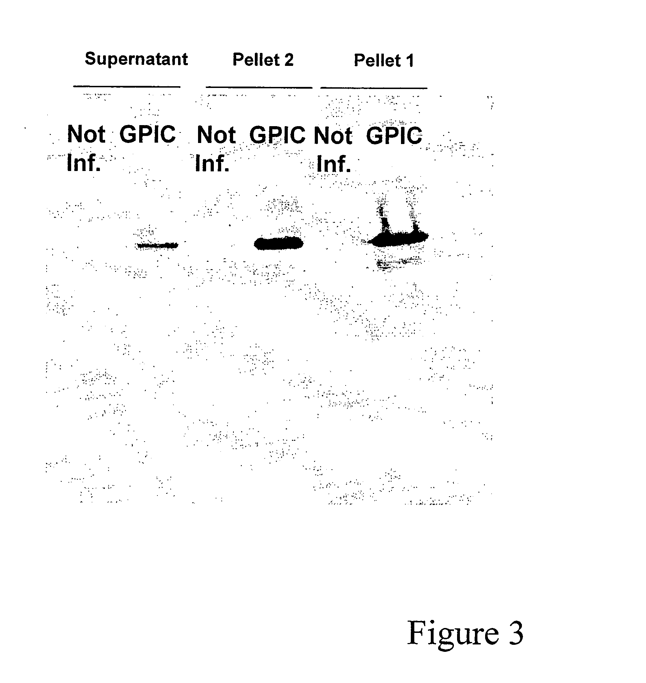

Figure 3: HeLa cells were infected or not for 24 hours with *C. psittaci* GPIC strain, scrapped and broken by 7 passages through a 2SG1^{1/2} syringe needle in homogenization buffer (0.25 M sucrose, 0.1% gelatin, 0.5 mM EGTA, 3 mM imidazole pH 7.4). The cells were centrifuged for 15 minutes at 800 g, the pellet was resuspended in gel sample buffer (pellet 1 contains unbroken cells, bacteria and cell nuclei) and the supernatant was centrifuged for 45 minutes at 541 000 g (pellet 2 contains cellular membranes and bacteria, the supernatant contains proteins present in the cytosol). Equal fractions of pellets and supernatant were analyzed by electrophoresis in 8% polyacrylamide gels in the presence of sodium dodecyl sulfate (SDS-PAGE). After electrophoresis, proteins were transferred on a PVDF membrane (Millipore Corporation, Bedford, MA), and the membrane was used for blotting with anti-Psi0705 antibody. Western blotting and revelation were performed by enhanced chemiluminescence (Amersham Pharmacia Biotech) according to the manufacturer's instructions. Psi0705 was present in the pellet fractions as well as in the supernatant fraction (cytosolic material). As a control MOMP (major outer membrane protein) was present only in the pellet fractions.

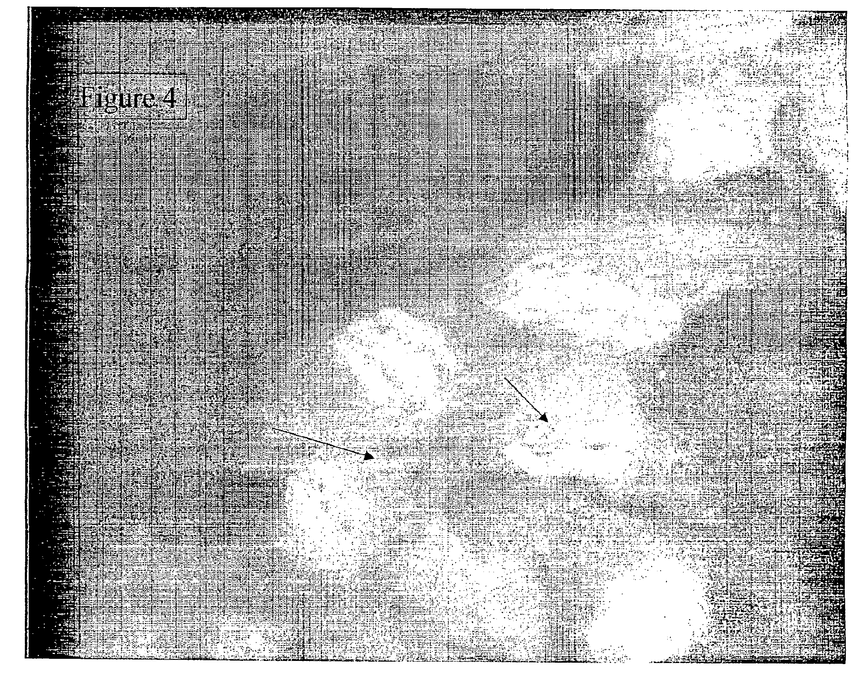

Figure 4: HeLa cells were infected for 24 hours with *C. psittaci* GPIC strain, fixed and permeabilized with 0.05% saponin. Psi0710 was labeled with specific rabbit antibodies raised against the purified protein, followed by anti-rabbit Alexa488-coupled secondary antibodies (Molecular Probes). Cells were examined under a epifluorescence microscope attached to a cooled CCD-camera. Arrowheads point to fibers extending from the membrane of inclusion, to which Psi0710 is associated.

Unless specifically defined, all technical and scientific terms used herein have the same meaning as commonly understood by a skilled artisan in biochemistry, molecular biology, enzymology, genetics, immunology, general medicine and veterinary medicine.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will control. Further, the materials, methods, and examples are illustrative only and are not intended to be limiting, unless otherwise specified.

A "polypeptide" as used herein is understood to mean a sequence of several amino acid residues linked by peptide bonds. Such amino acids are known in the art and encompass the unmodified and modified amino acids. In addition, one or more modifications known in the art such as glycosylation, phosphorylation, etc may modify the polypeptide.

The term "secreted *Chlamydia* polypeptide" as used herein is understood to mean a *Chlamydia* protein, or fragment of the protein, comprising at least 20 amino acids, detectable outside the bacteria.

The term "homologous" as used herein is understood to mean two or more proteins of *Chlamydia* from the same species or from a different species. Within the meaning of this term, said two or more proteins share at least 40% identity to a fragment having at least 50 amino acids. Preferably, homologous *Chlamydia* proteins share at least 50% identity to a fragment having at least 50 amino acids. More preferably, homologous *Chlamydia* proteins share at least 60% identity, at least 70% identity, at least 80% identity, at least 90% identity, at least 95% identity, or 100% identity to a fragment having at least 50 amino acids.

The term "outside the bacteria" as used herein is understood to mean that a certain portion of the polypeptide as defined above produced by the bacteria has crossed the inner membrane, the periplasm and the outer membrane of the bacteria and is either still bound to the outer membrane, found in the extracellular medium or found inside the host cell.

As used herein, the term "active molecule inhibiting the secretion of a secreted *Chlamydia* polypeptide" is understood to mean a molecule able to reduce, including to totally remove, said secretion by any means. For example, said reduction, including total removal, of secretion may result from an action of said molecule on the type III secretion system or on the expression of said polypeptide by the bacteria.

The term "type III secretion pathway" as used herein is understood to mean a complex association of various molecules, which are necessary for secretion of a polypeptide in a host in which it is normally expressed, as described in Hueck CJ. (1998), Type III protein secretion systems in bacterial pathogens of animals and plants. Microbiol Mol Biol Rev. 62: 379-433.

The term "immunogenic fragment" as it relates to polypeptides is understood to mean a polypeptide fragment of at least 6 amino acids and preferably at least 10 amino acids sufficient to induce an immune response when it is administered to a host eukaryotic organism.

The term "heterologous" as it relates to protein or polypeptide secretion systems is understood to mean that the protein or polypeptide is not normally expressed in a host.

Nucleic acids can be detected utilizing a nucleic acid amplification technique, such as polymerase chain reaction (PCR). Alternatively, the nucleic acid can be detected utilizing direct hybridization or by utilizing a restriction fragment length polymorphism.

Hybridization protocols are known in the art and are disclosed, for example, in Sambrook et al., Molecular Cloning: A Laboratory Manual, Cold Spring Harbor Laboratory, New York (1989).

The term "*Chlamydia* infection" as used herein means an infection in an animal, especially a human, that is caused by a species of *Chlamydia*; i.e., *Chlamydia trachomatis, Chlamydia psittaci, Chlamydia pneumoniae* and *Chlamydia pecorum.* According to the present invention, a *Chlamydia* infection is preferably a *Chlamydia pneumoniae* infection.
Within the context of the present invention, a *Chlamydia* infection causes a number of diseases, including trachoma, sexually transmitted disease, pelvic inflammatory disease, respiratory diseases, such as bronchitis, pneumonia and their sequelae, such as atherosclerosis.

As used herein, stringent hybridization conditions are those conditions which allow hybridization between polynucleotides that are 75%, 80%, 85%, 90%, 95%, or 98% homologous as determined using conventional homology programs, an example of which is UWGCG sequence analysis program available from the University of Wisconsin. (Devereaux et al., Nucl. Acids Res. 12: 387-397 (1984)). Such stringent hybridization conditions typically include washing the hybridization reaction mixture in 2X SSC and 0.5% SDS at 65°C (Sambrook et al., Molecular Cloning: A Laboratory Manual, Cold Spring Harbor Laboratory, New York (1989)). Of course, one of skill in the art will recognize that conditions can be varied depending on the length of the polynucleotides to be hybridized and the GC content of the polynucleotides see, for example, Sambrook et al., Molecular Cloning: A Laboratory Manual, Cold Spring Harbor Laboratory, New York (1989).

"Primer" or "probe" means a polynucleotide, especially an oligonucleotide, which is produced synthetically or biologically and includes a specific nucleotide sequence, which permits hybridization to a section containing the target nucleotide sequence. The phrase "primer pair selected from the list presented in Tables I and II" means a single forward primer and the corresponding reverse primer for the protein of interest.

Defined primers or probes, as well as all other oligonucleotides and polynucleotides as defined above, may be produced by any of several well-known methods, including automated solid-phase chemical synthesis using cyanoethyl-phosphoramidite precursors. Other well-known methods for construction of synthetic primers/oligonucleotides may, of course, be employed. J. Sambrook, E. F. Fritsch and T. Maniatis, Molecular Cloning 11 (2d ed. 1989).

The primers used to amplify the sample nucleic acids may be coupled to a detectable moiety. A preferred example of such a detectable moiety is fluorescein, which is a standard label used in nucleic acid sequencing systems using laser light as a detection system. Other detectable labels can also be employed, however, including other fluorophores, radio labels, chemical couplers such as biotin which can be detected with streptavidin-linked enzymes, and epitope tags such as digoxigenin detected using antibodies. The primers may be modified whereby another nucleotide is added to, removed from, or substituted for at least one nucleotide in the oligonucleotide. Introduction of known labels such as radioactive substances, enzymes, fluorescence substances, etc. after synthesis of oligonucleotide is also included therein.

Similarly, the probes/oligonucleotides used to hybridize with the polynucleotides coding for the polypeptides as defined above, for example for the purpose of detection of such a polynucleotide, may be coupled to a detectable moiety.

Examples of suitable primers for use as defined above are shown in Table I.

The inventors have constructed chimeras between the N-terminal domain of several chlamydial proteins and a reporter protein, the calmodulin-dependent adenylate cyclase (Cya) from *B. pertussis,* and have tested whether these chimeras were secreted by *S*. *flexneri.* Proteins analyzed in this study can be classified into five categories.
(i) Corresponding chimeras from *C*. *pneumoniae, C. trachomatis,* and *C. psittaci* that are homologous and are secreted. Sequence analysis has shown that these three species are rather distant, and, as a consequence, the percentage of amino acid identity of the N-terminal domain between homologous proteins is generally low. Consequently, the chimeras constructed from homologous proteins share little identity in their amino terminal region, which is the region containing the putative secretion signal. Therefore, the identification that the three homologous chimeras (one from each of the *Chlamydia* species above) are secreted strongly supports that the corresponding proteins are secreted during *Chlamydia* infection. This category comprises the following polypeptides: CPn0330; CT083; Psi0330; CPn0379; CT053; Psi0379; CPn0595; CT476; Psi0595; CPn0648; CT529; Psi0648; CPn 0671; CT550; Psi0671; CPn0710; CT666; Psi0710; CPn0761; CT610; Psi0761; CPn0774; CT606.1; Psi0774; CPn1002; CT845; Psi1002; CPn1005; CT848; Psi 1005; CPn1022; CT863; Psi1022.
(ii) Proteins of *C*. *pneumoniae* and *C. trachomatis* which are homologous and for which the 2 corresponding chimeras are secreted. For the same reasons as explained above in the case of category 1, these results support that these proteins are also secreted during *Chlamydia* infection. This category comprises the following polypeptides: CPn0490; CT387; CPn0705; CT671; CPn0711; CT665; CPn0712; CT664; CPn0725; CT652.1; CPn0770; CT642; CPn0859; CT718; CPn0906; CT763.
(iii) *C. pneumoniae* proteins that are secreted, which have no homolog in *C. trachomatis* or *C. psittaci.* They may be important proteins, being specific of *C. pneumoniae.* This category comprises the following polypeptides: CPn0009; CPn0012; CPn0028; CPn0049; CPn0063; CPn0066; CPn0067; CPn0130; CPn0132; CPn0146; CPn0167; CPn0174; CPn0175; CPn0181; CPn0210; CPn0211; CPn0220; CPn0223; CPn0226; CPn0243; CPn0267; CPn0277; CPn0284; CPn0357; CPn0365; CPn0585; CPn0829; CPn1027.
(iv) *C. pneumoniae* proteins that have a homolog in at least one of the other 2 sequenced *Chlamydia* species and for which secretion of the corresponding homologous chimera is undetermined. This category comprises the following polypeptides: CPn0026; CPn0104; CPn0186; CPn0206; CPn0291; CPn0292; CPn0405; CPn0443; CPn0480; CPn0489; CPn0556; CPn0673; CPn0681; CPn0720; CPn0746; CPn0853; CPn0879; CPn0939; CPn1019; CPn1020; CPn1032.
(v) *C. pneumoniae* proteins that have homologs in the other 2 sequenced *Chlamydia* species and for which secretion of the corresponding homologous chimeras are negative or undetermined. Although these results support that most proteins in this category are secreted by *Chlamydia* during infection, the Inventors regard them as somewhat less strong candidates than the proteins classified in the other 4 categories, until more data are obtained (see for example the result with Psi1058 below). This category comprises the following polypeptides: CPn0105; CPn0287; CPn0334; CPn0374; CPn0399; CPn0497; CPn0522; CPn0582; CPn0588; CPn0729; CPn0755; CPn0764; CPn0792; CPn0820; CPn0821; CPn1007; CPn1016; CPn1058.

The polypeptides as defined above are administered in a dose, which is effective to vaccinate an animal, preferably a human, against *Chlamydial* infection or to treat an animal, preferably a human, having a *Chlamydial* infection. As used herein, an effective amount of the polypeptides to achieve this goal is generally from about 2 ng to 2 mg/kg of body weight per week, preferably about 2 µg/kg per week. This range includes all specific values and subranges therebetween.

In a preferred embodiment, the *Chlamydia* polypeptide is homologous to one or more *Chlamydia pneumoniae* proteins selected from the group consisting of CPn0104 (SEQ ID NO: 2), CPn0206 (SEQ ID NO: 4), CPn0210 (SEQ ID NO: 6), CPn0399 (SEQ ID NO: 8), CPn0405 (SEQ ID NO: 10), CPn0443 (SEQ ID NO: 12), CPn0480 (SEQ ID NO: 14), CPn0489 (SEQ ID NO: 16), CPn0490 (SEQ ID NO: 18), CPn0497 (SEQ ID NO: 20), CPn0522 (SEQ ID NO: 22), CPn0556 (SEQ ID NO: 24), CPn0582 (SEQ ID NO: 26), CPn0588 (SEQ ID NO: 28), CPn0595 (SEQ ID NO: 30), CPn0671 (SEQ ID NO: 32), CPn0673 (SEQ ID NO: 34), CPn0681 (SEQ ID NO: 36), CPn0712 (SEQ ID NO: 38), CPn0720 (SEQ ID NO: 40), CPn0725 (SEQ ID NO: 42), CPn0729 (SEQ ID NO: 44), CPn0746 (SEQ ID NO: 46), CPn0755 (SEQ ID NO: 48), CPn0761 (SEQ ID NO: 50), CPn0764 (SEQ ID NO: 52), CPn0770 (SEQ ID NO: 54), CPn0774 (SEQ ID NO: 56), CPn0792 (SEQ ID NO: 58), CPn0853 (SEQ ID NO: 60), CPn0859 (SEQ ID NO: 62), CPn0879 (SEQ ID NO: 64), CPn0906 (SEQ ID NO: 66), CPn0939 (SEQ ID NO: 68), CPn1002 (SEQ ID NO: 70), CPn1005 (SEQ ID NO: 72), CPn1007 (SEQ ID NO: 74), CPn1019 (SEQ ID NO: 76), CPn1020 (SEQ ID NO: 78), CPn1032 (SEQ ID NO: 80), and CPn1058 (SEQ ID NO: 82); or a fragment thereof.

In another embodiment, the homologous *Chlamydia* polypeptide is a *Chlamydia trachomatis* protein selected from the group consisting of CT387 (SEQ ID NO: 84), CT476 (SEQ ID NO: 86), CT550 (SEQ ID NO: 88), CT606.1 (SEQ ID NO: 90), CT610 (SEQ ID NO: 92), CT642 (SEQ ID NO: 94), CT652.1 (SEQ ID NO: 96), CT664 (SEQ ID NO: 98), CT718 (SEQ ID NO: 100), CT763 (SEQ ID NO: 102), CT845 (SEQ ID NO: 104), and CT848 (SEQ ID NO: 106); or a fragment thereof.

In still another embodiment, the homologous *Chlamydia* polypeptide is a *Chlamydia psittaci* protein selected from the group consisting of Psi0330 (SEQ ID NO: 108), Psi0379 (SEQ ID NO: 110), Psi0595 (SEQ ID NO: 112), Psi0648 (SEQ ID NO: 114), Psi0671 (SEQ ID NO: 116), Psi0705 (SEQ ID NO: 118), Psi0710 (SEQ ID NO: 120), Psi0761 (SEQ ID NO: 122), Psi0774 (SEQ ID NO: 124), Psi1002 (SEQ ID NO: 126), Psi1005 (SEQ ID NO: 128), Psi1022 (SEQ ID NO: 130), and Psi1058 (SEQ ID NO: 132); or a fragment thereof.

A *Chlamydia* infection in an animal preferably a human, as used, herein includes a number of diseases, including trachoma, sexually transmitted disease, pelvic inflammatory disease, respiratory diseases, such as bronchitis, pneumonia and their sequelae, such as atherosclerosis.

Examples of other animals include: a horse (equine animal), a cow (bovine animal), a pig (porcine animal), a sheep (ovine animal), a goat (caprine animal), a bird, a dog, and a cat.

The polypeptides as defined above may be administered as a pharmaceutical composition containing the polypeptide compound and a pharmaceutically acceptable carrier or diluent. The active materials can also be mixed with other active materials, which do not impair the desired action and/or supplement the desired action. Any route can administer the active materials for example, orally, parenterally, intravenously, intradermally, subcutaneously, or topically, in liquid or solid form.

For the purposes of parenteral therapeutic administration, the active ingredient may be incorporated into a solution or suspension. The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic.

Another mode of administration of the polypeptides as defined above is oral. Oral compositions will generally include an inert diluent or an edible carrier. For the purpose of oral therapeutic administration, the aforesaid polypeptides may be incorporated with excipients and used in the form of tablets, gelatine capsules, troches, capsules, elixirs, suspensions, syrups, wafers, chewing gums and the like. Compositions may be prepared according to any method known to the art for the manufacture of pharmaceutical compositions and such compositions may contain one or more agents selected from the group consisting of sweetening agents, flavoring agents, coloring agents and preserving agents. Tablets containing the active ingredient in admixture with nontoxic pharmaceutically acceptable excipients, which are suitable for manufacture of tablets, are acceptable. These excipients may be, for example, inert diluents, such as calcium carbonate, sodium carbonate, lactose, calcium phosphate or sodium phosphate granulating and disintegrating agents, such as maize starch, or alginic acid; binding agents, such as starch, gelatin or acacia; and lubricating agents, such as magnesium stearate, stearic acid or talc. Tablets may be uncoated or may be coated by known techniques to delay disintegration and adsorption in the gastrointestinal tract and thereby provide a sustained action over a longer period. For example, a time delay material such as glyceryl monostearate or glyceryl distearate alone or with a wax may be employed. Formulations for oral use may also be presented as hard gelatin capsules wherein the active ingredient is mixed with an inert solid diluent, for example calcium carbonate, calcium phosphate or kaolin, or as soft gelatin capsules wherein the active ingredient is mixed with water or an oil medium, such as peanut oil, liquid paraffin or olive oil.

The tablets, pills, capsules, troches and the like may contain the following ingredients: a binder such as microcrystalline cellulose, gum tragacanth or gelatin; an excipient such as starch or lactose, a disintegrating agent such as alginic acid, Primogel, corn starch and the like; a lubricant such as magnesium stearate or Sterotes; a glidant such as colloidal silicon dioxide; and a sweetening agent such as sucrose or saccharin or flavoring agent such as peppermint, methyl salicylate, or orange flavoring may be added. When the dosage unit form is a capsule, it may contain, in addition to material of the above type, a liquid carrier such as fatty oil. Other dosage unit forms may contain other various materials, which modify the physical form of the dosage unit, for example, as coatings. Thus tablets or pills may be coated with sugar, shellac, or other enteric coating agents. A syrup may contain, in addition to the active polypeptides, sucrose as a sweetening agent and certain preservatives, dyes and colorings and flavors. Materials used in preparing these various compositions should be pharmaceutically or veterinary pure and non-toxic in the amounts used.

Aqueous suspensions contain the active materials in admixture with excipients suitable for the manufacture of aqueous suspensions. Such excipients include a suspending agent, such as sodium carboxymethylcellulose, methylcellulose, hydroxypropylethyl cellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethylene oxycetanol), a condensation product of ethylene oxide with a partial ester derived from a fatty acid and a hexitol (e.g., polyoxyethylene sorbitol mono-oleate), or a condensation product of ethylene oxide with a partial ester derived from fatty acid and a hexitol anhydride (e.g., polyoxyethylene sorbitan mono-oleate). The aqueous suspension may also contain one or more preservatives such as ethyl or n-propyl p-hydroxybenzoate, one or more coloring agents, one or more flavoring agents and one or more sweetening agents, such as sucrose, aspartame, saccharin, or sucralose.

Oil suspensions may be formulated by suspending the active ingredient in a vegetable oil, such as arachis oil, olive oil, sesame oil or coconut oil, or in a mineral oil such as liquid paraffin. The oil suspensions may contain a thickening agent, such as beeswax, hard paraffin or cetyl alcohol. Sweetening agents may be added to provide a palatable oral preparation. These compositions may be preserved by the addition of an antioxidant such as ascorbic acid.

Dispersible powders and granules suitable for preparation of an aqueous suspension by the addition of water may be formulated from the active ingredients in admixture with a dispersing, suspending and/or wetting agent, and one or more preservatives. Suitable dispersing or wetting agents and suspending agents are exemplified by those disclosed above. Additional excipients, for example sweetening, flavoring and coloring agents, may also be present.

The compositions as defined above may also be in the form of oil-in-water emulsions. The oily phase may be a vegetable oil, such as olive oil or arachis oil, a mineral oil, such as liquid paraffin, or a mixture of these. Suitable emulsifying agents include naturally occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsion may also contain sweetening and flavoring agents.

Syrups and elixirs may be formulated with sweetening agents, such as glycerol, sorbitol or sucrose. Such formulations may also contain a demulcent, a preservative, a flavoring or a coloring agent.

The compositions as defined above may be in the form of a sterile injectable preparation, such as a sterile injectable aqueous or oleaginous suspension. This suspension may be formulated according to the known art using those suitable dispersing or wetting agents and suspending agents, which have been mentioned above. The sterile injectable preparation may also be a sterile injectable solution or suspension in a nontoxic parenterally acceptable diluent or solvent, such as a solution of 1,3-butanediol. Among the acceptable vehicles and solvents that may be employed are water and Ringer's solution, an isotonic sodium chloride. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables. Sterilization may be performed by conventional methods known to those of ordinary skill in the art such as by aseptic filtration, irradiation or terminal sterilization (e.g. autoclaving).

Aqueous formulations (i.e oil-in-water emulsions, syrups, elixers and injectable preparations) may be formulated to achieve the pH of optimum stability.
The determination of the optimum pH may be performed by conventional methods known to those of ordinary skill in the art. Suitable buffers may also be used to maintain the pH of the formulation.

The polypeptides as defined above may also be administered in the form of suppositories for rectal administration of the drug. These compositions can be prepared by mixing the drug with a suitable nonirritating excipient, which is solid at ordinary temperatures but liquid at the rectal temperatures and will therefore melt in the rectum to release the drug. Non-limiting examples of such materials are cocoa butter and polyethylene glycols.

Methods for the production of antibodies directed to a specific peptide or fragment thereof are well known by those of skill in the art. Antibodies can be obtained by injecting an animal with an immunogenic peptide as defined above, or an immunogenic fragment thereof, and recovering the antibodies which are able to complex with said immunogenic peptide or fragment thereof from said animal.
Examples of such methods are disclosed in Antibodies, A Laboratory Manual, Harlow and Lane, Cold Spring Harbor Press, 1988, herein incorporated by reference.

Techniques which make it possible to humanize antibodies, either monoclonal or polyclonal, have been described in the following references: Waldmann T. (1991). Science. 252: 1657-1662; Winter G. et al. (1993). Immunology Today. 14(6): 243-246; Carter et al. (1992). Proc. Natl. Acad Sci, USA. 89: 4285-4289; Singer et al. (1993). Journal of Immunology. 150(7): 2844-2857; Kipriyanov et al, Mol. Biotechnol., 2004, 26 (1):39-60; U.S. Patent No. 6,693,176; U.S. Patent No. 5,225,539; U.S. Patent No. 6,572,857; US Patent No. 6,183,744, each of which are incorporated herein by reference.

An *ipaB* mutant strain of *S. flexneri* expresssing IncA/cya was deposited at C.N.C.M., 25, Rue de Docteur Roux, F-75724, Paris Cedex 15, France, on December 13, 2000, with accession number 1-2592.

A bacterial strain containing the vector pUC19cya was deposited at C.N.C.M., 25, Rue de Docteur Roux, F-75724, Paris Cedex 15, France, on December 13, 2000, with accession number I-2593.

An ipaB⁻ mutant strain of *S. flexneri* designated SF620 was deposited at C.N.C.M., 25, Rue de Docteur Roux, F-75724, Paris Cedex 15, France, on December 13, 2000, with accession number I-2594.

A *E. coli* bacterial strain containing Psi0710 in an expression vector (pQE trisystem, Qiagen) with a carboxy-terminal Histidine tag was deposited at C.N.C.M., 25, Rue de Docteur Roux, F-75724, Paris Cedex 15, France, on February 18, 2003, with accession number I-2974.

### EXAMPLES

Having generally described this invention, a further understanding can be obtained by reference to certain specific examples which are provided herein for purposes of illustration only, and are not intended to be limiting unless otherwise specified.

### Example 1

### Materials and Methods

### Bacterial strains and reagents

Strain M90T is the virulent, wild-type strain of *S. flexneri* 5 (Sansonetti *et al*., 1982). Strains SF401 and SF620 (deposited at C.N.C.M. with accession number I-2594) are derivatives of M90T in which the *mxiD and ipaB* genes, respectively, have been inactivated (Allaoui, A., P.J. Sansonetti, and C. Parsot. (1993). MxiD, an outer membrane protein necessary for the secretion of the Shigella flexneri lpa invasins. Mol. Microbiol. 7: 59-68; Ménard, R., P. Sansonetti and C. Parsot. (1994). The secretion of the Shigella flexneri Ipa invasins is activated by epithelial cells and controlled by IpaB and IpaD. EMBO J. 13: 5293-302). The *E. coli* strain TG1 (Sambrook et al., Molecular Cloning: A Laboratory Manual, Cold Spring Harbor, New York, 1989) was used for plasmid constructions. *S. flexneri and E. coli* strains were grown in Luria-Bertani (LB). Ampicillin was used at 0.1 mg/ml. Monoclonal antibodies against the calmodulin-dependent adenylate cyclase (Brézin et al, 1987). Evidence for the presence of cAMP, cAMP receptor and transcription termination factor Rho in different Gram-negative bacteria. J. Gen. Microbial. 131(11): 2953-2960) were used. Anti-IpaD antibody was used as described (Ménard, R., P.J. Sansonetti, and C. Parsot. (1993). Horseradish peroxidase-linked secondary antibodies for enhanced chemiluminescence were obtained from Amersham Pharmacia Biotech (Orsay, France). Alkaline phosphatase-linked secondary antibodies for enhanced chemifluorescence were obtained from Pierce (Rockford, IL, USA).

### Genomic data

*C. pneumoniae* protein sequences (CPnXXXX), obtained by sequencing of the CWL029 strain of *C*. *pneumoniae,* are available at http://chlamydia-www.berkeley.edu:4231/ (chromosome sequence Genbank Accession number: AE001363), see also Kalman et al. (1999) Nature Genetics. 21: 385-389. *C*. *trachomatis* protein sequences (CTXXX), obtained by sequencing serovar D (D/UW-3/Cx) trachoma biovar of *C*. *trachomatis*, are available at http://chlamydia-www.berkeley.edu:4231/ (chromosome sequence Genbank Accession number: AE001273), see also Stephens et al. (1998) Science. 282: 754-759. *Chlamydophila psittaci* (GPIC strain) sequencing by The Institute for Genomic Research (TIGR, Rockville, MD, Etats-Unis) is not complete but preliminary sequence data was obtained from The Institute for Genomic Research website at http://www.tigr.org. The present Inventors compared *C. pneumoniae* proteins with the translation in the six reading frames of the available sequence using a comparison tool available at http://tigrblast.tigr.org/ufmg/. Herein, the closest protein match form *C. psittaci* to the blasted protein from *C. pneumoniae* (CPnXXXX) is referred to as PsiXXXX.

The entire DNA and amino acid sequences of claimed CPnXXXX, CTXXX, and PsiXXXX designators are attached herewith. The amino acid sequences of the respective proteins used to construct the chimeras are indicated by capital letters.

### Construction of recombinant plasmids expressing hybrid proteins

Genomic DNA from *C*. *pneumoniae* strain TW183 (Kuo et al. (1995) Clinical Microbiology Reviews. 8(4):451-461), serovar D (D/UW-3/Cx) trachoma biovar of *C*. *trachomatis* and *Chlamydophila psittaci* GPIC strain (American Type Culture Collection, Virginia, U.S.A.; ATCC No. VR-2282), was prepared from purified bacteria using the RapidPrep Micro Genomic DNA isolation kit (Amersham Pharmacia Biotech). The *C*. *pneumoniae* DNA fragments used in the examples were amplified by PCR using the strain TW183 as a template. These fragments were sequenced and shown to be more than 99% identical to the sequences of the CWL029 strain. The 5' part of different chlamydial genes, including about 30 nucleotides located upstream from the proposed translation start sites and the first 20 to 99 codons, were amplified by PCR using the primers listed in Table 1. For the Inc/cya constructs, the forward and reverse primers contained additional HindIII *and Xba*I sites, respectively, to allow cloning of the PCR fragments between the HindIII *and Xba*I sites of the puc19cya vector. This vector was constructed by cloning the *Xba*I-*EcoR*I fragment of plasmid pMS109, which carried the *cya* gene of *Bordetella pertussis* (Sory, M.P., and G.R. Cornelis. (1994), between *Xba*I and *Eco*RI sites of the pUC 19 vector. In the recombinant plasmids, transcription of the hybrid genes was under the control of the *lac* promoter of the vector. Recombinant plasmids were amplified in *E. coli* TGI and sequencing checked the sequence of all constructs. Methods of sequencing nucleic acids are known in the art and are described in, for example, Sambrook et al., Molecular Cloning: A Laboratory Manual, Cold Spring Harbor Laboratory, New York (1989) and Current Protocols in Molecular Biology, Ausebel et al, eds., John Wiley and Sons, Inc., New York (2000).

**TABLE I: Oligonucleotides used to construct Inc/cya chimeras.**

| **Protein** | **Forward primer** | **Reverse primer** |
|---|---|---|
| CPn0009 | AGTCAAGCTTATGATTGTACGGACATAGAGACCG (SEQ ID NO: 133) | AGTCTCTAGATGCTTTTCGGACCATAGTC (SEQ ID NO: 250) |
| CPn0012 | AGTCAAGCTTGTAGGTTTATTAAAGGGGATGTACC (SEQ ID NO: 134) | AGTCTCTAGAATCCTCTTCCCAAGGAATCAG (SEQ ID NO: 251) |
| CPn0026 | AGTCAAGCTTGAGCTGTTCTTTTCAGAGAGCTT (SEQ ID NO: 135) | AGTCTCTAGATCGAATCGATTTGGAAGGAG (SEQ ID NO: 252) |
| CPn0028 | AGTCAAGCTTTACTTTTTGAAGGCTAGTACGTT (SEQ ID NO: 136) | AGTCTCTAGATTCAATAATGCCAGAGCTTTTTC (SEQ ID NO: 253) |
| CPn0049 | AGTCAAGCTTGGAAGGAATATCGTTTACCTGCT (SEQ ID NO: 137) | AGTCTCTAGATTCATCCACCCAAATAGCAC (SEQ ID NO: 254) |
| CPn0063 | AGTCAAGCTTGTAGATGCCCATCCTACCAACA G (SEQ ID NO: 138) | AGTCTCTAGAAGTAAGAGGGAGCCCAGGA (SEQ ID NO: 255) |
| CPn0066 | AGTCAAGCTTTCCCTAAAATGAATAAACTAAGGA (SEQ ID NO: 139) | AGTCTCTAGATGACGGGGGAGGTGTATTAG (SEQ ID NO: 256) |
| CPn0067 | AGTCAAGCTCAAGTGTCATTGAAAAGGTTCAGG (SEQ ID NO: 140) | AGTCTCTAGATAGGAGGCCTTTCGATTGTTT (SEQ ID NO: 257) |
| CPn0104 (SEQ ID NO: 2) | AGTCAAGCTTGCCAACCAACGGTTAGACGAAA (SEQ ID NO: 141) | AGCTTCTAGATGGAAAGGATACGGATTGG (SEQ ID NO: 258) |
| CPn0105 | AGTCAAGCTTGCGTGCCTTTTAGAAAATTTAGCA (SEQ ID NO: 142) | AGTCTCTAGAGAATGGGGGAATACAAATCA (SEQ ID NO: 259) |
| CPn0130 | AGTCAAGCTTGTAACGACTCCTTCTTTCAACGATT (SEQ ID NO: 143) | AGTCTCTAGAGGCGAGAGAAAGTTTCGTTA (SEQ ID NO: 260) |
| CPn0132 | AGTCAAGCTTCCATTAAGGATCTAAAACAATTT (SEQ ID NO: 144) | AGTCTCTAGACAACTTGTTCATGCGACAG (SEQ ID NO: 261) |
| CPn0146 | AGTCAAGCTTTGTTTGAGATGAATTCGCATTTT (SEQ ID NO: 145) | AGCTTCTAGACGCATCCGAAAGACTTTTCT (SEQ ID NO: 262) |
| CPn0167 | AGTCAAGCTTTTGCATGAATTCGTAAAATAGAAA (SEQ ID NO: 146) | AGTCTCTAGAACGTTGTAAGCCGTAATTCTCG (SEQ ID NO: 263) |
| CPn0174 | AGTCAAGCTTGTAGGGTTTGTGGAGAAAATTGTTA (SEQ ID NO: 147) | AGTCTCTAGATGCGGGGATCGTATAAGCTA (SEQ ID NO: 264) |
| CPn0175 | AGTCAAGCTTCTTCTATTAGCCTGTTTCCAATT (SEQ ID NO: 148) | AGTCTCTAGAAGGTGATTGCTTCCCAAGTCT (SEQ ID NO: 265) |
| CPn0181 | AGTCAAGCTTCTGTTGTTGAATTAATAGCTTCTT (SEQ ID NO: 149) | AGTCTCTAGAATAAGAGTCGAGGGCTTGCAC (SEQ ID NO: 266) |
| CPn0186 | AGTCAAGCTTGTGAGAAAAACAACAATTCTTATCC (SEQ ID NO: 150) | AGTCTCTAGATGTAGGAATACCTGGAGTCGTG (SEQ ID NO: 267) |
| CPn0206 (SEQ ID NO: 4) | AGTCAAGCTTACGACTCAATTAACAGTGATGCAA (SEQ ID NO: 151) | AGTCTCTAGAGCGCTGAGTAGCGTCGTGTAA (SEQ ID NO: 268) |
| CPn0210 (SEQ ID NO: 6) | AGTCAAGCTTTCAAGACAATTAGAGAGAAAGAGC (SEQ ID NO: 152) | AGCTTCTAGAGAATTCAAGATGATCCAAACA (SEQ ID NO: 269) |
| CPn0211 | AGTCAAGCTTGTAGGCATTGCTTAAAAATAAAATG (SEQ ID NO: 153) | AGTCTCTAGAAGTCTTATGAAGCAAAGCAG (SEQ ID NO: 270) |
| CPn0220 | AGTCAAGCTTGTAATCGGATTTTAAACCAACTTTT (SEQ ID NO: 154) | AGTCTCTAGAATTTATATGCCACGCCTTCTTTC (SEQ ID NO: 271) |
| CPn0223 | AGTCAAGCTTGTAGGAATTTTTATTACGAGTTTCA (SEQ ID NO: 155) | AGTCTCTAGAGGAGGTTTCCGAGACGATT (SEQ ID NO: 272) |
| CPn0226 | AGTCAAGCTTCAAGAAGCGTTAAGAAAACGAAA (SEQ ID NO: 156) | AGTCTCTAGAATCCCAGTCGTGAGAAGCAT (SEQ ID NO: 273) |
| CPn0243 | AGTCAAGCTTTTATTAAAATTTGTTAAAGGGAGG (SEQ ID NO: 157) | AGTCTCTAGAGAGAAGAATATCTGCAACTAGAGC (SEQ ID NO: 274) |
| CPn0267 | AGTCAAGCTTGTAAACTAATTCGGATTAAAAATGA (SEQ ID NO: 158) | AGTCTCTAGAGGTAACAGTGCATGGGAAAG (SEQ ID NO: 275) |
| CPn0277 | AGTCAAGCTTGAAGGAAAAGAAGTTCTCATAAAG (SEQ ID NO: 159) | AGTCTCTAGACAAAATAGGAATAGCAGCTTGG (SEQ ID NO: 276) |
| CPn0284 | AGTCAAGCTTGTAGCGTCTGGAAAAATTCTGG (SEQ ID NO: 160) | AGTCTCTAGACGGTCTTAAACTACTTTTCAATG (SEQ ID NO: 277) |
| CPn0287 | AGTCAAGCTTGGGTAAGAACACCTTCTAATATG (SEQ ID NO: 161) | AGTCTCTAGAATCAACCACATCTTCTGGACAA (SEQ ID NO: 278) |
| CPn0291 | GACTAAGCTTGTAATCTATTTTTAGATAGGAA (SEQ ID NO: 162) | GACTTCTAGATCCAGGTTTTTCGGAAGCAG A (SEQ ID NO: 279) |
| CPn0292 | AGTCAAGCTTCCCGTAATTACTGTCCGTACAAC (SEQ ID NO: 163) | AGTCTCTAGAAGTAGACGGGAGTTGCTG (SEQ ID NO: 280) |
| CPn0308 | GACTAAGCTTATTATATAGACAGATTAAAAT (SEQ ID NO: 164) | GACTTCTAGACTTAAAAAATACCCAGGAACA (SEQ ID NO: 281) |
| CPn0330 | AGTCAAGCTTAGGGTTTTAAGTCGGTTTGATG (SEQ ID NO: 165) | AGTCTCTAGACCGAATCGCTTCATTCGTAG (SEQ ID NO: 282) |
| CPn0334 | AGTCAAGCTTTCGATAATCATAATTCAAAGCGTA (SEQ ID NO: 166) | AGTCTCTAGAAAGGATCAGATGTGCTTTAGGG (SEQ ID NO: 283) |
| CPn0357 | AGTCAAGCTTTCCATGAATTCTAAAATGATTAGG (SEQ ID NO: 167) | AGTCTCTAGACGAAAATTGGGTAGCCTGAC (SEQ ID NO: 284) |
| CPn0365 | AGTCAAGCTTAATTTAAAGGAACTCAGGTGAA (SEQ ID NO: 168) | AGTCTCTAGAATCAAAGTTGCTGCAGGATT (SEQ ID NO: 285) |
| CPn0374 | AGTCAAGCTTGTAACATGGGGTATGGAAAGACC (SEQ ID NO: 169) | AGTCTCTAGACTCGGCATCCTTTTGTTTTG (SEQ ID NO: 286) |
| CPn0379 | AGTCAAGCTTGTAAGCACCTGCTCTCGAATACA (SEQ ID NO: 170) | AGTCTCTAGATTCTTCTTGGTGCCTGCTAA (SEQ ID NO: 287) |
| CPn0399 (SEQ ID NO: 8) | AGTCAAGCTTGTAACGGTCTCTTTGCTTGTTTTT (SEQ ID NO: 171) | AGTCTCTAGAAGTGCAGCTGGATAGGGTTG (SEQ ID NO: 288) |
| CPn0405 (SEQ ID NO: 10) | AGTCAAGCTTGTAACGATCCGAACTTATCGTAGT (SEQ ID NO: 172) | AGTCTCTAGATCCGGATGCGCTAGTAGTTG (SEQ ID NO: 289) |
| CPn0443 (SEQ ID NO: 12) | AGTCAAGCTTGTAAATTCATGGGCCTAATGATAA (SEQ ID NO: 173) | AGTCTCTAGATAACCCTGTGGATGACGTTTT (SEQ ID NO: 290) |
| CPn0480 (SBQ ID NO: 14) | AGTCAAGCTTGTAATTGGGGAATCTCTGGGAGAC (SEQ ID NO: 174) | AGTCTCTAGAGGGACTAACGACCTCAGCAC (SEQ ID NO: 291) |
| CPn0489 (SEQ ID NO: 16) | AGTCAAGCTTGTAATGGAGGATTGGCTAAGGA (SEQ ID NO: 175) | AGTCTCTAGAAACACCACCGACATCACAAA (SEQ ID NO: 292) |
| CPn0490 (SEQ ID NO: 18) | AGTCAAGCTTGTAAGAGGGACCTTACCTATTGCTT (SEQ ID NO: 176) | AGTCTCTAGAAATGAGGACCTCTCCTTCGT (SEQ ID NO: 293) |
| CPn0497 (SEQ ID NO: 20) | AGTCAAGCTTGCGGAATGGTTAAAGGAAACG (SEQ ID NO: 177) | AGTCTCTAGATTGTCCATCAAAGCCTACAAT (SEQ ID NO: 294) |
| CPn0522 (SEQ ID NO: 22) | AGTCAAGCTTGTAAAACCAGCCTAGCCTTCAAA (SEQ ID NO: 178) | AGTCTCTAGAGCTTTTTGCATAGGGGAAG (SEQ ID NO: 295) |
| CPn0556 (SEQ ID NO: 24) | AGTCAAGCTTGTAACCTGCTTCTTTAGGAACTAC (SEQ ID NO: 179) | AGTCTCTAGATTGTAGAGCAGCGGTGACAC T (SEQ ID NO: 296) |
| CPn0582 (SEQ ID NO: 26) | AGTCAAGCTTGTAAAATTCAGCACAGGCTTGTAA (SEQ ID NO: 180) | AGTCTCTAGAGCGTTCTGGAGTGACGAAAC (SEQ ID NO: 297) |
| CPn0585 | GACTAAGCTTGTAAATTGGAGAITGTAGTAGC (SEQ ID NO: 181) | GACTTCTAGAAACAATTGTATGATTCCATCC (SEQ ID NO: 298) |
| CPn0588 (SEQ ID NO: 28) | ATGCAAGCTTACGAGCAAGAGCATAAAATCCA (SEQ ID NO: 182) | ATGCTCTAGACCTTTGGCGGACTTTTTCTT (SEQ ID NO: 299) |
| CPn0595 (SEQ ID NO: 30) | AGTCAAGCTTGAATTGCTAACAGACACTAAAGG (SEQ ID NO: 183) | AGTCTCTAGATACCCATTCTTGACCGGAAA (SEQ ID NO: 300) |
| CPn0648 | AGTCAAGCTTGTTTTAAAAAGCCTTGTAAGGAGGT (SEQ ID NO: 184) | AGTCTCTAGACTGAGCAAAGGAGCTGACAG (SEQ ID NO: 301) |
| CPn0671 (SEQ ID NO: 32) | AGTCAAGCTTGTAAGAATTACCATAAATCAGAGGAA (SEQ ID NO: 185) | AGTCCCTAGAAGCATCAGTGCAATGAGGATAA (SEQ ID NO: 302) |
| CPn0673 (SEQ ID NO: 34) | AGTCAAGCTTGTAACAGAAGGAAAAGCATACCACTG (SEQ ID NO: 186) | AGTCTCTAGATCCAAAACGATCCTGTTCC (SEQ ID NO: 303) |
| CPn0681 (SEQ ID NO: 36) | AGTCAAGCTTGCCATAAGTCGTTTACAAGATCG (SEQ ID NO: 187) | AGTCTCTAGATTCCACACAAGAGACCACCA (SEQ ID NO: 304) |
| CPn0705 | AGTCAAGCTTGTGAATCAGGGGGAAGCTAGT (SEQ ID NO: 188) | AGTCTCTAGATCGTGTTTGCTTTCCTTCCA (SEQ ID NO: 305) |
| CPn0710 | AGTCAAGCTTCGCAAGATGATATAAAGGTCCA (SEQ ID NO: 189) | AGTCTCTAGAGACAGTGCCTTGTGTTGATG (SEQ ID NO: 306) |
| CPn0711 | AGTCAAGCTTTAAATAACCAAGTATTGGGGTTTA (SEQ ID NO: 190) | AGTCTCTAGATCGAAGCAGCGCATGTAACT (SEQ ID NO: 307) |
| CPn0712 (SEQ ID NO: 38) | AGTCAAGCTTGTAGACATGGCCGTCAGATCTTGG (SEQ ID NO: 191) | AGTCTCTAGAAGAGTCGCGTCCTATAGACCA (SEQ ID NO: 308) |
| CPn0720 (SEQ ID NO: 40) | AGTCAAGCTTCTCTTTTTAAAGACAACGCGAAT (SEQ ID NO: 192) | AGCTTCTAGACGTGTGAGTCCCCTGAACTT (SEQ ID NO: 309) |
| CPn0725 (SEQ ID NO: 42) | AGTCAAGCTTGTAATTAATCTTGCGGAGATGTTGG (SEQ ID NO: 193) | AGTCTCTAGACTCTGTAGTCAGCTGGTCGTTG (SEQ ID NO: 310) |
| CPn0729 (SEQ ID NO: 44) | AGTCAAGCTTGTAAGCCTGCTTGGTCTTCTGTT (SEQ ID NO: 194) | AGTCTCTAGACGAGAGAGGAAGTTCAGCGTA (SEQ ID NO: 311) |
| CPn0746 (SEQ ID NO: 46) | ATGCAAGCTTGTAAGGGAAGATGTCCGTTCAGTT (SEQ ID NO: 195) | ATGCTCTAGAAGCTCCCTTAACGACTTCTGG (SEQ ID NO: 312) |
| CPn0755 (SEQ ID NO: 48) | AGTCAAGCTTATGG*C*TATGAAGAGCAATTTATTC (SEQ ID NO: 196) | AGTCTCTAGAAGGGTAACACACTAGGGGAAGA (SEQ ID NO: 313) |
| CPn0761 (SEQ ID NO: 50) | AGTCAAGCTTGTAACTCCCGCACCAACCTC (SEQ ID NO: 197) | AGTCTCTAGATCCTTCAGACCAACGCTGATA (SEQ ID NO: 314) |
| CPn0764 (SEQ ID NO: 52) | AGTCAAGCTTGTAATCTGGTTAACACATGCTGAGG (SEQ ID NO: 198) | AGTCTCTAGATGACAGGCCGTTTCTATCAA (SEQ ID NO: 315) |
| CPn0770 (SEQ ID NO: 54) | AGTCAAGCTTGTAAGAGCCGAGCATAGATAGAAAC (SEQ ID NO: 199) | AGTCTCTAGAGCCTGCAATCAATCCCTGT (SEQ ID NO: 316) |
| CPn0774 (SEQ ID NO: 56) | AGTCAAGCTTTTAATAAGCTGAATAAACCAATGA (SEQ ID NO: 200) | AGTCTCTAGATTCTGCGGAAGGAAGCTGT (SEQ ID NO: 317) |
| CPn0792 (SEQ IDNO: 58) | AGTCAAGCTTGTAACATGACGACATCACCTTATT (SEQ ID NO: 201) | AGTCTCTAGAAGCGGCAGAAAATGAGAAA A (SEQ ID NO: 318) |
| CPn0820 | AGTCAAGCTTGTAAGTCAGGAAGTCCGTGGTGAT (SEQ ID NO: 202) | AGTCTCTAGACAAAGCAATTAGAGTGAACG ACA (SEQ ID NO: 319) |
| CPn0821 | AGTCAAGCTTGTAATTTGCGGTTGGGAAATAA (SEQ ID NO: 203) | AGTTCTAGAAGCACAGGCACAACGTTTAC (SEQ ID NO: 320) |
| CPnOS29 | AGTCAAGCTTGTAACCCTTGCCTCTATTTTGAGA (SEQ ID NO: 204) | AGTCTCTAGACGGTACCAAGGGCCATTTTG (SEQ ID NO: 321) |
| CPn0853 (SEQ ID NO: 60) | AGTCAAGCTTGTAATTGCGAGAAGGATTAAATCTTAG (SEQ ID NO: 205) | AGTCTCTAGAAAGATGTCGGGAAAGTGTCG (SEQ ID NO: 322) |
| CPn0859 (SEQ ID NO: 62) | AGTCAAGCTTATCTCAAACATCAAGTGCTGAA (SEQ ID NO: 206) | AGTCTCTAGATTGGCTTGCCTTATCATTCG (SEQ ID NO: 323) |
| CPn0879 (SEQ ID NO: 64) | AGTCAAGCTTGTAAGCCTCGTCAAATCCTGA (SEQ ID NO: 207) | AGTCTCTAGAGACGACTTGCTTGCTCACT (SEQ ID NO: 324) |
| CPn0906 (SEQ ID NO: 66) | AGTCAAGCTTTCTTCAGGAACTTTAAAAACA (SEQ ID NO: 208) | AGTCTCTAGATGCTGCOACACOAATTTCTA (SEQ ID NO: 325) |
| CPn0939 (SEQ ID NO: 68) | AGTCAAGCTTTAATAGAGCTCATTGGAGGAGGA (SEQ ID NO: 209) | AGTCTCTAGAGAGGAGGGAAACACCGTTAAT (SEQ ID NO: 326) |
| CPn1002 (SEQ ID NO: 70) | AGTCAAGCTTGTAATGTAGAAAAGCGCAGAGAGAAA (SEQ ID NO: 210) | AGTCTCTAGACCCTTCCAATTGAGGAAAA (SEQ ID NO: 327) |
| CPn1005 (SEQ ID NO: 72) | AGTCAAGCTTGTAATCGAAAACCAGTTAGGTTGAA (SEQ ID NO: 211) | AGTCTCTAGATGCAATGATATCGTCAGCAG (SEQ ID NO: 328) |
| CPn1007 (SEQ ID NO: 74) | AGTCAAGCTTGTAATGGGTCCTAATGGAGGCTTT (SEQ ID NO: 212) | AGTCTCTAGACACCTGGATTTTGCCTTCAG (SEQ ID NO: 329) |
| CPn1016 | AGTCAAGCTTGACCTCCTTGTAACCATTCTCG (SEQ ID NO: 213) | AGTCTCTAGACTTCCATGGTAAAGGAGCA (SEQ ID NO: 330) |
| CPn1019 (SEQ ID NO: 76) | AGTCAAGCTTTCCTTAAGAGGATAAATCCACAG (SEQ ID NO: 214) | AGTCTCTAGATTCTGCAGCAACAACAGCTT (SEQ ID NO: 331) |
| CPn1020 (SEQ ID NO: 78) | AGTCAAGCTTTTCGAAAACATAAATAATGTGGA (SEQ ID NO: 215) | AGTCTCTAGAAGCTTCTTGAGGCGCTACAG (SEQ ID NO: 332) |
| CPn1022 | AGTCAAGCTTGTGCAGGAAAATGATGTTTTAGC (SEQ ID NO: 216) | AGTCTCTAGATCCAGAGGCTGTGGAAACTG T (SEQ ID NO: 333) |
| CPn1027 | AGTCAAGCTTCAGCCTGGTTTCGTAATTTT (SEQ IDNO: 217) | AGTCTCTAGAAGCATGAGGCTGTATGAGG (SEQ ID NO: 334) |
| CPn1032 (SEQ ID NO: 80) | AGTCAAGCTTGTAAACCACACCAATATTATTTAGGA (SEQ ID NO: 218) | AGTCTCTAGATGCTTGTAGAAGAGCAGAAT CG (SEQ ID NO: 335) |
| CPn1058 (SEQ ID NO: 82) | AGTCAAGCTTGTAACCTGGGCAGGTCAAAATCTA (SEQ ID NO: 219) | AGTCTCTAGACCCAGATGAAGTCACTGGAAC (SEQ ID NO: 336) |
| CT053 | AGTCAAGCTTGTAATGGGTTTCCGGTTTCAATCA (SEQ ID NO: 220) | AGTCTCTAGAACGGATTTCTTCCTGGTGTCT (SEQ ID NO: 337) |
| CT083 | AGTCAAGCTTGTAAGCCAAGAGCAGGATAAAACGA (SEQ ID NO: 221) | AGTCTCTAGATGATATGCTCCCATTCTTTCG (SEQ ID NO: 338) |
| CT387 (SEQ ID NO: 84) | AGTCAAGCTTGTAAATTTTTCCTATAACFTGGTTC (SEQ ID NO: 222) | AGTCTCTAGATCCTTCGTATACGCCAGTACC (SEQ ID NO: 339) |
| CT476 (SEQ ID NO: 86) | AGTCAAGCTTTTGATAAGAAATTAOCCAACATAGG (SEQ ID NO: 223) | AGTCTCTAGAGCATCCACGTTTGTTCCATT (SEQ ID NO: 340) |
| CT529 | AGTCAAGCTTTTCGGTTTAAGTAATAGAAGTGG (SEQ ID NO: 224) | AGTCTCTAGAAGCATTCCCTGTACCAGACC (SEQ ID NO: 341) |
| CT550 (SEQ ID NO: 88) | AGTCAAGCTTGTAAGACGGTGCCTTCAATAACAAA (SEQ ID NO: 225) | AGTCTCCAGAAGCCGAACGAGCTAAGACAT (SEQ ID NO: 342) |
| CT6U6.1 (SEQ ID NO: 90) | AGTCAAGCTTGTAAAAACAGGGAAAGACGATGA (SEQ ID NO: 226) | AGTCTCTAGAGACCAAAGCTCCCTCTTGAA (SEQ ID NO: 343) |
| CT610 (SEQ ID NO: 92) | AGTCAAGCTTGTAAGCGGTTACGTGGAGTCAA (SEQ ID NO: 227) | AGTCTCTAGAGGCATACGCCTGTAATTGCT (SEQ ID NO: 344) |
| CT642 (SEQ ID NO: 94) | AGTCAAGCTTGTAATCCGGAGCCTTTCTCCTAT (SEQ ID NO: 228) | AGTCTCTAGATTCTTCAGGGCCAGCAA (SEQ ID NO: 345) |
| CT652.1 (SEQ ID NO: 96) | AGTCAAGCTTGTAAAGATGAATGATTCCAGAAAG (SEQ ID NO: 229) | AGTCTCTAGAAGGAAAGCCTATCGCACACA (SEQ ID NO: 346) |
| CT664 (SEQ ID NO: 98) | AGTCAAGCTTGGACTAAGTAAAACGGAGCAGGA (SEQ ID NO: 230) | AGTCTCTAGAAGACCAACTCGTCCCATTTT C (SEQ ID NO: 347) |
| CT665 | AGTCAAGCTTTTCGAGGTTTATTTAATTCTTCCA (SEQ ID NO: 231) | AGTCTCTAGAGTGGGCTTTGGTTACATCGT (SEQ ID NO: 348) |
| CT666 | AGTCAAGCTTACGTATCAACCGTAAAATGGTG (SEQ ID NO: 232) | AGTCTCTAGAAGTTCCTTGCGTGGATGTTT (SEQ ID NO: 349) |
| CT671 | AGTCAAGCTTGTAAGCAAAAACAACGGGAATC (SEQ ID NO: 233) | AGTCTCTAGACATCCGATCTGCTTTCTCTTG (SEQ ID NO: 350) |
| CT718 (SEQ ID NO: 100) | AGTCAAGCTTGTAAATGAAGAGTGCTGTGTTAAAGT (SEQ ID NO: 234) | AGTCTCTAGAATGGGAGAGAGCTTCTTGCT (SEQ ID NO: 351) |
| CT763 (SEQ ID NO: 102) | AGTCAAGCTTGTAACACAATCTTGGGCAAGAGAC (SEQ ID NO: 235) | AGTCTCTAGAGATCTCCAGCTTAAGGGTTG C (SEQ ID NO: 352) |
| CT845 (SEQ ID NO: 104) | AGTCAAGCTTGTAAGAGAAGAGAAAGGCTAA GGATG (SEQ ID NO: 236) | AGTCTCTAGAAGGGTTCTCCTCAAGTTCATC (SEQ ID NO: 353) |
| CT848 (SEQ ID NO: 106) | AGTCAAGCTTATAAACCCCTGTCTTAACCCA (SEQ ID NO: 237) | AGTCPCTAGACGTTGCAGAGTTCGTTGTTC (SEQ ID NO: 354) |
| CT863 | AGTCAAGCTTTCCTTTCTAAAAGGCCTGGA (SEQ ID NO: 238) | AGCTTCTAGACAGAACAACGTCTCCTACGC (SEQ ID NO: 355) |
| Psi0330 (SEQ ID NO: 108) | AGTCAAGCTTGCAAATCTAAGGAGTTAGGTTAGGG (SEQ ID NO: 239) | AGCTTCTAGAAGCCAAACGCATAGCTTCAT (SEQ ID NO: 356) |
| Psi0379 (SEQ ID NO: 110) | AGTCAAGCTTGTAATACGGATGGTGCCCACT (SEQ ID NO: 240) | AGTCTCTAGATAGAAGTTGCAGGCGTTCCT (SEQ ID NO: 357) |
| Psi0595 (SEQ ID NO: 112) | AGTCAAGCTTGCGCAAATAAACGATACAA (SEQ ID NO: 241) | AGTCTCTAGATCCGTAGTTGTTACGGAAGG TT (SEQ ID NO: 358) |
| Psi0648 (SEQ ID NO: 114) | AGTCAAGCTTCATTGTGTTATAATTGCAGCCTA (SEQ ID NO: 242) | AGTCTCTAGATTTCTTGGCTCCCTGCATAG (SEQ ID NO: 359) |
| Psi0671 (SEQ ID NO: 116) | AGTCAAGCTTTGACGCCCCTTAAAATAAAGA (SEQ ID NO: 243) | AGTCTCTAGACGCAGAATGGGATAGGACAT (SEQ ID NO: 360) |
| Psi0710 (SEQ ID NO: 120) | AGTCAAGCTTCCGCAAAATGGTTTAACAGA (SEQ ID NO: 244) | AGTCTCTAGATTGCACACCTTGGACGTACT (SEQ ID NO: 361) |
| Psi0761 (SEQ ID NO: 122) | AGTCAAGCTTGTAAGCTTGGGAATCTACACATTTTT (SEQ ID NO: 245) | AGTCTCTAGATTTCGTTAATTCTCCCTTAGA CCA (SEQ ID NO: 362) |
| Psi0774 (SEQ ID NO: 124) | AGTCAAGCTTGTAAAAGCTGAATAAGCCCATGA (SEQ ID NO: 246) | AGTCTCTAGAAAGTTTCTGTGTCGCTACGG (SEQ ID NO: 363) |
| Psi1002 (SEQ ID NO: 126) | AGTCAAGCTTGTAACCGTGAGGAGTCTGAACAA (SEQ ID NO: 247) | AGTCTCTAGAAGAAAAATCCATGGGCTGTA A (SEQ ID NO: 364) |
| Psi1005 (SEQ ID NO: 128) | AGTCAAGCTTTTAATCGAAGAAAATAGGTAGGAAA (SEQ ID NO: 248) | AGTCTCTAGATTCATCGGCTGTTGCTGTAT (SEQ ID NO: 365) |
| Psi1022 (SEQ ID NO: 130) | AGTCAAGCTTGTAACGCTTCTACATCCCCATAATT (SEQ ID NO: 249) | AGTCTCTAGAGAATACGGAAAGCGCAACAT (SEQ ID NO: 366) |

### Transformation of S. flexneri ipaB strain and test of secretion via the type III pathway

*Shigella flexneri* (*ipaB* strain, I-2594) were transformed with the constructs made by the process above and colonies expressing a CPn/cya, CT/cya, or Psi/cya chimera were isolated on plates containing the Congo Red dye. Only red colonies, indicative of a constitutive high level of type III secretion, were considered. Such colonies were picked on a LB plate and incubated for 6 hours at 37°C. A polyvinylidene fluoride membrane (Immobilon-P, Millipore) was deposited on top of the colonies, and the plate was incubated overnight at 37°C. The membrane was briefly incubated in ethanol and then processed for Western blotting using anti-cyclase antibody described (Subtil et al (2001) Mol. Microbiol. 39:792-800). Revelation was done by enhanced chemifluorescence (Amersham). The signal for colonies in which the CPn/cya, CT/cya, or Psi/cya chimeras were not secreted was restricted to the area of the membrane that had been in contact with the colonies. The signal for colonies in which the CPn/cya, CT/cya, or Psi/cya chimeras were secreted appeared as a halo around the area of the membrane that had been in contact with the colonies (see Figure 1).

### Negative controls-

Chlamydial proteins that are homologous to cytosolic proteins in other bacteria species are not expected to be secreted during *Chlamydia* infection and therefore should not be positive for secretion in the present assay. To validate the specificity of this assay, the Inventors constructed 9 chimeras between putative cytosolic chlamydial proteins and the cya reporter molecule. None of these chimeras were secreted by the *ipaB* strain of *S*. *flexneri.* This result shows that the test developed by the present Inventors is specific for detecting secreted proteins.

The 9 chimeras that were constructed used the amino terminal region of CPn0032, CPn0089, CPn0103, CPn0115, CPn0184, CPn0202, CPn0280, CPn0320, CPn0402.

### Negative results-

Most of the 280 chimera that the Inventors tested were not secreted by the *ipaB* strain. This result was expected since in other bacteria species such as *Shigella*, type III secretion allows for the secretion of only a limited number of proteins (up to date about 25 have been identified for *S. flexneri* (Buchrieser C., P. Glaser, C. Rusniok, H. Nedjari, H. d'Hauteville, F. Kunst, P. Sansonetti, and C. Parsot. (2000). The virulence plasmid pWR100 and the repertoire of proteins secreted by the type III secretion apparatus of Shigella flexneri. Mol. Microbiol. 38(4): 760-771).

Examples of Chlamydial proteins for which the corresponding chimera were not secreted in the *ipaB* strain are:
CPn0010, CPn0011, CPn0034, CPn0036, CPn0042, CPn0046, CPn0050, CPn0051, CPn0055, CPn0062, CPn0070, CPn0071, CPn0084, CPn0085, CPn0087, CPn0089, CPn0093, CPn0095, CPn0099, CPn0100.

Detecting secreted proteins by the type III secretion system by *Shigella flexneri*, the secretion of chimeric forms (consisting of the amino-terminal domain of these proteins fused with the Cya reporter) of *Chlamydia* spp. proteins was determined. The secreted chimeric proteins can be classified in 5 categories:
Category 1- Corresponding chimeras from *C. pneumoniae, C. trachomatis,* and *C. psittaci* that are homologous and are secreted. Sequence analysis has shown that these three species are rather distant, and, as a consequence, the percentage of amino acid identity between homologous proteins is generally low. Consequently, the chimeras constructed from homologous proteins share little identity in their amino terminal region, which is the region containing the putative secretion signal. Therefore, the identification that the three homologous chimeras (one from each of the *Chlamydia* species above) are secreted strongly supports that the corresponding proteins are secreted during *Chlamydia* infection.
   Proteins of *C*. *pneumoniae* (CPnXXXX), *C*. *trachomatis* (CTXXX), and C. *psittaci* (PsiXXXX) which are part of this category are:
   CPn0330 ; CT083 ; Psi0330 ; CPn0379 ; CT053 ; Psi0379 ; CPn0595 ; CT476 ; Psi0595 ; CPn0648 ; CT529 ; Psi0648 ; CPn 0671 ; C550 ; Psi0671 ; CPn0710 ; CT666 ; Psi0710 ; CPn0761 ; CT610 ; Psi0761 ; CPn0774 ; CT606.1 ; Psi0774 ; CPn1002 ; CT845 ; Psi1002 ; CPn1005 ; CT848 ; Psi1005 ; CPn1022 ; CT863 ; Psi1022.
Category 2- Proteins of C. *pneumoniae* and *C. trachomatis* which are homologous and for which the 2 corresponding chimeras are secreted. For the same reasons as explained above in the case of category 1, these results support that these proteins are secreted during *Chlamydia* infection.
   Proteins of *C. pneumoniae* (CPnXXXX) and *C. trachomatis* (CTXXX) which are part of this category are:
   CPn0490 ; CT387 ; CPn0705 ; CT671 ; CPn0711 ; CT665 ; CPn0712 ; CT664 ; CPn0725 ; CT652.1 ; CPn0770 ; CT642 ; CPn0859 ; CT718 ; CPn0906 ; CT763.
Category 3- *C. pneumoniae* proteins that are secreted, which have no homolog in *C. trachomatis* or *C*. *psittaci.* They may be important proteins, being specific of *C*. *pneumoniae.*
   *C. pneumoniae* proteins (CPnXXXX) which are part of this category are:
   Gun0009 ; CPn0012 ; CPn0028 ; CPn0049; CPn0063 ; CPn0066 ; CPn0067 ; CPn0130 ; CPn0132 ; CPn0146 ; CPn0167 ; CPn0174 ; CPn0175 ; CPn0181 ; CPn0210; CPn0211 ; CPn0220 ; CPn0223 ; CPn0226 ; CPn0243 ; CPn0267 ; CPn0277 ; CPn0284 ; CPn0357 ; CPn0365 ; CPn0585 ; CPn0829 ; CPn1027.
Category 4- *C*. *pneumoniae* proteins that have a homolog in at least one of the other 2 sequenced *Chlamydia* species and for which secretion of the corresponding homologous chimera have not been tested for technical or scientific reason (for example when the percentage of identity between the homologous proteins was very high).
   *C. pneumoniae* (CPnXXXX) proteins that have a homolog in at least one of the other 2 sequenced *Chlamydia* species and for which secretion of the corresponding homologous chimeras have not been tested, and are part of this category are:
   CPn0026 ; CPn0104 ; CPn0186 ; CPn0206 ; CPn0291 ; CPn0292 ; CPn0405 ; Con0443 CPn0480 ; CPn0489 ; CPn0556 ; CPn0673 ; CPn0681 ; CPn0720 ; CPn0746 ; CPn0853 ; CPn0879 ; CPn0939 ; CPn1019 ; CPn1020 ; CPn1032.
Category 5- *C. pneumoniae* proteins that have homologs in the other 2 sequenced *Chlamydia* species and for which secretion of the corresponding homologous chimeras have either not been tested or are negative or undetermined. Although these results support that most proteins in this category are secreted by *Chlamydia* during infection, the Inventors regard them as somewhat less strong candidates than the proteins classified in the other 4 categories, until more data are obtained (see for example the result with Psi1058 below).
   *C. pneumoniae* (CPnXXXX) proteins that have homologs in the other 2 sequenced *Chlamydia* species and for which secretion of the corresponding homologous chimeras have either not been tested or are negative or undetermined, and are part of this category are:
   CPn0105 ; CPn0287 ; CPn0334 ; CPn0374 ; CPn0399 ; CPn0497 ; CPn0522 ; CPn0582 ; CPn0588 ; CPn0729 ; CPn0755 ; CPn0764 ; CPn0792 ; CPn0820 ; CPn0821 ; CPn1007 ; CPn1016 ; CPn1058.

### Example 2

Chlamydial genes were cloned by PCR for expression of full-length chlamydial proteins with a carboxy-terminal Histidine-tag. The forward and reverse primers contained additional *NcoI* and *KpnI* sites, respectively, to allow cloning of the PCR fragments between the *NcoI* and *KpnI* sites of the pQE-TriSystem expression vector (Qiagen). Sequences of the primers are given in Table II. Recombinant plasmids were amplified in *E. coli* TG1 and the sequences were checked by sequencing.

Plasmids were used to transform the strains SF401 and SF620 which are derivatives of M90T in which the *mxiD* and *ipaB* genes, respectively, have been inactivated (Allaoui *et al*., 1993; Ménard *et al*., 1993). Transformed colonies were isolated on plates containing 100 µg/ml ampicillin.

Analysis of secreted proteins was performed as previously described (Allaoui *et al*., 1993). Briefly, 1 ml of an overnight culture at 30 °C was inoculated in 30 ml of LB and incubated at 37 °C for 3 h. Bacteria were harvested by centrifugation and the culture supernatant was filtered through 0.22 µm filters. Proteins present in 25 ml of the filtrates were precipitated by the addition of 1/10 (v/v) trichloroacetic acid, and resuspended in 0.5 ml of sample buffer for electrophoresis. Equal volumes of samples of the bacterial pellet and culture supernatants, the latter being concentrated 25-fold as compared to the pellet, were analyzed by electrophoresis in 12% polyacrylamide gels in the presence of sodium dodecyl sulfate (SDS-PAGE). After electrophoresis, proteins were transferred on a PVDF membrane (Millipore Corporation, Bedford, MA), and the membrane was used for blotting with anti-His antibody (H-15, sc-803, Santa Cruz Biotechnology). Western blotting and revelation were performed by enhanced chemiluminescence (Amersham Pharmacia Biotech) according to the manufacturer's instructions.

**Table II**

| **Protein** | **Forward primer** | **Reverse primer** |
|---|---|---|
| CPn0175 | ATCGTACCATGGAGCAACCCAATTGTG(SEQ ID NO: 367) | AGTCGGTACCAGCTTCCTTAACTTCGCTGAG (SEQ ID NO: 373) |
| Psi0705 (SEQ ID NO: 118) | ATCGTACCATGGAATTAAATAAAACATCCGAG TCTTTG (SEQ ID NO: 368) | AGTCGGTACCTTGGTTTIGTTCTTGATCTTGC (SEQ ID NO: 374) |
| Psi0725 | ATCGTACCATGGTTCTCGCTTCTTGTCTTCTTG (SEQ ID NO: 369) | AGTCGGTACCATCTAAGAAATCATCTTCTGTAA GGAC (SEQ ID NO: 375) |
| Psi0774 (SEQ ID NO: 124) | ATCGTACCATGGATGAAGGTATTCAAACCGTT TCT (SEQ ID NO: 370) | AGTCGGTACGGTCTCTAGGAACTAGCGTTTCCA (SEQ ID NO: 376) |
| Psi1005 (SEQ ID NO: 128) | ATCGTACCATGGGGTTCTCTTCTCCAGCACTTC (SEQ ID NO: 371) | AGTCGGTACCAATGTTTGCTATCAAACTTACAA TT (SEQ ID NO: 377) |
| Psi1058 (SEQ ID NO: 132) | ATCGTACCATGGAGAACAAAACACTAAGCGT TTTCT (SEQ ID NO: 372) | AGTCGGTACCCAATGAAAATAGACGCGGA (SEQ ID NO: 378) |

The present inventors have shown that CPn0175, Psi0705, Psi0725, Psi0774, Psi1005 and Psi1058, expressed as full-length proteins with a C-terminal histidine tag, are found in the supernatant of the transformed *ipaB* cultures. When they are expressed in the *mxiD* strain, which is deficient for type III secretion, these proteins are absent from the culture supernatants (see Figure 2). These results confirm that CPn0175, Psi0705, Psi0725, Psi0774, Psi1005 and Psi1058 are secreted by a type III mechanism in *S*. *flexneri.*

### Example 3

Proteins Psi0705 and Psi0710 were expressed in *E*. *coli* with a carboxy-terminal Histidine tag and purified by standard divalent metal column chromatography methods according to the manufacturer's instructions (Qiagen). Purified proteins were used to immunize rabbits and specific antibodies against Psi0705 and Psi0710 were obtained. These antibodies were used to study Psi0705 and Psi0710 localization during *Chlamydia* infection.

A: By immunofluorescence, the present inventors determined that Psi0710 is associated with the membrane of the inclusion in HeLa cells infected with C. *psittaci* GPIC strain (Figure 4). As a control, the present inventors determined that the pre-immune serum did not label infected cells, and that antibodies against the Major Outer Membrane Protein of *Chlamydia* did not label the membrane of the inclusion.

B: By Western Blot, the present inventors determined that Psi0705 is associated with the cytosolic fraction of HeLa cells infected with *C*. *psittaci* GPIC strain (Figure 3). The same result was obtained using antibodies specific for Psi0710. As a control, the present inventors determined that the Major Outer Membrane Protein of *Chlamydia* was not found in the cytosolic fraction of HeLa cells infected with *C*. *psittaci* GPIC strain.

These experiments demonstrate that Psi0705 and Psi0710 are secreted by *Chlamydia* during infection. Therefore, these data fully confirm the inventive approach to identify such proteins using heterologous type III secretion as described in the present invention.

## Claims

1. A method for *in vitro* diagnosing a *Chlamydia* infection in an animal, wherein said method comprises (a) providing a purified CPn0104 polypeptide from *Chlamydia pneumoniae,* or an immunogenic fragment thereof; (b) bringing said polypeptide or immunogenic fragment thereof into contact with a serum sample of said animal; and (c) detecting complexes formed between said polypeptide or immunogenic fragment thereof and antibodies contained in the serum sample; wherein said complexes are indicative of a *Chlamydia* infection in said animal.

2. The method of claim 1, wherein said purified CPn0104 polypeptide from *Chlamydia pneumoniae,* or said immunogenic fragment thereof is labelled.

3. A method of *in vitro* diagnosing a *Chlamydia* infection in an animal, wherein said method comprises (a) adding an antibody directed against the CPn0104 polypeptide from *Chlamydia pneumoniae,* under conditions suitable for antigen-antibody complex formation, to an animal sample of a tissue suspected to be infected by *Chlamydia* ; and (b) detecting antigen-antibody complex formation; wherein said complex formation is indicative of a *Chlamydia* infection in said animal.

4. The method of claim 3, wherein said antibody is labelled.

5. The method according to any of claims 1 to 4, wherein said *Chlamydia* infection is a *Chlamydia pneumoniae* infection.

## Patentansprüche

1. Verfahren zum in vitro-Diagnostizieren einer *Chlamydia-*Infektion bei einem Tier, wobei das Verfahren umfasst: (a) Bereitstellen eines gereinigten CPn0104-Polypeptids von *Chlamydia pneumoniae* oder eines immunogenen Fragments davon; (b) Inkontaktbringen des Polypeptids oder des immunogenen Fragments davon mit einer Serumprobe des Tiers und (c) Detektieren von Komplexen, die zwischen dem Polypeptid oder dem immunogenen Fragment davon und Antikörpern, die in der Serumprobe enthalten sind, gebildet wurden, wobei die Komplexe für eine Chlamydia-Infektion bei dem Tier indikativ sind.

2. Verfahren nach Anspruch 1, wobei das gereinigte CPn0104-Polypeptid von *Chlamydia pneumoniae* oder das immunogene Fragment davon markiert wird.

3. Verfahren zum in vitro-Diagnostizieren einer *Chlamydia-*Infektion bei einem Tier, wobei das Verfahren umfasst: (a) Zugeben eines Antikörpers, der gegen das CPn0104-Polypeptid von *Chlamydia pneumoniae* gerichtet ist, unter Bedingungen, die für eine Antigen-Antikörper-Komplexbildung geeignet sind, zu einer Tierprobe eines Gewebes, von der vermutet wird, dass sie durch *Chlamydia* infiziert ist, und (b) Detektieren der Antigen-Antikörper-Komplexbildung, wobei die Komplexbildung für eine Chlamydia-Infektion bei dem Tier indikativ ist.

4. Verfahren nach Anspruch 3, wobei der Antikörper markiert wird.

5. Verfahren nach einem der Ansprüche 1 bis 4, wobei die *Chlamydia*-Infektion eine *Chlamydia pneumoniae*-Infektion ist.

## Revendications

1. Procédé de diagnostic *in vitro* d'une infection par *Chlamydia* chez un animal, dans lequel ledit procédé comprend (a) l'obtention d'un polypeptide CPn0104 purifié issu de *Chlamydia pneumoniae,* ou d'un fragment immunogène de celui-ci ; (b) la mise en contact dudit polypeptide ou dudit fragment immunogène de celui-ci avec un échantillon de sérum dudit animal ; et (c) la détection des complexes formés entre ledit polypeptide ou ledit fragment immunogène de celui-ci et les anticorps contenus dans l'échantillon de sérum ; lesdits complexes indiquant une infection par *Chlamydia* chez ledit animal.

2. Procédé selon la revendication 1, dans lequel ledit polypeptide CPn0104 purifié issu de *Chlamydia pneumoniae* ou ledit fragment immunogène de celui-ci est marqué.

3. Procédé de diagnostic *in vitro* d'une infection par *Chlamydia* chez un animal, dans lequel ledit procédé comprend (a) l'ajout d'un anticorps dirigé contre le polypeptide CPn0104 issu de *Chlamydia pneumoniae,* dans des conditions appropriées pour la formation d'un complexe antigène-anticorps, à un échantillon de tissu d'un animal suspecté d'être infecté par *Chlamydia* ; et (b) la détection de la formation du complexe antigène-anticorps ; ladite formation du complexe indiquant une infection par *Chlamydia* chez ledit animal.

4. Procédé selon la revendication 3, dans lequel ledit anticorps est marqué.

5. Procédé selon l'une quelconque des revendications 1 à 4, dans lequel ladite infection par *Chlamydia* est une infection par *Chlamydia pneumoniae.*


### SEQUENCE LISTING

<110> INSTITUT PASTEUR CENTRE NATIONAL DE LA RECHERCHE SCIENTIFIQUE SUBTIL-SANDS, AGATHE DAUTRY-VARSAT, ALICE
<120> SECRETED CHLAMYDIA POLYPEPTIDES, POLYNUCLEOTIDES CODING THEREFOR, THERAPEUTIC AND DIAGNOSTIC USES THEREOF
<130> BLOcp226PCT114
<150> US 60/448,879
   <151> 2003-02-24
<160> 378
<170> PatentIn version 3.1
<210> 1
   <211> 1203
   <212> DNA
   <213> chlamydia pneumoniae
<400> 1
   atgagaatgc tccagatttc tatgcttctt ttagctttag gaactgcaat caactcacca     60
   gcaatctatg ctgccgattc ccaatccgta tcctttccag aacaacttcc ctcttcattt    120
   actggagaaa ttaagggaaa ccacgtacgg atgcgtctag cacctcatac tgatgggacc    180
   atcattaggg aattttctaa aggagatctt gttgctgtta tcggagaaag caaagactac    240
   tacgtaattt ctgcgcctcc aggaattaca ggttatgtgt tccgctcatt tgttttagat    300
   aatgtcgttg aaggtgaaca agtcaatgtt cgtttagaac cctcaacatc agctccagta    360
   cttgtgagac tctcccgagg cacacaaata cagccagctt ctcaagagcc acatgggaaa    420
   tggttagagg tggtcttgcc ctcacaatgc gtattctatg ttgcaaaaaa ctttgttgct    480
   aacaaaggac ccatcgagct gtatacgcaa cgcgagggac aaaaaaagat tgccatggac    540
   cttatcaatt ctgctttaaa ctttgctcat atagagcttg agaaaagcct caatgagatt    600
   gatctggaag caatttataa aaagatcaac cttgtacaat ccgaagagtt taaagatgtt    660
   ccaggaattc aagggcttat acaaaaagct ttagaagaaa tccaagatgc ctatctttct    720
   aaatctctag aatctcaaaa tacttcgatt gcaagctcac aatgttccac tcctaaggtt    780
   tcttcttctg aagttacaac ttcattactt tcacgtcata ttcgtaagca aactgcatta    840
   aaaacagctc ctcttaccca aggaagagaa aacctagagt attctctctt cagaatctgg    900
   gccagtatgc agcaaggcaa tgaccactct gaagcactaa cacaagaagc gttttatcgc    960
   gctgaacaga agaaaaaaca agtgcttgcg ggtgtattag aagtgtatcc tcatgtagta   1020
   aagaacaatc ccggggatta cctactaaaa gctcaggaaa acacgattgc ttttctttac   1080
   ggtacaagta tcaacttaga gcaatggtta ggaaagcgtg tcactgtgga atgtctccca   1140
   cgtcctaaca accattttgc ttttcctgct tattatgtag ttggaattaa agaagcttca   1200
   taa                                                                 1203
<210> 2
   <211> 310
   <212> PRT
   <213> Chlamydia pneumoniae
<400> 2
   Met Arg Met Leu Gln Ile Ser Met Leu Leu Leu Ala Leu Gly Thr Ala
   1               5                   10                  15
   Ile Asn Ser Pro Ala Ile Tyr Ala Ala Asp Ser Gln Ser Val Ser Phe
               20                  25                  30
   Pro Val Leu Leu Gly Leu Gly Gln Asp Lys Phe Leu Lys Ala Thr Glu
           35                  40                  45
   Asp Glu Asp Val Leu Phe Glu Ser Gln Lys Ala Ile Asp Ala Trp Asn
       50                  55                  60
   Ala Leu Leu Thr Lys Ala Arg Asp Val Leu Gly Leu Gly Asp Ile Gly
   65                  70                  75                  80
   Ala Ile Tyr Gln Thr Ile Glu Phe Leu Gly Ala Tyr Leu Ser Lys Val
                   85                  90                  95
   Asn Arg Arg Ala Phe Cys Ile Ala Ser Glu Ile His Phe Leu Lys Thr
               100                 105                 110
   Ala Ile Arg Asp Leu Asn Ala Tyr Tyr Leu Leu Asp Phe Arg Trp Pro
           115                 120                 125
   Leu Cys Lys Ile Glu Glu Phe Val Asp Trp Gly Asn Asp Cys Val Glu
       130                 135                 140
   Ile Ala Lys Arg Lys Leu Cys Thr Phe Glu Lys Glu Thr Lys Glu Leu
   145                 150                 155                 160
   Asn Glu Ser Leu Leu Arg Glu Glu His Ala Met Glu Lys Cys Ser Ile
                   165                 170                 175
   Gln Asp Leu Gln Arg Lys Leu Ser Asp Ile Ile Ile Glu Leu His Asp
               180                 185                 190
   Val Ser Leu Phe Cys Phe Ser Lys Thr Pro Ser Gln Glu Glu Tyr Gln
           195                 200                 205
   Lys Asp Cys Leu Tyr Gln Ser Arg Leu Arg Tyr Leu Leu Leu Leu Tyr
       210                 215                 220
   Glu Tyr Thr Leu Leu Cys Lys Thr Ser Thr Asp Phe Gln Glu Gln Ala
   225                 230                 235                 240
   Arg Ala Lys Glu Glu Phe Ile Arg Glu Lys Phe Ser Leu Leu Glu Leu
   245 250 255
   Glu Lys Gly Ile Lys Gln Thr Lys Glu Leu Glu Phe Ala Ile Ala Lys
   260 265 270
   Ser Lys Leu Glu Arg Gly Cys Leu Val Met Arg Lys Tyr Glu Ala Ala
   275 280 285
   Ala Lys His Ser Leu Asp Ser Met Phe Glu Glu Glu Thr Val Lys Ser
   290 295 300
   Pro Arg Lys Asp Thr Glu
   305 310
<210> 3
   <211> 756
   <212> DNA
   <213> Chlamydia pneumoniae
<400> 3
   ttgctaatgg atatttccca tatcctggaa gatcttgcct atgacgaagg gatccttcca      60
   agggaagcta tagaagcggc tattgttaaa caaatgcaaa ttacgcctta tttactgcat     120
   attttacacg acgctactca gcgcgtccct gagattgtaa atgatgggag ttatcaaggt     180
   cacctctatg ccatgtatct cctcgcacaa ttcagagaaa gtcgcgcact ccctctcatc     240
   attaaactct ttgcatttga agatgatact ccacacgcaa tagcaggtga tgtcctaacc     300
   gaagatctgc ctaggatcct agctagcgtc tgcaatgatg actcgctaat taaagagctc     360
   atagaaactc caaaaatcaa tccttatgtg aaggcagccg caatctctgg tcttgtaact     420
   cttgtaggag ccgggaaaat tcctagggat aaagttatcc gttattttgc agaacttcta     480
   aactatagat tagaaaaaca gccctcgttc gcttgggata acctaatcgc agggatctgt     540
   actctttacc ccggagagct cttctatcca ataagcaaag cctttgacgg aggacttgtt     600
   gatacatctt tcatcagcat ggaagatgtc gaaaatatta tccacgaaga aaccgtggaa     660
   tcttgtatcc ataccctctg ttcttctaca gaactcatta atgacactct agaagaaatg     720
   gaaaaatggt tagaagactt ccccatagaa ccgtga                               756
<210> 4
   <211> 251
   <212> PRT
   <213> Chlamydia pneumoniae
<400> 4
   Leu Leu Met Asp Ile Ser His Ile Leu Glu Asp Leu Ala Tyr Asp Glu
   1               5                   10                  15
   Gly Ile Leu Pro Arg Glu Ala Ile Glu Ala Ala Ile Val Lys Gln Met
               20                  25                  30
   Gln Ile Thr Pro Tyr Leu Leu His Ile Leu His Asp Ala Thr Gln Arg
           35                  40                  45
   Val Pro Glu Ile Val Asn Asp Gly Ser Tyr Gln Gly His Leu Tyr Ala
       50                  55                  60
   Met Tyr Leu Leu Ala Gln Phe Arg Glu Ser Arg Ala Leu Pro Leu Ile
   65                  70                  75                  80
   Ile Lys Leu Phe Ala Phe Glu Asp Asp Thr Pro His Ala Ile Ala Gly
                   85                  90                  95
   Asp Val Leu Thr Glu Asp Leu Pro Arg Ile Leu Ala Ser Val Cys Asn
               100                 105                 110
   Asp Asp Ser Leu Ile Lys Glu Leu Ile Glu Thr Pro Lys Ile Asn Pro
           115                 120                 125
   Tyr Val Lys Ala Ala Ala Ile Ser Gly Leu Val Thr Leu Val Gly Ala
       130                 135                 140
   Gly Lys Ile Pro Arg Asp Lys Val Ile Arg Tyr Phe Ala Glu Leu Leu
   145                 150                 155                 160
   Asn Tyr Arg Leu Glu Lys Gln Pro Ser Phe Ala Trp Asp Asn Leu Ile
                   165                 170                 175
   Ala Gly Ile Cys Thr Leu Tyr Pro Gly Glu Leu Phe Tyr Pro Ile Ser
               180                 185                 190
   Lys Ala Phe Asp Gly Gly Leu Val Asp Thr Ser Phe Ile Ser Met Glu
           195                 200                 205
   Asp Val Glu Asn Ile Ile His Glu Glu Thr Val Glu Ser Cys Ile His
       210                 215                 220
   Thr Leu Cys Ser Ser Thr Glu Leu Ile Asn Asp Thr Leu Glu Glu Met
   225                 230                 235                 240
   Glu Lys Trp Leu Glu Asp Phe Pro Ile Glu Pro
                   245                 250
<210> 5
   <211> 948
   <212> DNA
   <213> chlamydia pneumoniae
<400> 5
   atgctagtag agttagaggc tcttaaaaga gagtttgcgc atttaaaaga ccagaagccg 60
   acaagtgacc aagagatcac ttcactttat caatgtttgg atcatcttga attcgtttta 120
   ctcgggctgg gccaggacaa atttttaaag gctacggaag atgaagatgt gctttttgag 180
   tctcaaaaag caatcgatgc gtggaatgct ttattgacaa aagccagaga tgttttaggt 240
   cttggggaca taggtgctat ctatcagact atagaattct tgggtgccta tttatcaaaa 300
   gtgaatcgga gggctttttg tattgcttcg gagatacatt ttctaaaaac agcaatccga 360
   gatttgaatg catattacct gttagatttt agatggcctc tttgcaagat agaagagttt 420
   gtggattggg ggaatgattg tgttgaaata gcaaagagga agctatgcac ttttgaaaaa 480
   gaaaccaagg agctcaatga gagccttctt agagaggagc atgcgatgga gaaatgctcg 540
   attcaagatc tgcaaaggaa acttagcgac attattattg aattgcatga tgtttctctt 600
   ttttgttttt ctaagactcc cagtcaagag gagtatcaaa aggattgttt gtatcaatca 660
   cgattgaggt acttattgtt gctgtatgag tatacattgt tatgtaagac atccacagat 720
   tttcaagagc aggctagggc taaagaggag ttcattaggg agaaattcag ccttctagag 780
   ctcgaaaagg gaataaaaca aactaaagag cttgagtttg caattgctaa aagtaagtta 840
   gaacggggct gtttagttat gaggaagtat gaagctgccg ctaaacatag tttagattct 900
   atgttcgaag aagaaactgt gaagtcgccg cggaaagaca cagaataa 948
<210> 6
   <211> 315
   <212> PRT
   <213> Chlamydia pneumoniae
<400> 6
   Met Leu Val Glu Leu Glu Ala Leu Lys Arg Glu Phe Ala His Leu Lys
   1               5                   10                  15
   Asp Gln Lys Pro Thr Ser Asp Gln Glu Ile Thr Ser Leu Tyr Gln Cys
               20                  25                  30
   Leu Asp His Leu Glu Phe Val Leu Leu Gly Leu Gly Gln Asp Lys Phe
           35                  40                  45
   Leu Lys Ala Thr Glu Asp Glu Asp Val Leu Phe Glu Ser Gln Lys Ala
       50                  55                  60
   Ile Asp Ala Trp Asn Ala Leu Leu Thr Lys Ala Arg Asp Val Leu Gly
   65                  70                  75                  80
   Leu Gly Asp Ile Gly Ala Ile Tyr Gln Thr Ile Glu Phe Leu Gly Ala
                   85                  90                  95
   Tyr Leu Ser Lys Val Asn Arg Arg Ala Phe Cys Ile Ala Ser Glu Ile
               100                 105                 110
   His Phe Leu Lys Thr Ala Ile Arg Asp Leu Asn Ala Tyr Tyr Leu Leu
           115                 120                 125
   Asp Phe Arg Trp Pro Leu Cys Lys Ile Glu Glu Phe Val Asp Trp Gly
       130                 135                 140
   Asn Asp Cys Val Glu Ile Ala Lys Arg Lys Leu Cys Thr Phe Glu Lys
   145 150 155 160
   Glu Thr Lys Glu Leu Asn Glu Ser Leu Leu Arg Glu Glu His Ala Met
   165 170 175
   Glu Lys Cys Ser Ile Gln Asp Leu Gln Arg Lys Leu Ser Asp Ile Ile
   180 185 190
   Ile Glu Leu His Asp Val Ser Leu Phe Cys Phe Ser Lys Thr Pro Ser
   195 200 205
   Gln Glu Glu Tyr Gln Lys Asp Cys Leu Tyr Gln Ser Arg Leu Arg Tyr
   210 215 220
   Leu Leu Leu Leu Tyr Glu Tyr Thr Leu Leu Cys Lys Thr Ser Thr Asp
   225 230 235 240
   Phe Gln Glu Gln Ala Arg Ala Lys Glu Glu Phe Ile Arg Glu Lys Phe
   245 250 255
   Ser Leu Leu Glu Leu Glu Lys Gly Ile Lys Gln Thr Lys Glu Leu Glu
   260 265 270
   Phe Ala Ile Ala Lys Ser Lys Leu Glu Arg Gly Cys Leu Val Met Arg
   275 280 285
   Lys Tyr Glu Ala Ala Ala Lys His Ser Leu Asp Ser Met Phe Glu Glu
   290 295 300
   Glu Thr Val Lys Ser Pro Arg Lys Asp Thr Glu
   305 310 315
<210> 7
   <211> 648
   <212> DNA
   <213> Chlamydia pneumoniae
<400> 7
   atgcgaaaaa tgttggtatt attggcatct ttaggacttc tatccccaac cctatccagc      60
   tgcactcact taggctcttc aggaagttat catcctaagc tatacacttc agggagcaaa     120
   actaaaggtg tgattgcgat gcttcctgta tttcatcgcc caggaaagag tcttgaacct     180
   ttaccttgga acctccaagg agaatttact gaagagatca gcaaaaggtt ttatgcttcg     240
   gaaaaggtct tcctgatcaa gcacaatgct tcacctcaga cagtctctca gttctatgct     300
   ccgattgcga atcgtctacc cgaaacaatt attgagcaat ttcttcctgc agaattcatt     360
   gttgctacag aactgttaga acaaaagaca gggaaagaag caggtgtcga ttctgtaaca     420
   gcgtctgtac gtgttcgcgt ttttgatatc cgtcatcata aaatagctct catttatcaa     480
   gagattatcg aatgcagcca gcctttaact accctagtca atgattatca tcgctatggc     540
   tggaactcaa aacattttga ttcaacgccc atgggcttaa tgcatagccg tcttttccgc     600
   gaagttgttg ccagagttga gggctatgtt tgtgctaact actcgtag                  648
<210> 8
   <211> 173
   <212> PRT
   <213> Chlamydia pneumoniae
<400> 8
   Met Arg Lys Met Leu Val Leu Leu Ala Ser Leu Gly Leu Leu Ser Pro
   1               5                   10                  15
   Thr Leu Ser Ser Cys Thr Ala Ile Thr Ser Ser Pro Gly Met Val Asn
               20                  25                  30
   Leu Leu Ile Gly Trp Ala Lys Thr Lys Phe Ile Gln Pro Ile Arg Glu
           35                  40                  45
   Ser Lys Leu Phe Gln Ser Arg Ala Cys Gln Ile Thr Leu Leu Val Leu
       50                  55                  60
   Gly Ile Leu Leu Val Val Ala Gly Leu Ala Cys Met Phe Ile Phe His
   65                  70                  75                  80
   Ser Gln Leu Gly Ala Asn Ala Phe Trp Leu Ile Ile Pro Ala Ala Ile
                   85                  90                  95
   Gly Leu Ile Lys Leu Leu Val Thr Ser Leu Cys Phe Asp Glu Ala Cys
               100                 105                 110
   Thr Ser Glu Lys Leu Met Val Phe Gln Lys Trp Ala Gly Val Leu Glu
           115                 120                 125
   Asp Gln Leu Asp Asp Gly Ile Leu Asn Asn Ser Asn Lys Ile Phe Gly
       130                 135                 140
   His Val Lys Thr Glu Gly Asn Thr Ser Arg Ala Thr Thr Pro Val Leu
   145                 150                 155                 160
   Asn Asp Gly Arg Gly Thr Pro Val Leu Ser Pro Leu Val
                   165                 170
<210> 9
   <211> 777
   <212> DNA
   <213> Chlamydia pneumoniae
<400> 9
   ttgggtttca ctgattactt aggaggttgt ttgacgaatc ctttagggaa attcccctca      60
   ccacagaatc cacaggttgt tacgatagcg ccttcttcca caacaccaca agcagtctca     120
   tctgcagttc aaggttttct tcaaactgga ggagctgcct cctctacagc gacaactact     180
   accgcatccg gagcctctgc attaggactt tcacctgatc aagtgcaagc gttgcttact      240
   aatttattaa atgtgggaca accatcagtg ggacaaccat caacttcagc aggaacttcg      300
   ggagcctcct cttccagtgc aagtatgcag caacagcttt tgcaacttat cttagacaag      360
   acaacaggaa gtggcggatc gtccgtgagt tcagagcaat tacagcaact ccttagcttg      420
   gtgagccaga tgactacgtc tcaaggagga agtggtggaa ctcaggcagg acaggccgct      480
   tcggtactgt tgaatttgtt atcggcaaca ggatctgcag cagcaaatcc tttagggaca      540
   gctgcatcgt tggcacagat catttatgca gcagtaacaa gtcctggagc aaagaaaact      600
   agcgaatttt gttataatta ttgtggagag acctgccaag gcaactgcgg ttgtcctacc      660
   tgtggctgtc cagacggaca gtgcggttgt ggaggatttg gccgtttttt ctgtggtgta      720
   tggaaaaatt gttgcgggat aggagaggga tcccaagaac ccgcaatccc tttataa         777
<210> 10
   <211> 258
   <212> PRT
   <213> Chlamydia pneumoniae
<400> 10
   Leu Gly Phe Thr Asp Tyr Leu Gly Gly Cys Leu Thr Asn Pro Leu Gly
   1 5 10 15
   Lys Phe Pro Ser Pro Gln Asn Pro Gln Val Val Thr Ile Ala Pro Ser
   20 25 30
   Ser Thr Thr Pro Gln Ala Val Ser Ser Ala Val Gln Gly Phe Leu Gln
   35 40 45
   Thr Gly Gly Ala Ala Ser Ser Thr Ala Thr Thr Thr Thr Ala Ser Gly
   50 55 60
   Ala Ser Ala Leu Gly Leu Ser Pro Asp Gln Val Gln Ala Leu Leu Thr
   65 70 75 80
   Asn Leu Leu Asn Val Gly Gln Pro Ser Val Gly Gln Pro Ser Thr Ser
   85 90 95
   Ala Gly Thr Ser Gly Ala Ser Ser Ser Ser Ala Ser Met Gln Gln Gln
   100 105 110
   Leu Leu Gln Leu Ile Leu Asp Lys Thr Thr Gly Ser Gly Gly Ser Ser
   115 120 125
   Val Ser Ser Glu Gln Leu Gln Gln Leu Leu Ser Leu Val Ser Gln Met
   130 135 140
   Thr Thr Ser Gln Gly Gly Ser Gly Gly Thr Gln Ala Gly Gln Ala Ala
   145 150 155 160
   Ser Val Leu Leu Asn Leu Leu Ser Ala Thr Gly Ser Ala Ala Ala Asn
   165 170 175
   Pro Leu Gly Thr Ala Ala Ser Leu Ala Gln Ile Ile Tyr Ala Ala Val
   180 185 190
   Thr Ser Pro Gly Ala Lys Lys Thr Ser Glu Phe Cys Tyr Asn Tyr Cys
   195 200 205
   Gly Glu Thr Cys Gln Gly Asn Cys Gly Cys Pro Thr Cys Gly Cys Pro
   210 215 220
   Asp Gly Gln Cys Gly Cys Gly Gly Phe Gly Arg Phe Phe Cys Gly Val
   225 230 235 240
   Trp Lys Asn Cys Cys Gly Ile Gly Glu Gly Ser Gln Glu Pro Ala Ile
   245 250 255
   Pro Leu
<210> 11
   <211> 1254
   <212> DNA
   <213> Chlamydia pneumoniae
<400> 11
   atgagccaac cccctataaa ccctttaggt caacctcaag ttcctgcagc agcatcccca      60
   tcagggcagc caagcgtggt aaaacgttta aaaacgtcat ccacagggtt attcaaaaga     120
   tttattacta ttcctgataa atatcctaaa atgcgctatg tctatgacac aggcattatt     180
   gcccttgcgg caattgcgat cctttcgatt ctcctgactg cttcaggaaa cagccttatg     240
   ctttatgctc tcgctccggc acttgccctg ggagctttgg gagttactct acttatttct     300
   gatattctgg acagtccgaa ggccaagaaa atcggtgagg caatcactgc tatcgtcgtt     360
   cctatcattg tattagcgat tgctgcgggt cttattgcag gggctttcgt tgcctctagt      420
   gggacgatgt tagtctttgc caaccctatg tttgtcatgg gattgattac ggtggggcta     480
   tacttcatgt ccttgaataa gctcacctta gattatttcc gtagggaaca cctcttgagg     540
   atggaaaaga aaacccaaga gaccgcggag cctattctag tgactccatc cgccgacgat     600
   gcaaaaaaaa tcgcagtgga aaagaaaaaa gatctttctg catctgcccg catggaggaa     660
   cacgaagctt cacaacgcca agatgcccgt catcgtagga tcggtcggga ggctcaagga     720
   tctttcttct attcgtcacg aaatcctgag catagacgct ccttcggcag cctctcacgt     780
   tttaaaacaa aaccctcaga tgcggcttct acacgacccg catctataag tcctccattt     840
   aaggacgatt ttcagcctta tcacttcaaa gatttaagaa gcagttcatt cggtagtgga     900
   gcgagcagtg cgtttacacc cataatgcct gcaagttccc gctctcctaa tttctccacg     960
   gggacggttc tacaccctga gccggtctac cctaagggag gaaaagaacc ctcaattcct    1020
   cgagtttctt catcttcccg ccgttcccct cgtgatcgcc aagataaaca gcagcaacag    1080
   caaaatcaag atgaagaaca gaaacagcaa tctaagaaga aaagcgggaa atcgaatcaa    1140
   tctcttaaaa ctccgcctcc agacggaaaa agcacggcta acctcagccc ctccaatcca    1200
   ttctctgacg gttatgacga aagagaaaaa cggaaacaca gaaagaacaa ataa          1254
<210> 12
   <211> 417
   <212> PRT
   <213> Chlamydia pneumoniae
<400> 12
   Met Ser Gln Pro Pro Ile Asn Pro Leu Gly Gln Pro Gln Val Pro Ala
   1               5                   10                  15
   Ala Ala Ser Pro Ser Gly Gln Pro Ser Val Val Lys Arg Leu Lys Thr
               20                  25                  30
   Ser Ser Thr Gly Leu Phe Lys Arg Phe Ile Thr Ile Pro Asp Lys Tyr
           35                  40                  45
   Pro Lys Met Arg Tyr Val Tyr Asp Thr Gly Ile Ile Ala Leu Ala Ala
       50                  55                  60
   Ile Ala Ile Leu Ser Ile Leu Leu Thr Ala Ser Gly Asn Ser Leu Met
   65                  70                  75                  80
   Leu Tyr Ala Leu Ala Pro Ala Leu Ala Leu Gly Ala Leu Gly Val Thr
                   85                  90                  95
   Leu Leu Ile Ser Asp Ile Leu Asp Ser Pro Lys Ala Lys Lys Ile Gly
               100                 105                 110
   Glu Ala Ile Thr Ala Ile Val Val Pro Ile Ile Val Leu Ala Ile Ala
           115                 120                 125
   Ala Gly Leu Ile Ala Gly Ala Phe Val Ala Ser Ser Gly Thr Met Leu
       130                 135                 140
   Val Phe Ala Asn Pro Met Phe Val Met Gly Leu Ile Thr Val Gly Leu
   145                 150                 155                 160
   Tyr Phe Met Ser Leu Asn Lys Leu Thr Leu Asp Tyr Phe Arg Arg Glu
                   165                 170                 175
   His Leu Leu Arg Met Glu Lys Lys Thr Gln Glu Thr Ala Glu Pro Ile
               180                 185                 190
   Leu Val Thr Pro Ser Ala Asp Asp Ala Lys Lys Ile Ala Val Glu Lys
           195                 200                 205
   Lys Lys Asp Leu Ser Ala Ser Ala Arg Met Glu Glu His Glu Ala Ser
   210 215 220
   Gln Arg Gln Asp Ala Arg His Arg Arg Ile Gly Arg Glu Ala Gln Gly
   225 230 235 240
   Ser Phe Phe Tyr Ser Ser Arg Asn Pro Glu His Arg Arg Ser Phe Gly
   245 250 255
   Ser Leu Ser Arg Phe Lys Thr Lys Pro Ser Asp Ala Ala Ser Thr Arg
   260 265 270
   Pro Ala Ser Ile Ser Pro Pro Phe Lys Asp Asp Phe Gln Pro Tyr His
   275 280 285
   Phe Lys Asp Leu Arg Ser Ser Ser Phe Gly Ser Gly Ala Ser Ser Ala
   290 295 300
   Phe Thr Pro Ile Met Pro Ala Ser Ser Arg Ser Pro Asn Phe Ser Thr
   305 310 315 320
   Gly Thr Val Leu His Pro Glu Pro Val Tyr Pro Lys Gly Gly Lys Glu
   325 330 335
   Pro Ser Ile Pro Arg Val Ser Ser Ser Ser Arg Arg Ser Pro Arg Asp
   340 345 350
   Arg Gln Asp Lys Gln Gln Gln Gln Gln Asn Gln Asp Glu Glu Gln Lys
   355 360 365
   Gln Gln Ser Lys Lys Lys Ser Gly Lys Ser Asn Gln Ser Leu Lys Thr
   370 375 380
   Pro Pro Pro Asp Gly Lys Ser Thr Ala Asn Leu Ser Pro Ser Asn Pro
   385 390 395 400
   Phe Ser Asp Gly Tyr Asp Glu Arg Glu Lys Arg Lys His Arg Lys Asn
   405 410 415
   Lys
<210> 13
   <211> 657
   <212> DNA
   <213> Chlamydia pneumoniae
<400> 13
   atgttaggtt ctttgccatg ttatcctggt gctggcaata ttgaagaata caaaaatagg 60
   tatttctatt gtcagttatg tgctgaggtc gttagtccct atgttgttcc tgttattgta 120
   gttgatgtgc aaggggctcc tcctacaggt atcttgcagg tcttgcgttg taagcaacat 180
   aaatttcaag gcctacccgt acatggcccc attacttctt tatgggcttt ggagcccgtg 240
   ggtaagggag ctccgcagct ggagtctgca atgtacgagc tctgttctca agtaaggaat 300
   tttgacatct gctctattgt gagttgggtc tttggtgggt tgtgtatttt tgcaggtctg 360
   attgtcgggg taatggttga agcccctttg attgcgggat taagtgcttg ggtgattccc 420
   tgtatcattg gaggggttgg tgccatttta tgcttgtttg cgatcttgat ggcgtacttg 480
   ggaagaggga gagtccgtga gtggctcaat ctttcacacg aatatataac gcaatgtcat 540
   tgtcgtcaga tacaggcaca ttctcaaaac tattctgtga tcacagagta tcctgcaacc 600
   tgtgcattat ctcaaccgat tacaaagtta cctaatggat cacgcagaga taactaa 657
<210> 14
   <211> 218
   <212> PRT
   <213> Chlamydia pneumoniae
<400> 14
   Met Leu Gly Ser Leu Pro Cys Tyr Pro Gly Ala Gly Asn Ile Glu Glu
   1               5                   10                  15
   Tyr Lys Asn Arg Tyr Phe Tyr Cys Gln Leu Cys Ala Glu Val Val Ser
               20                  25                  30
   Pro Tyr Val Val Pro Val Ile Val Val Asp Val Gln Gly Ala Pro Pro
           35                  40                  45
   Thr Gly Ile Leu Gln Val Leu Arg Cys Lys Gln His Lys Phe Gln Gly
       50                  55                  60
   Leu Pro Val His Gly Pro Ile Thr Ser Leu Trp Ala Leu Glu Pro Val
   65                  70                  75                  80
   Gly Lys Gly Ala Pro Gln Leu Glu Ser Ala Met Tyr Glu Leu Cys Ser
                   85                  90                  95
   Gln Val Arg Asn Phe Asp Ile Cys Ser Ile Val Ser Trp Val Phe Gly
               100                 105                 110
   Gly Leu Cys Ile Phe Ala Gly Leu Ile Val Gly Val Met Val Glu Ala
           115                 120                 125
   Pro Leu Ile Ala Gly Leu Ser Ala Trp Val Ile Pro Cys Ile Ile Gly
       130                 135                 140
   Gly Val Gly Ala Ile Leu Cys Leu Phe Ala Ile Leu Met Ala Tyr Leu
   145                 150                 155                 160
   Gly Arg Gly Arg Val Arg Glu Trp Leu Asn Leu Ser His Glu Tyr Ile
                   165                 170                 175
   Thr Gln Cys His Cys Arg Gln Ile Gln Ala His Ser Gln Asn Tyr Ser
               180                 185                 190
   Val Ile Thr Glu Tyr Pro Ala Thr Cys Ala Leu Ser Gln Pro Ile Thr
           195                 200                 205
   Lys Leu Pro Asn Gly Ser Arg Arg Asp Asn
       210                 215
<210> 15
   <211> 873
   <212> DNA
   <213> Chlamydia pneumoniae
<400> 15
   atgcagattc caagaagcat tggtactcac gatggttctt tccatgcgga tgaggtcaca      60
   gcgtgtgctc tccttattat tttcgatctt gtggatgaaa ataaaattat acgctctcga     120
   gatcctgtcg tattatcgaa atgtgaatat gtttgtgatg tcggtggtgt ttattctata     180
   gaaaacaagc gttttgatca tcatcaagtc tcttatgatg gatcttggag tagtgcaggt     240
   atgattctgc attatcttaa agagtttggt tatatggatt gtgaagaata tcatttcctt     300
   aacaacactt tggtacatgg tgtggatgaa caagataatg gcagattctt ctctaaggag     360
   ggattttgtt cgttttctga tattattaaa atttataatc ctcgcgagga agaagaaact     420
   aattcggatg cggatttttc ttgtgctttg cattttacca tcgacttttt gtgtcggcta     480
   aggaagaagt ttcagtatga tcgagtttgt agggggattg tcagagaagc catggaaacc     540
   gaggatatgt gtttatattt tgatcgtcct ttagcatggc aagaaaattt ctttttttta      600
   gggggagaga agcaccctgc agcttttgtt tgttttcctt cctgcgatca atggatttta     660
   cgagggattc ctccgaattt agatcgccgt atggacgttc gtgttccttt ccctgagaat     720
   tgggcaggtt tgttaggtaa agagttgtcc aaagtatcag ggattcctgg ggctgtgttc     780
   tgccataaag gtcttttcct ttctgtatgg acaaatagag aaagttgcca acgtgctttg     840
   cggttaacgt tacaagatcg agggatcata tga                                 873
<210> 16
   <211> 290
   <212> PRT
   <213> Chlamydia pneumoniae
<400> 16
   Met Gln Ile Pro Arg Ser Ile Gly Thr His Asp Gly Ser Phe His Ala
   1               5                   10                  15
   Asp Glu Val Thr Ala Cys Ala Leu Leu Ile Ile Phe Asp Leu Val Asp
               20                  25                  30
   Glu Asn Lys Ile Ile Arg Ser Arg Asp Pro Val Val Leu Ser Lys Cys
           35                  40                  45
   Glu Tyr Val Cys Asp Val Gly Gly Val Tyr Ser Ile Glu Asn Lys Arg
   50 55 60
   Phe Asp His His Gln Val Ser Tyr Asp Gly Ser Trp Ser Ser Ala Gly
   65 70 75 80
   Met Ile Leu His Tyr Leu Lys Glu Phe Gly Tyr Met Asp Cys Glu Glu
   85 90 95
   Tyr His Phe Leu Asn Asn Thr Leu Val His Gly Val Asp Glu Gln Asp
   100 105 110
   Asn Gly Arg Phe Phe Ser Lys Glu Gly Phe Cys Ser Phe Ser Asp Ile
   115 120 125
   Ile Lys Ile Tyr Asn Pro Arg Glu Glu Glu Glu Thr Asn Ser Asp Ala
   130 135 140
   Asp Phe Ser Cys Ala Leu His Phe Thr Ile Asp Phe Leu Cys Arg Leu
   145 150 155 160
   Arg Lys Lys Phe Gln Tyr Asp Arg Val Cys Arg Gly Ile Val Arg Glu
   165 170 175
   Ala Met Glu Thr Glu Asp Met Cys Leu Tyr Phe Asp Arg Pro Leu Ala
   180 185 190
   Trp Gln Glu Asn Phe Phe Phe Leu Gly Gly Glu Lys His Pro Ala Ala
   195 200 205
   Phe Val Cys Phe Pro Ser Cys Asp Gln Trp Ile Leu Arg Gly Ile Pro
   210 215 220
   Pro Asn Leu Asp Arg Arg Met Asp Val Arg Val Pro Phe Pro Glu Asn
   225 230 235 240
   Trp Ala Gly Leu Leu Gly Lys Glu Leu Ser Lys Val Ser Gly Ile Pro
   245 250 255
   Gly Ala Val Phe Cys His Lys Gly Leu Phe Leu Ser Val Trp Thr Asn
   260 265 270
   Arg Glu Ser Cys Gln Arg Ala Leu Arg Leu Thr Leu Gln Asp Arg Gly
   275 280 285
   Ile Ile
   290
<210> 17
   <211> 2058
   <212> DNA
   <213> Chlamydia pneumoniae
<400> 17
   atgtataacc tactccacgc gcatcatgat gcagcctccc cagacggacg actcgtttcc      60
   catttgaaaa aactctcgcc ccacatttac gaaggagagg tcctcattga gaatattcct     120
   gcgtactttc ttggatttca tctgcctcaa cagtgtatac aagtaaattt aaaaagttcc     180
   ttagcccaac taggtgtcga agccgtttta aaccacttgg agctaaataa agcccgaaaa     240
   gaagctcgtc tacacgttct cttcatgagc caagatccta tagccactgc tatgttggag     300
   ctcctagagc ctggaagttt tgtctgcaag ctctttgctg ctgatgatcg ccgactcgta     360
   cgttcgcctt gttatctcaa caggatgttt acgcacacag accgtacagg atctccgctc     420
   ctacgctttg ggaaaaaact tgagcacttc atcactctag agatcattaa tgatcggctt     480
   gttgtcttcc ttccgatcct tccaggaaca atctgttacg aagagacaat ttatgggttc     540
   cttcccttaa tgagcaaatc actcacgcgt ccccatttaa aaatacgtaa gtttcttcct     600
   ttgtatcaaa tggtaacaga tcgtcctccc gttcccgaag atcataaaat tcttctcata     660
   aagacagagc ctctgcacat ccgaaccgta tttgcaagag tcgttcagga cttactcccc     720
   caagggcttc gtcacaccgc agcggatatt ctcgaaccta ccacacaaga atctggagat     780
   atttatgaat tctacggcag cacttcagaa cctattgaga gaataccttt agaatttttt     840
   actcttgagc cttacaaaga gcattcgttt ttcttctata gagatatgct ccaggaaacc     900
   ttagaatctc ctcaagaggt atttcgtgtt tttgaatcca taccggaagg cgaagatcaa     960
   gctgcgatgt ttatctccaa aggtagtgag ctgcttgagc tctcccaaga ctcttggatc    1020
   atcaaacctc gaatctcccc atcagatgaa agacatgcta gggaaattca aaagcacatt    1080
   gaagaccaac cttgtttccc ttttttaaaa gccatggaaa cagatcatat cacaagccaa    1140
   ggagttttat tttcccgcta cttcccttca gcatcgctga agggcatgtt cctctctaac    1200
   tactctcgct attacctgca acatatctat tttcagattc cctctcccac ttctggagag    1260
   tttttctcga atcgagatcg ctctttcctt ctcgatctat attttgcagg aatttctgta    1320
   ttttgggcag acttagaatc gaaacgactc ttacaataca tcaaacgcag aaataaagat    1380
   gtgggcatgt ttgtccctaa acatcaagct gaacagtttg ctcaatccta ctttatagga    1440
   attcatggtt cctgcctaat cgctggggat tatgatgagt ttctccgtga gctcctgaca    1500
   ggaatgcata ctctttctca gcaattcacg atcccagaat ttccaccaca gacaccgtta    1560
   gcaatcctta caggaggggg ttctggagct atggaactcg cgaatcgtgt agctacagaa    1620
   ctctccatac tctcttgtgg gaatctaatt agcttggata ccacgaatgc ctatgtagaa    1680
   gctaaaatga gctatgctat tcctgatctt ttagaacgtc aggccgactt ccatgtcgac    1740
   cttgctgtat ttgttatcgg aggcatggga accgatttcg aactccttct ggagcttatt    1800
   agtctcaaaa cagggaaaaa agctcttgtt cccgtcttcc taatcggacc tgtagactat    1860
   tggaaatcca agatcacagc tttgtataat tccaatcatg ctgtaggaac cattcgaggt    1920
   tctgaatggg tacacaactg cctattctgc ctatcctcag caaaggcagg cattgcaatc    1980
   ttccgcagat atctcaatca tacgctgccc ataggacctg aacaccctgt ccctgaagat 2040
   ggttttgtta tcgtttag 2058
<210> 18
   <211> 685
   <212> PRT
   <213> Chlamydia pneumoniae

<400> 18
   Met Tyr Asn Leu Leu His Ala His His Asp Ala Ala Ser Pro Asp Gly
   1               5                   10                  15
   Arg Leu Val Ser His Leu Lys Lys Leu Ser Pro His Ile Tyr Glu Gly
               20                  25                  30
   Glu Val Leu Ile Glu Asn Ile Pro Ala Tyr Phe Leu Gly Phe His Leu
           35                  40                  45
   Pro Gln Gln Cys Ile Gln Val Asn Leu Lys Ser Ser Leu Ala Gln Leu
       50                  55                  60
   Gly Val Glu Ala Val Leu Asn His Leu Glu Leu Asn Lys Ala Arg Lys
   65                  70                  75                  80
   Glu Ala Arg Leu His Val Leu Phe Met Ser Gln Asp Pro Ile Ala Thr
                   85                  90                  95
   Ala Met Leu Glu Leu Leu Glu Pro Gly Ser Phe Val Cys Lys Leu Phe
               100                 105                 110
   Ala Ala Asp Asp Arg Arg Leu Val Arg Ser Pro Cys Tyr Leu Asn Arg
           115                 120                 125
   Met Phe Thr His Thr Asp Arg Thr Gly Ser Pro Leu Leu Arg Phe Gly
       130                 135                 140
   Lys Lys Leu Glu His Phe Ile Thr Leu Glu Ile Ile Asn Asp Arg Leu
   145                 150                 155                 160
   Val Val Phe Leu Pro Ile Leu Pro Gly Thr Ile Cys Tyr Glu Glu Thr
                   165                 170                 175
   Ile Tyr Gly Phe Leu Pro Leu Met Ser Lys Ser Leu Thr Arg Pro His
               180                 185                 190
   Leu Lys Ile Arg Lys Phe Leu Pro Leu Tyr Gln Met Val Thr Asp Arg
           195                 200                 205
   Pro Pro Val Pro Glu Asp His Lys Ile Leu Leu Ile Lys Thr Glu Pro
       210                 215                 220
   Leu His Ile Arg Thr Val Phe Ala Arg Val Val Gln Asp Leu Leu Pro
   225 230 235 240
   Gln Gly Leu Arg His Thr Ala Ala Asp Ile Leu Glu Pro Thr Thr Gln
   245 250 255
   Glu Ser Gly Asp Ile Tyr Glu Phe Tyr Gly Ser Thr Ser Glu Pro Ile
   260 265 270
   Glu Arg Ile Pro Leu Glu Phe Phe Thr Leu Glu Pro Tyr Lys Glu His
   275 280 285
   Ser Phe Phe Phe Tyr Arg Asp Met Leu Gln Glu Thr Leu Glu Ser Pro
   290 295 300
   Gln Glu Val Phe Arg Val Phe Glu Ser Ile Pro Glu Gly Glu Asp Gln
   305 310 315 320
   Ala Ala Met Phe Ile Ser Lys Gly Ser Glu Leu Leu Glu Leu Ser Gln
   325 330 335
   Asp Ser Trp Ile Ile Lys Pro Arg Ile Ser Pro Ser Asp Glu Arg His
   340 345 350
   Ala Arg Glu Ile Gln Lys His Ile Glu Asp Gln Pro Cys Phe Pro Phe
   355 360 365
   Leu Lys Ala Met Glu Thr Asp His Ile Thr Ser Gln Gly Val Leu Phe
   370 375 380
   Ser Arg Tyr Phe Pro Ser Ala Ser Leu Lys Gly Met Phe Leu Ser Asn
   385 390 395 400
   Tyr Ser Arg Tyr Tyr Leu Gln His Ile Tyr Phe Gln Ile Pro Ser Pro
   405 410 415
   Thr Ser Gly Glu Phe Phe Ser Asn Arg Asp Arg Ser Phe Leu Leu Asp
   420 425 430
   Leu Tyr Phe Ala Gly Ile Ser Val Phe Trp Ala Asp Leu Glu Ser Lys
   435 440 445
   Arg Leu Leu Gln Tyr Ile Lys Arg Arg Asn Lys Asp Val Gly Met Phe
   450 455 460
   Val Pro Lys His Gln Ala Glu Gln Phe Ala Gln Ser Tyr Phe Ile Gly
   465 470 475 480
   Ile His Gly Ser Cys Leu Ile Ala Gly Asp Tyr Asp Glu Phe Leu Arg
   485 490 495
   Glu Leu Leu Thr Gly Met His Thr Leu Ser Gln Gln Phe Thr Ile Pro
   500 505 510
   Glu Phe Pro Pro Gln Thr Pro Leu Ala Ile Leu Thr Gly Gly Gly Ser
   515 520 525
   Gly Ala Met Glu Leu Ala Asn Arg Val Ala Thr Glu Leu Ser Ile Leu
   530 535 540
   Ser Cys Gly Asn Leu Ile Ser Leu Asp Thr Thr Asn Ala Tyr Val Glu
   545 550 555 560
   Ala Lys Met Ser Tyr Ala Ile Pro Asp Leu Leu Glu Arg Gln Ala Asp
   565 570 575
   Phe His Val Asp Leu Ala Val Phe Val Ile Gly Gly Met Gly Thr Asp
   580 585 590
   Phe Glu Leu Leu Leu Glu Leu Ile Ser Leu Lys Thr Gly Lys Lys Ala
   595 600 605
   Leu Val Pro Val Phe Leu Ile Gly Pro Val Asp Tyr Trp Lys Ser Lys
   610 615 620
   Ile Thr Ala Leu Tyr Asn Ser Asn His Ala Val Gly Thr Ile Arg Gly
   625 630 635 640
   Ser Glu Trp Val His Asn Cys Leu Phe Cys Leu Ser Ser Ala Lys Ala
   645 650 655
   Gly Ile Ala Ile Phe Arg Arg Tyr Leu Asn His Thr Leu Pro Ile Gly
   660 665 670
   Pro Glu His Pro Val Pro Glu Asp Gly Phe Val Ile Val
   675 680 685
<210> 19
   <211> 273
   <212> DNA
   <213> Chlamydia pneumoniae
<400> 19
   ttggatgatt catggatctt agaggttaaa gtcactccaa aagccaaaga gaacaaaatt      60
   gtaggctttg atggacaagc tttgaaggtc cgtgttaccg aacccccaga aaagggtaag     120
   gccaatgatg ctgtaatttc tttattagca aaagctttat ccttaccgaa gcgtgatgtc     180
   actttaattg caggagaaac ttctcgaaag aaaaagtttc ttcttcctaa cagagttcaa     240
   gacattattt tttctttgca tatagacgta tag                                  273
<210> 20
   <211> 90
   <212> PRT
   <213> Chlamydia pneumoniae
<400> 20
   Leu Asp Asp Ser Trp Ile Leu Glu Val Lys Val Thr Pro Lys Ala Lys
   1               5                   10                  15
   Glu Asn Lys Ile Val Gly Phe Asp Gly Gln Ala Leu Lys Val Arg Val
               20                  25                  30
   Thr Glu Pro Pro Glu Lys Gly Lys Ala Asn Asp Ala Val Ile Ser Leu
           35                  40                  45
   Leu Ala Lys Ala Leu Ser Leu Pro Lys Arg Asp Val Thr Leu Ile Ala
       50                  55                  60
   Gly Glu Thr Ser Arg Lys Lys Lys Phe Leu Leu Pro Asn Arg Val Gln
   65                  70                  75                  80
   Asp Ile Ile Phe Ser Leu His Ile Asp Val
                   85                  90
<210> 21
   <211> 672
   <212> DNA
   <213> Chlamydia pneumoniae
<400> 21
   atgaccctct acttaggatt gaatcaaaaa accgctcgta aataccaagc tcattatttg      60
   cctattctaa ctctcttccc ctatgcaaaa agcactccac aaaataagcg tgctcttcaa     120
   ttccttccac aagcaaccca tgtgattctc acaagtccct catccactca cctattcctt     180
   tccagaatga cttctcttct ttctaaggcc actctaaaaa caaagaccta cctctgtata     240
   ggagagtcca ccaaagaaag acttctctct ttccttggac aagtgaagta cgtagtagca     300
   actcaagaaa tcgctgaagg catcttccca ttgctacagg cactgccctc ttcagcccgc     360
   attctctacc cccactcctc cctcgcaaga cctgtgatca gagaatttct ttacaatcga     420
   tttacttttt tctcttaccc tcactacaca gtgaagccgc gaaaacttaa aaaaaatatt     480
   ttatctaaat acaaaaaaat tatcttcaca agcccttcaa ctgtaagagc tttcgccaaa     540
   atctttccgc gatttcctga aaaaacctac tggtgccaag gaaggatgac cttgcaggag     600
   tttcaaaagt tctcctctca aaagcaggta tctttgttag aaacgcttgg gaagtccagg     660
   acatctccgt ga                                                         672
<210> 22
   <211> 94
   <212> PRT
   <213> Chlamydia pneumoniae
<400> 22
   Met Thr Leu Tyr Leu Gly Leu Asn Gln Lys Thr Ala Arg Lys Tyr Gln
   1               5                   10                  15
   Ala His Tyr Leu Pro Ile Leu Thr Leu Phe Pro Tyr Ala Lys Ser Leu
               20                  25                  30
   Lys Val Arg Val Thr Glu Pro Pro Glu Lys Gly Lys Ala Asn Asp Ala
           35                  40                  45
   Val Ile Ser Leu Leu Ala Lys Ala Leu Ser Leu Pro Lys Arg Asp Val
       50                  55                  60
   Thr Leu Ile Ala Gly Glu Thr Ser Arg Lys Lys Lys Phe Leu Leu Pro
   65                  70                  75                  80
   Asn Arg Val Gln Asp Ile Ile Phe Ser Leu His Ile Asp Val
                   85                  90
<210> 23
   <211> 570
   <212> DNA
   <213> Chlamydia pneumoniae
<400> 23
   atgtcatcaa atctacatcc cgtaggagga acaggaacag gagcagctgc tcctgagtct      60
   gtgctaaaca tagtagagga aatagcagca tcggggagtg tcaccgctgg tctacaagca     120
   attacgtcca gtccaggaat ggtgaatcta ctcataggat gggcaaagac aaaatttatt     180
   caacctatac gtgaatcaaa gctctttcaa tccagagctt gccaaattac cctgctcgtt     240
   ttaggaattc ttttggttgt tgctggatta gcatgtatgt ttatcttcca tagccagtta     300
   ggggcaaatg cattttggtt gattattcct gctgccatag gattgattaa gttactagtt     360
   acatcattat gttttgatga agcttgtaca tctgaaaaac tcatggtttt ccaaaaatgg     420
   gcaggtgttt tagaagatca gctcgatgat gggatcctta ataactcaaa taagattttt     480
   ggccatgtga aaacagaagg aaatacctct agggctacta ccccagtact taatgatggc     540
   cgcggaactc ctgtactttc acctttagta                                      570
<210> 24
   <211> 190
   <212> PRT
   <213> Chlamydia pneumoniae
<400> 24
   Met Ser Ser Asn Leu His Pro Val Gly Gly Thr Gly Thr Gly Ala Ala
   1               5                   10                  15
   Ala Pro Glu Ser Val Leu Asn Ile Val Glu Glu Ile Ala Ala Ser Gly
               20                  25                  30
   Ser Val Thr Ala Gly Leu Gln Ala Ile Thr Ser Ser Pro Gly Met Val
           35                  40                  45
   Asn Leu Leu Ile Gly Trp Ala Lys Thr Lys Phe Ile Gln Pro Ile Arg
       50                  55                  60
   Glu Ser Lys Leu Phe Gln Ser Arg Ala Cys Gln Ile Thr Leu Leu Val
   65                  70                  75                  80
   Leu Gly Ile Leu Leu Val Val Ala Gly Leu Ala Cys Met Phe Ile Phe
                   85                  90                  95
   His Ser Gln Leu Gly Ala Asn Ala Phe Trp Leu Ile Ile Pro Ala Ala
               100                 105                 110
   Ile Gly Leu Ile Lys Leu Leu Val Thr Ser Leu Cys Phe Asp Glu Ala
           115                 120                 125
   Cys Thr Ser Glu Lys Leu Met Val Phe Gln Lys Trp Ala Gly Val Leu
       130                 135                 140
   Glu Asp Gln Leu Asp Asp Gly Ile Leu Asn Asn Ser Asn Lys Ile Phe
   145                 150                 155                 160
   Gly His Val Lys Thr Glu Gly Asn Thr Ser Arg Ala Thr Thr Pro Val
                   165                 170                 175
   Leu Asn Asp Gly Arg Gly Thr Pro Val Leu Ser Pro Leu Val
               180                 185                 190
<210> 25
   <211> 828
   <212> DNA
   <213> Chlamydia pneumoniae
<400> 25
   atgcacgata aaaacaaggt tctgtatcta caagcaaacc atctaaatca aaaaagaaaa      60
   cgtcataatc ctctaaatac ataccactcc tcaaacacaa ctgaaactcg tcgcttacca     120
   acatactata aatccaacat tgtcttaaaa atgattttac ggatctccac cgtaagcctt     180
   cttacaagtt gctccttctc gaaaaattct cgtacctgtt tcgtcactcc agaacgcatt     240
   acctcacaaa aagactgccc cgtccttctc catccaaaaa gcactacgat ttctccccct     300
   ctctatgact ggatctcccc aaatagagag gtaatcaccg cctattcttt ctactgccga     360
   ggtcaaggaa actctatcat aactcccgaa ggggttctct atgattgtga tggactccat     420
   cacagcataa ctaaagaaga gttccgttat atccatccta gattgattga ggtagtacga     480
   ctcttgcaac aagatcaccc taaagtctct attattgaag ccttttgttg tcctaaacac     540
   tttcattttt tagaagcctc aggaatctca ctctctcaac tccatctcca aggtactgca     600
   gctaccttcg ctctagatcc tcccctcccc atggagaaac tcttggcaac tataaagaaa     660
   ctgtataaaa aaaactccga tccttctctc tctaatttta tcgttacaga agctacactg     720
   accaatccag aactgcgact cacgcaacaa gatctcggct cgcatacaga aattactgta     780
   gaaattctcg ataatctaca aaacaaagag gctctttcct ccgcataa                  828
<210> 26
   <211> 142
   <212> PRT
   <213> Chlamydia pneumoniae
<400> 26
   Met His Asp Lys Asn Lys Val Leu Tyr Leu Gln Ala Asn His Leu Asn
   1               5                   10                  15
   Gln Lys Arg Lys Arg His Asn Pro Leu Asn Thr Tyr His Ser Ser Asn
               20                  25                  30
   Thr Thr Glu Thr Arg Arg Leu Pro Thr Tyr Tyr Lys Ser Asn Ile Val
           35                  40                  45
   Leu Lys Met Ile Leu Arg Ile Ser Thr Val Ser Leu Leu Thr Ser Cys
       50                  55                  60
   Ser Phe Ser Lys Asn Ser Arg Thr Cys Phe Val Thr Pro Glu Arg Leu
   65                  70                  75                  80
   Lys Val Arg Val Thr Glu Pro Pro Glu Lys Gly Lys Ala Asn Asp Ala
                   85                  90                  95
   Val Ile Ser Leu Leu Ala Lys Ala Leu Ser Leu Pro Lys Arg Asp Val
               100                 105                 110
   Thr Leu Ile Ala Gly Glu Thr Ser Arg Lys Lys Lys Phe Leu Leu Pro
           115                 120                 125
   Asn Arg Val Gln Asp Ile Ile Phe Ser Leu His Ile Asp Val
       130                 135                 140
<210> 27
   <211> 546
   <212> DNA
   <213> Chlamydia pneumoniae
<400> 27
   atgtccttat tgaaccttcc ctcaagccag gattctgcat ctgaggactc cacatcgcaa     60
   tctcaaatct tcgatcccat tagaaatcgg gagttagttt ctactcccga agaaaaagtc    120
   cgccaaaggt tgctctcctt cctaatgcat aagctgaact accctaagaa actcatcatc    180
   atagaaaaag aactcaaaac tctttttcct ctgcttatgc gtaaaggaac cctaatccca    240
   aaacgccgcc cagatattct catcatcact ccccccacat acacagacgc acagggaaac    300
   actcacaacc taggcgaccc aaaacccctg ctacttatcg aatgtaaggc cttagccgta    360
   aaccaaaatg cactcaaaca actccttagc tataactact ctatcggagc cacctgcatt    420
   gctatggcag ggaaacactc tcaagtgtca gctctcttca atccaaaaac acaaactctt    480
   gatttttatc ctggcctccc agagtattcc caactcctaa actactttat ttctttaaac    540
   ttatag                                                               546
<210> 28
   <211> 106
   <212> PRT
   <213> Chlaniydia pneumoniae
<400> 28
   Met Ser Leu Leu Asn Leu Pro Ser Ser Gln Asp Ser Ala Ser Glu Asp
   1               5                   10                  15
   Ser Thr Ser Gln Ser Gln Ile Phe Asp Pro Ile Arg Asn Arg Glu Leu
               20                  25                  30
   Val Ser Thr Pro Glu Glu Lys Val Arg Gln Arg Leu Lys Val Arg Val
           35                  40                  45
   Thr Glu Pro Pro Glu Lys Gly Lys Ala Asn Asp Ala Val Ile Ser Leu
       50                  55                  60
   Leu Ala Lys Ala Leu Ser Leu Pro Lys Arg Asp Val Thr Leu Ile Ala
   65                  70                  75                  80
   Gly Glu Thr Ser Arg Lys Lys Lys Phe Leu Leu Pro Asn Arg Val Gln
                   85                  90                  95
   Asp Ile Ile Phe Ser Leu His Ile Asp Val
               100                 105
<210> 29
   <211> 972
   <212> DNA
   <213> Chlamydia pneumoniae
<400> 29
   atgaaacaat tacttttctg tgtttgcgta tttgctatgt catgttctgc ttacgcatcc      60
   ccacgacgac aagatccttc tgttatgaag gaaacattcc gaaataatta tggcattatt     120
   gtttccggtc aagaatgggt aaagcgtggt tctgacggca ccatcaccaa agtactcaaa     180
   aatggagcta ccctgcatga agtttattct ggaggcctcc ttcatgggga aattacctta     240
   acgtttcccc ataccacagc attggacgtt gttcaaatct atgatcaagg tagactcgtt     300
   tctcgcaaaa ccttttttgt gaacggtctt ccatctcaag aagagctgtt caatgaagat     360
   ggcacgtttg tcctcacacg atggccggac aacaacgaca gtgataccat cacaaagcct     420
   tacttcatag aaacgacata tcaagggcat gtcatagaag gaagttatac ttcctttaat     480
   gggaaatact cctcatccat ccacaatgga gagggagttc gttctgtgtt ctcctccaat     540
   aacatccttc tttctgaaga gaccttcaat gaaggtgtca tggtgaaata taccacattc     600
   tatccgaatc gcgatcccga atcgattact cattatcaaa atggacagcc tcacggctta     660
   cggctaacat atctacaagg tggcatcccc aatacgatag aggagtggcg ttatggcttt    720
   caagacggaa cgaccatcgt atttaaaaat ggttgtaaga catctgagat cgcttatgtt    780
   aagggagtga aagaaggttt agaactgcgc tacaatgaac aggaaattgt agctgaagaa    840
   gtttcttggc gtaatgattt tctgcatgga gaacgtaaga tctatgctgg aggaatccaa    900
   aagcatgaat ggtattaccg cgggagatct gtatctaaag ccaaattcga gcggctaaat    960
   gctgcaggat ag                                                        972
<210> 30
   <211> 323
   <212> PRT
   <213> Chlamydia pneumoniae
<400> 30
   Met Lys Gln Leu Leu Phe Cys Val Cys Val Phe Ala Met Ser Cys Ser
   1               5                   10                  15
   Ala Tyr Ala Ser Pro Arg Arg Gln Asp Pro Ser Val Met Lys Glu Thr
               20                  25                  30
   Phe Arg Asn Asn Tyr Gly Ile Ile Val Ser Gly Gln Glu Trp Val Lys
           35                  40                  45
   Arg Gly Ser Asp Gly Thr Ile Thr Lys Val Leu Lys Asn Gly Ala Thr
       50                  55                  60
   Leu His Glu Val Tyr Ser Gly Gly Leu Leu His Gly Glu Ile Thr Leu
   65                  70                  75                  80
   Thr Phe Pro His Thr Thr Ala Leu Asp Val Val Gln Ile Tyr Asp Gln
                   85                  90                  95
   Gly Arg Leu Val Ser Arg Lys Thr Phe Phe Val Asn Gly Leu Pro Ser
               100                 105                 110
   Gln Glu Glu Leu Phe Asn Glu Asp Gly Thr Phe Val Leu Thr Arg Trp
           115                 120                 125
   Pro Asp Asn Asn Asp Ser Asp Thr Ile Thr Lys Pro Tyr Phe Ile Glu
       130                 135                 140
   Thr Thr Tyr Gln Gly His Val Ile Glu Gly Ser Tyr Thr Ser Phe Asn
   145                 150                 155                 160
   Gly Lys Tyr Ser Ser Ser Ile His Asn Gly Glu Gly Val Arg Ser Val
                   165                 170                 175
   Phe Ser Ser Asn Asn Ile Leu Leu Ser Glu Glu Thr Phe Asn Glu Gly
               180                 185                 190
   Val Met Val Lys Tyr Thr Thr Phe Tyr Pro Asn Arg Asp Pro Glu Ser
           195                 200                 205
   Ile Thr His Tyr Gln Asn Gly Gln Pro His Gly Leu Arg Leu Thr Tyr
       210                 215                 220
   Leu Gln Gly Gly Ile Pro Asn Thr Ile Glu Glu Trp Arg Tyr Gly Phe
   225                 230                 235                 240
   Gln Asp Gly Thr Thr Ile Val Phe Lys Asn Gly Cys Lys Thr Ser Glu
                   245                 250                 255
   Ile Ala Tyr Val Lys Gly Val Lys Glu Gly Leu Glu Leu Arg Tyr Asn
               260                 265                 270
   Glu Gln Glu Ile Val Ala Glu Glu Val Ser Trp Arg Asn Asp Phe Leu
           275                 280                 285
   His Gly Glu Arg Lys Ile Tyr Ala Gly Gly Ile Gln Lys His Glu Trp
       290                 295                 300
   Tyr Tyr Arg Gly Arg Ser Val Ser Lys Ala Lys Phe Glu Arg Leu Asn
   305                 310                 315                 320
   Ala Ala Gly
<210> 31
   <211> 429
   <212> DNA
   <213> Chlamydia pneumoniae
<400> 31
   atgagtttag attttttcga ggagttctat catcagtcaa tactcaatac agggacgtcc      60
   ttccccgaag gatacttaaa tattgcagaa atactctctt atcctcattg cactgatgct     120
   aacactgact ttctctgtag ccagtctgac aacgatttta ttattgcaga atctaaagat     180
   aaactcacat tatttaacgc tgattttgct atttggctcg ttcctgagct tgttcaagga     240
   caggcagtca ctcggggata tattgcggtt tcccaaggag aaggaaacta tgaaccagaa     300
   atggctttcg aagcctctgg acaatacaat cagtcgtcgc tgattctcga agccctgcag     360
   ttatatctta aggatattaa agatactgaa aatgctctgc gttctttccg ctttaataac     420
   gatcactag                                                             429
<210> 32
   <211> 142
   <212> PRT
   <213> Chlamydia pneumoniae
<400> 32
   Met Ser Leu Asp Phe Phe Glu Glu Phe Tyr His Gln Ser Ile Leu Asn
   1 5 10 15
   Thr Gly Thr Ser Phe Pro Glu Gly Tyr Leu Asn Ile Ala Glu Ile Leu
   20 25 30
   Ser Tyr Pro His Cys Thr Asp Ala Asn Thr Asp Phe Leu Cys Ser Gln
   35 40 45
   Ser Asp Asn Asp Phe Ile Ile Ala Glu Ser Lys Asp Lys Leu Thr Leu
   50 55 60
   Phe Asn Ala Asp Phe Ala Ile Trp Leu Val Pro Glu Leu Val Gln Gly
   65 70 75 80
   Gln Ala Val Thr Arg Gly Tyr Ile Ala Val Ser Gln Gly Glu Gly Asn
   85 90 95
   Tyr Glu Pro Glu Met Ala Phe Glu Ala Ser Gly Gln Tyr Asn Gln Ser
   100 105 110
   Ser Leu Ile Leu Glu Ala Leu Gln Leu Tyr Leu Lys Asp Ile Lys Asp
   115 120 125
   Thr Glu Asn Ala Leu Arg Ser Phe Arg Phe Asn Asn Asp His
   130 135 140
<210> 33
   <211> 180
   <212> DNA
   <213> Chlamydia pneumoniae
<400> 33
   gtgtctattg ccttaaaccg agaagaagtt tgggataatc cccatcactt aatgtttatc 60
   ttaatgcaat tccaacaatt ttcaggggaa caggatcgtt ttggaagttt cttagaagca 120
   accatccgtg atcgggtctc ttttttagtc ttacaagaaa agattgccac tttaaagtag 180
<210> 34
   <211> 59
   <212> PRT
   <213> Chlamydia pneumoniae
<400> 34
   Val Ser Ile Ala Leu Asn Arg Glu Glu Val Trp Asp Asn Pro His His
   1               5                   10                  15
   Leu Met Phe Ile Leu Met Gln Phe Gln Gln Phe Ser Gly Glu Gln Asp
               20                  25                  30
   Arg Phe Gly Ser Phe Leu Glu Ala Thr Ile Arg Asp Arg Val Ser Phe
           35                  40                  45
   Leu Val Leu Gln Glu Lys Ile Ala Thr Leu Lys
                   50                  55
<210> 35
   <211> 675
   <212> DNA
   <213> Chlamydia pneumoniae
<400> 35
   atgcaaaccc ttgctcgtct atttggccaa tctccatttg ctcctttaca agctcatctg      60
   gaaatggtgg tctcttgtgt ggaatacatg cttcctatat tcactgctct ccgagatgga     120
   agatatgaag aattattaga aatggcaaaa cttgtttctg ataaagagta tcaagcagat     180
   tgtataaaaa atgatatgag gaatcatctt cctgcaggat tattcatgcc gatatctcga     240
   gcggggattc tagaaattat ttctatacaa gatagcatcg cggatactgc tgaagatgtt     300
   gctatcttat taaccatcag acgattaaac ttttatccat ctatggaaac gctttttttc     360
   cgatttttgg aaaaaaatct agaagctttt gagttaacta tgacattgct acatgaattc     420
   aaccaattgc ttgaaagttc atttgggggg aggaaggcag ataaagcacg cttgcttgta     480
   gggcgtgtgg ctaaatctga acatgaatcg gatgttttgc aacgagaact tatgcaaata     540
   tttttttctg atgattttat aattcctgaa aaagagtttt atctttggtt acaagtaatt     600
   cgacgcactg cggggatttc agatagttct gaaaagctcg cacatagaat taatatgacc     660
   ctagaagaaa agtaa                                                      675
<210> 36
   <211> 224
   <212> PRT
   <213> Chlamydial pneumoniae
<400> 36
   Met Gln Thr Leu Ala Arg Leu Phe Gly Gln Ser Pro Phe Ala Pro Leu
   1               5                   10                  15
   Gln Ala His Leu Glu Met Val Val Ser Cys Val Glu Tyr Met Leu Pro
               20                  25                  30
   Ile Phe Thr Ala Leu Arg Asp Gly Arg Tyr Glu Glu Leu Leu Glu Met
           35                  40                  45
   Ala Lys Leu Val Ser Asp Lys Glu Tyr Gln Ala Asp Cys Ile Lys Asn
       50                  55                  60
   Asp Met Arg Asn His Leu Pro Ala Gly Leu Phe Met Pro Ile Ser Arg
   65                  70                  75                  80
   Ala Gly Ile Leu Glu Ile Ile Ser Ile Gln Asp Ser Ile Ala Asp Thr
                   85                  90                  95
   Ala Glu Asp Val Ala Ile Leu Leu Thr Ile Arg Arg Leu Asn Phe Tyr
               100                 105                 110
   Pro Ser Met Glu Thr Leu Phe Phe Arg Phe Leu Glu Lys Asn Leu Glu
           115                 120                 125
   Ala Phe Glu Leu Thr Met Thr Leu Leu His Glu Phe Asn Gln Leu Leu
       130                 135                 140
   Glu Ser Ser Phe Gly Gly Arg Lys Ala Asp Lys Ala Arg Leu Leu Val
   145                 150                 155                 160
   Gly Arg Val Ala Lys Ser Glu His Glu Ser Asp Val Leu Gln Arg Glu
                   165                 170                 175
   Leu Met Gln Ile Phe Phe Ser Asp Asp Phe Ile Ile Pro Glu Lys Glu
               180                 185                 190
   Phe Tyr Leu Trp Leu Gln Val Ile Arg Arg Thr Ala Gly Ile Ser Asp
           195                 200                 205
   Ser Ser Glu Lys Leu Ala His Arg Ile Asn Met Thr Leu Glu Glu Lys
       210                 215                 220
<210> 37
   <211> 2538
   <212> DNA
   <213> Chlamydia pneumoniae
<400> 37
   atggcagtac gattaattgt tgatgaaggc cccttgtctg gtgtaatttt tgttctggaa 60
   gatgggataa gctggtctat aggacgcgac tctagtgcta atgacattcc tattgaagat 120
   cctaaactcg gtgcatcgca agccattatc aataagactg acggaagcta ctacatcaca 180
   aatttagatg atacaattcc tattgttgta aatggcgtag cgatccaaga aactacacag 240
   ttaaaaaatg aagatactat cttattagga agcaatcagt attctttctt atcagatgaa 300
   tttgatcctc aagatcttgt ttatgatttt gatattcccg aagaaaattt ttctaatgat 360
   tcaggggatt tgtccgatag taatgaacag ggaaaagatc ttgagcctcg gcaaacttcg 420
   gaaacaaatc attcaccgaa gcctaaggaa aagctgacca aagatcaggg aagtagcgat 480
   ccaattacaa gtggggatca ggagcttgct gatgcttttt tagcatcagc aaaagcggaa 540
   aaaaatcaac caagagccaa agttgctaag aagggtttaa aagaatcttc aaacgagtct 600
   ttgaatccaa aggaacaaaa tgcaaaggat tctccgaaag gagaggaaag aaccaacaaa 660
   ccccagaacg ccattatgga agataacgga gcttcgccta ggcaagatcc gcaaccaaag 720
   tcagcagaac cctctcttaa aaacacagcc agggatgaga ctcccttgaa agaaaataaa 780
   cctgtagaag agaaggctaa taagaaagca acaccggatt ctccagaaaa aaaagatcaa 840
   cctgaggaag gttctaaaaa ggaaggctct aaaatagaag caacaccttt ggattcacaa 900
   aaagaatccg aggataagga agcagaagaa gcctttgtac aagaagaaga agagaacctt 960
   acggaagata ataaagaaga ttctgacagt gccgctgatg caaatgacga cacggcaagt 1020
   gaccatactg cagaggataa caaagaaact cctaaaaaag tcgagaacga aaagagcgca 1080
   gttctatccc catttcatgt tcaagactta tttcgattcg atcaaacaat ttttccagca 1140
   gagattgatg atattgcgaa aaaaaatatc tctgtagact tgacgcagcc ttctcgtttt 1200
   ttactcaaag ttttagccgg agctaatatt ggagcagagt tccatttaga ctcaggaaaa 1260
   acctatattt taggtacgga tcctacaact tgtgacatag tatttaatga cttaagtgtt 1320
   tctcatcaac atgctaaaat tactgtcggt aatgacgggg gcattcttat cgaggatctc 1380
   gatagtaaaa acggtgtcat tgttgaagga cgaaaaattg ataagacctc tacattgagc 1440
   tcgaatcaag ttgtggcttt aggaacgaca ttatttttac ttatagatca tcatgccccc 1500
   gctgatacta tagttgcttc tctatcccca gacgattaca gtttgtttgg gagacagcaa 1560
   gacgccgaag ccttagaaag acaagaggcc caagaagaag aagaaaaaca aaaacgcgct 1620
   acactacccg caggatcttt cattcttacc ctgtttgttg gaggattggc tattctcttt 1680
   ggtataggaa cagcttctct tttccatacc aaagaagtgg ttcctttaga aaatattgat 1740
   tatcaagaag atcttgccca ggttatcaat cagttcccta cggtgcgtta tacgtttaat 1800
   aaaacgaaca gccaactttt cttaatcgga catgtcaaaa atagtacgga caaaagcgag 1860
   ctgctgtata aagtagacgc cctttccttt gtgaaatccg tagatgataa tgttattgat 1920
   gatgaagctg tttggcagga gatgaacatc ctgttatcaa agcgacccga gtttaaaggc 1980
   atcagcatgc attccccaga acctgggaaa ttcatcatca caggctatgt caagactgag 2040
   gagcaagcag cttgcctcgt tgattattta aatatacatt ttaattacct ctcgttacta 2100
   gagaataaag ttgttgttga aacccagatg ttaaaagcaa ttgcaggcca tcttcttcaa 2160
   ggaggttttg caaacatcca tgtggccttt gtgaacggtg aagttatcct tactggttac 2220
   gtcaataacg atgatgcaga gaagttccgt gctgtagtgc aagagctgtc ggggattcct 2280
   ggtgtgaggt tggtcaagaa ttttgctgtc ttactcccag ctgaagaggg aatcatagat 2340
   ttaaacctac gttaccccaa tcgctatcgt gttacaggct attctagata cggagaaata 2400
   agtatcaatg tagttgtcaa tggcagaatc ctcacaagag gggacgtgat tgatggtatg 2460
   acagtaacaa gtatacaacc taacgcgatc tttttagaga aggaagggtt gaaatataaa 2520
   atagactaca ataaataa 2538
<210> 38
   <211> 845
   <212> PRT
   <213> Chlamydia pneumoniae
<400> 38
   Met Ala Val Arg Leu Ile Val Asp Glu Gly Pro Leu Ser Gly Val Ile
   1               5                   10                  15
   Phe Val Leu Glu Asp Gly Ile Ser Trp Ser Ile Gly Arg Asp Ser Ser
               20                  25                  30
   Ala Asn Asp Ile Pro Ile Glu Asp Pro Lys Leu Gly Ala Ser Gln Ala
   35 40 45
   Ile Ile Asn Lys Thr Asp Gly Ser Tyr Tyr Ile Thr Asn Leu Asp Asp
   50 55 60
   Thr Ile Pro Ile Val Val Asn Gly Val Ala Ile Gln Glu Thr Thr Gln
   65 70 75 80
   Leu Lys Asn Glu Asp Thr Ile Leu Leu Gly Ser Asn Gln Tyr Ser Phe
   85 90 95
   Leu Ser Asp Glu Phe Asp Pro Gln Asp Leu Val Tyr Asp Phe Asp Ile
   100 105 110
   Pro Glu Glu Asn Phe Ser Asn Asp Ser Gly Asp Leu Ser Asp Ser Asn
   115 120 125
   Glu Gln Gly Lys Asp Leu Glu Pro Arg Gln Thr Ser Glu Thr Asn His
   130 135 140
   Ser Pro Lys Pro Lys Glu Lys Leu Thr Lys Asp Gln Gly Ser Ser Asp
   145 150 155 160
   Pro Ile Thr Ser Gly Asp Gln Glu Leu Ala Asp Ala Phe Leu Ala Ser
   165 170 175
   Ala Lys Ala Glu Lys Asn Gln Pro Arg Ala Lys Val Ala Lys Lys Gly
   180 185 190
   Leu Lys Glu Ser Ser Asn Glu Ser Leu Asn Pro Lys Glu Gln Asn Ala
   195 200 205
   Lys Asp Ser Pro Lys Gly Glu Glu Arg Thr Asn Lys Pro Gln Asn Ala
   210 215 220
   Ile Met Glu Asp Asn Gly Ala Ser Pro Arg Gln Asp Pro Gln Pro Lys
   225 230 235 240
   Ser Ala Glu Pro Ser Leu Lys Asn Thr Ala Arg Asp Glu Thr Pro Leu
   245 250 255
   Lys Glu Asn Lys Pro Val Glu Glu Lys Ala Asn Lys Lys Ala Thr Pro
   260 265 270
   Asp Ser Pro Glu Lys Lys Asp Gln Pro Glu Glu Gly Ser Lys Lys Glu
   275 280 285
   Gly Ser Lys Ile Glu Ala Thr Pro Leu Asp Ser Gln Lys Glu Ser Glu
   290 295 300
   Asp Lys Glu Ala Glu Glu Ala Phe Val Gln Glu Glu Glu Glu Asn Leu
   305                 310                 315                 320
   Thr Glu Asp Asn Lys Glu Asp Ser Asp Ser Ala Ala Asp Ala Asn Asp
                   325                 330                 335
   Asp Thr Ala Ser Asp His Thr Ala Glu Asp Asn Lys Glu Thr Pro Lys
               340                 345                 350
   Lys Val Glu Asn Glu Lys Ser Ala Val Leu Ser Pro Phe His Val Gln
           355                 360                 365
   Asp Leu Phe Arg Phe Asp Gln Thr Ile Phe Pro Ala Glu Ile Asp Asp
       370                 375                 380
   Ile Ala Lys Lys Asn Ile Ser Val Asp Leu Thr Gln Pro Ser Arg Phe
   385                 390                 395                 400
   Leu Leu Lys Val Leu Ala Gly Ala Asn Ile Gly Ala Glu Phe His Leu
                   405                 410                 415
   Asp Ser Gly Lys Thr Tyr Ile Leu Gly Thr Asp Pro Thr Thr Cys Asp
               420                 425                 430
   Ile Val Phe Asn Asp Leu Ser Val Ser His Gln His Ala Lys Ile Thr
           435                 440                 445
   Val Gly Asn Asp Gly Gly Ile Leu Ile Glu Asp Leu Asp Ser Lys Asn
       450                 455                 460
   Gly Val Ile Val Glu Gly Arg Lys Ile Asp Lys Thr Ser Thr Leu Ser
   465                 470                 475                 480
   Ser Asn Gln Val Val Ala Leu Gly Thr Thr Leu Phe Leu Leu Ile Asp
                   485                 490                 495
   His His Ala Pro Ala Asp Thr Ile Val Ala Ser Leu Ser Pro Asp Asp
               500                 505                 510
   Tyr Ser Leu Phe Gly Arg Gln Gln Asp Ala Glu Ala Leu Glu Arg Gln
           515                 520                 525
   Glu Ala Gln Glu Glu Glu Glu Lys Gln Lys Arg Ala Thr Leu Pro Ala
       530                 535                 540
   Gly Ser Phe Ile Leu Thr Leu Phe Val Gly Gly Leu Ala Ile Leu Phe
   545                 550                 555                 560
   Gly Ile Gly Thr Ala Ser Leu Phe His Thr Lys Glu Val Val Pro Leu
                   565                 570                 575
   Glu Asn Ile Asp Tyr Gln Glu Asp Leu Ala Gln Val Ile Asn Gln Phe
               580                 585                 590
   Pro Thr Val Arg Tyr Thr Phe Asn Lys Thr Asn Ser Gln Leu Phe Leu
           595                 600                 605
   Ile Gly His Val Lys Asn Ser Thr Asp Lys Ser Glu Leu Leu Tyr Lys
       610                 615                 620
   Val Asp Ala Leu Ser Phe Val Lys Ser Val Asp Asp Asn Val Ile Asp
   625                 630                 635                 640
   Asp Glu Ala Val Trp Gln Glu Met Asn Ile Leu Leu Ser Lys Arg Pro
                   645                 650                 655
   Glu Phe Lys Gly Ile Ser Met His Ser Pro Glu Pro Gly Lys Phe Ile
               660                 665                 670
   Ile Thr Gly Tyr Val Lys Thr Glu Glu Gln Ala Ala Cys Leu Val Asp
           675                 680                 685
   Tyr Leu Asn Ile His Phe Asn Tyr Leu Ser Leu Leu Glu Asn Lys Val
       690                 695                 700
   Val Val Glu Thr Gln Met Leu Lys Ala Ile Ala Gly His Leu Leu Gln
   705                 710                 715                 720
   Gly Gly Phe Ala Asn Ile His Val Ala Phe Val Asn Gly Glu Val Ile
                   725                 730                 735
   Leu Thr Gly Tyr Val Asn Asn Asp Asp Ala Glu Lys Phe Arg Ala Val
               740                 745                 750
   Val Gln Glu Leu Ser Gly Ile Pro Gly Val Arg Leu Val Lys Asn Phe
           755                 760                 765
   Ala Val Leu Leu Pro Ala Glu Glu Gly Ile Ile Asp Leu Asn Leu Arg
       770                 775                 780
   Tyr Pro Asn Arg Tyr Arg Val Thr Gly Tyr Ser Arg Tyr Gly Glu Ile
   785                 790                 795                 800
   Ser Ile Asn Val Val Val Asn Gly Arg Ile Leu Thr Arg Gly Asp Val
                   805                 810                 815
   Ile Asp Gly Met Thr Val Thr Ser Ile Gln Pro Asn Ala Ile Phe Leu
               820                 825                 830
   Glu Lys Glu Gly Leu Lys Tyr Lys Ile Asp Tyr Asn Lys
           835                 840                 845
<210> 39
   <211> 237
   <212> DNA
   <213> Chlamydia pneumoniae
<400> 39
   atgaaagaat ttttagccta tatcattaag aatctagtgg accgccctga agaagtccgt     60
   attaaagaag ttcaggggac tcacacgatt atttatgaac taagtgtagc taaacctgat    120
   atcgggaaga tcattggcaa agaaggccgt acgatcaaag cgattcgtac tcttctggtt    180
   tctgtagcaa gcaggaacaa tgtaagggtc agtttagaaa ttatggaaga aaagtag       237
<210> 40
   <211> 78
   <212> PRT
   <213> Chlamydia pneumoniae
<400> 40
   Met Lys Glu Phe Leu Ala Tyr Ile Ile Lys Asn Leu Val Asp Arg Pro
   1               5                   10                  15
   Glu Glu Val Arg Ile Lys Glu Val Gln Gly Thr His Thr Ile Ile Tyr
               20                  25                  30
   Glu Leu Ser Val Ala Lys Pro Asp Ile Gly Lys Ile Ile Gly Lys Glu
           35                  40                  45
   Gly Arg Thr Ile Lys Ala Ile Arg Thr Leu Leu Val Ser Val Ala Ser
       50                  55                  60
   Arg Asn Asn Val Arg Val Ser Leu Glu Ile Met Glu Glu Lys
   65                  70                  75
<210> 41
   <211> 228
   <212> DNA
   <213> Chlamydia pneumoniae
<400> 41
   atgtttttcg ctcctcttct ttatgaatcg ttacggaggg gtcttatgca tccaacatct     60
   catatgcaac agcaactcgc tcgcttggag tttatcaacg accagctgac tacagagtta    120
   gaacatgtaa atgaattatt atgtagttta ggtttccctg aaggtctcac tacaatcaag    180
   gcaatcgcag aggaagtcct ctctgatgac gaacctctac tagattag                 228
<210> 42
   <211> 75
   <212> PRT
   <213> Chlamydia pneumoniae
<400> 42
   Met Phe Phe Ala Pro Leu Leu Tyr Glu Ser Leu Arg Arg Gly Leu Met
   1 5 10 15
   His Pro Thr Ser His Met Gln Gln Gln Leu Ala Arg Leu Glu Phe Ile
   20 25 30
   Asn Asp Gln Leu Thr Thr Glu Leu Glu His Val Asn Glu Leu Leu Cys
   35 40 45
   Ser Leu Gly Phe Pro Glu Gly Leu Thr Thr Ile Lys Ala Ile Ala Glu
   50 55 60
   Glu Val Leu Ser Asp Asp Glu Pro Leu Leu Asp
   65 70 75
<210> 43
   <211> 1269
   <212> DNA
   <213> Chlamydia pneumoniae
<400> 43
   atgaaaaaac aggtatatca atggttagcg agtgtggttc ttttagcgct gacaatttca     60
   ggatacgctg aacttcctct ctcggaacaa aaagtaaaaa gtcacactta tacaacttta    120
   gacgaagtca aagactactt aagtaaacgg ggttttgtag aaacgcgaaa gcaagatggc    180
   gttttaagaa tagcaggaga tgttagagcc cggtggttgt atttcagaga agatatcaaa    240
   aacccctcag ataaagataa atacaatccc ttaccagtaa atcgttatcg tagtgaattt    300
   tatctctata ttgattatcg cgctgagagg aactggctgt cttcaaagat gaattggaca    360
   gcaattgcag gaggggaaaa cactgcagct ggtgttgata tcaacagagc atttctagga    420
   tatcgttttt ataagaatcc cgaaacacgt acagatttct ttatggaaat cggacgttct    480
   ggtttaggag atctctttga gtcagaagtc caattccaaa gtaattttga cggactacat    540
   atatattgga ctcgagaact ttctaaggac tatccttatc aagtgattgt tcatggaggt    600
   cctttcgtcg tgaacatgac aaaaaaacat tatgcttggg ttgtagaagg gattctcaat    660
   cgtttgccta aacagttttt tgtgaaatgt agtgttgtcg actggaacac attcgttcct    720
   tcagaaacct ccactacaga aaaagctgct acaaacgcta tgaaatacaa atactgtgtt    780
   tggcagtggc tcgtcggaaa gcatagtcag gttccttgga tcaatggaca gaaaaagcct    840
   ctatatcttt atggagcttt cttaatgaac cctttagcaa aggctacgaa gactacgtta    900
   aatggaaaag aaaacctagc ttggtttatt ggaggaactt tagggggact cagaaaagct    960
   ggagactggt ctgccacagt acgttatgag tatgtcgaag ccttgtcggt tccagaaata   1020
   gatgtttcag ggattggccg tggtaattta ttaaagtttt ggttcgccca agcaattgct   1080
   gctaactatg atcctaaaga ggctaatggt tttacaaatt ataaaggatt ttccgctcta   1140
   tatatgtatg gcatcacaga ttctctatca ttcagagctt atggggctta ctccaaacca   1200
   gcaaacgata aactcggcag tgattttact ttccgaaagt ttgatctagg tataatttca   1260
   gcgttttaa                                                           1269
<210> 44
   <211> 422
   <212> PRT
   <213> Chlamydia pneumoniae

<400> 44
   Met Lys Lys Gln Val Tyr Gln Trp Leu Ala Ser Val Val Leu Leu Ala
   1               5                   10                  15
   Leu Thr Ile Ser Gly Tyr Ala Glu Leu Pro Leu Ser Glu Gln Lys Val
               20                  25                  30
   Lys Ser His Thr Tyr Thr Thr Leu Asp Glu Val Lys Asp Tyr Leu Ser
           35                  40                  45
   Lys Arg Gly Phe Val Glu Thr Arg Lys Gln Asp Gly Val Leu Arg Ile
       50                  55                  60
   Ala Gly Asp Val Arg Ala Arg Trp Leu Tyr Phe Arg Glu Asp Ile Lys
   65                  70                  75                  80
   Asn Pro Ser Asp Lys Asp Lys Tyr Asn Pro Leu Pro Val Asn Arg Tyr
                   85                  90                  95
   Arg Ser Glu Phe Tyr Leu Tyr Ile Asp Tyr Arg Ala Glu Arg Asn Trp
               100                 105                 110
   Leu Ser Ser Lys Met Asn Trp Thr Ala Ile Ala Gly Gly Glu Asn Thr
           115                 120                 125
   Ala Ala Gly Val Asp Ile Asn Arg Ala Phe Leu Gly Tyr Arg Phe Tyr
       130                 135                 140
   Lys Asn Pro Glu Thr Arg Thr Asp Phe Phe Met Glu Ile Gly Arg Ser
   145                 150                 155                 160
   Gly Leu Gly Asp Leu Phe Glu Ser Glu Val Gln Phe Gln Ser Asn Phe
                   165                 170                 175
   Asp Gly Leu His Ile Tyr Trp Thr Arg Glu Leu Ser Lys Asp Tyr Pro
               180                 185                 190
   Tyr Gln Val Ile Val His Gly Gly Pro Phe Val Val Asn Met Thr Lys
           195                 200                 205
   Lys His Tyr Ala Trp Val Val Glu Gly Ile Leu Asn Arg Leu Pro Lys
       210                 215                 220
   Gln Phe Phe Val Lys Cys Ser Val Val Asp Trp Asn Thr Phe Val Pro
   225                 230                 235                 240
   Ser Glu Thr Ser Thr Thr Glu Lys Ala Ala Thr Asn Ala Met Lys Tyr
   245 250 255
   Lys Tyr Cys Val Trp Gln Trp Leu Val Gly Lys His Ser Gln Val Pro
   260 265 270
   Trp Ile Asn Gly Gln Lys Lys Pro Leu Tyr Leu Tyr Gly Ala Phe Leu
   275 280 285
   Met Asn Pro Leu Ala Lys Ala Thr Lys Thr Thr Leu Asn Gly Lys Glu
   290 295 300
   Asn Leu Ala Trp Phe Ile Gly Gly Thr Leu Gly Gly Leu Arg Lys Ala
   305 310 315 320
   Gly Asp Trp Ser Ala Thr Val Arg Tyr Glu Tyr Val Glu Ala Leu Ser
   325 330 335
   Val Pro Glu Ile Asp Val Ser Gly Ile Gly Arg Gly Asn Leu Leu Lys
   340 345 350
   Phe Trp Phe Ala Gln Ala Ile Ala Ala Asn Tyr Asp Pro Lys Glu Ala
   355 360 365
   Asn Gly Phe Thr Asn Tyr Lys Gly Phe Ser Ala Leu Tyr Met Tyr Gly
   370 375 380
   Ile Thr Asp Ser Leu Ser Phe Arg Ala Tyr Gly Ala Tyr Ser Lys Pro
   385 390 395 400
   Ala Asn Asp Lys Leu Gly Ser Asp Phe Thr Phe Arg Lys Phe Asp Leu
   405 410 415
   Gly Ile Ile Ser Ala Phe
   420
<210> 45
   <211> 1596
   <212> DNA
   <213> Chlamydia pneumoniae
<400> 45
   atgttgggca aagaagaaga gtttacgtgt aaacaaaagc agtgtttgtc acattttgtt 60
   accaatctga cgtccgatgt atttgcttta aaaaatcttc cagaagtcgt taagggagct 120
   ttattttcta aatactcccg ttcagtttta ggtttgcgag cacttttgtt aaaagaattt 180
   ctatctaatg aagaggatgg agatgtttgt gacgaagcct atgacttcga aaccgatgta 240
   cagaaagctg cggactttta ccaaagggtt cttgataatt ttggggatga ttctgtagga 300
   gagcttggcg gagcccacct ggctatggaa aatgtctcta ttttggctgc taaagtttta 360
   gaggatgctc gaattggcgg atccccgcta gaaaagtcca caagatacgt ctatttcgat 420
   caaaaggtac ggggggagta tttatattac cgagacccta ttttgatgac ttcggccttt 480
   aaagacatgt ttttgggtac ttgtgatttt ttattcgata cctattctgc tttaatccct 540
   caagttcgtg cctattttga aaaactgtat cctaaagatt ctaaaacacc cgcatctgcc 600
   tatgccacat cattacgagc taaagtttta gattgtatac ggggacttct tcctgcggca 660
   actttgacaa atctaggatt tttcggtaac ggtaggtttt ggcaaaatct gattcacaag 720
   ttacaaggtc ataaccttgc agagttgcga cgtttaggag atgaatccct aacagagctt 780
   atgaaagtta ttccttcatt tgtaagtaga gccgagcctc atcatcacca tcatcaagct 840
   atgatgcaat atcgaagagc tttaaaagag cagctcaagg gacttgctga acaagcaaca 900
   tttagtgagg agatgtcttc ttcaccgagt gttcagttgg tatacggaga ccctgatggc 960
   atttataaag tagctgctgg atttcttttt ccttattcaa atcgttctct tacagatctc 1020
   atagactatt gtaaaaaaat gcctcatgaa gatcttgtac agattttaga gagcagtgtt 1080
   tctgcaagag aaaaccgccg gcataagtct cctcgtggtt tagaatgcgt agaatttggc 1140
   tttgatatac ttgctgattt cggtgcatac cgcgatttgc aacgacatcg gacgctgact 1200
   caagaacgac agttactctc tacacatcat ggatacaatt ttcctgtgga gcttctagat 1260
   actcctatgg aaaaatctta tcgagaagct atggagaggg cgaatgaaac ctataatgag 1320
   attgttcagg agttccctga ggaagctcag tatatggttc ccatggctta caatatacgt 1380
   tggtttttcc atgtaaatgc tcgggctttg caatggattt gtgagttacg ctcacagcct 1440
   caaggtcatc aaaattaccg cactatagct acaggtttag tgcgagaggt tgtcaagttc 1500
   aatcctatgt acgaattatt tttcaaattt gtagattatt ctgacataga tttaggacgg 1560
   ttaaatcagg aaatgcgaaa agaaccaacg acctaa 1596
<210> 46
   <211> 531
   <212> PRT
   <213> Chlamydia pneumoniae
<400> 46
   Met Leu Gly Lys Glu Glu Glu Phe Thr Cys Lys Gln Lys Gln Cys Leu
   1 5 10 15
   Ser His Phe Val Thr Asn Leu Thr Ser Asp Val Phe Ala Leu Lys Asn
   20 25 30
   Leu Pro Glu Val Val Lys Gly Ala Leu Phe Ser Lys Tyr Ser Arg Ser
   35 40 45
   Val Leu Gly Leu Arg Ala Leu Leu Leu Lys Glu Phe Leu Ser Asn Glu
   50 55 60
   Glu Asp Gly Asp Val Cys Asp Glu Ala Tyr Asp Phe Glu Thr Asp Val
   65 70 75 80
   Gln Lys Ala Ala Asp Phe Tyr Gln Arg Val Leu Asp Asn Phe Gly Asp
   85 90 95
   Asp Ser Val Gly Glu Leu Gly Gly Ala His Leu Ala Met Glu Asn Val
   100 105 110
   Ser Ile Leu Ala Ala Lys Val Leu Glu Asp Ala Arg Ile Gly Gly Ser
   115 120 125
   Pro Leu Glu Lys Ser Thr Arg Tyr Val Tyr Phe Asp Gln Lys Val Arg
   130 135 140
   Gly Glu Tyr Leu Tyr Tyr Arg Asp Pro Ile Leu Met Thr Ser Ala Phe
   145 150 155 160
   Lys Asp Met Phe Leu Gly Thr Cys Asp Phe Leu Phe Asp Thr Tyr Ser
   165 170 175
   Ala Leu Ile Pro Gln Val Arg Ala Tyr Phe Glu Lys Leu Tyr Pro Lys
   180 185 190
   Asp Ser Lys Thr Pro Ala Ser Ala Tyr Ala Thr Ser Leu Arg Ala Lys
   195 200 205
   Val Leu Asp Cys Ile Arg Gly Leu Leu Pro Ala Ala Thr Leu Thr Asn
   210 215 220
   Leu Gly Phe Phe Gly Asn Gly Arg Phe Trp Gln Asn Leu Ile His Lys
   225 230 235 240
   Leu Gln Gly His Asn Leu Ala Glu Leu Arg Arg Leu Gly Asp Glu Ser
   245 250 255
   Leu Thr Glu Leu Met Lys Val Ile Pro Ser Phe Val Ser Arg Ala Glu
   260 265 270
   Pro His His His His His Gln Ala Met Met Gln Tyr Arg Arg Ala Leu
   275 280 285
   Lys Glu Gln Leu Lys Gly Leu Ala Glu Gln Ala Thr Phe Ser Glu Glu
   290 295 300
   Met Ser Ser Ser Pro Ser Val Gln Leu Val Tyr Gly Asp Pro Asp Gly
   305 310 315 320
   Ile Tyr Lys Val Ala Ala Gly Phe Leu Phe Pro Tyr Ser Asn Arg Ser
   325 330 335
   Leu Thr Asp Leu Ile Asp Tyr Cys Lys Lys Met Pro His Glu Asp Leu
   340 345 350
   Val Gln Ile Leu Glu Ser Ser Val Ser Ala Arg Glu Asn Arg Arg His
   355 360 365
   Lys Ser Pro Arg Gly Leu Glu Cys Val Glu Phe Gly Phe Asp Ile Leu
   370 375 380
   Ala Asp Phe Gly Ala Tyr Arg Asp Leu Gln Arg His Arg Thr Leu Thr
   385 390 395 400
   Gln Glu Arg Gln Leu Leu Ser Thr His His Gly Tyr Asn Phe Pro Val
   405 410 415
   Glu Leu Leu Asp Thr Pro Met Glu Lys Ser Tyr Arg Glu Ala Met Glu
   420 425 430
   Arg Ala Asn Glu Thr Tyr Asn Glu Ile Val Gln Glu Phe Pro Glu Glu
   435 440 445
   Ala Gln Tyr Met Val Pro Met Ala Tyr Asn Ile Arg Trp Phe Phe His
   450 455 460
   Val Asn Ala Arg Ala Leu Gln Trp Ile Cys Glu Leu Arg Ser Gln Pro
   465 470 475 480
   Gln Gly His Gln Asn Tyr Arg Thr Ile Ala Thr Gly Leu Val Arg Glu
   485 490 495
   Val Val Lys Phe Asn Pro Met Tyr Glu Leu Phe Phe Lys Phe Val Asp
   500 505 510
   Tyr Ser Asp Ile Asp Leu Gly Arg Leu Asn Gln Glu Met Arg Lys Glu
   515 520 525
   Pro Thr Thr
   530
<210> 47
   <211> 1206
   <212> DNA
   <213> Chlamydia pneumoniae
<400> 47
   atgttattgg taaggaaatg gttgcatact tgtttcaaat attggattta ctttcttccg 60
   gtggtaacgc tacttcttcc cctagtgtgt tacccttttc tgtcgattag tcaaaaaatt 120
   tatggatact ttgtttttac tacaatttct tctttaggct ggttttttgc attgagacgt 180
   agggaaaatc aattaaaaac agcagctgtt cagcttcttc aaacaaaaat tagaaaatta 240
   acagaaaata atgaagggtt aagacaaatt cgagaatctc ttaaagaaca tcagcaagag 300
   agtgctcaac tgcaaattca aagtcagaag cttaaaaaca gcctatttca tcttcagggt 360
   ttacttgtga aaactaaggg agaggggcaa aaattagaaa ctttgttact tcatagaaca 420
   gaagagaatc gatgtttgaa aatgcaagta gattctttaa ttcaggaatg cggagaaaaa 480
   acagaggaag tacaaacttt aaatcgagag ttggctgaga ctttagccta ccagcaagct 540
   ttaaatgacg agtatcaagc gaccttctct gagcaacgca atatgctgga taagcggcag 600
   atctacattg gaaagctgga aaacaaggtt caggatttaa tgtatgagat ccgtaacttg 660
   cttcagttag agtcagacat agcagagaat attccttctc aagaatcgaa tgctgttacg 720
   ggaaatattt ctttacaatt gtctagtgag ttaaaaaaaa ttgcttttaa ggctgaaaac 780
   atagaggcag cctcttcttt aacagcatca cgttaccttc atacagatac gagtgtgcat 840
   aactactctt tagagtgtcg ccagttattt gatagcttaa gagaagaaaa tctcgggatg 900
   ctttttgtct atgctcgtca atcccaacgt gcggtttttg ctaatgcgtt atttaaaacg 960
   tggacggggt attgtgcaga agatttttta aaatttggta gtgacatagt gatttctggg 1020
   ggcaaacagt ggatggagga tcttcattcc tctagagaag aatgctctgg tagattagtg 1080
   attaaaacga aatcacgagg tcatcttcct ttccgttatt gtttaatggc tttgaataaa 1140
   ggccctcttt gctatcatgt tttgggggtt ctttatcctc tccataaaga agtgcttcag 1200
   agttga 1206
<210> 48
   <211> 401
   <212> PRT
   <213> Chlamydia pneumoniae
<400> 48
<210> 49
   <211> 675
   <212> DNA
   <213> Chlamydia pneumoniae
<400> 49
   atgacatcct ggatagaatt acttgataag caaattgaag atcaacatat gttaaagcac     60
   gaattttatc agcgttggtc tgaaggaaag ttagaaaaac aacaacttca agcttatgcc    120
   aaagattact atttacatat taaagcattt ccttgttacc tttcagcgct gcatgctcgc    180
   tgtgatgact tgcagattcg tagacaaatt cttgagaatc tcatggatga agaagctgga    240
   aatcctaatc acatagattt atggagacag tttgctttat ctcttggagt ttctgaagag    300
   gagcttgcca atcatgaatt cagtcaggct gctcaagata tggtagcgac atttcgccgc    360
   ttatgcgaca tgccacaact tgccgtgggt ttaggcgctc tctatactta tgagattcag    420
   attcctcaag tctgtgtaga gaaaatccgt ggtttgaaag aatattttgg agtttctgct    480
   cgaggctatg catactttac tgtacatcaa gaagctgata ttaaacatgc cagcgaagag    540
   aaagaaatgc tacaaacttt ggtaggcaga gagaatcctg atgctgtttt gcaaggatca    600
   caagaagttt tagatactct atggaacttt ttgagctctt ttattaattc aacggagcct    660
   tgttcttgta agtag                                                     675
<210> 50
   <211> 224
   <212> PRT
   <213> Chlamydia pneumoniae
<400> 50
   Met Thr Ser Trp Ile Glu Leu Leu Asp Lys Gln Ile Glu Asp Gln His
   1               5                   10                  15
   Met Leu Lys His Glu Phe Tyr Gln Arg Trp Ser Glu Gly Lys Leu Glu
               20                  25                  30
   Lys Gln Gln Leu Gln Ala Tyr Ala Lys Asp Tyr Tyr Leu His Ile Lys
           35                  40                  45
   Ala Phe Pro Cys Tyr Leu Ser Ala Leu His Ala Arg Cys Asp Asp Leu
       50                  55                  60
   Gln Ile Arg Arg Gln Ile Leu Glu Asn Leu Met Asp Glu Glu Ala Gly
   65                  70                  75                  80
   Asn Pro Asn His Ile Asp Leu Trp Arg Gln Phe Ala Leu Ser Leu Gly
                   85                  90                  95
   Val Ser Glu Glu Glu Leu Ala Asn His Glu Phe Ser Gln Ala Ala Gln
               100                 105                 110
   Asp Met Val Ala Thr Phe Arg Arg Leu Cys Asp Met Pro Gln Leu Ala
           115                 120                 125
   Val Gly Leu Gly Ala Leu Tyr Thr Tyr Glu Ile Gln Ile Pro Gln Val
       130                 135                 140
   Cys Val Glu Lys Ile Arg Gly Leu Lys Glu Tyr Phe Gly Val Ser Ala
   145                 150                 155                 160
   Arg Gly Tyr Ala Tyr Phe Thr Val His Gln Glu Ala Asp Ile Lys His
                   165                 170                 175
   Ala Ser Glu Glu Lys Glu Met Leu Gln Thr Leu Val Gly Arg Glu Asn
               180                 185                 190
   Pro Asp Ala Val Leu Gln Gly Ser Gln Glu Val Leu Asp Thr Leu Trp
           195                 200                 205
   Asn Phe Leu Ser Ser Phe Ile Asn Ser Thr Glu Pro Cys Ser Cys Lys
       210                 215                 220
<210> 51
   <211> 1287
   <212> DNA
   <213> Chlamydia pneumoniae
<400> 51
   atggatataa aaaaactctt ttgcttattt ctatgttctt ctctaattgc catgagtccc      60
   atttatggga aaacaggtga ctatgagaaa ctcaccctta cagggatcaa tatcattgat     120
   agaaacggcc tgtcagaaac tatttgctct aaagagaagc taaagaaata caccaaggta     180
   gactttcttg ctccccagcc ctatcaaaag gtcatgagga tgtataaaaa caaacgcgga     240
   gataacgttt cttgtttaac agcctatcac actaacgggc aaattaagca gtacctggag     300
   tgtctcaata atcgtgctta tggaagatat cgtgaatggc acgtcaacgg gaatatcaaa     360
   atccaagctg aggttatcgg aggtattgcg gatcttcatc cctcagcaga gtctggctgg     420
   ctatttgatc aaactacatt tgcctataat gatgaaggta tcttagaagc cgctatcgtc     480
   tatgaaaaag ggctgctcga aggatcttcg gtgtattacc atactaatgg gaatatttgg     540
   aaagagtgtc cctatcataa gggagttcct caaggtaaat tcctgacata cacatcttcg     600
   gggaaactgc tcaaagaaca gaattaccaa caaggcaaaa gacacggtct ttcgattcgc     660
   tacagcgaag attccgaaga agatgtttta gcctgggaag aatatcatga gggacgactc     720
   ctaaaagcag agtacttaga tcctcaaact cacgaaatct atgcgactat acacgaaggg     780
   aacggcattc aagcaatcta cggcaagtat gccgttatag aaactagggc attttaccga     840
   ggggaacctt atggaaaagt taccagattc gacaactccg gaacacagat tgtccaaacg     900
   tataaccttt tgcaaggcgc gaagcacgga gaagaatttt tcttttatcc tgagacaggg     960
   aaacccaagc tgcttcttaa ttggcatgaa ggaattttaa atgggatagt aaaaacttgg    1020
   tatcccggag gaaccttaga aagttgtaaa gaactcgtaa ataacaaaaa atccgggtta    1080
   ctgaccattt actaccctga aggacagatc atggcgaccg aagagtatga taatgatctt    1140
   ctaattaaag gagagtactt ccgccctgga gaccgtcatc cctactctaa aatagatcgt    1200
   ggttgtggga ctgcagtatt tttctcgtcg gcgggaacta ttactaaaaa aatcccctat    1260
   caggacggca aacctttgct caactag                                        1287
<210> 52
   <211> 428
   <212> PRT
   <213> Chlamydia pneumoniae
<400> 52
   Met Asp Ile Lys Lys Leu Phe Cys Leu Phe Leu Cys Ser Ser Leu Ile
   1               5                   10                  15
   Ala Met Ser Pro Ile Tyr Gly Lys Thr Gly Asp Tyr Glu Lys Leu Thr
               20                  25                  30
   Leu Thr Gly Ile Asn Ile Ile Asp Arg Asn Gly Leu Ser Glu Thr Ile
           35                  40                  45
   Cys Ser Lys Glu Lys Leu Lys Lys Tyr Thr Lys Val Asp Phe Leu Ala
       50                  55                  60
   Pro Gln Pro Tyr Gln Lys Val Met Arg Met Tyr Lys Asn Lys Arg Gly
   65                  70                  75                  80
   Asp Asn Val Ser Cys Leu Thr Ala Tyr His Thr Asn Gly Gln Ile Lys
                   85                  90                  95
   Gln Tyr Leu Glu Cys Leu Asn Asn Arg Ala Tyr Gly Arg Tyr Arg Glu
               100                 105                 110
   Trp His Val Asn Gly Asn Ile Lys Ile Gln Ala Glu Val Ile Gly Gly
           115                 120                 125
   Ile Ala Asp Leu His Pro Ser Ala Glu Ser Gly Trp Leu Phe Asp Gln
       130                 135                 140
   Thr Thr Phe Ala Tyr Asn Asp Glu Gly Ile Leu Glu Ala Ala Ile Val
   145                 150                 155                 160
   Tyr Glu Lys Gly Leu Leu Glu Gly Ser Ser Val Tyr Tyr His Thr Asn
                   165                 170                 175
   Gly Asn Ile Trp Lys Glu Cys Pro Tyr His Lys Gly Val Pro Gln Gly
               180                 185                 190
   Lys Phe Leu Thr Tyr Thr Ser Ser Gly Lys Leu Leu Lys Glu Gln Asn
   195 200 205
   Tyr Gln Gln Gly Lys Arg His Gly Leu Ser Ile Arg Tyr Ser Glu Asp
   210 215 220
   Ser Glu Glu Asp Val Leu Ala Trp Glu Glu Tyr His Glu Gly Arg Leu
   225 230 235 240
   Leu Lys Ala Glu Tyr Leu Asp Pro Gln Thr His Glu Ile Tyr Ala Thr
   245 250 255
   Ile His Glu Gly Asn Gly Ile Gln Ala Ile Tyr Gly Lys Tyr Ala Val
   260 265 270
   Ile Glu Thr Arg Ala Phe Tyr Arg Gly Glu Pro Tyr Gly Lys Val Thr
   275 280 285
   Arg Phe Asp Asn Ser Gly Thr Gln Ile Val Gln Thr Tyr Asn Leu Leu
   290 295 300
   Gln Gly Ala Lys His Gly Glu Glu Phe Phe Phe Tyr Pro Glu Thr Gly
   305 310 315 320
   Lys Pro Lys Leu Leu Leu Asn Trp His Glu Gly Ile Leu Asn Gly Ile
   325 330 335
   Val Lys Thr Trp Tyr Pro Gly Gly Thr Leu Glu Ser Cys Lys Glu Leu
   340 345 350
   Val Asn Asn Lys Lys Ser Gly Leu Leu Thr Ile Tyr Tyr Pro Glu Gly
   355 360 365
   Gln Ile Met Ala Thr Glu Glu Tyr Asp Asn Asp Leu Leu Ile Lys Gly
   370 375 380
   Glu Tyr Phe Arg Pro Gly Asp Arg His Pro Tyr Ser Lys Ile Asp Arg
   385 390 395 400
   Gly Cys Gly Thr Ala Val Phe Phe Ser Ser Ala Gly Thr Ile Thr Lys
   405 410 415
   Lys Ile Pro Tyr Gln Asp Gly Lys Pro Leu Leu Asn
   420 425
<210> 53
   <211> 795
   <212> DNA
   <213> Chlamydia pneumoniae
<400> 53
   gtggaaaaac ttgagtttgt caccagcctt tcttctcctg atgatgattt gattactttc 60
   aataaacagg gattgattgc aggcccagaa gaagaaaagg tagcgtttct tgtacgtagc 120
   aatgctatgc tagatgcagg acccgaaacc cccgcgtcgt ttcctgaatc tttaagggaa 180
   caattcgata ttttccctga gtatgttgaa gtgctctact ctaatgaagg attagatgtc 240
   tgggaagcag gatgtacgtg gattctaaat aatgaagtga ccatccaact gcgtaaacat 300
   caccggaaag cttcgcgatg gctaggaatg tattccagag atgaggtact cgctcacgaa 360
   gccgtgcatg ctgtgagaat gaaatttcat gagcctgtct ttgaagaggt gttagcttat 420
   caaacttctc gttggggttg gagaaggttt ttcggtcctc tatttcgctc tccaggagag 480
   agctacttgc tattattctt caccatttta ggtttaggaa tctccttatg gtatcctgcc 540
   ggtatactga ttatgctggt tttacctatg tattttttga tgcgattgtg catggcgcag 600
   agctatttgt atcgggccat gaaaaagatt cgtaaaatgc tcggagtacc tcccttatgg 660
   gtgctgctaa ggctgacgga taaggaaata aaaatgtttg ctaaagagcc tattcctgtt 720
   ttggaacact atgctagaaa acgaaagctt gaaaatgtcc gttggaagca aatttatcaa 780
   tcctactttg tttaa 795
<210> 54
   <211> 264
   <212> PRT
   <213> Chlamydia pneumoniae
<400> 54
   Val Glu Lys Leu Glu Phe Val Thr Ser Leu Ser Ser Pro Asp Asp Asp
   1 5 10 15
   Leu Ile Thr Phe Asn Lys Gln Gly Leu Ile Ala Gly Pro Glu Glu Glu
   20 25 30
   Lys Val Ala Phe Leu Val Arg Ser Asn Ala Met Leu Asp Ala Gly Pro
   35 40 45
   Glu Thr Pro Ala Ser Phe Pro Glu Ser Leu Arg Glu Gln Phe Asp Ile
   50 55 60
   Phe Pro Glu Tyr Val Glu Val Leu Tyr Ser Asn Glu Gly Leu Asp Val
   65 70 75 80
   Trp Glu Ala Gly Cys Thr Trp Ile Leu Asn Asn Glu Val Thr Ile Gln
   85 90 95
   Leu Arg Lys His His Arg Lys Ala Ser Arg Trp Leu Gly Met Tyr Ser
   100 105 110
   Arg Asp Glu Val Leu Ala His Glu Ala Val His Ala Val Arg Met Lys
   115 120 125
   Phe His Glu Pro Val Phe Glu Glu Val Leu Ala Tyr Gln Thr Ser Arg
   130 135 140
   Trp Gly Trp Arg Arg Phe Phe Gly Pro Leu Phe Arg Ser Pro Gly Glu
   145 150 155 160
   Ser Tyr Leu Leu Leu Phe Phe Thr Ile Leu Gly Leu Gly Ile Ser Leu
   165 170 175
   Trp Tyr Pro Ala Gly Ile Leu Ile Met Leu Val Leu Pro Met Tyr Phe
   180 185 190
   Leu Met Arg Leu Cys Met Ala Gln Ser Tyr Leu Tyr Arg Ala Met Lys
   195 200 205
   Lys Ile Arg Lys Met Leu Gly Val Pro Pro Leu Trp Val Leu Leu Arg
   210 215 220
   Leu Thr Asp Lys Glu Ile Lys Met Phe Ala Lys Glu Pro Ile Pro Val
   225 230 235 240
   Leu Glu His Tyr Ala Arg Lys Arg Lys Leu Glu Asn Val Arg Trp Lys
   245 250 255
   Gln Ile Tyr Gln Ser Tyr Phe Val
   260
<210> 55
   <211> 234
   <212> DNA
   <213> Chlamydia pneumoniae
<400> 55
   atgaatgaag gtatccactc tgtctgtttt caaaaaacac ctcggcttac tgcgaagtcc 60
   gtagtgagta tggagatgct cttaactact caacagcttc cttccgcaga agggatgccc 120
   tcggttgcta atttggaagc ggatttttta cgagcagaag ctctgttagc agaaatgcga 180
   gaaattcgtg gttgcttgga gcaatctttg cgaacactag tccctagtga gtag 234
<210> 56
   <211> 77
   <212> PRT
   <213> Chlamydia pneumoniae
<400> 56
   Met Asn Glu Gly Ile His Ser Val Cys Phe Gln Lys Thr Pro Arg Leu
   1 5 10 15
   Thr Ala Lys Ser Val Val Ser Met Glu Met Leu Leu Thr Thr Gln Gln
   20 25 30
   Leu Pro Ser Ala Glu Gly Met Pro Ser Val Ala Asn Leu Glu Ala Asp
   35 40 45
   Phe Leu Arg Ala Glu Ala Leu Leu Ala Glu Met Arg Glu Ile Arg Gly
   50 55 60
   Cys Leu Glu Gln Ser Leu Arg Thr Leu Val Pro Ser Glu
   65 70 75
<210> 57
   <211> 1815
   <212> DNA
   <213> Chlamydia pneumoniae
<400> 57
   atgaaacata cctttaccaa gcgtgttcta ttttttttct ttttagtgat tcccattccc      60
   ctactcctca atcttatggt cgtaggtttt ttctcatttt ctgccgctaa agcaaattta     120
   gtacaggtcc tccatacccg tgctacgaac ttaagtatag aattcgaaaa aaaactgacg     180
   atacacaagc ttttcctcga tagacttgcc aacacattag ccttaaaatc ctatgcatct     240
   ccttctgcag agccctatgc acaggcatac aatgagatga tggcactctc caatacagac     300
   ttttccttat gccttataga tccctttgat ggatctgtaa ggacgaaaaa tcctggagac     360
   cctttcattc gctatctaaa acagcatcct gaaatgaaga aaaagctatc cgcagctgta     420
   gggaaagcct ttttattgac cattccaggt aaaccacttt tacattatct tattctagtt     480
   gaagatgtcg catcttggga ttctacaacg acttcaggac tgcttgtaag tttctatccc     540
   atgtcttttt tacagaaaga tttattccaa tccttacaca tcaccaaagg aaatatctgc     600
   cttgtaaata agtatggcga ggtcctcttc tgtgctcagg acagtgaatc ttcttttgta     660
   ttttctctag atctccctaa tttaccgcaa ttccaagcaa gaagcccctc tgccatagaa     720
   attgagaaag cttctggaat tcttggtggg gagaacctaa tcacagtgag tatcaacaag     780
   aaacgctacc taggattggt actgaataaa attcctatcc aagggaccta cactctatct     840
   ttagttccag tttctgatct catccaatcc gccttgaaag ttcctctcaa tatttgtttt     900
   ttctatgtac ttgctttcct cctcatgtgg tggattttct ctaagatcaa caccaaactt     960
   aacaagcctc ttcaagaact gaccttctgt atggaagctg cctggcgagg aaaccataac    1020
   gtgaggtttg aaccccagcc ttacggttat gaattcaatg aactaggaaa tattttcaat    1080
   tgcactctcc tactcttatt gaattccatt gagaaagcag atatcgatta ccattcaggc    1140
   gaaaaattac aaaaagaatt agggatttta tcttcactac aaagtgcgtt actaagtccg    1200
   gatttcccta cgttccctaa agttaccttt agttcccaac atctccggag aaggcaactt    1260
   tccggtcatt ttaatggttg gacagttcaa gatggtggcg ataccctttt agggatcata    1320
   gggctcgctg gcgatattgg tcttccttcc tatctctatg ctttatccgc acggagtctt    1380
   tttcttgcct atgcttcctc ggacgtttcg ttacaaaaaa tcagcaagga tactgccgac    1440
   agcttctcaa aaacaacaga aggcaatgag gctgtagttg ctatgacttt cattaaatat    1500
   gtagaaaaag atcgatctct agagctcctc tcgttaagcg agggagctcc taccatgttt    1560
   ctacaacgag gagaatcttt cgtacgtctc cccttagaga ctcaccaagc tctacagcct    1620
   ggagatcggt tgatctgcct cactggagga gaagacatcc tcaagtactt ttctcagctt    1680
   cctattgaag agctcttaaa agatccttta aaccctctaa atacagagaa tcttattgat    1740
   tctctaacca tgatgttaaa caacgaaacc gaacattctg cagatggaac tctgaccatc    1800
   ctttcatttt cataa                                                      1815
<210> 58
   <211> 604
   <212> PRT
   <213> Chlamydia pneumoniae
<400> 58
   Met Lys His Thr Phe Thr Lys Arg Val Leu Phe Phe Phe Phe Leu Val
   1               5                   10                  15
   Ile Pro Ile Pro Leu Leu Leu Asn Leu Met Val Val Gly Phe Phe Ser
               20                  25                  30
   Phe Ser Ala Ala Lys Ala Asn Leu Val Gln Val Leu His Thr Arg Ala
           35                  40                  45
   Thr Asn Leu Ser Ile Glu Phe Glu Lys Lys Leu Thr Ile His Lys Leu
       50                  55                  60
   Phe Leu Asp Arg Leu Ala Asn Thr Leu Ala Leu Lys Ser Tyr Ala Ser
   65                  70                  75                  80
   Pro Ser Ala Glu Pro Tyr Ala Gln Ala Tyr Asn Glu Met Met Ala Leu
                   85                  90                  95
   Ser Asn Thr Asp Phe Ser Leu Cys Leu Ile Asp Pro Phe Asp Gly Ser
               100                 105                 110
   Val Arg Thr Lys Asn Pro Gly Asp Pro Phe Ile Arg Tyr Leu Lys Gln
           115                 120                 125
   His Pro Glu Met Lys Lys Lys Leu Ser Ala Ala Val Gly Lys Ala Phe
       130                 135                 140
   Leu Leu Thr Ile Pro Gly Lys Pro Leu Leu His Tyr Leu Ile Leu Val
   145                 150                 155                 160
   Glu Asp Val Ala Ser Trp Asp Ser Thr Thr Thr Ser Gly Leu Leu Val
                   165                 170                 175
   Ser Phe Tyr Pro Met Ser Phe Leu Gln Lys Asp Leu Phe Gln Ser Leu
               180                 185                 190
   His Ile Thr Lys Gly Asn Ile Cys Leu Val Asn Lys Tyr Gly Glu Val
           195                 200                 205
   Leu Phe Cys Ala Gln Asp Ser Glu Ser Ser Phe Val Phe Ser Leu Asp
   210 215 220
   Leu Pro Asn Leu Pro Gln Phe Gln Ala Arg Ser Pro Ser Ala Ile Glu
   225 230 235 240
   Ile Glu Lys Ala Ser Gly Ile Leu Gly Gly Glu Asn Leu Ile Thr Val
   245 250 255
   Ser Ile Asn Lys Lys Arg Tyr Leu Gly Leu Val Leu Asn Lys Ile Pro
   260 265 270
   Ile Gln Gly Thr Tyr Thr Leu Ser Leu Val Pro Val Ser Asp Leu Ile
   275 280 285
   Gln Ser Ala Leu Lys Val Pro Leu Asn Ile Cys Phe Phe Tyr Val Leu
   290 295 300
   Ala Phe Leu Leu Met Trp Trp Ile Phe Ser Lys Ile Asn Thr Lys Leu
   305 310 315 320
   Asn Lys Pro Leu Gln Glu Leu Thr Phe Cys Met Glu Ala Ala Trp Arg
   325 330 335
   Gly Asn His Asn Val Arg Phe Glu Pro Gln Pro Tyr Gly Tyr Glu Phe
   340 345 350
   Asn Glu Leu Gly Asn Ile Phe Asn Cys Thr Leu Leu Leu Leu Leu Asn
   355 360 365
   Ser Ile Glu Lys Ala Asp Ile Asp Tyr His Ser Gly Glu Lys Leu Gln
   370 375 380
   Lys Glu Leu Gly Ile Leu Ser Ser Leu Gln Ser Ala Leu Leu Ser Pro
   385 390 395 400
   Asp Phe Pro Thr Phe Pro Lys Val Thr Phe Ser Ser Gln His Leu Arg
   405 410 415
   Arg Arg Gln Leu Ser Gly His Phe Asn Gly Trp Thr Val Gln Asp Gly
   420 425 430
   Gly Asp Thr Leu Leu Gly Ile Ile Gly Leu Ala Gly Asp Ile Gly Leu
   435 440 445
   Pro Ser Tyr Leu Tyr Ala Leu Ser Ala Arg Ser Leu Phe Leu Ala Tyr
   450 455 460
   Ala Ser Ser Asp Val Ser Leu Gln Lys Ile Ser Lys Asp Thr Ala Asp
   465 470 475 480
   Ser Phe Ser Lys Thr Thr Glu Gly Asn Glu Ala Val Val Ala Met Thr
   485 490 495
   Phe Ile Lys Tyr Val Glu Lys Asp Arg Ser Leu Glu Leu Leu Ser Leu
   500 505 510
   Ser Glu Gly Ala Pro Thr Met Phe Leu Gln Arg Gly Glu Ser Phe Val
   515 520 525
   Arg Leu Pro Leu Glu Thr His Gln Ala Leu Gln Pro Gly Asp Arg Leu
   530 535 540
   Ile Cys Leu Thr Gly Gly Glu Asp Ile Leu Lys Tyr Phe Ser Gln Leu
   545 550 555 560
   Pro Ile Glu Glu Leu Leu Lys Asp Pro Leu Asn Pro Leu Asn Thr Glu
   565 570 575
   Asn Leu Ile Asp Ser Leu Thr Met Met Leu Asn Asn Glu Thr Glu His
   580 585 590
   Ser Ala Asp Gly Thr Leu Thr Ile Leu Ser Phe Ser
   595 600
<210> 59
   <211> 1170
   <212> DNA
   <213> Chlamydia pneumoniae
<400> 59
   atgcatccga aaatagaaaa aagaaatagc cttccactta cggcagtcgc tcctgtgttt     60
   gaagaatcgt atcatccttc tgtagctaca actgtagatt atgtagatgc cacgacactt    120
   tcccgacatc ttacagtctt aaaagatgtg ataaaagaag ctcgaaactt agatttaggg    180
   aaggcattcc tgacatctat gaaacagggt tttataaata cgggtacgga acttgccatt    240
   atacaagcat ctctggcaga tcagagtagt cgcgagtcgc gtaagaagga agagaagatc    300
   ttccatcagc acttaggaaa ggcagcccca caagcggcaa cagcaacttc aggagtgcag    360
   cctactgcgg atcctgttgc tgataagatg cctttacaat ctgcatttgc ctatgttctc    420
   cttgataagt acattcctgc tcaagaggaa gccctttatg ctcttggaag ggagttaaac    480
   ctatcaggat atgcgcaaaa tttatttagt cctcttttag atatgattaa gagctttaac    540
   tctgctccta tcaactacaa tttaggatcg tacatatctc agacgagtgg cactgcgaat    600
   ttcgcgtatg gttatgagat gattttatcg cgctataaca acgaagtctc tcaatgtcgc    660
   ctggacatag caagtacagt aaaagctaaa gctgcgttag cgaacatgtc ggcttctgtt    720
   aaagcaaatg tgagtctgac tgatgcacag aagaaacaaa ttgaggatat cattgccagc    780
   tatacgaaat ctttagatgt gattcataca cagttaactg atgtgatgac aaatttagca    840
   tccataacct ttgttcctgg tttaaataaa tatgatcctt cgtatcgcat tgttggtggg    900
   gatttatcta tcattgcctt gcagaatgac gagaaggtac ttgtcgatgg taaggtggat    960
   atcacgactg ctgtgaatga aggaggccta cttaatttct tcactacagt ccttacggat   1020
   gtgcagaatt atggagactt agctcaaacg caacagctga tgttggactt agagcttaag   1080
   gcgatgcaac aacaatggag tttagtatct gcatctttga aattattgaa tgggatgtat   1140
   accacagtaa tttctggatt taaaaactaa                                     1170
<210> 60
   <211> 389
   <212> PRT
   <213> Chlamydia pneumoniae
<400> 60
   Met His Pro Lys Ile Glu Lys Arg Asn Ser Leu Pro Leu Thr Ala Val
   1               5                   10                  15
   Ala Pro Val Phe Glu Glu Ser Tyr His Pro Ser Val Ala Thr Thr Val
               20                  25                  30
   Asp Tyr Val Asp Ala Thr Thr Leu Ser Arg His Leu Thr Val Leu Lys
           35                  40                  45
   Asp Val Ile Lys Glu Ala Arg Asn Leu Asp Leu Gly Lys Ala Phe Leu
       50                  55                  60
   Thr Ser Met Lys Gln Gly Phe Ile Asn Thr Gly Thr Glu Leu Ala Ile
   65                  70                  75                  80
   Ile Gln Ala Ser Leu Ala Asp Gln Ser Ser Arg Glu Ser Arg Lys Lys
                   85                  90                  95
   Glu Glu Lys Ile Phe His Gln His Leu Gly Lys Ala Ala Pro Gln Ala
               100                 105                 110
   Ala Thr Ala Thr Ser Gly Val Gln Pro Thr Ala Asp Pro Val Ala Asp
           115                 120                 125
   Lys Met Pro Leu Gln Ser Ala Phe Ala Tyr Val Leu Leu Asp Lys Tyr
       130                 135                 140
   Ile Pro Ala Gln Glu Glu Ala Leu Tyr Ala Leu Gly Arg Glu Leu Asn
   145                 150                 155                 160
   Leu Ser Gly Tyr Ala Gln Asn Leu Phe Ser Pro Leu Leu Asp Met Ile
                   165                 170                 175
   Lys Ser Phe Asn Ser Ala Pro Ile Asn Tyr Asn Leu Gly Ser Tyr Ile
               180                 185                 190
   Ser Gln Thr Ser Gly Thr Ala Asn Phe Ala Tyr Gly Tyr Glu Met Ile
           195                 200                 205
   Leu Ser Arg Tyr Asn Asn Glu Val Ser Gln Cys Arg Leu Asp Ile Ala
   210 215 220
   Ser Thr Val Lys Ala Lys Ala Ala Leu Ala Asn Met Ser Ala Ser Val
   225 230 235 240
   Lys Ala Asn Val Ser Leu Thr Asp Ala Gln Lys Lys Gln Ile Glu Asp
   245 250 255
   Ile Ile Ala Ser Tyr Thr Lys Ser Leu Asp Val Ile His Thr Gln Leu
   260 265 270
   Thr Asp Val Met Thr Asn Leu Ala Ser Ile Thr Phe Val Pro Gly Leu
   275 280 285
   Asn Lys Tyr Asp Pro Ser Tyr Arg Ile Val Gly Gly Asp Leu Ser Ile
   290 295 300
   Ile Ala Leu Gln Asn Asp Glu Lys Val Leu Val Asp Gly Lys Val Asp
   305 310 315 320
   Ile Thr Thr Ala Val Asn Glu Gly Gly Leu Leu Asn Phe Phe Thr Thr
   325 330 335
   Val Leu Thr Asp Val Gln Asn Tyr Gly Asp Leu Ala Gln Thr Gln Gln
   340 345 350
   Leu Met Leu Asp Leu Glu Leu Lys Ala Met Gln Gln Gln Trp Ser Leu
   355 360 365
   Val Ser Ala Ser Leu Lys Leu Leu Asn Gly Met Tyr Thr Thr Val Ile
   370 375 380
   Ser Gly Phe Lys Asn
   385
<210> 61
   <211> 537
   <212> DNA
   <213> Chlamydia pneumoniae
<400> 61
   gtgacaacac cacaatctcc tggttcgctt tctcaatctc accttcctca tccacatgat     60
   ccttgggata cagaacctac aagtcttccc gaagatccga atgataaggc aagccaagaa    120
   ctccattcgt tagttcatct ctttcgcaag ctatccattc acctacttag tgaagtcgaa    180
   aaaacagtac aacagctaaa acccgaccta ctagaactgg ctcttctcat ctgtgaaaaa    240
   tttctctata agaagctaga aaatcctcaa gaactggccc tgctcctctc taccgcgctc    300
   caaagacata cgacattaag atctctgact cccatcaaag tatttctcca tcccgaggat    360
   ctcaaaacac ttacagattg gatctccacc cacgaactcc ccatgattaa gcatgctgag    420
   tttttccctg acacttcttg tagacgatca ggattcaaaa tagaaacccc taacggaatt    480
   ctgagacaag aaatcagcga agaactagac catctacttt ctgttttgac agcatga       537
<210> 62
   <211> 178
   <212> PRT
   <213> Chlamydia pneumoniae
<400> 62
   Val Thr Thr Pro Gln Ser Pro Gly Ser Leu Ser Gln Ser His Leu Pro
   1 5 10 15
   His Pro His Asp Pro Trp Asp Thr Glu Pro Thr Ser Leu Pro Glu Asp
   20 25 30
   Pro Asn Asp Lys Ala Ser Gln Glu Leu His Ser Leu Val His Leu Phe
   35 40 45
   Arg Lys Leu Ser Ile His Leu Leu Ser Glu Val Glu Lys Thr Val Gln
   50 55 60
   Gln Leu Lys Pro Asp Leu Leu Glu Leu Ala Leu Leu Ile Cys Glu Lys
   65 70 75 80
   Phe Leu Tyr Lys Lys Leu Glu Asn Pro Gln Glu Leu Ala Leu Leu Leu
   85 90 95
   Ser Thr Ala Leu Gln Arg His Thr Thr Leu Arg Ser Leu Thr Pro Ile
   100 105 110
   Lys Val Phe Leu His Pro Glu Asp Leu Lys Thr Leu Thr Asp Trp Ile
   115 120 125
   Ser Thr His Glu Leu Pro Met Ile Lys His Ala Glu Phe Phe Pro Asp
   130 135 140
   Thr Ser Cys Arg Arg Ser Gly Phe Lys Ile Glu Thr Pro Asn Gly Ile
   145 150 155 160
   Leu Arg Gln Glu Ile Ser Glu Glu Leu Asp His Leu Leu Ser Val Leu
   165 170 175
   Thr Ala
<210> 63
   <211> 798
   <212> DNA
   <213> Chlamydia pneumoniae
<400> 63
   atgcaaggtt ttttcccttt agcttctgga tccaaaggga attctgctta tctaggcacg 60
   gattcttgta agattcttat tgatttagga gtgagcaagc aagtcgtcac tcgggaatta    120
   ctctctatga atatcgatcc tgaagatatt caggcaattt ttgttacgca cgaacattct    180
   gatcatatct ccgggattaa aagttttgtt aaggcgtata acactcccat tgtttgcaac    240
   ttggagacgg ctcgtgcttt atgccatcta ctagatagcc atccagaatt caaaatattt    300
   tccacagggt cttcattttg ttttcaagat ctcgaagtac agacgttcaa tgtacctcat    360
   gatgctgtag atcctgtggc ttttattttt cattatcgcg aagagaaact gggtttttgc    420
   acagatttag gttgggtcac ctcttggatc acacatgaac tctatgattg tgattactta    480
   ttaattgagt ccaatcattc ccctgaattg gtacgtcaat ctcaacgtcc tgatgtttac    540
   aaaaagcgtg tattgagtaa attaggtcat atttctaacc aagagtgtgg tcagctttta    600
   caaaagatta tcactccgaa gttaaagaag ttatatcttg ctcacctgtc taccgagtgt    660
   aacactgcgg agctagcact ttctacagta tctgaatcga tagcttctat cacttctata    720
   gcaccagaaa ttgcattagc gcagggcatt acatctccaa tatacttttc tcgtcttgag    780
   gttgcatgtc cacggtaa                                                  798
<210> 64
   <211> 265
   <212> PRT
   <213> Chlamydia pneumoniae
<400> 64
   Met Gln Gly Phe Phe Pro Leu Ala Ser Gly Ser Lys Gly Asn Ser Ala
   1 5 10 15
   Tyr Leu Gly Thr Asp Ser Cys Lys Ile Leu Ile Asp Leu Gly Val Ser
   20 25 30
   Lys Gln Val Val Thr Arg Glu Leu Leu Ser Met Asn Ile Asp Pro Glu
   35 40 45
   Asp Ile Gln Ala Ile Phe Val Thr His Glu His Ser Asp His Ile Ser
   50 55 60
   Gly Ile Lys Ser Phe Val Lys Ala Tyr Asn Thr Pro Ile Val Cys Asn
   65 70 75 80
   Leu Glu Thr Ala Arg Ala Leu Cys His Leu Leu Asp Ser His Pro Glu
   85 90 95
   Phe Lys Ile Phe Ser Thr Gly Ser Ser Phe Cys Phe Gln Asp Leu Glu
   100 105 110
   Val Gln Thr Phe Asn Val Pro His Asp Ala Val Asp Pro Val Ala Phe
   115 120 125
   Ile Phe His Tyr Arg Glu Glu Lys Leu Gly Phe Cys Thr Asp Leu Gly
   130 135 140
   Trp Val Thr Ser Trp Ile Thr His Glu Leu Tyr Asp Cys Asp Tyr Leu
   145 150 155 160
   Leu Ile Glu Ser Asn His Ser Pro Glu Leu Val Arg Gln Ser Gln Arg
   165 170 175
   Pro Asp Val Tyr Lys Lys Arg Val Leu Ser Lys Leu Gly His Ile Ser
   180 185 190
   Asn Gln Glu Cys Gly Gln Leu Leu Gln Lys Ile Ile Thr Pro Lys Leu
   195 200 205
   Lys Lys Leu Tyr Leu Ala His Leu Ser Thr Glu Cys Asn Thr Ala Glu
   210 215 220
   Leu Ala Leu Ser Thr Val Ser Glu Ser Ile Ala Ser Ile Thr Ser Ile
   225 230 235 240
   Ala Pro Glu Ile Ala Leu Ala Gln Gly Ile Thr Ser Pro Ile Tyr Phe
   245 250 255
   Ser Arg Leu Glu Val Ala Cys Pro Arg
   260 265
<210> 65
   <211> 429
   <212> DNA
   <213> Chlamydia pneumoniae
<400> 65
   atggaagagt ttgtagcata tattgttaag aatttagtta ctaatcctga agctgttgaa 60
   attcgttcta ttgaagacga ggataacgaa tctattaagt tagaaattcg tgtcgcagca 120
   gaggatattg ggaaaattat tggaagaaga gggaacacaa tacatgcgct aagaacgatt 180
   cttagacgtg tatgttctag attaaaaaag aaagtgcaga tagatttggt tcagcctgaa 240
   aatggaactg atgtgattgc tgatcaagat tacatctgtg ataatgactc ttctaactct 300
   actgaagata ctttcggcga gtctgacacc tgctgcagtg gacactgtca ttacgacgaa 360
   gatcttaatc aagaagaaca agaagaaggc aatatgcatc attcttgcga atgtagtaac 420
   caccattaa 429
<210> 66
   <211> 142
   <212> PRT
   <213> Chlamydia pneumoniae
<400> 66
   Met Glu Glu Phe Val Ala Tyr Ile Val Lys Asn Leu Val Thr Asn Pro
   1               5                   10                  15
   Glu Ala Val Glu Ile Arg Ser Ile Glu Asp Glu Asp Asn Glu Ser Ile
               20                  25                  30
   Lys Leu Glu Ile Arg Val Ala Ala Glu Asp Ile Gly Lys Ile Ile Gly
           35                  40                  45
   Arg Arg Gly Asn Thr Ile His Ala Leu Arg Thr Ile Leu Arg Arg Val
       50                  55                  60
   Cys Ser Arg Leu Lys Lys Lys Val Gln Ile Asp Leu Val Gln Pro Glu
   65                  70                  75                  80
   Asn Gly Thr Asp Val Ile Ala Asp Gln Asp Tyr Ile Cys Asp Asn Asp
                   85                  90                  95
   Ser Ser Asn Ser Thr Glu Asp Thr Phe Gly Glu Ser Asp Thr Cys Cys
               100                 105                 110
   Ser Gly His Cys His Tyr Asp Glu Asp Leu Asn Gln Glu Glu Gln Glu
           115                 120                 125
   Glu Gly Asn Met His His Ser Cys Glu Cys Ser Asn His His
       130                 135                 140
<210> 67
   <211> 471
   <212> DNA
   <213> Chlamydia pneumoniae
<400> 67
   atggaaaaac aaaatttaaa attagatgtc aaagagattg agtttcctga aacggtattc 60
   agccgtgata tcgaaactcg tgttatccaa gtaattattt tgcattgttt agcaaaaatt 120
   aacggtgttt ccctcctcgg aggaaatcta atagacgctc tgttcggtag agatatcgaa 180
   agaatgaagg ggatctatgt agaacaggat tcaaaaaatc atctggtcaa agttcgtgtc 240
   gaagtgaacg tagattacgg tgtttctata ccagagaaaa cagaagaaat ccagggatgc 300
   attgtttcag aaatttcaga atatacagga cttcatgtgg ccgctgtcca cgtgatcatt 360
   aaagggttga cacaaccaaa agatcgtatt gatgaagaaa ttgaagagga agtctctgtt 420
   caagatcttc cttcacctga agacttctta cttgagaatt ctgaagggta g 471
<210> 68
   <211> 156
   <212> PRT
   <213> Chlamydia pneumoniae
<400> 68
   Met Glu Lys Gln Asn Leu Lys Leu Asp Val Lys Glu Ile Glu Phe Pro
   1 5 10 15
   Glu Thr Val Phe Ser Arg Asp Ile Glu Thr Arg Val Ile Gln Val Ile
   20 25 30
   Ile Leu His Cys Leu Ala Lys Ile Asn Gly Val Ser Leu Leu Gly Gly
   35 40 45
   Asn Leu Ile Asp Ala Leu Phe Gly Arg Asp Ile Glu Arg Met Lys Gly
   50 55 60
   Ile Tyr Val Glu Gln Asp Ser Lys Asn His Leu Val Lys Val Arg Val
   65 70 75 80
   Glu Val Asn Val Asp Tyr Gly Val Ser Ile Pro Glu Lys Thr Glu Glu
   85 90 95
   Ile Gln Gly Cys Ile Val Ser Glu Ile Ser Glu Tyr Thr Gly Leu His
   100 105 110
   Val Ala Ala Val His Val Ile Ile Lys Gly Leu Thr Gln Pro Lys Asp
   115 120 125
   Arg Ile Asp Glu Glu Ile Glu Glu Glu Val Ser Val Gln Asp Leu Pro
   130 135 140
   Ser Pro Glu Asp Phe Leu Leu Glu Asn Ser Glu Gly
   145 150 155
<210> 69
   <211> 231
   <212> DNA
   <213> Chlamydia pneumoniae
<400> 69
   gtgaaaaata aaattgttac attattagat cagctctacg aggatcagga gtcacgactt 60
   cagaagttag gagaggaaat tgttcctaac ctcactcctg aagatttatt gcaacctatg 120
   gattttcctc aattggaagg gaatccggca tttcgttttg aagagggtgt cttatcagga 180
   attggtgagg tgcgagctgc gattttagcg gcgctctctc aagagaacta g 231
<210> 70
   <211> 76
   <212> PRT
   <213> Chlamydia pneumoniae
<400> 70
   Val Lys Asn Lys Ile Val Thr Leu Leu Asp Gln Leu Tyr Glu Asp Gln
   1 5 10 15
   Glu Ser Arg Leu Gln Lys Leu Gly Glu Glu Ile Val Pro Asn Leu Thr
   20 25 30
   Pro Glu Asp Leu Leu Gln Pro Met Asp Phe Pro Gln Leu Glu Gly Asn
   35 40 45
   Pro Ala Phe Arg Phe Glu Glu Gly Val Leu Ser Gly Ile Gly Glu Val
   50 55 60
   Arg Ala Ala Ile Leu Ala Ala Leu Ser Gln Glu Asn
   65 70 75
<210> 71
   <211> 522
   <212> DNA
   <213> Chlamydia pneumoniae
<400> 71
   atgtggatca tagaccctct atcagcaaaa aaacctctac aagcagccat aaatgttcct      60
   ggcactccaa ttacaggagg acctaataca gcaactgctg acgatatcat tgcaaaattc     120
   tccaaagact caaaccctct gattgttact gtttactacg tctatcaatc cgtattagtt     180
   gctcaagata acctctccat cattgcccaa gaactccaag caaactcttc agctcaaacc     240
   tacctaaata accaagaagc cttataccaa tacgtcagta ttcctaaaaa taaactgaac     300
   gataactcct ctagctatct acaaaacatc caatccgata accaagcgat aggagcttct     360
   cggcaagcta tccaaaacca aatttccagt ttaggaaacg cggctcaggt aatctccagt     420
   aacttgaaca caaataataa catcatccaa caatccttac aggtaggaca ggctcttatc     480
   cagaccttct ctcaaattgt aagcctaatt gctaacatct aa                        522
<210> 72
   <211> 173
   <212> PRT
   <213> Chlamydia pneumoniae
<400> 72
   Met Trp Ile Ile Asp Pro Leu Ser Ala Lys Lys Pro Leu Gln Ala Ala
   1               5                   10                  15
   Ile Asn Val Pro Gly Thr Pro Ile Thr Gly Gly Pro Asn Thr Ala Thr
               20                  25                  30
   Ala Asp Asp Ile Ile Ala Lys Phe Ser Lys Asp Ser Asn Pro Leu Ile
           35                  40                  45
   Val Thr Val Tyr Tyr Val Tyr Gln Ser Val Leu Val Ala Gln Asp Asn
       50                  55                  60
   Leu Ser Ile Ile Ala Gln Glu Leu Gln Ala Asn Ser Ser Ala Gln Thr
   65                  70                  75                  80
   Tyr Leu Asn Asn Gln Glu Ala Leu Tyr Gln Tyr Val Ser Ile Pro Lys
                   85                  90                  95
   Asn Lys Leu Asn Asp Asn Ser Ser Ser Tyr Leu Gln Asn Ile Gln Ser
               100                 105                 110
   Asp Asn Gln Ala Ile Gly Ala Ser Arg Gln Ala Ile Gln Asn Gln Ile
   115 120 125
   Ser Ser Leu Gly Asn Ala Ala Gln Val Ile Ser Ser Asn Leu Asn Thr
   130 135 140
   Asn Asn Asn Ile Ile Gln Gln Ser Leu Gln Val Gly Gln Ala Leu Ile
   145 150 155 160
   Gln Thr Phe Ser Gln Ile Val Ser Leu Ile Ala Asn Ile
   165 170
<210> 73
   <211> 222
   <212> DNA
   <213> Chlamydia pneumoniae
<400> 73
   ttgtggtata aatctttagc tggagaggag aaggacgtgt ctgggaatga gtgcaatgac 60
   tatccagaag tttttaaaga tgacgtaagt gcttacgtat tggtaacttg tggtcagatg 120
   tcttctgaag gcaaaatcca ggtggagatg acttatgaag gagatccagc tgtgatcagc 180
   tatttattaa caaaagcacg agactcttta gatgagtctt aa 222
<210> 74
   <211> 73
   <212> PRT
   <213> Chlamydia pneumoniae
<400> 74
   Leu Trp Tyr Lys Ser Leu Ala Gly Glu Glu Lys Asp Val Ser Gly Asn
   1 5 10 15
   Glu Cys Asn Asp Tyr Pro Glu Val Phe Lys Asp Asp Val Ser Ala Tyr
   20 25 30
   Val Leu Val Thr Cys Gly Gln Met Ser Ser Glu Gly Lys Ile Gln Val
   35 40 45
   Glu Met Thr Tyr Glu Gly Asp Pro Ala Val Ile Ser Tyr Leu Leu Thr
   50 55 60
   Lys Ala Arg Asp Ser Leu Asp Glu Ser
   65 70
<210> 75
   <211> 1494
   <212> DNA
   <213> Chlamydia pneumoniae
<400> 75
   atgactgtat cttaccaatc catatcgact cctcctcctg aaggagagtt tgatattttt      60
   gtagatggaa atgccactga agaagctgtt gttgctgcag aagtacaagt agctctccct     120
   gcaggagagc aatatgcgat gctgagagca acttccgagc tatgttttgg gattttaaca     180
   caatcggaat gtgctttgac acaagcactg cctcccaagg aaaaaccatt acaagaagag     240
   caatttctag taaaaaatgg catattaatg cgatcaacat ctctgccgaa cctaaaacca     300
   ggacaatcgc agcagactag cttagcttca catagaaatc ccttagcgca gcaatccaca     360
   tcttcgaatt ccacagggaa agcatcaaca gaaacaacct cgtcttcctt tccgtttttc     420
   tcatgcaaag ctcctgaagg agactccagt gtggacaaaa cgttcacagt gtctgtccaa     480
   acaccaaaag cacaagagca acaagaagca agcgcttctc aaagccaagc acaatttcat     540
   gtaaggtcct attctagcag caccataaaa gaacatagtg ctaaagaaaa agtctcacaa     600
   agcaccaagt cggcagagac acaaaaacac acacaaacta agtctgatgc gactcttagt     660
   ccgatgtcac tctatagcac cttacataag gaagtacctc aagcactatc ctctacaaaa     720
   agccaacaaa aagatgaaga acatcgagat caaaggcaac aagaaggata cgagcaagaa     780
   caagagcaag aagaaggcaa gaaaaagaca ccatggtgca ctgtggaatc tctacaacag     840
   acttcaagtt cgaaccaggt gtacgagtct tatactccta ttattcctga tcctattgtg     900
   gagtttgctt tgtctgaatc acagctaagc gtgcttgcag gaaagcgtgt gaccaacctt     960
   gatgtcctta gaatatgtac agagctaatg aagttgatgc tcaaaagtag agctaacgac    1020
   acaatgacac gtcttgaaga aagagagctc atggaaaggg aagctcatga attggcggcg    1080
   agttattcac gtcaagctaa atacgcccgc tggctaggga tcgcgacagc aacgttaggt    1140
   attttaggag cgattgctcc tatggttgga gaaatttccg gagatagcat tttagggttt    1200
   gtccaaagga tttctggaag attcaaagat gcgactgcga aaaccttctt taaaggaata    1260
   ggaaaagttt tcacctctct atcacaactt actgaagccg cttctaaagt acatgagtta    1320
   tcagagagtg ctgtacgagc tgttgctgag tacagaaaag aagtcttcag aatgagacag    1380
   gatgaagtca cacgtaccat tgaggaagtc aaagacaact ggaaaagtat ggacaatttc    1440
   cttctgaaca ttctccaaac agaacatgac gctgctcgca gtctgtatca gtag            1494
<210> 76
   <211> 497
   <212> PRT
   <213> Chlamydia pneumoniae

<400> 76
   Met Thr Val Ser Tyr Gln Ser Ile Ser Thr Pro Pro Pro Glu Gly Glu
   1 5 10 15
   Phe Asp Ile Phe Val Asp Gly Asn Ala Thr Glu Glu Ala Val Val Ala
   20 25 30
   Ala Glu Val Gln Val Ala Leu Pro Ala Gly Glu Gln Tyr Ala Met Leu
   35 40 45
   Arg Ala Thr Ser Glu Leu Cys Phe Gly Ile Leu Thr Gln Ser Glu Cys
   50 55 60
   Ala Leu Thr Gln Ala Leu Pro Pro Lys Glu Lys Pro Leu Gln Glu Glu
   65 70 75 80
   Gln Phe Leu Val Lys Asn Gly Ile Leu Met Arg Ser Thr Ser Leu Pro
   85 90 95
   Asn Leu Lys Pro Gly Gln Ser Gln Gln Thr Ser Leu Ala Ser His Arg
   100 105 110
   Asn Pro Leu Ala Gln Gln Ser Thr Ser Ser Asn Ser Thr Gly Lys Ala
   115 120 125
   Ser Thr Glu Thr Thr Ser Ser Ser Phe Pro Phe Phe Ser Cys Lys Ala
   130 135 140
   Pro Glu Gly Asp Ser Ser Val Asp Lys Thr Phe Thr Val Ser Val Gln
   145 150 155 160
   Thr Pro Lys Ala Gln Glu Gln Gln Glu Ala Ser Ala Ser Gln Ser Gln
   165 170 175
   Ala Gln Phe His Val Arg Ser Tyr Ser Ser Ser Thr Ile Lys Glu His
   180 185 190
   Ser Ala Lys Glu Lys Val Ser Gln Ser Thr Lys Ser Ala Glu Thr Gln
   195 200 205
   Lys His Thr Gln Thr Lys Ser Asp Ala Thr Leu Ser Pro Met Ser Leu
   210 215 220
   Tyr Ser Thr Leu His Lys Glu Val Pro Gln Ala Leu Ser Ser Thr Lys
   225 230 235 240
   Ser Gln Gln Lys Asp Glu Glu His Arg Asp Gln Arg Gln Gln Glu Gly
   245 250 255
   Tyr Glu Gln Glu Gln Glu Gln Glu Glu Gly Lys Lys Lys Thr Pro Trp
   260 265 270
   Cys Thr Val Glu Ser Leu Gln Gln Thr Ser Ser Ser Asn Gln Val Tyr
   275 280 285
   Glu Ser Tyr Thr Pro Ile Ile Pro Asp Pro Ile Val Glu Phe Ala Leu
   290 295 300
   Ser Glu Ser Gln Leu Ser Val Leu Ala Gly Lys Arg Val Thr Asn Leu
   305 310 315 320
   Asp Val Leu Arg Ile Cys Thr Glu Leu Met Lys Leu Met Leu Lys Ser
   325 330 335
   Arg Ala Asn Asp Thr Met Thr Arg Leu Glu Glu Arg Glu Leu Met Glu
   340 345 350
   Arg Glu Ala His Glu Leu Ala Ala Ser Tyr Ser Arg Gln Ala Lys Tyr
   355 360 365
   Ala Arg Trp Leu Gly Ile Ala Thr Ala Thr Leu Gly Ile Leu Gly Ala
   370 375 380
   Ile Ala Pro Met Val Gly Glu Ile Ser Gly Asp Ser Ile Leu Gly Phe
   385 390 395 400
   Val Gln Arg Ile Ser Gly Arg Phe Lys Asp Ala Thr Ala Lys Thr Phe
   405 410 415
   Phe Lys Gly Ile Gly Lys Val Phe Thr Ser Leu Ser Gln Leu Thr Glu
   420 425 430
   Ala Ala Ser Lys Val His Glu Leu Ser Glu Ser Ala Val Arg Ala Val
   435 440 445
   Ala Glu Tyr Arg Lys Glu Val Phe Arg Met Arg Gln Asp Glu Val Thr
   450 455 460
   Arg Thr Ile Glu Glu Val Lys Asp Asn Trp Lys Ser Met Asp Asn Phe
   465 470 475 480
   Leu Leu Asn Ile Leu Gln Thr Glu His Asp Ala Ala Arg Ser Leu Tyr
   485 490 495
   Gln
<210> 77
   <211> 1533
   <212> DNA
   <213> Chlamydia pneumoniae
<400> 77
   atgtcctctt ggttatctca agcaagtgag gttcttctca atcaagatcc ttatatccct     60
   gatgctccta ggtcacaaga atcttcggtt cctaagatta gctattctat cactgtagcg    120
   cctcaagaag ctcaaaagtc tcttcccaag tttttcactc agaaatttca gtcacaatgt    180
   aagtctgagc ctcctatcac ccatcataaa acattcatta ttgcaacacc aagagagaga    240
   atcttgcgtt tcggcagctc tttcgaatct caacttcaca acacatcgca agctcaaact    300
   tcatctcctt ggaatctttt ttctcaaaaa aatagcacag aagcaagtaa agctctgatg    360
   caagaactga ctatgccaaa atctccggag aaaacttcag agaaggctct agataaaaac    420
   ctgagttcta aacaagaagg ctcttgcaaa aattttgata cgctgcacct acaacaacat    480
   ctcaagttgt ttggaaccgt tgactcgcta tattctcaaa gcctagatag tgaacagcaa    540
   gaactcctcc aatcacgaag agaagagcgc agtgaaacct atgcaaacca gcagagttct    600
   gagaaaaaaa tagaaaccaa agttcagata aaagatctct gtaaagacct cttttctcag    660
   gatcaggatt ccaatcaaaa acaaaagaaa tcccctttcc aacaagatac atcacgtaaa   720
   aatagaatag ccaaagcagc acaagctgtt cctgtaattc ctcctccaag cataggagtg    780
   tttacattga gctatctact cacaaaacaa gggattcttt cagacttttc ttcgtatgga    840
   tgccacaaag attccgtgga gtcgacacaa cgagagctcg atgctctaca tgaaaagaga    900
   atcgagacta tcaaggtcag catcgagaaa gaaaaacgag aaagattatg gggatctctt    960
   tctgacatta tcggttggct agctccgttt gtttctatag ggatcggcat tgttgctatc   1020
   ttgagtggag gcggtatctt tgcttttgca ggtttttttg cagggctaat ttctcttgtt  1080
   atcaagtgtt tagagaaact aaagttctgg gattggctag aaaaacatct gcctataaaa   1140
   aatgaagaac taagacgaaa aattataacc ataatccagt gggtcgtcta tttaacccct   1200
   gtcattctct ccatatgcac attaaaagta gaaaatttag gtttttcccc tattatagaa   1260
   ggagctatta aaggaatcca acccgcgata gaatctacga tggctgcttt aagatgcgct   1320
   atactgtttt ctcaagcaga aatctataaa ctcaaaggaa aactcactaa aatccaactc   1380
   gacattgaat tgaaaagttt tgatagggac gatcattacg aacgttctca agaactttta   1440
   gataacatgg agagttcttt cgaagccctt tcaagaatct taaattacat gcgtgaacta   1500
   gatcaagtgt atctccactc cttaagagga taa                                1533
<210> 78
   <211> 510
   <212> PRT
   <213> Chlamydia pneumoniae
<400> 78
   Met Ser Ser Trp Leu Ser Gln Ala Ser Glu Val Leu Leu Asn Gln Asp
   1               5                   10                  15
   Pro Tyr Ile Pro Asp Ala Pro Arg Ser Gln Glu Ser Ser Val Pro Lys
               20                  25                  30
   Ile Ser Tyr Ser Ile Thr Val Ala Pro Gln Glu Ala Gln Lys Ser Leu
           35                  40                  45
   Pro Lys Phe Phe Thr Gln Lys Phe Gln Ser Gln Cys Lys Ser Glu Pro
       50                  55                  60
   Pro Ile Thr His His Lys Thr Phe Ile Ile Ala Thr Pro Arg Glu Arg
   65                  70                  75                  80
   Ile Leu Arg Phe Gly Ser Ser Phe Glu Ser Gln Leu His Asn Thr Ser
                   85                  90                  95
   Gln Ala Gln Thr Ser Ser Pro Trp Asn Leu Phe Ser Gln Lys Asn Ser
   100 105 110
   Thr Glu Ala Ser Lys Ala Leu Met Gln Glu Leu Thr Met Pro Lys Ser
   115 120 125
   Pro Glu Lys Thr Ser Glu Lys Ala Leu Asp Lys Asn Leu Ser Ser Lys
   130 135 140
   Gln Glu Gly Ser Cys Lys Asn Phe Asp Thr Leu His Leu Gln Gln His
   145 150 155 160
   Leu Lys Leu Phe Gly Thr Val Asp Ser Leu Tyr Ser Gln Ser Leu Asp
   165 170 175
   Ser Glu Gln Gln Glu Leu Leu Gln Ser Arg Arg Glu Glu Arg Ser Glu
   180 185 190
   Thr Tyr Ala Asn Gln Gln Ser Ser Glu Lys Lys Ile Glu Thr Lys Val
   195 200 205
   Gln Ile Lys Asp Leu Cys Lys Asp Leu Phe Ser Gln Asp Gln Asp Ser
   210 215 220
   Asn Gln Lys Gln Lys Lys Ser Pro Phe Gln Gln Asp Thr Ser Arg Lys
   225 230 235 240
   Asn Arg Ile Ala Lys Ala Ala Gln Ala Val Pro Val Ile Pro Pro Pro
   245 250 255
   Ser Ile Gly Val Phe Thr Leu Ser Tyr Leu Leu Thr Lys Gln Gly Ile
   260 265 270
   Leu Ser Asp Phe Ser Ser Tyr Gly Cys His Lys Asp Ser Val Glu Ser
   275 280 285
   Thr Gln Arg Glu Leu Asp Ala Leu His Glu Lys Arg Ile Glu Thr Ile
   290 295 300
   Lys Val Ser Ile Glu Lys Glu Lys Arg Glu Arg Leu Trp Gly Ser Leu
   305 310 315 320
   Ser Asp Ile Ile Gly Trp Leu Ala Pro Phe Val Ser Ile Gly Ile Gly
   325 330 335
   Ile Val Ala Ile Leu Ser Gly Gly Gly Ile Phe Ala Phe Ala Gly Phe
   340 345 350
   Phe Ala Gly Leu Ile Ser Leu Val Ile Lys Cys Leu Glu Lys Leu Lys
   355 360 365
   Phe Trp Asp Trp Leu Glu Lys His Leu Pro Ile Lys Asn Glu Glu Leu
   370 375 380
   Arg Arg Lys Ile Ile Thr Ile Ile Gln Trp Val Val Tyr Leu Thr Pro
   385 390 395 400
   Val Ile Leu Ser Ile Cys Thr Leu Lys Val Glu Asn Leu Gly Phe Ser
   405 410 415
   Pro Ile Ile Glu Gly Ala Ile Lys Gly Ile Gln Pro Ala Ile Glu Ser
   420 425 430
   Thr Met Ala Ala Leu Arg Cys Ala Ile Leu Phe Ser Gln Ala Glu Ile
   435 440 445
   Tyr Lys Leu Lys Gly Lys Leu Thr Lys Ile Gln Leu Asp Ile Glu Leu
   450 455 460
   Lys Ser Phe Asp Arg Asp Asp His Tyr Glu Arg Ser Gln Glu Leu Leu
   465 470 475 480
   Asp Asn Met Glu Ser Ser Phe Glu Ala Leu Ser Arg Ile Leu Asn Tyr
   485 490 495
   Met Arg Glu Leu Asp Gln Val Tyr Leu His Ser Leu Arg Gly
   500 505 510
<210> 79
   <211> 588
   <212> DNA
   <213> Chlamydia pneumoniae
<400> 79
   atggcttacg gaactcgtta tcccacgcta gcattccata cagggggcat tggtgaatct 60
   gatgacggta tgcccccaca accctttgaa actttctgct acgattctgc tcttctacaa 120
   gcaaaaatcg aaaattttaa tatcgtccct tatacatctg tacttcctaa agagctcttt 180
   gggaatatcg ttcctgtaga tacctgtgta aaatctttca aacacggcgc tgtccttgaa 240
   gtaatcatgg cgggtcgtgg tgctgctctc tctgacggaa cccatgcgat cgccaccgga 300
   atcggcattt gctggggtaa agataaaaat ggagagctta ttggcggatg ggcagcagaa 360
   tatgttgaat tcttccctac atggataaat gacgaaattg cagaaaccca tgctaaaatg 420
   tggttgaaaa aatccctaca acacgaactt gacctacgct ctatagcaaa gcatagcgaa 480
   ttccaattct tccataatta catcaacatc aaacaaaaat tcggtttttg cttaactgca 540
   ttaggattcc taaatttcga aaatgctgaa ccagctaagg taaattaa 588
<210> 80
   <211> 195
   <212> PRT
   <213> Chlamydia pneumoniae
<400> 80
   Met Ala Tyr Gly Thr Arg Tyr Pro Thr Leu Ala Phe His Thr Gly Gly
   1 5 10 15
   Ile Gly Glu Ser Asp Asp Gly Met Pro Pro Gln Pro Phe Glu Thr Phe
   20 25 30
   Cys Tyr Asp Ser Ala Leu Leu Gln Ala Lys Ile Glu Asn Phe Asn Ile
   35 40 45
   Val Pro Tyr Thr Ser Val Leu Pro Lys Glu Leu Phe Gly Asn Ile Val
   50 55 60
   Pro Val Asp Thr Cys Val Lys Ser Phe Lys His Gly Ala Val Leu Glu
   65 70 75 80
   Val Ile Met Ala Gly Arg Gly Ala Ala Leu Ser Asp Gly Thr His Ala
   85 90 95
   Ile Ala Thr Gly Ile Gly Ile Cys Trp Gly Lys Asp Lys Asn Gly Glu
   100 105 110
   Leu Ile Gly Gly Trp Ala Ala Glu Tyr Val Glu Phe Phe Pro Thr Trp
   115 120 125
   Ile Asn Asp Glu Ile Ala Glu Thr His Ala Lys Met Trp Leu Lys Lys
   130 135 140
   Ser Leu Gln His Glu Leu Asp Leu Arg Ser Ile Ala Lys His Ser Glu
   145 150 155 160
   Phe Gln Phe Phe His Asn Tyr Ile Asn Ile Lys Gln Lys Phe Gly Phe
   165 170 175
   Cys Leu Thr Ala Leu Gly Phe Leu Asn Phe Glu Asn Ala Glu Pro Ala
   180 185 190
   Lys Val Asn
   195
<210> 81
   <211> 1092
   <212> DNA
   <213> Chlamydia pneumoniae
<400> 81
   atgaaattat atcagacctt gcgaggtatt gttttagtaa gtacgggatg catattctta      60
   ggaatgcacg gaggatatgc cgctgaggtt ccagtgactt catctgggta tgagaatctt     120
   ttagaatcta aggaacagga tccttcaggt ctagcgatcc acgatcgcat tttgtttaag     180
   gtagatgaag agaatgtagt gactgcctta gatgtgatcc ataaattaaa cttactattt     240
   tataattcgt atcctcatct tatagattct ttccctgcac gatcccagta ctatactgcg     300
   atgtggcctg tggttcttga atctgtgatt gatgagtttt tgatggtggc agatgccaag     360
   gcaaagagaa tcgctacaga tcccaccgca gtgaatcaag aaatcgaaga gatgttcgga     420
   agagatctct ctcctttgta tgcgcatttt gaaatgagtc ccaacgatat ttttaatgtg     480
   atcgatcgca ctttgacagc acaaagggtg atgggtatga tggtgcgctc taaggtaatg     540
   ttgaaggtga ctccagggaa aattcgagaa tattaccgaa agctagaaga agaagcctct     600
   aggaaagtca tctggaagta tcgtgtgttg acgattaaag ccaacacaga atccttggct     660
   agccagattg ctgataaagt gcgtgctcgt ctaaatgaag cgaaaacctg ggataaagat     720
   cgtttaactg ctcttgtgat ctctcaggga gggcaactcg tctgctccga agagttttct     780
   cgagagaata gtgagctctc ccaaagccat aagcaagagc tggacttgat tggctatcct     840
   aaagagctct gtgggttgcc taaggcacat aagtcaggat ataaactcta tatgttgtta     900
   gacaaaacct caggttctat agagccttta gatgttatgg agtccaagat caaacagcat     960
   ctttttgctt tagaagctga gagtgtagag aaacaatata aagacagatt acgcaagcgc    1020
   tacggctatg atgcttctat gattgcgaaa cttctttctg aagaagctcc acctctattt    1080
   tccttattat ag                                                        1092
<210> 82
   <211> 363
   <212> PRT
   <213> Chlamydia pneumoniae
<400> 82
   Met Lys Leu Tyr Gln Thr Leu Arg Gly Ile Val Leu Val Ser Thr Gly
   1               5                   10                  15
   Cys Ile Phe Leu Gly Met His Gly Gly Tyr Ala Ala Glu Val Pro Val
               20                  25                  30
   Thr Ser Ser Gly Tyr Glu Asn Leu Leu Glu Ser Lys Glu Gln Asp Pro
           35                  40                  45
   Ser Gly Leu Ala Ile His Asp Arg Ile Leu Phe Lys Val Asp Glu Glu
       50                  55                  60
   Asn Val Val Thr Ala Leu Asp Val Ile His Lys Leu Asn Leu Leu Phe
   65                  70                  75                  80
   Tyr Asn Ser Tyr Pro His Leu Ile Asp Ser Phe Pro Ala Arg Ser Gln
                   85                  90                  95
   Tyr Tyr Thr Ala Met Trp Pro Val Val Leu Glu Ser Val Ile Asp Glu
               100                 105                 110
   Phe Leu Met Val Ala Asp Ala Lys Ala Lys Arg Ile Ala Thr Asp Pro
   115 120 125
   Thr Ala Val Asn Gln Glu Ile Glu Glu Met Phe Gly Arg Asp Leu Ser
   130 135 140
   Pro Leu Tyr Ala His Phe Glu Met Ser Pro Asn Asp Ile Phe Asn Val
   145 150 155 160
   Ile Asp Arg Thr Leu Thr Ala Gln Arg Val Met Gly Met Met Val Arg
   165 170 175
   Ser Lys Val Met Leu Lys Val Thr Pro Gly Lys Ile Arg Glu Tyr Tyr
   180 185 190
   Arg Lys Leu Glu Glu Glu Ala Ser Arg Lys Val Ile Trp Lys Tyr Arg
   195 200 205
   Val Leu Thr Ile Lys Ala Asn Thr Glu Ser Leu Ala Ser Gln Ile Ala
   210 215 220
   Asp Lys Val Arg Ala Arg Leu Asn Glu Ala Lys Thr Trp Asp Lys Asp
   225 230 235 240
   Arg Leu Thr Ala Leu Val Ile Ser Gln Gly Gly Gln Leu Val Cys Ser
   245 250 255
   Glu Glu Phe Ser Arg Glu Asn Ser Glu Leu Ser Gln Ser His Lys Gln
   260 265 270
   Glu Leu Asp Leu Ile Gly Tyr Pro Lys Glu Leu Cys Gly Leu Pro Lys
   275 280 285
   Ala His Lys Ser Gly Tyr Lys Leu Tyr Met Leu Leu Asp Lys Thr Ser
   290 295 300
   Gly Ser Ile Glu Pro Leu Asp Val Met Glu Ser Lys Ile Lys Gln His
   305 310 315 320
   Leu Phe Ala Leu Glu Ala Glu Ser Val Glu Lys Gln Tyr Lys Asp Arg
   325 330 335
   Leu Arg Lys Arg Tyr Gly Tyr Asp Ala Ser Met Ile Ala Lys Leu Leu
   340 345 350
   Ser Glu Glu Ala Pro Pro Leu Phe Ser Leu Leu
   355 360
<210> 83
   <211> 2076
   <212> DNA
   <213> Chlamydia trachomatis
<400> 83
   atgacgctct ttcattctca tcatgatgcc gtctctccag acagctacct atgttcttcc     60
   cttcagttag ttggtactgg cgtatacgaa ggagaaatcg agattcaaaa tatcccctct    120
   tatttccttg gattccaatt accctctcat tgcatacacc ttaatttaaa gagctctcta    180
   gctcaattag gaatagatgc ctcccttctt cactgcgaat tgagcaaaaa tcaacatcga    240
   gcacatatac atgctcaatt taccggtcat ggccccattg ctgaatctat gctagccctt    300
   ctccaaccag gagatcgtgt agcaaaacta tttgctgcag acgatcgcag actggtccga    360
   tctccagatt acctcgaaag catgctgaaa aatacagata aagctggcca tcctttgctc    420
   tgttttggga aaaaattaga acacttgatt tcttttgatg tggtagatga tcgccttgtc    480
   gtctcccttc ctaccctgcc gggagttgtt cgttatgatt cggatattta tggactcctt    540
   cctcttattc aaaaatcact cagtaatccc aaactcagca ttcgtcactt tttagctctg    600
   taccaacaga ttgtggaagg gcaacatgtc tcttgcggaa accatattct tctgatcaaa    660
   acagaaccgc tgcacatccg cactgtattt gctcgcgtgg taaatcaact cctccctcaa    720
   ggtctctccc acacttctgc caatattttg gaaccaacca ctcgagaatc cggggatatc    780
   tttgaatttt ttgggaaccc ttctgcacag atagaaagaa ttcctttaga atttttcact    840
   atcgaaccct ataaagaaca ttcttacttc tgtaatcggg atttattaca aaccatctta    900
   caatcagaaa gcgaaatcaa aaaaatattc gaaacagcgc ccaaagaacc tgtcaaagct    960
   gccacctatt tatcaaaagg cagtgaaatc tcttccctgc acacagactc ttggctcaca   1020
   ggatccgcag ctgcctatca atatagtgag caagcagata aaaacgagta cactcatgct   1080
   caaccttgct atcctttctt agaagcaatg gaaatgggcc tgatcaatag cgaaggagcc   1140
   ttactcactc gttatttccc ttcagctagc ttaaaaggaa tgttgatttc ctaccatgtg   1200
   cgccactatc tcaaacaaat ctactttcaa gttccctctt atacacatgg aaactatttc   1260
   tctcataatg acagaggttt gctattagat ctgcagcaag cagatattga tgttttctgg   1320
   gcagatgaag aaagcggccg tgtgttgcaa tatacaaaac gacgcgataa gaatagcggt   1380
   atgttcgtga tcaaaaatcg tgttgaagag tttcgatcag cttattttat tgctatttat   1440
   ggctctcgtc tccttgagaa taatttctct gctcagctcc ataccctcct agcgggctta   1500
   cagcaagcag cacatactct cggcattcct ggattctcaa agcctacccc acttgcagtc   1560
   atcaccggag gcggcactgg agttatggcc acaggaaatc gtgtagctaa agaactagga   1620
   atcctatctt gtggaaccgt tcttgattta gaagcttctc cagcacaaat cgaccaacct   1680
   accaatgaat tcttagatgc taaaatgaca taccgcctac ctcaacttat agaaaggcaa   1740
   gaacactttt atgcagacct tcctatcctt gtagttggcg gtgtaggaac cgatttcgaa   1800
   ctctacctag aacttgtcta tctcaaaaca ggagctaaac caccgactcc cattttccta   1860
   attggaccta ttgaatactg gaaagaaaaa gtggcccacg cctacgagat caacctcaaa   1920
   gcaggaacca tccgtggatc cgaatggatc agcaactgcc tatattgtat cacttctccg   1980
   gaagctggaa ttgccgtatt cgaacaattc ctagctggag aactccctat aggatacgac   2040
   tatcctccag ctccagatgg attagtgatc gtctaa 2076
<210> 84
   <211> 691
   <212> PRT
   <213> Chlamydia trachomatis
<400> 84
   Met Thr Leu Phe His Ser His His Asp Ala Val Ser Pro Asp Ser Tyr
   1 5 10 15
   Leu Cys Ser Ser Leu Gln Leu Val Gly Thr Gly Val Tyr Glu Gly Glu
   20 25 30
   Ile Glu Ile Gln Asn Ile Pro Ser Tyr Phe Leu Gly Phe Gln Leu Pro
   35 40 45
   Ser His Cys Ile His Leu Asn Leu Lys Ser Ser Leu Ala Gln Leu Gly
   50 55 60
   Ile Asp Ala Ser Leu Leu His Cys Glu Leu Ser Lys Asn Gln His Arg
   65 70 75 80
   Ala His Ile His Ala Gln Phe Thr Gly His Gly Pro Ile Ala Glu Ser
   85 90 95
   Met Leu Ala Leu Leu Gln Pro Gly Asp Arg Val Ala Lys Leu Phe Ala
   100 105 110
   Ala Asp Asp Arg Arg Leu Val Arg Ser Pro Asp Tyr Leu Glu Ser Met
   115 120 125
   Leu Lys Asn Thr Asp Lys Ala Gly His Pro Leu Leu Cys Phe Gly Lys
   130 135 140
   Lys Leu Glu His Leu Ile Ser Phe Asp Val Val Asp Asp Arg Leu Val
   145 150 155 160
   Val Ser Leu Pro Thr Leu Pro Gly Val Val Arg Tyr Asp Ser Asp Ile
   165 170 175
   Tyr Gly Leu Leu Pro Leu Ile Gln Lys Ser Leu Ser Asn Pro Lys Leu
   180 185 190
   Ser Ile Arg His Phe Leu Ala Leu Tyr Gln Gln Ile Val Glu Gly Gln
   195 200 205
   His Val Ser Cys Gly Asn His Ile Leu Leu Ile Lys Thr Glu Pro Leu
   210 215 220
   His Ile Arg Thr Val Phe Ala Arg Val Val Asn Gln Leu Leu Pro Gln
   225 230 235 240
   Gly Leu Ser His Thr Ser Ala Asn Ile Leu Glu Pro Thr Thr Arg Glu
   245 250 255
   Ser Gly Asp Ile Phe Glu Phe Phe Gly Asn Pro Ser Ala Gln Ile Glu
   260 265 270
   Arg Ile Pro Leu Glu Phe Phe Thr Ile Glu Pro Tyr Lys Glu His Ser
   275 280 285
   Tyr Phe Cys Asn Arg Asp Leu Leu Gln Thr Ile Leu Gln Ser Glu Ser
   290 295 300
   Glu Ile Lys Lys Ile Phe Glu Thr Ala Pro Lys Glu Pro Val Lys Ala
   305 310 315 320
   Ala Thr Tyr Leu Ser Lys Gly Ser Glu Ile Ser Ser Leu His Thr Asp
   325 330 335
   Ser Trp Leu Thr Gly Ser Ala Ala Ala Tyr Gln Tyr Ser Glu Gln Ala
   340 345 350
   Asp Lys Asn Glu Tyr Thr His Ala Gln Pro Cys Tyr Pro Phe Leu Glu
   355 360 365
   Ala Met Glu Met Gly Leu Ile Asn Ser Glu Gly Ala Leu Leu Thr Arg
   370 375 380
   Tyr Phe Pro Ser Ala Ser Leu Lys Gly Met Leu Ile Ser Tyr His Val
   385 390 395 400
   Arg His Tyr Leu Lys Gln Ile Tyr Phe Gln Val Pro Ser Tyr Thr His
   405 410 415
   Gly Asn Tyr Phe Ser His Asn Asp Arg Gly Leu Leu Leu Asp Leu Gln
   420 425 430
   Gln Ala Asp Ile Asp Val Phe Trp Ala Asp Glu Glu Ser Gly Arg Val
   435 440 445
   Leu Gln Tyr Thr Lys Arg Arg Asp Lys Asn Ser Gly Met Phe Val Ile
   450 455 460
   Lys Asn Arg Val Glu Glu Phe Arg Ser Ala Tyr Phe Ile Ala Ile Tyr
   465 470 475 480
   Gly Ser Arg Leu Leu Glu Asn Asn Phe Ser Ala Gln Leu His Thr Leu
   485 490 495
   Leu Ala Gly Leu Gln Gln Ala Ala His Thr Leu Gly Ile Pro Gly Phe
   500 505 510
   Ser Lys Pro Thr Pro Leu Ala Val Ile Thr Gly Gly Gly Thr Gly Val
   515 520 525
   Met Ala Thr Gly Asn Arg Val Ala Lys Glu Leu Gly Ile Leu Ser Cys
   530 535 540
   Gly Thr Val Leu Asp Leu Glu Ala Ser Pro Ala Gln Ile Asp Gln Pro
   545 550 555 560
   Thr Asn Glu Phe Leu Asp Ala Lys Met Thr Tyr Arg Leu Pro Gln Leu
   565 570 575
   Ile Glu Arg Gln Glu His Phe Tyr Ala Asp Leu Pro Ile Leu Val Val
   580 585 590
   Gly Gly Val Gly Thr Asp Phe Glu Leu Tyr Leu Glu Leu Val Tyr Leu
   595 600 605
   Lys Thr Gly Ala Lys Pro Pro Thr Pro Ile Phe Leu Ile Gly Pro Ile
   610 615 620
   Glu Tyr Trp Lys Glu Lys Val Ala His Ala Tyr Glu Ile Asn Leu Lys
   625 630 635 640
   Ala Gly Thr Ile Arg Gly Ser Glu Trp Ile Ser Asn Cys Leu Tyr Cys
   645 650 655
   Ile Thr Ser Pro Glu Ala Gly Ile Ala Val Phe Glu Gln Phe Leu Ala
   660 665 670
   Gly Glu Leu Pro Ile Gly Tyr Asp Tyr Pro Pro Ala Pro Asp Gly Leu
   675 680 685
   Val Ile Val
   690
<210> 85
   <211> 966
   <212> DNA
   <213> Chlamydia trachomatis
<400> 85
   atgaagcgtt tattttttat ctgcgccctc gccctttctc ctctagcata tggagctgtt     60
   caaaaggatc ctatgttaat gaaggagact ttccgtaata actacgggat cattgtctct    120
   aagcaagaat ggaacaaacg tggatgcgat ggctccatca ctagagtatt caaagatgga    180
   actacaacct tagaagttta tgcgcaaggt gctttacatg gggaagtcac acgaacgttt    240
   cctcactcta ctaccctggc cgttatagaa acttatgatc agggaaggct tctttctaag    300
   aagaccttct tcccaaatgc tttgcctgct aaagaagaag tttaccacga agatgggtct    360
   ttctccctaa cacgttggcc tgacaataac aactctgaca caatcacaga cccctgcttt    420
   gtagaaaaaa cttatggggg aagagtattg gaaggtcatt acacctcttt taatggaaaa    480
   tactcttcaa caatccttaa cggcgaggga gttcgctcta ctttttcttc ggatagtatc    540
   ttgttgacag aagagtcgtt taatgatggc gtaatggtca aaaaaacgac attttactcg    600
   actcgagaac ccgaaaccgt cactcattat gtcaatgggt accctcacgg agttcggttt    660
   acctatcttc ctggtgggat tccaaatacg attgaagaat ggcgatatgg acatcaagac    720
   ggccttacaa tcttatttaa aaatggttgt aagattgctg aagtcccatt tgtacgcgga    780
   gcaaaaaatg gaatcgaact ccgatacaat gaacaagaga atatcgctga agagatttct    840
   tggcagcaca acatcttgca tggagtccgt aaaatccatg cggcgggggt atgcaaatcc    900
   gaatggtatt acaaaggcaa acctgtctcg caaatcaagt ttgaacgact cagcgctgcc    960
   agataa                                                               966
<210> 86
   <211> 321
   <212> PRT
   <213> Chlamydia trachomatis
<400> 86
   Met Lys Arg Leu Phe Phe Ile Cys Ala Leu Ala Leu Ser Pro Leu Ala
   1               5                   10                  15
   Tyr Gly Ala Val Gln Lys Asp Pro Met Leu Met Lys Glu Thr Phe Arg
               20                  25                  30
   Asn Asn Tyr Gly Ile Ile Val Ser Lys Gln Glu Trp Asn Lys Arg Gly
           35                  40                  45
   Cys Asp Gly Ser Ile Thr Arg Val Phe Lys Asp Gly Thr Thr Thr Leu
       50                  55                  60
   Glu Val Tyr Ala Gln Gly Ala Leu His Gly Glu Val Thr Arg Thr Phe
   65                  70                  75                  80
   Pro His Ser Thr Thr Leu Ala Val Ile Glu Thr Tyr Asp Gln Gly Arg
                   85                  90                  95
   Leu Leu Ser Lys Lys Thr Phe Phe Pro Asn Ala Leu Pro Ala Lys Glu
               100                 105                 110
   Glu Val Tyr His Glu Asp Gly Ser Phe Ser Leu Thr Arg Trp Pro Asp
           115                 120                 125
   Asn Asn Asn Ser Asp Thr Ile Thr Asp Pro Cys Phe Val Glu Lys Thr
       130                 135                 140
   Tyr Gly Gly Arg Val Leu Glu Gly His Tyr Thr Ser Phe Asn Gly Lys
   145                 150                 155                 160
   Tyr Ser Ser Thr Ile Leu Asn Gly Glu Gly Val Arg Ser Thr Phe Ser
                   165                 170                 175
   Ser Asp Ser Ile Leu Leu Thr Glu Glu Ser Phe Asn Asp Gly Val Met
               180                 185                 190
   Val Lys Lys Thr Thr Phe Tyr Ser Thr Arg Glu Pro Glu Thr Val Thr
           195                 200                 205
   His Tyr Val Asn Gly Tyr Pro His Gly Val Arg Phe Thr Tyr Leu Pro
       210                 215                 220
   Gly Gly Ile Pro Asn Thr Ile Glu Glu Trp Arg Tyr Gly His Gln Asp
   225                 230                 235                 240
   Gly Leu Thr Ile Leu Phe Lys Asn Gly Cys Lys Ile Ala Glu Val Pro
                   245                 250                 255
   Phe Val Arg Gly Ala Lys Asn Gly Ile Glu Leu Arg Tyr Asn Glu Gln
               260                 265                 270
   Glu Asn Ile Ala Glu Glu Ile Ser Trp Gln His Asn Ile Leu His Gly
           275                 280                 285
   Val Arg Lys Ile His Ala Ala Gly Val Cys Lys Ser Glu Trp Tyr Tyr
       290                 295                 300
   Lys Gly Lys Pro Val Ser Gln Ile Lys Phe Glu Arg Leu Ser Ala Ala
   305                 310                 315                 320
   Arg
<210> 87
   <211> 426
   <212> DNA
   <213> Chlamydia trachomatis
<400> 87
   gtgagtttag attttttaga ggattttttc cgtcgctcaa ttaccaatca caacacagct   60
   tttccagaag gctttctgga tatatctgat gtcttagctc gttcggcttt agattttaag   120
   gctgaggaac ttgctgacag tgctgttaat gacttcatcg tatcagaatc ttcagataaa   180
   ctcactttat ttaacacaaa ttttgctgtg tggttggtac ctacattagt tgatggtgag   240
   gcaattactc gcggctacat cgctttaaac cagggtgaag agttctctcc tgaattagct   300
   tttgaagcat caggaaagta taataattcg agtttaatct tagaggcgtt gcgaagatat   360
   ttgtgtgata tccaggatac agaaaaagaa ttacgggcat tacgcccgcc ttcaatagat   420
   ggatag                                                              426
<210> 88
   <211> 141
   <212> PRT
   <213> Chlamydia trachomatis
<400> 88
   Val Ser Leu Asp Phe Leu Glu Asp Phe Phe Arg Arg Ser Ile Thr Asn
   1               5                   10                  15
   His Asn Thr Ala Phe Pro Glu Gly Phe Leu Asp Ile Ser Asp Val Leu
               20                  25                  30
   Ala Arg Ser Ala Leu Asp Phe Lys Ala Glu Glu Leu Ala Asp Ser Ala
           35                  40                  45
   Val Asn Asp Phe Ile Val Ser Glu Ser Ser Asp Lys Leu Thr Leu Phe
       50                  55                  60
   Asn Thr Asn Phe Ala Val Trp Leu Val Pro Thr Leu Val Asp Gly Glu
   65                  70                  75                  80
   Ala Ile Thr Arg Gly Tyr Ile Ala Leu Asn Gln Gly Glu Glu Phe Ser
                   85                  90                  95
   Pro Glu Leu Ala Phe Glu Ala Ser Gly Lys Tyr Asn Asn Ser Ser Leu
               100                 105                 110
   Ile Leu Glu Ala Leu Arg Arg Tyr Leu Cys Asp Ile Gln Asp Thr Glu
           115                 120                 125
   Lys Glu Leu Arg Ala Leu Arg Pro Pro Ser Ile Asp Gly
       130                 135                 140
<210> 89
   <211> 240
   <212> DNA
   <213> Chlamydia trachomatis
<400> 89
   ttggaagata gaatgatcga cgggattcag acgtgttctt ttagccctac gcatcgctta    60
   actgcgaaat ccgcagtgag tatagagatg cctttagcaa cacaaaatct tcaagaggga   120
   gctttggtca atgcaaagct cgaagcggat ttcgcgagag cagagcagat tcttacagag   180
   atgcaagaaa tccgttctag tttagagagg tctttagaga ctctctttcc ccgcgagtaa   240
<210> 90
   <211> 79
   <212> PRT
   <213> Chlamydia trachomatis
<400> 90
   Leu Glu Asp Arg Met Ile Asp Gly Ile Gln Thr Cys Ser Phe Ser Pro
   1               5                   10                  15
   Thr His Arg Leu Thr Ala Lys Ser Ala Val Ser Ile Glu Met Pro Leu
               20                  25                  30
   Ala Thr Gln Asn Leu Gln Glu Gly Ala Leu Val Asn Ala Lys Leu Glu
           35                  40                  45
   Ala Asp Phe Ala Arg Ala Glu Gln Ile Leu Thr Glu Met Gln Glu Ile
       50                  55                  60
   Arg Ser Ser Leu Glu Arg Ser Leu Glu Thr Leu Phe Pro Arg Glu
   65                  70                  75
<210> 91
   <211> 696
   <212> DNA
   <213> Chlamydia trachomatis
<400> 91
   atgatggagg tgtttatgaa ttttttagat cagttagatt taattattca aaataagcat    60
   atgctagaac acacatttta tgtgaaatgg tcgaaggggg agcttactaa agagcaatta   120
   caggcgtatg ccaaagacta ttatttacat atcaaagcct ttcctaaata tttatctgcg   180
   attcatagtc gttgcgatga tttagaggcg cgtaagttat tgttagataa cttgatggat   240
   gaagagaacg gttaccctaa tcatattgat ttgtggaagc agtttgtgtt tgctctagga   300
   gttactccag aagagttaga ggctcatgag cctagtgaag cagcaaaagc gaaagtagct   360
   actttcatgc ggtggtgtac aggagattct ttagctgcag gagtggctgc tttgtattct   420
   tatgagagtc aaattccacg tatcgctaga gagaaaattc gtggattgac tgagtacttt   480
   ggattttcca atcctgaaga ctatgcatat ttcacagaac atgaagaagc ggatgtgcgg   540
   catgctagag aagaaaaagc gctcattgag atgcttctca aagatgacgc tgataaagtg   600
   ttagaggcat cgcaagaagt aacgcaatct ttgtatggct ttttagattc ttttttggat   660
   ccaggaactt gttgtagttg tcatcaatct tattaa                              696
<210> 92
   <211> 231
   <212> PRT
   <213> Chlamydia trachomatis
<400> 92
   Met Met Glu Val Phe Met Asn Phe Leu Asp Gln Leu Asp Leu Ile Ile
   1               5                   10                  15
   Gln Asn Lys His Met Leu Glu His Thr Phe Tyr Val Lys Trp Ser Lys
               20                  25                  30
   Gly Glu Leu Thr Lys Glu Gln Leu Gln Ala Tyr Ala Lys Asp Tyr Tyr
           35                  40                  45
   Leu His Ile Lys Ala Phe Pro Lys Tyr Leu Ser Ala Ile His Ser Arg
       50                  55                  60
   Cys Asp Asp Leu Glu Ala Arg Lys Leu Leu Leu Asp Asn Leu Met Asp
   65                  70                  75                  80
   Glu Glu Asn Gly Tyr Pro Asn His Ile Asp Leu Trp Lys Gln Phe Val
                   85                  90                  95
   Phe Ala Leu Gly Val Thr Pro Glu Glu Leu Glu Ala His Glu Pro Ser
               100                 105                 110
   Glu Ala Ala Lys Ala Lys Val Ala Thr Phe Met Arg Trp Cys Thr Gly
           115                 120                 125
   Asp Ser Leu Ala Ala Gly Val Ala Ala Leu Tyr Ser Tyr Glu Ser Gln
       130                 135                 140
   Ile Pro Arg Ile Ala Arg Glu Lys Ile Arg Gly Leu Thr Glu Tyr Phe
   145                 150                 155                 160
   Gly Phe Ser Asn Pro Glu Asp Tyr Ala Tyr Phe Thr Glu His Glu Glu
                   165                 170                 175
   Ala Asp Val Arg His Ala Arg Glu Glu Lys Ala Leu Ile Glu Met Leu
               180                 185                 190
   Leu Lys Asp Asp Ala Asp Lys Val Leu Glu Ala Ser Gln Glu Val Thr
           195                 200                 205
   Gln Ser Leu Tyr Gly Phe Leu Asp Ser Phe Leu Asp Pro Gly Thr Cys
       210                 215                 220
   Cys Ser Cys His Gln Ser Tyr
   225                 230
<210> 93
   <211> 816
   <212> DNA
   <213> Chlamydia trachomatis
<400> 93
   ttgagagcag ccttgaatac acttgaattt ctttcttctc ccccctcttc ggatccttac 60
   gatgatttat tacaattaaa caaagaagga ttccttgctg gccctgaaga agaaaaacaa 120
   gctttttttc ttcgggtaga aaggacatta gcagaagctc ctgtacatcc cacccctttt 180
   cccatagaat tacagaaact cttcgatgtg aacccttctt ttttagaggt tgtgtactct 240
   aatgaaagtt tagatgcctg ggaagcagga tgtacatgga tcaccgataa cagagtatcg 300
   attcaactac gcaaacgttt tcaaaaagct tctttctggt ttggtttttt ttccaaagaa 360
   gaagtgctgt ctcacgaagc tgttcatgct gtgcgtatga aattttatga accgatcttt 420
   gaagaggtct tggcatacag cacttctaaa cacttttgga gacgcttttt tggtcccctg 480
   ttccgatcag cagaagaaac gcatttcttt ctgtttttcg ttttatttgg agcgttttta 540
   ttcccttggt ttccttggat aggcctttct tgtattcttg ctcctaatat gttctttttt 600
   tttcgcttat tccgaacaca aatcctattt cgtaaagcaa agaaaaaaat tcgaaaactt 660
   ttaggtatag aacctctctg ggtcttacta cgcttaacag atagagaaat tcgcctattt 720
   gctacacagc ccttagctgt gatagaagac ttcgctagga aagaaaagct gaaaagtgtg 780
   cgctggaggc aaatctatca aagttacttc acctaa 816
<210> 94
   <211> 271
   <212> PRT
   <213> Chlamydia trachomatis
<400> 94
   Leu Arg Ala Ala Leu Asn Thr Leu Glu Phe Leu Ser Ser Pro Pro Ser
   1 5 10 15
   Ser Asp Pro Tyr Asp Asp Leu Leu Gln Leu Asn Lys Glu Gly Phe Leu
   20 25 30
   Ala Gly Pro Glu Glu Glu Lys Gln Ala Phe Phe Leu Arg Val Glu Arg
   35 40 45
   Thr Leu Ala Glu Ala Pro Val His Pro Thr Pro Phe Pro Ile Glu Leu
   50 55 60
   Gln Lys Leu Phe Asp Val Asn Pro Ser Phe Leu Glu Val Val Tyr Ser
   65 70 75 80
   Asn Glu Ser Leu Asp Ala Trp Glu Ala Gly Cys Thr Trp Ile Thr Asp
   85 90 95
   Asn Arg Val Ser Ile Gln Leu Arg Lys Arg Phe Gln Lys Ala Ser Phe
   100 105 110
   Trp Phe Gly Phe Phe Ser Lys Glu Glu Val Leu Ser His Glu Ala Val
   115 120 125
   His Ala Val Arg Met Lys Phe Tyr Glu Pro Ile Phe Glu Glu Val Leu
   130 135 140
   Ala Tyr Ser Thr Ser Lys His Phe Trp Arg Arg Phe Phe Gly Pro Leu
   145 150 155 160
   Phe Arg Ser Ala Glu Glu Thr His Phe Phe Leu Phe Phe Val Leu Phe
   165 170 175
   Gly Ala Phe Leu Phe Pro Trp Phe Pro Trp Ile Gly Leu Ser Cys Ile
   180 185 190
   Leu Ala Pro Asn Met Phe Phe Phe Phe Arg Leu Phe Arg Thr Gln Ile
   195 200 205
   Leu Phe Arg Lys Ala Lys Lys Lys Ile Arg Lys Leu Leu Gly Ile Glu
   210 215 220
   Pro Leu Trp Val Leu Leu Arg Leu Thr Asp Arg Glu Ile Arg Leu Phe
   225 230 235 240
   Ala Thr Gln Pro Leu Ala Val Ile Glu Asp Phe Ala Arg Lys Glu Lys
   245 250 255
   Leu Lys Ser Val Arg Trp Arg Gln Ile Tyr Gln Ser Tyr Phe Thr
   260 265 270
<210> 95
   <211> 180
   <212> DNA
   <213> Chlamydia trachomatis
<400> 95
   atggaccagt tatcacagat acatcaagag ctagctcgtt tagaatttat caatgatcaa 60
   ctgcagtcgg aaagagcgta catccatgat ttattgtgtg cgataggctt tcctgaaggg 120
   ttaaagacga tagccgcgat agctaacgaa gtgctttccg aagaagattc ccaaggttaa 180
<210> 96
   <211> 59
   <212> PRT
   <213> Chlamydia trachomatis
<400> 96
   Met Asp Gln Leu Ser Gln Ile His Gln Glu Leu Ala Arg Leu Glu Phe
   1 5 10 15
   Ile Asn Asp Gln Leu Gln Ser Glu Arg Ala Tyr Ile His Asp Leu Leu
   20 25 30
   Cys Ala Ile Gly Phe Pro Glu Gly Leu Lys Thr Ile Ala Ala Ile Ala
   35 40 45
   Asn Glu Val Leu Ser Glu Glu Asp Ser Gln Gly
   50 55
<210> 97
   <211> 2490
   <212> DNA
   <213> Chlamydia trachomatis
<400> 97
   atgggtatac gcttagttat tgataaaggg cccttgtctg gaactgttct tattttagaa 60
   aatgggacga gttggtctct tggcagtgat ggaaaagcta gtgatattct cctgcaagat 120
   gaaaagcttg ctccctctca gattcgcatc actttaaaag atggcgagta ttatttagaa 180
   aatttagatg ctttgaggcc ggtttctgtt gatggaacag ttatcactgc ccctgttttg 240
   ttaaaagatg gggtttcctt tgtaatggga agctgccaag tctcgttttt taaaggggaa 300
   gaggtagaag gagatataga gttatcgttc cagacagaag gtggtaatga gggagagcct 360
   gcagcgcaag gctcttcaag cgtttcgtcc gaagctccta aaaaggagac agggaatcca 420
   agtcttcctt cggaggcaaa ggcttctgga gaagtatcta gttcagcaat agcgaaagaa 480
   caagagttag cggcgtcctt tttagcttct gttgagaagg agcctggaac accaaaagaa 540
   gtctctgagc caaaggtctc ttcacaagaa ggacagactc cttctgttac aggagaaaaa 600
   aaggatcttg agcttccttt ggcaagtcaa gaacaaccta aacaaactac tccatcaggc 660
   agtggtgaac caacccaatc tcaaaacgcg agtatggaag aaaacagaac gtcgcccgat 720
   caaaatcagc agccacagct ttcttctgct tcagaatcgg gttctcaaag tcccgaaaat 780
   caggagcaac aaccttctca aacgcctccc ccatccccgg aaactccaga gccgtcagga 840
   gaacctaata gcgctacgga agaaaactcg ccatctccaa tggagaaagc ttccgtaaca 900
   gaagaaggca gctcagggac gagtgaagaa gaaaaagagg gtgaagaaga tactgctgaa 960
   agcgcagcaa atgaagagcc aaaggcagag gcttctcaag aagaagagaa gaaagaggaa 1020
   gataaaggag aggttcttgc tccctttaat gttcaggatc ttttccgttt tgatcaagga 1080
   atcttccctg ctgagataga agatcttgca cagaaacaag ttgcggttga tttgacgcaa 1140
   ccatcacgat ttttgttgaa ggttcttgct ggtgcgaata tcggtgctga attccatttg 1200
   gatagtggga aaacctatat cgtaggaagt gatccgcagg ttgcagacat tgtcttaagt 1260
   gatatgagta tttcgcgcca acatgcgaag atcattatcg gaaatgataa ttcagttttg 1320
   attgaagatc tgggtagtaa gaatggcgtg attgttgaag ggcgcaagat tgaacatcaa 1380
   tctacgctct ctgcgaatca agttgttgct ctaggaacaa cgttattctt acttgtcgac 1440
   tatgctgctc cttccgatac ggtaatggcg acgatttctt ctgaagatta tgggttattt 1500
   ggtcgtccgc aatctcctga agagattgct gccagagctg cggaagagga agaagagaag 1560
   agaaaacgtg ctacgttgcc aacaggtgct tttatattaa ccttgttcat tggagggtta 1620
   gctctgctct ttggaatagg aacagcttct ttgttccata cgaaggaagt agtttctata 1680
   gatcaaatcg atttgattca tgatattgaa catgtaattc agcagtttcc aactgtacgg 1740
   tttacgttca ataagaacaa cggacagttg ttcttaattg ggcatgtaag aaatagcatt 1800
   gataagagcg agttacttta caaagtggat gctctctcgt ttgtcaagtc ggtagatgat 1860
   aacgtgatcg atgacgaggc agtatggcaa gagatgaata ttctcttgtc taagaatcca 1920
   gaatttaaag gtatcagcat gcaatctcca gagccgggga tttttgtaat cagcgggtat 1980
   ctaaagacag aagaacaagc agcttgtttg gctgattatc taaatctaca ttttaattac 2040
   ctttcactat tggataataa ggtgattatc gaatcacaag tcatgaaagc tcttgctgga 2100
   catcttgtgc aatcaggttt tgcgaacgtt catgtgtcct tcaccaatgg tgaagctgtt 2160
   ttgacaggat atatcaataa taaagatgca gataaattcc gaacggttgt gcaagaactg 2220
   caagatattg cagggattcg tgcggtgaag aattttgtcg ttttgctgcc tgcagaagaa 2280
   ggtgttattg atctaaatat gcggtatcca ggccgttatc gggtaaccgg tttttcaaag 2340
   tgcggggata ttagtattaa tgttgtagtt aatgggcgta ttttaactcg aggcgatatt 2400
   ttagatggaa tgacggtaac aagcattcaa tcgcattgta tctttttaga acgggaaggg 2460
   ttgaaatata aaattgagta caataaatag 2490
<210> 98
   <211> 829
   <212> PRT
   <213> Chlamydia trachomatis

<400> 98
   Met Gly Ile Arg Leu Val Ile Asp Lys Gly Pro Leu Ser Gly Thr Val
   1 5 10 15
   Leu Ile Leu Glu Asn Gly Thr Ser Trp Ser Leu Gly Ser Asp Gly Lys
   20 25 30
   Ala Ser Asp Ile Leu Leu Gln Asp Glu Lys Leu Ala Pro Ser Gln Ile
   35 40 45
   Arg Ile Thr Leu Lys Asp Gly Glu Tyr Tyr Leu Glu Asn Leu Asp Ala
   50 55 60
   Leu Arg Pro Val Ser Val Asp Gly Thr Val Ile Thr Ala Pro Val Leu
   65 70 75 80
   Leu Lys Asp Gly Val Ser Phe Val Met Gly Ser Cys Gln Val Ser Phe
   85 90 95
   Phe Lys Gly Glu Glu Val Glu Gly Asp Ile Glu Leu Ser Phe Gln Thr
   100 105 110
   Glu Gly Gly Asn Glu Gly Glu Pro Ala Ala Gln Gly Ser Ser Ser Val
   115 120 125
   Ser Ser Glu Ala Pro Lys Lys Glu Thr Gly Asn Pro Ser Leu Pro Ser
   130 135 140
   Glu Ala Lys Ala Ser Gly Glu Val Ser Ser Ser Ala Ile Ala Lys Glu
   145 150 155 160
   Gln Glu Leu Ala Ala Ser Phe Leu Ala Ser Val Glu Lys Glu Pro Gly
   165 170 175
   Thr Pro Lys Glu Val Ser Glu Pro Lys Val Ser Ser Gln Glu Gly Gln
   180 185 190
   Thr Pro Ser Val Thr Gly Glu Lys Lys Asp Leu Glu Leu Pro Leu Ala
           195                 200                 205
   Ser Gln Glu Gln Pro Lys Gln Thr Thr Pro Ser Gly Ser Gly Glu Pro
       210                 215                 220
   Thr Gln Ser Gln Asn Ala Ser Met Glu Glu Asn Arg Thr Ser Pro Asp
   225                 230                 235                 240
   Gln Asn Gln Gln Pro Gln Leu Ser Ser Ala Ser Glu Ser Gly Ser Gln
                   245                 250                 255
   Ser Pro Glu Asn Gln Glu Gln Gln Pro Ser Gln Thr Pro Pro Pro Ser
               260                 265                 270
   Pro Glu Thr Pro Glu Pro Ser Gly Glu Pro Asn Ser Ala Thr Glu Glu
           275                 280                 285
   Asn Ser Pro Ser Pro Met Glu Lys Ala Ser Val Thr Glu Glu Gly Ser
       290                 295                 300
   Ser Gly Thr Ser Glu Glu Glu Lys Glu Gly Glu Glu Asp Thr Ala Glu
   305                 310                 315                 320
   Ser Ala Ala Asn Glu Glu Pro Lys Ala Glu Ala Ser Gln Glu Glu Glu
                   325                 330                 335
   Lys Lys Glu Glu Asp Lys Gly Glu Val Leu Ala Pro Phe Asn Val Gln
               340                 345                 350
   Asp Leu Phe Arg Phe Asp Gln Gly Ile Phe Pro Ala Glu Ile Glu Asp
           355                 360                 365
   Leu Ala Gln Lys Gln Val Ala Val Asp Leu Thr Gln Pro Ser Arg Phe
       370                 375                 380
   Leu Leu Lys Val Leu Ala Gly Ala Asn Ile Gly Ala Glu Phe His Leu
   385                 390                 395                 400
   Asp Ser Gly Lys Thr Tyr Ile Val Gly Ser Asp Pro Gln Val Ala Asp
                   405                 410                 415
   Ile Val Leu Ser Asp Met Ser Ile Ser Arg Gln His Ala Lys Ile Ile
               420                 425                 430
   Ile Gly Asn Asp Asn Ser Val Leu Ile Glu Asp Leu Gly Ser Lys Asn
           435                 440                 445
   Gly Val Ile Val Glu Gly Arg Lys Ile Glu His Gln Ser Thr Leu Ser
       450                 455                 460
   Ala Asn Gln Val Val Ala Leu Gly Thr Thr Leu Phe Leu Leu Val Asp
   465 470 475 480
   Tyr Ala Ala Pro Ser Asp Thr Val Met Ala Thr Ile Ser Ser Glu Asp
   485 490 495
   Tyr Gly Leu Phe Gly Arg Pro Gln Ser Pro Glu Glu Ile Ala Ala Arg
   500 505 510
   Ala Ala Glu Glu Glu Glu Glu Lys Arg Lys Arg Ala Thr Leu Pro Thr
   515 520 525
   Gly Ala Phe Ile Leu Thr Leu Phe Ile Gly Gly Leu Ala Leu Leu Phe
   530 535 540
   Gly Ile Gly Thr Ala Ser Leu Phe His Thr Lys Glu Val Val Ser Ile
   545 550 555 560
   Asp Gln Ile Asp Leu Ile His Asp Ile Glu His Val Ile Gln Gln Phe
   565 570 575
   Pro Thr Val Arg Phe Thr Phe Asn Lys Asn Asn Gly Gln Leu Phe Leu
   580 585 590
   Ile Gly His Val Arg Asn Ser Ile Asp Lys Ser Glu Leu Leu Tyr Lys
   595 600 605
   Val Asp Ala Leu Ser Phe Val Lys Ser Val Asp Asp Asn Val Ile Asp
   610 615 620
   Asp Glu Ala Val Trp Gln Glu Met Asn Ile Leu Leu Ser Lys Asn Pro
   625 630 635 640
   Glu Phe Lys Gly Ile Ser Met Gln Ser Pro Glu Pro Gly Ile Phe Val
   645 650 655
   Ile Ser Gly Tyr Leu Lys Thr Glu Glu Gln Ala Ala Cys Leu Ala Asp
   660 665 670
   Tyr Leu Asn Leu His Phe Asn Tyr Leu Ser Leu Leu Asp Asn Lys Val
   675 680 685
   Ile Ile Glu Ser Gln Val Met Lys Ala Leu Ala Gly His Leu Val Gln
   690 695 700
   Ser Gly Phe Ala Asn Val His Val Ser Phe Thr Asn Gly Glu Ala Val
   705 710 715 720
   Leu Thr Gly Tyr Ile Asn Asn Lys Asp Ala Asp Lys Phe Arg Thr Val
   725 730 735
   Val Gln Glu Leu Gln Asp Ile Ala Gly Ile Arg Ala Val Lys Asn Phe
   740 745 750
   Val Val Leu Leu Pro Ala Glu Glu Gly Val Ile Asp Leu Asn Met Arg
   755 760 765
   Tyr Pro Gly Arg Tyr Arg Val Thr Gly Phe Ser Lys Cys Gly Asp Ile
   770 775 780
   Ser Ile Asn Val Val Val Asn Gly Arg Ile Leu Thr Arg Gly Asp Ile
   785 790 795 800
   Leu Asp Gly Met Thr Val Thr Ser Ile Gln Ser His Cys Ile Phe Leu
   805 810 815
   Glu Arg Glu Gly Leu Lys Tyr Lys Ile Glu Tyr Asn Lys
   820 825
<210> 99
   <211> 525
   <212> DNA
   <213> Chlamydia trachomatis
<400> 99
   ttggaggatt acgtggcttc tcctcatctt cgttccctag cgtgtttaga taacccacaa 60
   ctacccatag aaacacctct ctttgagcaa gaagctctct cccatgagct tctttctctt 120
   attcaggtgt tccgtaaatt atctgtccat cttctctctg aaatcgaaaa attatctcag 180
   aaactaaaac ctgagcttct tgaacttgct gtcctcgtct gtgaaaaatt tctgtacaga 240
   aagcttgcct gtacagaaga acttgctctc ctaatctccg cagctctgca acatcattta 300
   gctacttatg ccgtctctcc cataaaaata ggtttacatc ctgaagatct ttcaaaccta 360
   tctaaatggt taatccttca cgatgttccc ttactcaaaa atatcgaatt cattgcagat 420
   cctttatgca agaaagctag ctataaaata gaactccctt caggaattct gagacaagac 480
   atcggggaag agctgtccca tctactgagt gtactcactc cataa 525
<210> 100
   <211> 174
   <212> PRT
   <213> Chlamydia trachomatis
<400> 100
   Leu Glu Asp Tyr Val Ala Ser Pro His Leu Arg Ser Leu Ala Cys Leu
   1               5                   10                  15
   Asp Asn Pro Gln Leu Pro Ile Glu Thr Pro Leu Phe Glu Gln Glu Ala
               20                  25                  30
   Leu Ser His Glu Leu Leu Ser Leu Ile Gln Val Phe Arg Lys Leu Ser
           35                  40                  45
   Val His Leu Leu Ser Glu Ile Glu Lys Leu Ser Gln Lys Leu Lys Pro
       50                  55                  60
   Glu Leu Leu Glu Leu Ala Val Leu Val Cys Glu Lys Phe Leu Tyr Arg
   65                  70                  75                  80
   Lys Leu Ala Cys Thr Glu Glu Leu Ala Leu Leu Ile Ser Ala Ala Leu
                   85                  90                  95
   Gln His His Leu Ala Thr Tyr Ala Val Ser Pro Ile Lys Ile Gly Leu
               100                 105                 110
   His Pro Glu Asp Leu Ser Asn Leu Ser Lys Trp Leu Ile Leu His Asp
           115                 120                 125
   Val Pro Leu Leu Lys Asn Ile Glu Phe Ile Ala Asp Pro Leu Cys Lys
       130                 135                 140
   Lys Ala Ser Tyr Lys Ile Glu Leu Pro Ser Gly Ile Leu Arg Gln Asp
   145                 150                 155                 160
   Ile Gly Glu Glu Leu Ser His Leu Leu Ser Val Leu Thr Pro
                   165                 170
<210> 101
   <211> 420
   <212> DNA
   <213> Chlamydia trachomatis
<400> 101
   atggaagatt ttgcagagta catcgttaaa aatttagtta ccgatcccaa cgccgttgag      60
   attcggtcat ccgaggacaa agccagcgca acccttaagc tggagatcca tgccgcttct     120
   gaagatattg gaaagatcat cgggagaaaa ggacaaacca tacaagcgct aagaaccatt     180
   ctaaaacgtg taggcgctag attgcagaaa aaaatccttg ttgagcttgc tcaacctgaa     240
   aacggctctc tcacagacga agaagttttg tctttagatt atatctctgc agcttctgcc     300
   gaagatttcg aagaagattc ttcttttgct gaaaatagca tcgaagaaga accttcggtc     360
   attgtacgat ctcttgcagg tgtatgccca ggttgtagct gctctcatca tcatgattag     420
<210> 102
   <211> 139
   <212> PRT
   <213> Chlamydia trachomatis
<400> 102
   Met Glu Asp Phe Ala Glu Tyr Ile Val Lys Asn Leu Val Thr Asp Pro
   1               5                   10                  15
   Asn Ala Val Glu Ile Arg Ser Ser Glu Asp Lys Ala Ser Ala Thr Leu
               20                  25                  30
   Lys Leu Glu Ile His Ala Ala Ser Glu Asp Ile Gly Lys Ile Ile Gly
           35                  40                  45
   Arg Lys Gly Gln Thr Ile Gln Ala Leu Arg Thr Ile Leu Lys Arg Val
       50                  55                  60
   Gly Ala Arg Leu Gln Lys Lys Ile Leu Val Glu Leu Ala Gln Pro Glu
   65                  70                  75                  80
   Asn Gly Ser Leu Thr Asp Glu Glu Val Leu Ser Leu Asp Tyr Ile Ser
                   85                  90                  95
   Ala Ala Ser Ala Glu Asp Phe Glu Glu Asp Ser Ser Phe Ala Glu Asn
               100                 105                 110
   Ser Ile Glu Glu Glu Pro Ser Val Ile Val Arg Ser Leu Ala Gly Val
           115                 120                 125
   Cys Pro Gly Cys Ser Cys Ser His His His Asp
       130                 135
<210> 103
   <211> 279
   <212> DNA
   <213> Chlamydia trachomatis
<400> 103
   atgaaggaag aaattctcgc gctacttgat catttatata cggagcagga aagacgatta     60
   atgtcgctag ggacgacgat tgttcctgga ttgacgaaag aggatctttt acagcctatg    120
   gattatgatg aacttgagga gaacccttct tttagatttg aagaaggagt tttgaatgga    180
   ataggagaga ctcgagccgc attatattct tttttttctg atctagaaga ctccttttgc     240
   gtggagtctt ctagcgatac gagcctctgt aaggattag                           279
<210> 104
   <211> 92
   <212> PRT
   <213> Chlamydia trachomatis
<400> 104
   Met Lys Glu Glu Ile Leu Ala Leu Leu Asp His Leu Tyr Thr Glu Gln
   1               5                   10                  15
   Glu Arg Arg Leu Met Ser Leu Gly Thr Thr Ile Val Pro Gly Leu Thr
               20                  25                  30
   Lys Glu Asp Leu Leu Gln Pro Met Asp Tyr Asp Glu Leu Glu Glu Asn
           35                  40                  45
   Pro Ser Phe Arg Phe Glu Glu Gly Val Leu Asn Gly Ile Gly Glu Thr
       50                  55                  60
   Arg Ala Ala Leu Tyr Ser Phe Phe Ser Asp Leu Glu Asp Ser Phe Cys
   65                  70                  75                  80
   Val Glu Ser Ser Ser Asp Thr Ser Leu Cys Lys Asp
                   85                  90
<210> 105
   <211> 507
   <212> DNA
   <213> Chlamydia trachomatis
<400> 105
   atgtggcata aagaaccaat gcatgctgta ttacagctac ctgaaacacc tctggttaca     60
   ggaacaacga actctgcaac ggctgatgag attataacac gatttgctaa ggattctaac    120
   cctctcatcg ttactgttta ctatatttac cagtctgttc tcgtcgcgca aaataacttg    180
   tctctagttg cagagcaatt gcaagctaat gctgccgcgc aaacattcct gaacaatgaa    240
   gaagcgctat accaatacac taccattcca aaaaaccaag taaactctca aaactcctct    300
   tatttacaaa acgtacaatc ggttaaccaa gcagtcggag catctagaca agcgattcaa    360
   aaccaaatat ctggtcttgg aaatgcttct caagtgattt ccagtaactt gaacacgaat    420
   aataatatta ttcaacagtc cctacaagtc ggtcaggcgc tcattcaaac gttttcacaa    480
   atcgttagct tgatcgcgaa tatctaa                                        507
<210> 106
   <211> 168
   <212> PRT
   <213> Chlamydia trachomatis
<400> 106
   Met Trp His Lys Glu Pro Met His Ala Val Leu Gln Leu Pro Glu Thr
   1               5                   10                  15
   Pro Leu Val Thr Gly Thr Thr Asn Ser Ala Thr Ala Asp Glu Ile Ile
               20                  25                  30
   Thr Arg Phe Ala Lys Asp Ser Asn Pro Leu Ile Val Thr Val Tyr Tyr
           35                  40                  45
   Ile Tyr Gln Ser Val Leu Val Ala Gln Asn Asn Leu Ser Leu Val Ala
       50                  55                  60
   Glu Gln Leu Gln Ala Asn Ala Ala Ala Gln Thr Phe Leu Asn Asn Glu
   65                  70                  75                  80
   Glu Ala Leu Tyr Gln Tyr Thr Thr Ile Pro Lys Asn Gln Val Asn Ser
                   85                  90                  95
   Gln Asn Ser Ser Tyr Leu Gln Asn Val Gln Ser Val Asn Gln Ala Val
               100                 105                 110
   Gly Ala Ser Arg Gln Ala Ile Gln Asn Gln Ile Ser Gly Leu Gly Asn
   115 120 125
   Ala Ser Gln Val Ile Ser Ser Asn Leu Asn Thr Asn Asn Asn Ile Ile
   130 135 140
   Gln Gln Ser Leu Gln Val Gly Gln Ala Leu Ile Gln Thr Phe Ser Gln
   145 150 155 160
   Ile Val Ser Leu Ile Ala Asn Ile
   165
<210> 107
   <211> 486
   <212> DNA
   <213> Chlamydia psittaci
<400> 107
   gtgcgcatac taccctttga tccttatgga gcattacctc ctcaaggagt aaataaagat 60
   cctcatagca atataccttt aaatcagaag atttctgatg aaatagctaa aaatgaagct 120
   atgcgtttgg ctttgcttgc tattactgat caagaaaaag acaaaagaaa gagaaaacat 180
   cggtttaaaa ttcttaaccg caaacaagca aaagtgctcc tatctcaatt gcgaaatgta 240
   gatttagact ttcaaagttt gaaaaatgct ggtttaaaag aagaagaaga taacgaagaa 300
   gataatgaag aatcttcaaa acaagggaaa actttccata ttgctagtag taagaagcct 360
   gtgaagatag gggcatctgc agctcaggct attgctgatg ctgcagaggc ttgggttatt 420
   gctcgcaata gaggagtctt ggatatggcc tccctattat tctggcataa agatgatgag 480
   tgttaa 486
<210> 108
   <211> 161
   <212> PRT
   <213> Chlamydia psittaci
<400> 108
   Val Arg Ile Leu Pro Phe Asp Pro Tyr Gly Ala Leu Pro Pro Gln Gly
   1 5 10 15
   Val Asn Lys Asp Pro His Ser Asn Ile Pro Leu Asn Gln Lys Ile Ser
   20 25 30
   Asp Glu Ile Ala Lys Asn Glu Ala Met Arg Leu Ala Leu Leu Ala Ile
   35 40 45
   Thr Asp Gln Glu Lys Asp Lys Arg Lys Arg Lys His Arg Phe Lys Ile
   50 55 60
   Leu Asn Arg Lys Gln Ala Lys Val Leu Leu Ser Gln Leu Arg Asn Val
   65 70 75 80
   Asp Leu Asp Phe Gln Ser Leu Lys Asn Ala Gly Leu Lys Glu Glu Glu
   85 90 95
   Asp Asn Glu Glu Asp Asn Glu Glu Ser Ser Lys Gln Gly Lys Thr Phe
   100 105 110
   His Ile Ala Ser Ser Lys Lys Pro Val Lys Ile Gly Ala Ser Ala Ala
   115 120 125
   Gln Ala Ile Ala Asp Ala Ala Glu Ala Trp Val Ile Ala Arg Asn Arg
   130 135 140
   Gly Val Leu Asp Met Ala Ser Leu Leu Phe Trp His Lys Asp Asp Glu
   145 150 155 160
   Cys
<210> 109
   <211> 447
   <212> DNA
   <213> Chlamydia psittaci
<400> 109
   atgaaaagtg aacgtctgaa aaaactcgaa tctgaattac gtgacttaac ccagtggatg      60
   caattgggtt tagttccaaa aaaagaaatc gatagacata aggaagaaat acgttcttta     120
   gaaaacaaaa tccatgaaga gaaggaacgc ctgcaacttc taaaagaaag tggcgaagtt     180
   gaagagtttg ttactccaag acgtagccct gcaaaaacgg tatatcctga cggtccaagc     240
   atgtcagata tggaatttgt tgaagctaca gagacagaaa ttgatattga tccaggtgaa     300
   accgtagagc tcgaacttcc tgatgaagaa cgtgaagaag gcgctgtaga aatcgattat     360
   tccagtgatg acgatgaaga tcctttcagt gatcgcaatc gttggagacg tgggggcatt     420
   gttgaccccg atgctaatga atggtaa                                         447
<210> 110
   <211> 148
   <212> PRT
   <213> Chlamydia psittaci
<400> 110
   Met Lys Ser Glu Arg Leu Lys Lys Leu Glu Ser Glu Leu Arg Asp Leu
   1               5                   10                  15
   Thr Gln Trp Met Gln Leu Gly Leu Val Pro Lys Lys Glu Ile Asp Arg
               20                  25                  30
   His Lys Glu Glu Ile Arg Ser Leu Glu Asn Lys Ile His Glu Glu Lys
           35                  40                  45
   Glu Arg Leu Gln Leu Leu Lys Glu Ser Gly Glu Val Glu Glu Phe Val
   50 55 60
   Thr Pro Arg Arg Ser Pro Ala Lys Thr Val Tyr Pro Asp Gly Pro Ser
   65 70 75 80
   Met Ser Asp Met Glu Phe Val Glu Ala Thr Glu Thr Glu Ile Asp Ile
   85 90 95
   Asp Pro Gly Glu Thr Val Glu Leu Glu Leu Pro Asp Glu Glu Arg Glu
   100 105 110
   Glu Gly Ala Val Glu Ile Asp Tyr Ser Ser Asp Asp Asp Glu Asp Pro
   115 120 125
   Phe Ser Asp Arg Asn Arg Trp Arg Arg Gly Gly Ile Val Asp Pro Asp
   130 135 140
   Ala Asn Glu Trp
   145
<210> 111
   <211> 972
   <212> DNA
   <213> Chlamydia psittaci
<400> 111
   atgaaacggc tgcttttttg cgtttgcgca ctctctttct catgctttac ctacggttca    60
   gctctaaaac aagattcttc tgttatgaag gaaaccttcc gtaacaacta cggaattatt   120
   gtatcaggaa aagattgggt aaaacgaggc tgtgatggga caatcaccaa agttttaaag   180
   gatgggtcta ctctttatga aatttacgtt cagggtcttc ttcatggcga gataacgtta    240
   acattccccc attctacgac tcttgctgta ataaaaactt acgatcaggg aaggcttgtt   300
   tcatataaaa cattcttttc taatggttta ccttctcaag aagaattata ccaagaagat   360
   ggctctcttg ttgtgactcg ctggcctgac aacaaaaata gcgataccat caccgatcct   420
   tattttactg agactaccta ccagggtcgt gtacttgaag ggagctactc ctcatttaat   480
   gggaaatata catcaacaat tcgcaatgga gaaggcatac gctcgaactt ttctccaagt   540
   aacgtccttc tttctgaaga aacctttaac gatggcgtta tggtaaaaag aactaccttc   600
   tatgctacta gagatcctga aactataacg cattatatta atggccaacc tcatggattg   660
   cgtttaacct atctccctgg agggattcct aacactattg aagaatggcg ttatggttac   720
   caagatggaa ctacaacagt atttaaaaac ggttgcaaag ctgctgagat tccttttgta   780
   aaaggatcta aggaaggatg tgaattacgc tataatgaag acgaagttat tgccgaagaa   840
   gtgtcttgga gaaataactt ccctcatggt atgagaaaga tctatgctgc cggtgtttat   900
   aaatgcgagt ggtaccaccg cggacgccta gtctcaaaag ctaagttcga gagactcaat   960
   aatgcaggat aa                                                       972
<210> 112
   <211> 323
   <212> PRT
   <213> Chlamydia psittaci
<400> 112
   Met Lys Arg Leu Leu Phe Cys Val Cys Ala Leu Ser Phe Ser Cys Phe
   1 5 10 15
   Thr Tyr Gly Ser Ala Leu Lys Gln Asp Ser Ser Val Met Lys Glu Thr
   20 25 30
   Phe Arg Asn Asn Tyr Gly Ile Ile Val Ser Gly Lys Asp Trp Val Lys
   35 40 45
   Arg Gly Cys Asp Gly Thr Ile Thr Lys Val Leu Lys Asp Gly Ser Thr
   50 55 60
   Leu Tyr Glu Ile Tyr Val Gln Gly Leu Leu His Gly Glu Ile Thr Leu
   65 70 75 80
   Thr Phe Pro His Ser Thr Thr Leu Ala Val Ile Lys Thr Tyr Asp Gln
   85 90 95
   Gly Arg Leu Val Ser Tyr Lys Thr Phe Phe Ser Asn Gly Leu Pro Ser
   100 105 110
   Gln Glu Glu Leu Tyr Gln Glu Asp Gly Ser Leu Val Val Thr Arg Trp
   115 120 125
   Pro Asp Asn Lys Asn Ser Asp Thr Ile Thr Asp Pro Tyr Phe Thr Glu
   130 135 140
   Thr Thr Tyr Gln Gly Arg Val Leu Glu Gly Ser Tyr Ser Ser Phe Asn
   145 150 155 160
   Gly Lys Tyr Thr Ser Thr Ile Arg Asn Gly Glu Gly Ile Arg Ser Asn
   165 170 175
   Phe Ser Pro Ser Asn Val Leu Leu Ser Glu Glu Thr Phe Asn Asp Gly
   180 185 190
   Val Met Val Lys Arg Thr Thr Phe Tyr Ala Thr Arg Asp Pro Glu Thr
   195 200 205
   Ile Thr His Tyr Ile Asn Gly Gln Pro His Gly Leu Arg Leu Thr Tyr
   210 215 220
   Leu Pro Gly Gly Ile Pro Asn Thr Ile Glu Glu Trp Arg Tyr Gly Tyr
   225 230 235 240
   Gln Asp Gly Thr Thr Thr Val Phe Lys Asn Gly Cys Lys Ala Ala Glu
                       245                 250                 255
   Ile Pro Phe Val Lys Gly Ser Lys Glu Gly Cys Glu Leu Arg Tyr Asn
               260                 265                 270
   Glu Asp Glu Val Ile Ala Glu Glu Val Ser Trp Arg Asn Asn Phe Pro
           275                 280                 285
   His Gly Met Arg Lys Ile Tyr Ala Ala Gly Val Tyr Lys Cys Glu Trp
       290                 295                 300
   Tyr His Arg Gly Arg Leu Val Ser Lys Ala Lys Phe Glu Arg Leu Asn
   305                 310                 315                 320
   Asn Ala Gly
<210> 113
   <211> 975
   <212> DNA
   <213> Chlamydia psittaci
<400> 113
   atgacatctg caatgcctcg tgtggctagc cttgtagtgg gcagtagaaa tgtgtttatg      60
   caaacagcta tgcagggagc caagaaaggc gatgtaggct gctgcattag gcagtttgtt     120
   acgaatggaa acaaccattt agcacgcttt gtcggaagta caaaaaatat agataaggca     180
   tttaagtttg ctaaatccgt ctctgaattt agctgcggtg ttattgaaag cacaggtgat     240
   acaggacctg ctcttcagat aagtaaaaac gttgcatcga ctttaagcac ggctagaaat     300
   gttgtcgcct tgtctaatgt gttcactggt gcgattcctg gattcgtcct ttcctcaaag     360
   aattgctata atcatattaa gaaatgtttt gctccagaaa ctgagtacga ttgcggtagt     420
   attgagaaag gcttgcctta taacaagctt tatctcacta agggtgatca tgtgttgggt     480
   gcgattaaag aaggttgctc tgcagttggc gcgggaactt acgttgcaac gtttggtgtc     540
   agccgtccgg tgctcttggc aaacaagctt gctcacaagc ctttcctctc ttcaggagtg     600
   aaggcagcat tttgtaacag cgtaacctat atgatgacag caaaccacgc tgcaggggtt     660
   atcggcggag cagcagcctt gctctatgaa aaccgtgtat ataaacgcgc ttctgaggga     720
   ttgttagctt ctaaaatgac agagagtctg gattctgaag tttacgatca agtgtctaaa     780
   ggcttgaaag agtcgcacta ccaagctgtt aaaaaaacaa tacttggaat tttagaaaaa     840
   gcatttgaat tgatcgctga tatattcgat ttcattgctt taccaattac tgcttcagtt     900
   cgcttggcaa taaaagcggg attagtcaca gtatccagtg gtttcggtct ttacagcgtc     960
   tgggtcaatt cttaa                                                      975
<210> 114
   <211> 324
   <212> PRT
   <213> Chlamydia psittaci
<400> 114
   Met Thr Ser Ala Met Pro Arg Val Ala Ser Leu Val Val Gly Ser Arg
   1 5 10 15
   Asn Val Phe Met Gln Thr Ala Met Gln Gly Ala Lys Lys Gly Asp Val
   20 25 30
   Gly Cys Cys Ile Arg Gln Phe Val Thr Asn Gly Asn Asn His Leu Ala
   35 40 45
   Arg Phe Val Gly Ser Thr Lys Asn Ile Asp Lys Ala Phe Lys Phe Ala
   50 55 60
   Lys Ser Val Ser Glu Phe Ser Cys Gly Val Ile Glu Ser Thr Gly Asp
   65 70 75 80
   Thr Gly Pro Ala Leu Gln Ile Ser Lys Asn Val Ala Ser Thr Leu Ser
   85 90 95
   Thr Ala Arg Asn Val Val Ala Leu Ser Asn Val Phe Thr Gly Ala Ile
   100 105 110
   Pro Gly Phe Val Leu Ser Ser Lys Asn Cys Tyr Asn His Ile Lys Lys
   115 120 125
   Cys Phe Ala Pro Glu Thr Glu Tyr Asp Cys Gly Ser Ile Glu Lys Gly
   130 135 140
   Leu Pro Tyr Asn Lys Leu Tyr Leu Thr Lys Gly Asp His Val Leu Gly
   145 150 155 160
   Ala Ile Lys Glu Gly Cys Ser Ala Val Gly Ala Gly Thr Tyr Val Ala
   165 170 175
   Thr Phe Gly Val Ser Arg Pro Val Leu Leu Ala Asn Lys Leu Ala His
   180 185 190
   Lys Pro Phe Leu Ser Ser Gly Val Lys Ala Ala Phe Cys Asn Ser Val
   195 200 205
   Thr Tyr Met Met Thr Ala Asn His Ala Ala Gly Val Ile Gly Gly Ala
   210 215 220
   Ala Ala Leu Leu Tyr Glu Asn Arg Val Tyr Lys Arg Ala Ser Glu Gly
   225 230 235 240
   Leu Leu Ala Ser Lys Met Thr Glu Ser Leu Asp Ser Glu Val Tyr Asp
   245 250 255
   Gln Val Ser Lys Gly Leu Lys Glu Ser His Tyr Gln Ala Val Lys Lys
   260 265 270
   Thr Ile Leu Gly Ile Leu Glu Lys Ala Phe Glu Leu Ile Ala Asp Ile
   275 280 285
   Phe Asp Phe Ile Ala Leu Pro Ile Thr Ala Ser Val Arg Leu Ala Ile
   290 295 300
   Lys Ala Gly Leu Val Thr Val Ser Ser Gly Phe Gly Leu Tyr Ser Val
   305 310 315 320
   Trp Val Asn Ser
<210> 115
   <211> 423
   <212> DNA
   <213> Chlamydia psittaci
<400> 115
   atgagtttgg attttttaga agaattttac cgtcgttcta tttgtaacaa aggaacggca 60
   tttcctgagg gcttcatgga tattgccgat gtcctatccc attctgcgtc tgaacttaaa 120
   atcgagtcta tgagcgatct tcctgttaac aatttcatca ttgcagaatc ggcagataaa 180
   ctcactttat ttaatgcaga ttttgctgtt tggttagtgc ctgagcttgt ccaaggagaa 240
   gctgtgactc gaggatacat cgctttatac cattctggag gggattatac tccagaaatg 300
   gcatttcaag cctctgggga gtacaatcaa tcagcattaa ttcttgaagc acttcagata 360
   tatctacaag acataaaaga taccgaaagc acgctacgct ctttccgctt taatcaagac 420
   tag 423
<210> 116
   <211> 140
   <212> PRT
   <213> Chlamydia psittaci
<400> 116
   Met Ser Leu Asp Phe Leu Glu Glu Phe Tyr Arg Arg Ser Ile Cys Asn
   1 5 10 15
   Lys Gly Thr Ala Phe Pro Glu Gly Phe Met Asp Ile Ala Asp Val Leu
   20 25 30
   Ser His Ser Ala Ser Glu Leu Lys Ile Glu Ser Met Ser Asp Leu Pro
   35 40 45
   Val Asn Asn Phe Ile Ile Ala Glu Ser Ala Asp Lys Leu Thr Leu Phe
   50 55 60
   Asn Ala Asp Phe Ala Val Trp Leu Val Pro Glu Leu Val Gln Gly Glu
   65 70 75 80
   Ala Val Thr Arg Gly Tyr Ile Ala Leu Tyr His Ser Gly Gly Asp Tyr
   85 90 95
   Thr Pro Glu Met Ala Phe Gln Ala Ser Gly Glu Tyr Asn Gln Ser Ala
   100 105 110
   Leu Ile Leu Glu Ala Leu Gln Ile Tyr Leu Gln Asp Ile Lys Asp Thr
   115 120 125
   Glu Ser Thr Leu Arg Ser Phe Arg Phe Asn Gln Asp
   130 135 140
<210> 117
   <211> 849
   <212> DNA
   <213> Chlamydia psittaci
<400> 117
   atggaattaa ataaaacatc cgagtctttg tacaattgca agacagatcg ccattcagta    60
   caacaagaag taggtccaga gcctaaagat aaccgtgacg ttaaagtgtt ttctttagaa   120
   ggccgtcaac aatcaaaaca agatcgtcag gataaagttt ccagtaaaga ttctcgtcaa   180
   gaatctcgag gagctgatga taagcatgta gaagagaaaa catcagcagt atcttctaaa   240
   gaagaagata aagaagagag tgatggtttc atggcttatg acaatcctac agcaggcatg   300
   gcatttgtag atgttgctgc ttctgtatct agtgaagccg ttgtagaaag tactacagta   360
   gctatcggca gtgcagattt acagtgggtg caagatgtta ttgctagtac tgtagaatct   420
   atgatggttg ctgatgttaa cggtcagcaa ttaatcgagt tagttttaga tgctgaaggt   480
   aatgttcctg acatttttgc aggtgcgaat ttaacattag tacaaacagg aacagaccta   540
   tctgtaaaat tctctaattt tgtagataat gctcagatgg cagaagcaat gagtctcatt   600
   gtgaataacc cttctcagct tgctggttta gtagaggcat taaaaaatcg tcatttgaat   660
   ttgacagaat tggctgttgg atcaagtatt gtacaattgc caactattga agaggtgcaa   720
   acacctctac atatgattgc tgctacaatt catcaaagag atgaagagag agatcaagaa   780
   ggacaagatc agcagcaaca gcaagatcaa gaacaaaacc aatataaagt tgaagaagca   840
   cgtttgtaa                                                           849
<210> 118
   <211> 282
   <212> PRT
   <213> Chlamydia psittaci
<400> 118
   Met Glu Leu Asn Lys Thr Ser Glu Ser Leu Tyr Asn Cys Lys Thr Asp
   1               5                   10                  15
   Arg His Ser Val Gln Gln Glu Val Gly Pro Glu Pro Lys Asp Asn Arg
               20                  25                  30
   Asp Val Lys Val Phe Ser Leu Glu Gly Arg Gln Gln Ser Lys Gln Asp
   35 40 45
   Arg Gln Asp Lys Val Ser Ser Lys Asp Ser Arg Gln Glu Ser Arg Gly
   50 55 60
   Ala Asp Asp Lys His Val Glu Glu Lys Thr Ser Ala Val Ser Ser Lys
   65 70 75 80
   Glu Glu Asp Lys Glu Glu Ser Asp Gly Phe Met Ala Tyr Asp Asn Pro
   85 90 95
   Thr Ala Gly Met Ala Phe Val Asp Val Ala Ala Ser Val Ser Ser Glu
   100 105 110
   Ala Val Val Glu Ser Thr Thr Val Ala Ile Gly Ser Ala Asp Leu Gln
   115 120 125
   Trp Val Gln Asp Val Ile Ala Ser Thr Val Glu Ser Met Met Val Ala
   130 135 140
   Asp Val Asn Gly Gln Gln Leu Ile Glu Leu Val Leu Asp Ala Glu Gly
   145 150 155 160
   Asn Val Pro Asp Ile Phe Ala Gly Ala Asn Leu Thr Leu Val Gln Thr
   165 170 175
   Gly Thr Asp Leu Ser Val Lys Phe Ser Asn Phe Val Asp Asn Ala Gln
   180 185 190
   Met Ala Glu Ala Met Ser Leu Ile Val Asn Asn Pro Ser Gln Leu Ala
   195 200 205
   Gly Leu Val Glu Ala Leu Lys Asn Arg His Leu Asn Leu Thr Glu Leu
   210 215 220
   Ala Val Gly Ser Ser Ile Val Gln Leu Pro Thr Ile Glu Glu Val Gln
   225 230 235 240
   Thr Pro Leu His Met Ile Ala Ala Thr Ile His Gln Arg Asp Glu Glu
   245 250 255
   Arg Asp Gln Glu Gly Gln Asp Gln Gln Gln Gln Gln Asp Gln Glu Gln
   260 265 270
   Asn Gln Tyr Lys Val Glu Glu Ala Arg Leu
   275 280
<210> 119
   <211> 261
   <212> DNA
   <213> Chlamydia psittaci
<400> 119
   atgagtagtg gtagcgggag cagttgctca gcatttaatt ttaatgacat gctcaatggc    60
   gtatgtaagt acgtccaagg tgtgcaacaa tatttaacag aattagaaac ctcaacgcaa   120
   ggtactgttg acttaggtac gatgtttaat ttgcagtttc gtatgcaaat tttatcacag   180
   tacatggaag cagtatccaa catcttgaca gcagtgaaca cagagatgat tactatggca   240
   agagctgtta aaggaagtta a                                              261
<210> 120
   <211> 86
   <212> PRT
   <213> Chlamydia psittaci
<400> 120
   Met Ser Ser Gly Ser Gly Ser Ser Cys Ser Ala Phe Asn Phe Asn Asp
   1               5                   10                  15
   Met Leu Asn Gly Val Cys Lys Tyr Val Gln Gly Val Gln Gln Tyr Leu
               20                  25                  30
   Thr Glu Leu Glu Thr Ser Thr Gln Gly Thr Val Asp Leu Gly Thr Met
           35                  40                  45
   Phe Asn Leu Gln Phe Arg Met Gln Ile Leu Ser Gln Tyr Met Glu Ala
       50                  55                  60
   Val Ser Asn Ile Leu Thr Ala Val Asn Thr Glu Met Ile Thr Met Ala
   65                  70                  75                  80
   Arg Ala Val Lys Gly Ser
                   85
<210> 121
   <211> 729
   <212> DNA
   <213> Chlamydia psittaci
<400> 121
   atggaacctt atttagattt attagataaa aacatcaaag aaaaacatat gttagatcat      60
   cccttttata tgaagtggtc taagggagaa ttaacgaaag agcagttaaa agaatacgct     120
   aaggattact acctccacat caaagctttt cctcgttatc tttcagcagt tcatagccgc     180
   tgtgataatt tagaagcgcg taaattgctt cttgataacc ttatggatga agaaacaggc     240
   catcctaatc acatagatct atggaaaaat tttgcctatg cgttaggtgt tactgaggaa     300
   gaattagaaa atcatgttcc tagtgcagca gcacaaaaga aagtggatac atttttacgt     360
   tggtgtactg gagattcctt atctgctggt gtagctgctt tatataccta tgaaagccaa     420
   atccctacag ttgcagagac taaaatctcg ggattaaaac agtatttcgg ctttacggct     480
   cctgaagatt atgagtactt cacagtacat caagatgttg atgtaagaca ttctcgtgaa     540
   gaaaaagaat taatagagtc gttgctaaat aatgatagcg ataaggttct acaagcttca     600
   aaagaagttt gtgatgcttt atacggcttt ttagatactt tcttagatga aaaagacgcc     660
   tgctcagcaa cttcatcttc tgttgcggat tctaaaccat cttcatgttg ttgtcgttgc     720
   cgtcattaa                                                             729
<210> 122
   <211> 242
   <212> PRT
   <213> Chlamydia psittaci
<400> 122
   Met Glu Pro Tyr Leu Asp Leu Leu Asp Lys Asn Ile Lys Glu Lys His
   1               5                   10                  15
   Met Leu Asp His Pro Phe Tyr Met Lys Trp Ser Lys Gly Glu Leu Thr
               20                  25                  30
   Lys Glu Gln Leu Lys Glu Tyr Ala Lys Asp Tyr Tyr Leu His Ile Lys
           35                  40                  45
   Ala Phe Pro Arg Tyr Leu Ser Ala Val His Ser Arg Cys Asp Asn Leu
       50                  55                  60
   Glu Ala Arg Lys Leu Leu Leu Asp Asn Leu Met Asp Glu Glu Thr Gly
   65                  70                  75                  80
   His Pro Asn His Ile Asp Leu Trp Lys Asn Phe Ala Tyr Ala Leu Gly
                   85                  90                  95
   Val Thr Glu Glu Glu Leu Glu Asn His Val Pro Ser Ala Ala Ala Gln
               100                 105                 110
   Lys Lys Val Asp Thr Phe Leu Arg Trp Cys Thr Gly Asp Ser Leu Ser
           115                 120                 125
   Ala Gly Val Ala Ala Leu Tyr Thr Tyr Glu Ser Gln Ile Pro Thr Val
       130                 135                 140
   Ala Glu Thr Lys Ile Ser Gly Leu Lys Gln Tyr Phe Gly Phe Thr Ala
   145                 150                 155                 160
   Pro Glu Asp Tyr Glu Tyr Phe Thr Val His Gln Asp Val Asp Val Arg
                   165                 170                 175
   His Ser Arg Glu Glu Lys Glu Leu Ile Glu Ser Leu Leu Asn Asn Asp
               180                 185                 190
   Ser Asp Lys Val Leu Gln Ala Ser Lys Glu Val Cys Asp Ala Leu Tyr
           195                 200                 205
   Gly Phe Leu Asp Thr Phe Leu Asp Glu Lys Asp Ala Cys Ser Ala Thr
       210                 215                 220
   Ser Ser Ser Val Ala Asp Ser Lys Pro Ser Ser Cys Cys Cys Arg Cys
   225                 230                 235                 240
   Arg His
<210> 123
   <211> 234
   <212> DNA
   <213> Chlamydia psittaci
<400> 123
   atgaatgaag gtattcaaac cgtttctttt aacaagacac accgactcac cgcgaaatct    60
   acagttagtt tagaaatgcc cgtagcgaca cagaaacttc aaggtaaaga aggcatgccc   120
   gcagcggcaa gtttagaagc tgatttctta agagcagaag ctatacttgc agaaatgcgt   180
   gaaattcgcg gctgtttaga acattcattg gaaacgctag ttcctagaga ctag         234
<210> 124
   <211> 77
   <212> PRT
   <213> Chlamydia psittaci
<400> 124
   Met Asn Glu Gly Ile Gln Thr Val Ser Phe Asn Lys Thr His Arg Leu
   1               5                   10                  15
   Thr Ala Lys Ser Thr Val Ser Leu Glu Met Pro Val Ala Thr Gln Lys
               20                  25                  30
   Leu Gln Gly Lys Glu Gly Met Pro Ala Ala Ala Ser Leu Glu Ala Asp
           35                  40                  45
   Phe Leu Arg Ala Glu Ala Ile Leu Ala Glu Met Arg Glu Ile Arg Gly
       50                  55                  60
   Cys Leu Glu His Ser Leu Glu Thr Leu Val Pro Arg Asp
   65                  70                  75
<210> 125
   <211> 231
   <212> DNA
   <213> Chlamydia psittaci
<400> 125
   atgaaaaata aactacatga tttgttgaat cagctttatg aaaatcaaaa atctcgttta    60
   caaagcatgg gggagcagat cgtcccgaat ttaacttctg atgatgtatt acagcccatg   120
   gatttttcta tgctggaaga aaatcctttc tttcggtttg aggaaggtgt tctttctggt   180
   cttggagagg ctcgggcggc gattttggca ttatttgccg aagaaggtta a            231
<210> 126
   <211> 76
   <212> PRT
   <213> Chlamydia psittaci
<400> 126
   Met Lys Asn Lys Leu His Asp Leu Leu Asn Gln Leu Tyr Glu Asn Gln
   1               5                   10                  15
   Lys Ser Arg Leu Gln Ser Met Gly Glu Gln Ile Val Pro Asn Leu Thr
               20                  25                  30
   Ser Asp Asp Val Leu Gln Pro Met Asp Phe Ser Met Leu Glu Glu Asn
           35                  40                  45
   Pro Phe Phe Arg Phe Glu Glu Gly Phe Phe Leu Val Leu Glu Arg Leu
       50                  55                  60
   Gly Arg Arg Phe Trp His Tyr Leu Pro Lys Lys Val
   65                  70                  75
<210> 127
   <211> 519
   <212> DNA
   <213> Chlamydia psittaci
<400> 127
   atgtggttct cttctccagc acttcaaact agccctagag ccgctattga cgttcccggg      60
   acatctatca caggtggacc aaatacagca acagccgatg aaattattgc aaaatttgcg     120
   aaagattcca atcctctgat tatcaccgtg tactatgtat accagtctgt attggtagcg     180
   caggacaact tatctattat cgctcaagaa cttcagtcta atgcttctgc ccagactttt     240
   ttaaacaacc aagaagcttt ataccaatac gtaaccatac ctaagaacaa attaaacgat     300
   aactcgtcgg cgttcttaca agatgttcaa tcaacaaacc aagctgttgg agcttctcga     360
   caagcactac aaaaccaaat ttcaggttta ggaaatggtg ctcaggttat ctcaagtaac     420
   ttgaacacga acaataacat tatccaacag tctctacaag taggccaggc gttaattcaa     480
   acgttctcac aaattgtaag tttgatagca aacatttaa                            519
<210> 128
   <211> 172
   <212> PRT
   <213> Chlamydia psittaci
<400> 128
   Met Trp Phe Ser Ser Pro Ala Leu Gln Thr Ser Pro Arg Ala Ala Ile
   1 5 10 15
   Asp Val Pro Gly Thr Ser Ile Thr Gly Gly Pro Asn Thr Ala Thr Ala
   20 25 30
   Asp Glu Ile Ile Ala Lys Phe Ala Lys Asp Ser Asn Pro Leu Ile Ile
   35 40 45
   Thr Val Tyr Tyr Val Tyr Gln Ser Val Leu Val Ala Gln Asp Asn Leu
   50 55 60
   Ser Ile Ile Ala Gln Glu Leu Gln Ser Asn Ala Ser Ala Gln Thr Phe
   65 70 75 80
   Leu Asn Asn Gln Glu Ala Leu Tyr Gln Tyr Val Thr Ile Pro Lys Asn
   85 90 95
   Lys Leu Asn Asp Asn Ser Ser Ala Phe Leu Gln Asp Val Gln Ser Thr
   100 105 110
   Asn Gln Ala Val Gly Ala Ser Arg Gln Ala Leu Gln Asn Gln Ile Ser
   115 120 125
   Gly Leu Gly Asn Gly Ala Gln Val Ile Ser Ser Asn Leu Asn Thr Asn
   130 135 140
   Asn Asn Ile Ile Gln Gln Ser Leu Gln Val Gly Gln Ala Leu Ile Gln
   145 150 155 160
   Thr Phe Ser Gln Ile Val Ser Leu Ile Ala Asn Ile
   165 170
<210> 129
   <211> 1443
   <212> DNA
   <213> Chlamydia psittaci
<400> 129
   atgaacactc ccctgccttc agcagttccc tctaccaaca tgacgttaaa ggaagatgcc 60
   tcctcttcat cctcagcatc aacatcttcc agtattttaa agactgcagc aggagatgtt 120
   gcgctttccg tattcacttc cgaaggaacc acaccagctt ccttaaactc tctagttgct 180
   cttgcccttg cgcaaatttc tgcagcttct ggagaaaatg ccaatccttt acaagattgt 240
   gctcataatc ttgtcttcct ttctccagaa actattgaag ttgaaatact catctctgat 300
   cttcttcaaa ctttagagac tacagatctt ataaacacac aggaagagtc ctcaagttta 360
   ggaaaacaag aacagcgcct tcctcaagaa ggatgcaaac cacaggattt agcaccaaga 420
   tctacaatag attcgtacgg cacaccgaaa gcattacaac aacctgccgt gaaactcgct 480
   gtccgctatt cttctgcaaa ggctcctgat tctcggcctt atacaagttc ctcctcaccg 540
   cagcatactt cgggacaatt tgcccaacgt gctgcccagg cgccaggaat actgcaacac 600
   tcccaaacaa aaaaagatgg agagcaaatt tcttctcaat ctcacaattc ctttattgcg 660
   gagaaaaaag aacagcagat tttcaccaca aagtctcaag aatctcagca ggatcgtgaa 720
   aaccgagatc aaaaacaaga tcgccaacat gatggccaac atcaagatga tgatgatgat 780
   cagcaaaaag gtagggggaa aaaatataaa tcaaagactt ctgccgaagc agttcctgct 840
   gatctctccg tagcgcatct acgctatctt aatgaggtac gccaatctcg agaaattcat 900
   gttgaggaag aaaaaacatt taagaagaaa gcgcaatctc cgatggcact tttttctgct 960
   ccaactcctc cagcaggatt taccccaatt cctactccaa agattgaaaa cgtattcata 1020
   cgttttatga aacttatgga aaggattctg ggacaggccg aagccgaagc ccaagaatta 1080
   tatcttcgcg ttaaagagcg taccgataat gtagacacat taatgctgct catttctaaa 1140
   attaactctg aaaaaggtgc tattgactgg cgagatgatt cagaaatgaa agctctcgtg 1200
   gatcaagcaa aaaaactggg tgtaacgata acagatactc tgcaatggtc tgaagaagag 1260
   aaaaaacttc taaaagaaaa tattcagatg cgtaaagaaa acctggaaaa aatcacccag 1320
   cttgaacgaa cagacatgca aaggcatctt caagaagtct cacaatgtca ccaagcacgc 1380
   tccaacgcct tgaaacttct caaagaactc atggatacct ttatttacaa tttgcgtccg 1440
   taa 1443
<210> 130
   <211> 480
   <212> PRT
   <213> Chlamydia psittaci

<400> 130
   Met Asn Thr Pro Leu Pro Ser Ala Val Pro Ser Thr Asn Met Thr Leu
   1               5                   10                  15
   Lys Glu Asp Ala Ser Ser Ser Ser Ser Ala Ser Thr Ser Ser Ser Ile
               20                  25                  30
   Leu Lys Thr Ala Ala Gly Asp Val Ala Leu Ser Val Phe Thr Ser Glu
           35                  40                  45
   Gly Thr Thr Pro Ala Ser Leu Asn Ser Leu Val Ala Leu Ala Leu Ala
       50                  55                  60
   Gln Ile Ser Ala Ala Ser Gly Glu Asn Ala Asn Pro Leu Gln Asp Cys
   65                  70                  75                  80
   Ala His Asn Leu Val Phe Leu Ser Pro Glu Thr Ile Glu Val Glu Ile
                   85                  90                  95
   Leu Ile Ser Asp Leu Leu Gln Thr Leu Glu Thr Thr Asp Leu Ile Asn
               100                 105                 110
   Thr Gln Glu Glu Ser Ser Ser Leu Gly Lys Gln Glu Gln Arg Leu Pro
           115                 120                 125
   Gln Glu Gly Cys Lys Pro Gln Asp Leu Ala Pro Arg Ser Thr Ile Asp
       130                 135                 140
   Ser Tyr Gly Thr Pro Lys Ala Leu Gln Gln Pro Ala Val Lys Leu Ala
   145 150 155 160
   Val Arg Tyr Ser Ser Ala Lys Ala Pro Asp Ser Arg Pro Tyr Thr Ser
   165 170 175
   Ser Ser Ser Pro Gln His Thr Ser Gly Gln Phe Ala Gln Arg Ala Ala
   180 185 190
   Gln Ala Pro Gly Ile Leu Gln His Ser Gln Thr Lys Lys Asp Gly Glu
   195 200 205
   Gln Ile Ser Ser Gln Ser His Asn Ser Phe Ile Ala Glu Lys Lys Glu
   210 215 220
   Gln Gln Ile Phe Thr Thr Lys Ser Gln Glu Ser Gln Gln Asp Arg Glu
   225 230 235 240
   Asn Arg Asp Gln Lys Gln Asp Arg Gln His Asp Gly Gln His Gln Asp
   245 250 255
   Asp Asp Asp Asp Gln Gln Lys Gly Arg Gly Lys Lys Tyr Lys Ser Lys
   260 265 270
   Thr Ser Ala Glu Ala Val Pro Ala Asp Leu Ser Val Ala His Leu Arg
   275 280 285
   Tyr Leu Asn Glu Val Arg Gln Ser Arg Glu Ile His Val Glu Glu Glu
   290 295 300
   Lys Thr Phe Lys Lys Lys Ala Gln Ser Pro Met Ala Leu Phe Ser Ala
   305 310 315 320
   Pro Thr Pro Pro Ala Gly Phe Thr Pro Ile Pro Thr Pro Lys Ile Glu
   325 330 335
   Asn Val Phe Ile Arg Phe Met Lys Leu Met Glu Arg Ile Leu Gly Gln
   340 345 350
   Ala Glu Ala Glu Ala Gln Glu Leu Tyr Leu Arg Val Lys Glu Arg Thr
   355 360 365
   Asp Asn Val Asp Thr Leu Met Leu Leu Ile Ser Lys Ile Asn Ser Glu
   370 375 380
   Lys Gly Ala Ile Asp Trp Arg Asp Asp Ser Glu Met Lys Ala Leu Val
   385 390 395 400
   Asp Gln Ala Lys Lys Leu Gly Val Thr Ile Thr Asp Thr Leu Gln Trp
   405 410 415
   Ser Glu Glu Glu Lys Lys Leu Leu Lys Glu Asn Ile Gln Met Arg Lys
   420 425 430
   Glu Asn Leu Glu Lys Ile Thr Gln Leu Glu Arg Thr Asp Met Gln Arg
   435 440 445
   His Leu Gln Glu Val Ser Gln Cys His Gln Ala Arg Ser Asn Ala Leu
   450 455 460
   Lys Leu Leu Lys Glu Leu Met Asp Thr Phe Ile Tyr Asn Leu Arg Pro
   465 470 475 480
<210> 131
   <211> 1083
   <212> DNA
   <213> Chlamydia psittaci
<400> 131
   atgaagaaca aaacactaag cgttttctct ttggtggggt tggtttgttc ggtaggagtt 60
   ttaccaggca atgaaagttc tctccctgta caacgggagt atccaagtcg tacagaaaga 120
   atccctgaag atcctgccgg aattgctatt catgatcgtg tgttatttaa aatcgatgaa 180
   gataacgttg ttacgacttt agatgttata cagaagctta accttctttt tgcttcttca 240
   taccctcagc ttatggattc ttatcctgcg cgttcgcaat actatacagc catgtggcca 300
   gtagttttag aatctgtaat agacgagttt cttatggttg ccgatgctaa gactaaaaaa 360
   atccaggtgg actctactac agtgaatgaa gaaattgaag caatgtttgg tagagatcta 420
   tctcctttgt atgtacattt tgacatgacc cccgaagatg ttttcaacgt ggtaaatcgt 480
   accctaattg ctcagagagt catgggtatg atggtgcgct ctaaggtaat gttgaaagtt 540
   accccaggga aaattcgcga acattataat cagctggctg aagatgcagc aaacactact 600
   gtatggaaat acagagttgt tacgattaaa gcagctacag agtcattatc gagccaaatt 660
   gccgataaag tctgcgctag gttaaatgaa acgcaaagct ggaataaaga gcgtttatct 720
   gctcttactc tttctcaagg agggcagttt gtctgttctg aagagtttac acgcaatgat 780
   aaagaattat cagaagctca taaaatggaa ttgtcctcgg taaactaccc acaaactatt 840
   tgcagcctac ctaaagccca taagtcagga cataagcttt atgtgcttct tgataaatct 900
   gcaatggcaa tgcagcccct agaagaaatg gaaacgcaga taaagcagac gctatttatg 960
   aattatgctg ggactataga aagtcagtat aaaatgaaat tgcgtacacg ctatggattt 1020
   gactcttcaa cgattgctaa attgctttct gaagaagctc cgcctctatt ttcattgctt 1080
   tag 1083
<210> 132
   <211> 360
   <212> PRT
   <213> Chlamydia psittaci
<400> 132
   Met Lys Asn Lys Thr Leu Ser Val Phe Ser Leu Val Gly Leu Val Cys
   1 5 10 15
   Ser Val Gly Val Leu Pro Gly Asn Glu Ser Ser Leu Pro Val Gln Arg
   20 25 30
   Glu Tyr Pro Ser Arg Thr Glu Arg Ile Pro Glu Asp Pro Ala Gly Ile
   35 40 45
   Ala Ile His Asp Arg Val Leu Phe Lys Ile Asp Glu Asp Asn Val Val
   50 55 60
   Thr Thr Leu Asp Val Ile Gln Lys Leu Asn Leu Leu Phe Ala Ser Ser
   65 70 75 80
   Tyr Pro Gln Leu Met Asp Ser Tyr Pro Ala Arg Ser Gln Tyr Tyr Thr
   85 90 95
   Ala Met Trp Pro Val Val Leu Glu Ser Val Ile Asp Glu Phe Leu Met
   100 105 110
   Val Ala Asp Ala Lys Thr Lys Lys Ile Gln Val Asp Ser Thr Thr Val
   115 120 125
   Asn Glu Glu Ile Glu Ala Met Phe Gly Arg Asp Leu Ser Pro Leu Tyr
   130 135 140
   Val His Phe Asp Met Thr Pro Glu Asp Val Phe Asn Val Val Asn Arg
   145 150 155 160
   Thr Leu Ile Ala Gln Arg Val Met Gly Met Met Val Arg Ser Lys Val
   165 170 175
   Met Leu Lys Val Thr Pro Gly Lys Ile Arg Glu His Tyr Asn Gln Leu
   180 185 190
   Ala Glu Asp Ala Ala Asn Thr Thr Val Trp Lys Tyr Arg Val Val Thr
   195 200 205
   Ile Lys Ala Ala Thr Glu Ser Leu Ser Ser Gln Ile Ala Asp Lys Val
   210 215 220
   Cys Ala Arg Leu Asn Glu Thr Gln Ser Trp Asn Lys Glu Arg Leu Ser
   225 230 235 240
   Ala Leu Thr Leu Ser Gln Gly Gly Gln Phe Val Cys Ser Glu Glu Phe
   245 250 255
   Thr Arg Asn Asp Lys Glu Leu Ser Glu Ala His Lys Met Glu Leu Ser
   260 265 270
   Ser Val Asn Tyr Pro Gln Thr Ile Cys Ser Leu Pro Lys Ala His Lys
   275 280 285
   Ser Gly His Lys Leu Tyr Val Leu Leu Asp Lys Ser Ala Met Ala Met
   290 295 300
   Gln Pro Leu Glu Glu Met Glu Thr Gln Ile Lys Gln Thr Leu Phe Met
   305 310 315 320
   Asn Tyr Ala Gly Thr Ile Glu Ser Gln Tyr Lys Met Lys Leu Arg Thr
   325 330 335
   Arg Tyr Gly Phe Asp Ser Ser Thr Ile Ala Lys Leu Leu Ser Glu Glu
   340 345 350
   Ala Pro Pro Leu Phe Ser Leu Leu
   355 360
<210> 133
   <211> 34
   <212> DNA
   <213> Artificial sequence
<220>
   <223> synthetic DNA
<400> 133
   agtcaagctt atgattgtac ggacatagag accg 34
<210> 134
   <211> 35
   <212> DNA
   <213> Artificial sequence
<220>
   <223> synthetic DNA
<400> 134
   agtcaagctt gtaggtttat taaaggggat gtacc 35
<210> 135
   <211> 34
   <212> DNA
   <213> Artificial sequence
<220>
   <223> synthetic DNA
<400> 135
   agtcaagctt gtagctgttc ttttcagaga gctt 34
<210> 136
   <211> 33
   <212> DNA
   <213> Artificial sequence
<220>
   <223> synthetic DNA
<400> 136
   agtcaagctt tactttttga aggctagtac gtt 33
<210> 137
   <211> 33
   <212> DNA
   <213> Artificial Sequence
<220>

   <223> synthetic DNA
<400> 137
   agtcaagctt ggaaggaata tcgtttacct gct 33
<210> 138
   <211> 33
   <212> DNA
   <213> Artificial sequence
<220>
   <223> Synthetic DNA
<400> 138
   agtcaagctt gtagatgccc atcctaccaa cag 33
<210> 139
   <211> 34
   <212> DNA
   <213> Artificial Sequence
<220>
   <223> synthetic DNA
<400> 139
   agtcaagctt tccctaaaat gaataaacta agga 34
<210> 140
   <211> 33
   <212> DNA
   <213> Artificial sequence
<220>
   <223> Synthetic DNA
<400> 140
   agtcaagctt aagtgtcatt gaaaaggttc agg 33
<210> 141
   <211> 32
   <212> DNA
   <213> Artificial Sequence
<220>
   <223> Synthetic DNA
<400> 141
   agtcaagctt gccaaccaac ggttagacga aa 32
<210> 142
   <211> 34
   <212> DNA
   <213> Artificial Sequence
<220>
   <223> Synthetic DNA
<400> 142
   agtcaagctt gcgtgccttt tagaaaattt agca 34
<210> 143
   <211> 35
   <212> DNA
   <213> Artificial Sequence
<220>
   <223> Synthetic DNA
<400> 143
   agtcaagctt gtaacgactc cttctttcaa cgatt 35
<210> 144
   <211> 33
   <212> DNA
   <213> Artificial sequence
<220>
   <223> Synthetic DNA
<400> 144
   agtcaagctt ccattaagga tctaaaacaa ttt 33
<210> 145
   <211> 33
   <212> DNA
   <213> Artificial Sequence
<220>
   <223> Synthetic DNA
<400> 145
   agtcaagctt tgtttgagat gaattcgcat ttt 33
<210> 146
   <211> 34
   <212> DNA
   <213> Artificial Sequence
<220>
   <223> Synthetic DNA
<400> 146
   agtcaagctt ttgcatgaat tcgtaaaata gaaa 34
<210> 147
   <211> 35
   <212> DNA
   <213> Artificial Sequence
<220>
   <223> Synthetic DNA
<400> 147
   agtcaagctt gtagggtttg tggagaaaat tgtta 35
<210> 148
   <211> 33
   <212> DNA
   <213> Artificial Sequence
<220>
   <223> Synthetic DNA
<400> 148
   agtcaagctt cttctattag cctgtttcca att 33
<210> 149
   <211> 34
   <212> DNA
   <213> Artificial sequence
<220>
   <223> synthetic DNA
<400> 149
   agtcaagctt ctgttgttga attaatagct tctt 34
<210> 150
   <211> 35
   <212> DNA
   <213> Artificial Sequence
<220>

   <223> synthetic DNA
<400> 150
   agtcaagctt gtgagaaaaa caacaattct tatcc 35
<210> 151
   <211> 34
   <212> DNA
   <213> Artificial Sequence
<220>
   <223> Synthetic DNA
<400> 151
   agtcaagctt acgactcaat taacagtgat gcaa 34
<210> 152
   <211> 34
   <212> DNA
   <213> Artificial Sequence
<220>
   <223> Synthetic DNA
<400> 152
   agtcaagctt tcaagacaat tagagagaaa gagc 34
<210> 153
   <211> 35
   <212> DNA
   <213> Artificial Sequence
<220>
   <223> synthetic DNA
<400> 153
   agtcaagctt gtaggcattg cttaaaaata aaatg 35
<210> 154
   <211> 35
   <212> DNA
   <213> Artificial Sequence
<220>
   <223> Synthetic DNA
<400> 154
   agtcaagctt gtaatcggat tttaaaccaa ctttt 35
<210> 155
   <211> 35
   <212> DNA
   <213> Artificial Sequence
<220>
   <223> Synthetic DNA
<400> 155
   agtcaagctt gtaggaattt ttattacgag tttca 35
<210> 156
   <211> 33
   <212> DNA
   <213> Artificial Sequence
<220>
   <223> Synthetic DNA
<400> 156
   agtcaagctt caagaagcgt taagaaaacg aaa 33
<210> 157
   <211> 34
   <212> DNA
   <213> Artificial Sequence
<220>
   <223> Synthetic DNA
<400> 157
   agtcaagctt ttattaaaat ttgttaaagg gagg 34
<210> 158
   <211> 35
   <212> DNA
   <213> Artificial Sequence
<220>
   <223> Synthetic DNA
<400> 158
   agtcaagctt gtaaactaat tcggattaaa aatga 35
<210> 159
   <211> 34
   <212> DNA
   <213> Artificial Sequence
<220>
   <223> Synthetic DNA

<400> 159
   agtcaagctt gaaggaaaag aagttctcat aaag 34
<210> 160
   <211> 32
   <212> DNA
   <213> Artificial Sequence
<220>
   <223> Synthetic DNA
<400> 160
   agtcaagctt gtagcgtctg gaaaaattct gg 32
<210> 161
   <211> 33
   <212> DNA
   <213> Artificial Sequence
<220>
   <223> Synthetic DNA
<400> 161
   agtcaagctt gggtaagaac accttctaat atg 33
<210> 162
   <211> 32
   <212> DNA
   <213> Artificial Sequence
<220>
   <223> Synthetic DNA
<400> 162
   gactaagctt gtaatctatt tttagatagg aa 32
<210> 163
   <211> 33
   <212> DNA
   <213> Artificial Sequence
<220>
   <223> Synthetic DNA
<400> 163
   agtcaagctt cccgtaatta ctgtccgtac aac 33
<210> 164
   <211> 31
   <212> DNA
   <213> Artificial Sequence
<220>
   <223> Synthetic DNA
<400> 164
   gactaagctt attatataga cagattaaaa t 31
<210> 165
   <211> 32
   <212> DNA
   <213> Artificial Sequence
<220>
   <223> Synthetic DNA
<400> 165
   agtcaagctt agggttttaa gtcggtttga tg 32
<210> 166
   <211> 34
   <212> DNA
   <213> Artificial Sequence
<220>
   <223> Synthetic DNA
<400> 166
   agtcaagctt tcgataatca taattcaaag cgta 34
<210> 167
   <211> 34
   <212> DNA
   <213> Artificial Sequence
<220>
   <223> Synthetic DNA
<400> 167
   agtcaagctt tccatgaatt ctaaaatgat tagg 34
<210> 168
   <211> 32
   <212> DNA
   <213> Artificial Sequence
<220>
   <223> Synthetic DNA
<400> 168
   agtcaagctt aatttaaagg aactcaggtg aa 32
<210> 169
   <211> 33
   <212> DNA
   <213> Artificial Sequence
<220>
   <223> synthetic DNA
<400> 169
   agtcaagctt gtaacatggg gtatggaaag acc 33
<210> 170
   <211> 33
   <212> DNA
   <213> Artificial Sequence
<220>
   <223> Synthetic DNA
<400> 170
   agtcaagctt gtaagcacct gctctcgaat aca 33
<210> 171
   <211> 34
   <212> DNA
   <213> Artificial Sequence
<220>
   <223> Synthetic DNA
<400> 171
   agtcaagctt gtaacggtct ctttgcttgt tttt 34
<210> 172
   <211> 34
   <212> DNA
   <213> Artificial Sequence
<220>
   <223> Synthetic DNA
<400> 172
   agtcaagctt gtaacgatcc gaacttatcg tagt 34
<210> 173
   <211> 34
   <212> DNA
   <213> Artificial Sequence
<220>
   <223> synthetic DNA
<400> 173
   agtcaagctt gtaaattcat gggcctaatg ataa 34
<210> 174
   <211> 34
   <212> DNA
   <213> Artificial Sequence
<220>
   <223> Synthetic DNA
<400> 174
   agtcaagctt gtaattgggg aatctctggg agac 34
<210> 175
   <211> 32
   <212> DNA
   <213> Artificial Sequence
<220>
   <223> Synthetic DNA
<400> 175
   agtcaagctt gtaatggagg attggctaag ga 32
<210> 176
   <211> 35
   <212> DNA
   <213> Artificial Sequence
<220>
   <223> synthetic DNA
<400> 176
   agtcaagctt gtaagaggga ccttacctat tgctt 35
<210> 177
   <211> 31
   <212> DNA
   <213> Artificial Sequence
<220>
   <223> Synthetic DNA
<400> 177
   agtcaagctt gcggaatggt taaaggaaac g 31
<210> 178
   <211> 33
   <212> DNA
   <213> Artificial sequence
<220>
   <223> synthetic DNA
<400> 178
   agtcaagctt gtaaaaccag cctagccttc aaa 33
<210> 179
   <211> 34
   <212> DNA
   <213> Artificial Sequence
<220>
   <223> synthetic DNA
<400> 179
   agtcaagctt gtaacctgct tctttaggaa ctac 34
<210> 180
   <211> 34
   <212> DNA
   <213> Artificial Sequence
<220>
   <223> Synthetic DNA
<400> 180
   agtcaagctt gtaaaattca gcacaggctt gtaa 34
<210> 181
   <211> 32
   <212> DNA
   <213> Artificial Sequence
<220>
   <223> Synthetic DNA
<400> 181
   gactaagctt gtaaattgga gattgtagta gc 32
<210> 182
   <211> 32
   <212> DNA
   <213> Artificial Sequence
<220>
   <223> Synthetic DNA
<400> 182
   atgcaagctt acgagcaaga gcataaaatc ca 32
<210> 183
   <211> 33
   <212> DNA
   <213> Artificial Sequence
<220>
   <223> Synthetic DNA
<400> 183
   agtcaagctt gaattgctaa cagacactaa agg 33
<210> 184
   <211> 35
   <212> DNA
   <213> Artificial Sequence
<220>
   <223> Synthetic DNA
<400> 184
   agtcaagctt gttttaaaaa gccttgtaag gaggt 35
<210> 185
   <211> 36
   <212> DNA
   <213> Artificial Sequence
<220>
   <223> Synthetic DNA
<400> 185
   agtcaagctt gtaagaatta ccataaatca gaggaa 36
<210> 186
   <211> 36
   <212> DNA
   <213> Artificial Sequence
<220>
   <223> synthetic DNA
<400> 186
   agtcaagctt gtaacagaag gaaaagcata ccactg 36
<210> 187
   <211> 33
   <212> DNA
   <213> Artificial Sequence
<220>
   <223> Synthetic DNA
<400> 187
   agtcaagctt gccataagtc gtttacaaga tcg 33
<210> 188
   <211> 31
   <212> DNA
   <213> Artificial Sequence
<220>
   <223> synthetic DNA
<400> 188
   agtcaagctt gtgaatcagg gggaagctag t 31
<210> 189
   <211> 32
   <212> DNA
   <213> Artificial Sequence
<220>
   <223> Synthetic DNA
<400> 189
   agtcaagctt cgcaagatga tataaaggtc ca 32
<210> 190
   <211> 34
   <212> DNA
   <213> Artificial Sequence
<220>
   <223> Synthetic DNA
<400> 190
   agtcaagctt taaataacca agtattgggg ttta 34
<210> 191
   <211> 34
   <212> DNA
   <213> Artificial Sequence
<220>
   <223> Synthetic DNA
<400> 191
   agtcaagctt gtagacatgg ctgtcagatc ttgg 34
<210> 192
   <211> 33
   <212> DNA
   <213> Artificial Sequence
<220>
   <223> synthetic DNA
<400> 192
   agtcaagctt ctctttttaa agacaacgcg aat 33
<210> 193
   <211> 35
   <212> DNA
   <213> Artificial Sequence
<220>
   <223> Synthetic DNA
<400> 193
   agtcaagctt gtaattaatc ttgcggagat gttgg 35
<210> 194
   <211> 33
   <212> DNA
   <213> Artificial sequence
<220>
   <223> Synthetic DNA
<400> 194
   agtcaagctt gtaagcctgc ttggtcttct gtt 33
<210> 195
   <211> 34
   <212> DNA
   <213> Artificial Sequence
<220>
   <223> Synthetic DNA
<400> 195
   atgcaagctt gtaagggaag atgtccgttc agtt 34
<210> 196
   <211> 34
   <212> DNA
   <213> Artificial Sequence
<220>
   <223> Synthetic DNA
<400> 196
   agtcaagctt atggctatga agagcaattt attc 34
<210> 197
   <211> 30
   <212> DNA
   <213> Artificial Sequence
<220>
   <223> synthetic DNA
<400> 197
   agtcaagctt gtaactcccg caccaacctc 30
<210> 198
   <211> 35
   <212> DNA
   <213> Artificial Sequence
<220>
   <223> synthetic DNA
<400> 198
   agtcaagctt gtaatctggt taacacatgc tgagg 35
<210> 199
   <211> 35
   <212> DNA
   <213> Artificial Sequence
<220>
   <223> Synthetic DNA
<400> 199
   agtcaagctt gtaagagccg agcatagata gaaac 35
<210> 200
   <211> 34
   <212> DNA
   <213> Artificial Sequence
<220>
   <223> Synthetic DNA
<400> 200
   agtcaagctt ttaataagct gaataaacca atga 34
<210> 201
   <211> 34
   <212> DNA
   <213> Artificial sequence
<220>
   <223> Synthetic DNA
<400> 201
   agtcaagctt gtaacatgac gacatcacct tatt 34
<210> 202
   <211> 34
   <212> DNA
   <213> Artificial Sequence
<220>
   <223> Synthetic DNA
<400> 202
   agtcaagctt gtaagtcagg aagtccgtgg tgat 34
<210> 203
   <211> 32
   <212> DNA
   <213> Artificial Sequence
<220>
   <223> synthetic DNA
<400> 203
   agtcaagctt gtaatttgcg gttgggaaat aa 32
<210> 204
   <211> 34
   <212> DNA
   <213> Artificial sequence
<220>
   <223> synthetic DNA
<400> 204
   agtcaagctt gtaacccttg cctctatttt gaga 34
<210> 205
   <211> 37
   <212> DNA
   <213> Artificial Sequence
<220>
   <223> Synthetic DNA
<400> 205
   agtcaagctt gtaattgcga gaaggattaa atcttag 37
<210> 206
   <211> 32
   <212> DNA
   <213> Artificial Sequence
<220>
   <223> synthetic DNA
<400> 206
   agtcaagctt atctcaaaca tcaagtgctg aa 32
<210> 207
   <211> 31
   <212> DNA
   <213> Artificial Sequence
<220>
   <223> Synthetic DNA
<400> 207
   agtcaagctt gtaagcctcg tcaaatcctg a 31
<210> 208
   <211> 31
   <212> DNA
   <213> Artificial Sequence
<220>
   <223> Synthetic DNA
<400> 208
   agtcaagctt tcttcaggaa ctttaaaaac a 31
<210> 209
   <211> 33
   <212> DNA
   <213> Artificial Sequence
<220>
   <223> synthetic DNA
<400> 209
   agtcaagctt taatagagct cattggagga gga 33
<210> 210
   <211> 36
   <212> DNA
   <213> Artificial sequence
<220>
   <223> Synthetic DNA
<400> 210
   agtcaagctt gtaatgtaga aaagcgcaga gagaaa 36
<210> 211
   <211> 35
   <212> DNA
   <213> Artificial Sequence
<220>
   <223> Synthetic DNA
<400> 211
   agtcaagctt gtaatcgaaa accagttagg ttgaa 35
<210> 212
   <211> 34
   <212> DNA
   <213> Artificial Sequence
<220>
   <223> synthetic DNA
<400> 212
   agtcaagctt gtaatgggtc ctaatggagg cttt 34
<210> 213
   <211> 32
   <212> DNA
   <213> Artificial Sequence
<220>
   <223> synthetic DNA
<400> 213
   agtcaagctt gacctccttg taaccattct cg 32
<210> 214
   <211> 33
   <212> DNA
   <213> Artificial Sequence
<220>
   <223> Synthetic DNA
<400> 214
   agtcaagctt tccttaagag gataaatcca cag 33
<210> 215
   <211> 33
   <212> DNA
   <213> Artificial Sequence
<220>
   <223> Synthetic DNA
<400> 215
   agtcaagctt ttcgaaaaca taaataatgt gga 33
<210> 216
   <211> 33
   <212> DNA
   <213> Artificial Sequence
<220>
   <223> synthetic DNA
<400> 216
   agtcaagctt gtgcaggaaa atgatgtttt agc 33
<210> 217
   <211> 30
   <212> DNA
   <213> Artificial Sequence
<220>
   <223> Synthetic DNA
<400> 217
   agtcaagctt cagcctggtt tcgtaatttt 30
<210> 218
   <211> 36
   <212> DNA
   <213> Artificial Sequence
<220>
   <223> Synthetic DNA
<400> 218
   agtcaagctt gtaaaccaca ccaatattat ttagga 36
<210> 219
   <211> 34
   <212> DNA
   <213> Artificial Sequence
<220>
   <223> Synthetic DNA
<400> 219
   agtcaagctt gtaacctggg caggtcaaaa tcta 34
<210> 220
   <211> 34
   <212> DNA
   <213> Artificial Sequence
<220>
   <223> synthetic DNA
<400> 220
   agtcaagctt gtaatgggtt tccggtttca atca 34
<210> 221
   <211> 35
   <212> DNA
   <213> Artificial sequence
<220>
   <223> Synthetic DNA
<400> 221
   agtcaagctt gtaagccaag agcaggataa aacga 35
<210> 222
   <211> 35
   <212> DNA
   <213> Artificial Sequence
<220>
   <223> Synthetic DNA
<400> 222
   agtcaagctt gtaaattttt cctataagct ggttc 35
<210> 223
   <211> 35
   <212> DNA
   <213> Artificial Sequence
<220>
   <223> Synthetic DNA
<400> 223
   agtcaagctt ttgataagaa attagccaac atagg 35
<210> 224
   <211> 33
   <212> DNA
   <213> Artificial Sequence
<220>
   <223> synthetic DNA
<400> 224
   agtcaagctt ttcggtttaa gtaatagaag tgg 33
<210> 225
   <211> 35
   <212> DNA
   <213> Artificial Sequence
<220>
   <223> Synthetic DNA
<400> 225
   agtcaagctt gtaagacggt gccttcaata acaaa 35
<210> 226
   <211> 33
   <212> DNA
   <213> Artificial Sequence
<220>
   <223> synthetic DNA
<400> 226
   agtcaagctt gtaaaaacag ggaaagacga tga 33
<210> 227
   <211> 32
   <212> DNA
   <213> Artificial sequence
<220>
   <223> Synthetic DNA
<400> 227
   agtcaagctt gtaagcggtt acgtggagtc aa 32
<210> 228
   <211> 33
   <212> DNA
   <213> Artificial Sequence
<220>
   <223> Synthetic DNA
<400> 228
   agtcaagctt gtaatccgga gcctttctcc tat 33
<210> 229
   <211> 34
   <212> DNA
   <213> Artificial Sequence
<220>
   <223> synthetic DNA
<400> 229
   agtcaagctt gtaaagatga atgattccag aaag 34
<210> 230
   <211> 33
   <212> DNA
   <213> Artificial Sequence
<220>
   <223> Synthetic DNA
<400> 230
   agtcaagctt ggactaagta aaacggagca gga 33
<210> 231
   <211> 34
   <212> DNA
   <213> Artificial Sequence
<220>
   <223> Synthetic DNA
<400> 231
   agtcaagctt ttcgaggttt atttaattct tcca 34
<210> 232
   <211> 32
   <212> DNA
   <213> Artificial Sequence
<220>
   <223> Synthetic DNA
<400> 232
   agtcaagctt acgtatcaac cgtaaaatgg tg 32
<210> 233
   <211> 32
   <212> DNA
   <213> Artificial Sequence
<220>
   <223> Synthetic DNA
<400> 233
   agtcaagctt gtaagcaaaa acaacgggaa tc 32
<210> 234
   <211> 36
   <212> DNA
   <213> Artificial Sequence
<220>
   <223> Synthetic DNA
<400> 234
   agtcaagctt gtaaatgaag agtgctgtgt taaagt 36
<210> 235
   <211> 34
   <212> DNA
   <213> Artificial Sequence
<220>
   <223> Synthetic DNA
<400> 235
   agtcaagctt gtaacacaat cttgggcaag agac 34
<210> 236
   <211> 36
   <212> DNA
   <213> Artificial Sequence
<220>
   <223> Synthetic DNA
<400> 236
   agtcaagctt gtaagagaag agaaaggcta aggatg 36
<210> 237
   <211> 31
   <212> DNA
   <213> Artificial Sequence
<220>
   <223> synthetic DNA
<400> 237
   agtcaagctt ataaacccct gtcttaaccc a 31
<210> 238
   <211> 30
   <212> DNA
   <213> Artificial sequence
<220>
   <223> Synthetic DNA
<400> 238
   agtcaagctt tcctttctaa aaggcctgga 30
<210> 239
   <211> 35
   <212> DNA
   <213> Artificial sequence
<220>
   <223> Synthetic DNA
<400> 239
   agtcaagctt gcaaatctaa ggagttaggt taggg 35
<210> 240
   <211> 32
   <212> DNA
   <213> Artificial Sequence
<220>
   <223> Synthetic DNA

<400> 240
   agtcaagctt gtaatacagg atggtgccca ct 32
<210> 241
   <211> 29
   <212> DNA
   <213> Artificial Sequence
<220>
   <223> Synthetic DNA
<400> 241
   agtcaagctt gcgcaaataa acgatacaa 29
<210> 242
   <211> 33
   <212> DNA
   <213> Artificial Sequence
<220>
   <223> Synthetic DNA
<400> 242
   agtcaagctt cattgtgtta taattgcagg cta 33
<210> 243
   <211> 31
   <212> DNA
   <213> Artificial Sequence
<220>
   <223> synthetic DNA
<400> 243
   agtcaagctt tgacgcccct taaaataaag a 31
<210> 244
   <211> 30
   <212> DNA
   <213> Artificial sequence
<220>
   <223> Synthetic DNA
<400> 244
   agtcaagctt ccgcaaaatg gtttaacaga 30
<210> 245
   <211> 36
   <212> DNA
   <213> Artificial Sequence
<220>
   <223> Synthetic DNA
<400> 245
   agtcaagctt gtaagcttgg gaatctacac attttt 36
<210> 246
   <211> 33
   <212> DNA
   <213> Artificial Sequence
<220>
   <223> Synthetic DNA
<400> 246
   agtcaagctt gtaaaagctg aataagccca tga 33
<210> 247
   <211> 33
   <212> DNA
   <213> Artificial Sequence
<220>
   <223> synthetic DNA
<400> 247
   agtcaagctt gtaaccgtga ggagtctgaa caa 33
<210> 248
   <211> 35
   <212> DNA
   <213> Artificial Sequence
<220>
   <223> synthetic DNA
<400> 248
   agtcaagctt ttaatcgaag aaaataggta ggaaa 35
<210> 249
   <211> 35
   <212> DNA
   <213> Artificial Sequence
<220>
   <223> Synthetic DNA
<400> 249
   agtcaagctt gtaacgcttc tacatcccca taatt 35
<210> 250
   <211> 29
   <212> DNA
   <213> Artificial Sequence
<220>
   <223> Synthetic DNA
<400> 250
   agtctctaga tgcttttcgg accatagtc 29
<210> 251
   <211> 31
   <212> DNA
   <213> Artificial Sequence
<220>
   <223> Synthetic DNA
<400> 251
   agtctctaga atcctcttcc caaggaatca g 31
<210> 252
   <211> 30
   <212> DNA
   <213> Artificial Sequence
<220>
   <223> Synthetic DNA
<400> 252
   agtctctaga tcgaatcgat ttggaaggag 30
<210> 253
   <211> 33
   <212> DNA
   <213> Artificial Sequence
<220>
   <223> Synthetic DNA
<400> 253
   agtctctaga ttcaataatg ccagagcttt ttc 33
<210> 254
   <211> 30
   <212> DNA
   <213> Artificial Sequence
<220>
   <223> Synthetic DNA
<400> 254
   agtctctaga ttcatccacc caaatagcac 30
<210> 255
   <211> 29
   <212> DNA
   <213> Artificial Sequence
<220>
   <223> synthetic DNA
<400> 255
   agtctctaga agtaagaggg agcccagga 29
<210> 256
   <211> 30
   <212> DNA
   <213> Artificial Sequence
<220>
   <223> Synthetic DNA
<400> 256
   agtctctaga tgacggggga ggtgtattag 30
<210> 257
   <211> 31
   <212> DNA
   <213> Artificial Sequence
<220>
   <223> synthetic DNA
<400> 257
   agtctctaga taggaggcct ttcgattgtt t 31
<210> 258
   <211> 29
   <212> DNA
   <213> Artificial Sequence
<220>
   <223> Synthetic DNA
<400> 258
   agcttctaga tggaaaggat acggattgg 29
<210> 259
   <211> 30
   <212> DNA
   <213> Artificial Sequence
<220>
   <223> Synthetic DNA
<400> 259
   agtctctaga gaatggggga atacaaatca 30
<210> 260
   <211> 30
   <212> DNA
   <213> Artificial Sequence
<220>
   <223> synthetic DNA
<400> 260
   agtctctaga ggcgagagaa agtttcgtta 30
<210> 261
   <211> 29
   <212> DNA
   <213> Artificial Sequence
<220>
   <223> synthetic DNA
<400> 261
   agtctctaga caacttgttc atgcgacag 29
<210> 262
   <211> 30
   <212> DNA
   <213> Artificial Sequence
<220>
   <223> Synthetic DNA
<400> 262
   agcttctaga cgcatccgaa agacttttct 30
<210> 263
   <211> 32
   <212> DNA
   <213> Artificial Sequence
<220>
   <223> Synthetic DNA
<400> 263
   agtctctaga acgttctaag ccgtaattct cg 32
<210> 264
   <211> 30
   <212> DNA
   <213> Artificial Sequence
<220>
   <223> Synthetic DNA
<400> 264
   agtctctaga tgcggggatc gtataagcta 30
<210> 265
   <211> 31
   <212> DNA
   <213> Artificial Sequence
<220>
   <223> synthetic DNA
<400> 265
   agtctctaga aggtgattgc ttcccaagtc t 31
<210> 266
   <211> 31
   <212> DNA
   <213> Artificial Sequence
<220>
   <223> Synthetic DNA
<400> 266
   agtctctaga ataagagtcg agggcttgca c 31
<210> 267
   <211> 32
   <212> DNA
   <213> Artificial Sequence
<220>
   <223> Synthetic DNA
<400> 267
   agtctctaga tgtaggaata cctggagtcg tg 32
<210> 268
   <211> 31
   <212> DNA
   <213> Artificial Sequence
<220>
   <223> Synthetic DNA
<400> 268
   agtctctaga gcgctgagta gcgtcgtgta a 31
<210> 269
   <211> 31
   <212> DNA
   <213> Artificial Sequence
<220>
   <223> Synthetic DNA
<400> 269
   agcttctaga gaattcaaga tgatccaaac a 31
<210> 270
   <211> 30
   <212> DNA
   <213> Artificial Sequence
<220>
   <223> Synthetic DNA
<400> 270
   agtctctaga agtcttatga agcaaagcag 30
<210> 271
   <211> 33
   <212> DNA
   <213> Artificial Sequence
<220>
   <223> synthetic DNA
<400> 271
   agtctctaga atttatatgc cacgccttct ttc 33
<210> 272
   <211> 29
   <212> DNA
   <213> Artificial Sequence
<220>
   <223> synthetic DNA
<400> 272
   agtctctaga ggaggtttcc gagacgatt 29
<210> 273
   <211> 30
   <212> DNA
   <213> Artificial Sequence
<220>
   <223> synthetic DNA
<400> 273
   agtctctaga atcccagtcg tgagaagcat 30
<210> 274
   <211> 34
   <212> DNA
   <213> Artificial Sequence
<220>
   <223> Synthetic DNA
<400> 274
   agtctctaga gagaagaata tctgcaacta gagc 34
<210> 275
   <211> 30
   <212> DNA
   <213> Artificial Sequence
<220>
   <223> Synthetic DNA
<400> 275
   agtctctaga ggtaacagtg catgcgaaag 30
<210> 276
   <211> 32
   <212> DNA
   <213> Artificial Sequence
<220>
   <223> Synthetic DNA
<400> 276
   agtctctaga caaaatagga atagcagctt gg 32
<210> 277
   <211> 33
   <212> DNA
   <213> Artificial Sequence
<220>
   <223> Synthetic DNA
<400> 277
   agtctctaga cggtcttaaa ctacttttca atg 33
<210> 278
   <211> 32
   <212> DNA
   <213> Artificial Sequence
<220>
   <223> synthetic DNA
<400> 278
   agtctctaga atcaaccaca tcttctggac aa 32
<210> 279
   <211> 31
   <212> DNA
   <213> Artificial Sequence
<220>
   <223> Synthetic DNA
<400> 279
   gacttctaga tccaggtttt tcggaagcag a 31
<210> 280
   <211> 28
   <212> DNA
   <213> Artificial Sequence
<220>
   <223> Synthetic DNA
<400> 280
   agtctctaga agtagacggg agttgctg 28
<210> 281
   <211> 31
   <212> DNA
   <213> Artificial Sequence
<220>
   <223> Synthetic DNA
<400> 281
   gacttctaga cttaaaaaat acccaggaac a 31
<210> 282
   <211> 30
   <212> DNA
   <213> Artificial Sequence
<220>
   <223> Synthetic DNA
<400> 282
   agtctctaga ccgaatcgct tcattcgtag 30
<210> 283
   <211> 32
   <212> DNA
   <213> Artificial Sequence
<220>
   <223> Synthetic DNA
<400> 283
   agtctctaga aaggatcaga tgtgctttag gg 32
<210> 284
   <211> 30
   <212> DNA
   <213> Artificial Sequence
<220>
   <223> Synthetic DNA
<400> 284
   agtctctaga cgaaaattgg gtagcctgac 30
<210> 285
   <211> 30
   <212> DNA
   <213> Artificial Sequence
<220>
   <223> Synthetic DNA
<400> 285
   agtctctaga atcaaagttg ctgcaggatt 30
<210> 286
   <211> 30
   <212> DNA
   <213> Artificial Sequence
<220>
   <223> Synthetic DNA
<400> 286
   agtctctaga ctcggcatcc ttttgttttg 30
<210> 287
   <211> 30
   <212> DNA
   <213> Artificial Sequence
<220>
   <223> Synthetic DNA
<400> 287
   agtctctaga ttcttcttgg tgcctgctaa 30
<210> 288
   <211> 30
   <212> DNA
   <213> Artificial Sequence
<220>
   <223> synthetic DNA
<400> 288
   agtctctaga agtgcagctg gatagggttg 30
<210> 289
   <211> 30
   <212> DNA
   <213> Artificial Sequence
<220>
   <223> Synthetic DNA
<400> 289
   agtctctaga tccggatgcg gtagtagttg 30
<210> 290
   <211> 31
   <212> DNA
   <213> Artificial Sequence
<220>
   <223> Synthetic DNA
<400> 290
   agtctctaga taaccctgtg gatgacgttt t 31
<210> 291
   <211> 30
   <212> DNA
   <213> Artificial Sequence
<220>
   <223> Synthetic DNA
<400> 291
   agtctctaga gggactaacg acctcagcac 30
<210> 292
   <211> 30
   <212> DNA
   <213> Artificial Sequence
<220>
   <223> Synthetic DNA
<400> 292
   agtctctaga aacaccaccg acatcacaaa 30
<210> 293
   <211> 30
   <212> DNA
   <213> Artificial Sequence
<220>
   <223> Synthetic DNA
<400> 293
   agtctctaga aatgaggacc tctccttcgt 30
<210> 294
   <211> 31
   <212> DNA
   <213> Artificial Sequence
<220>
   <223> Synthetic DNA
<400> 294
   agtctctaga ttgtccatca aagcctacaa t 31
<210> 295
   <211> 29
   <212> DNA
   <213> Artificial Sequence
<220>
   <223> synthetic DNA
<400> 295
   agtctctaga gctttttgca taggggaag 29
<210> 296
   <211> 31
   <212> DNA
   <213> Artificial Sequence
<220>
   <223> Synthetic DNA
<400> 296
   agtctctaga ttgtagacca gcggtgacac t 31
<210> 297
   <211> 30
   <212> DNA
   <213> Artificial Sequence
<220>
   <223> Synthetic DNA
<400> 297
   agtctctaga gcgttctgga gtgacgaaac 30
<210> 298
   <211> 31
   <212> DNA
   <213> Artificial Sequence
<220>
   <223> Synthetic DNA
<400> 298
   gacttctaga aacaattgta tgattccatc c 31
<210> 299
   <211> 30
   <212> DNA
   <213> Artificial Sequence
<220>
   <223> Synthetic DNA
<400> 299
   atgctctaga cctttggcgg actttttctt 30
<210> 300
   <211> 30
   <212> DNA
   <213> Artificial Sequence
<220>
   <223> Synthetic DNA
<400> 300
   agtctctaga tacccattct tgaccggaaa 30
<210> 301
   <211> 30
   <212> DNA
   <213> Artificial Sequence
<220>
   <223> Synthetic DNA
<400> 301
   agtctctaga ctgagcaaag gagctgacag 30
<210> 302
   <211> 32
   <212> DNA
   <213> Artificial Sequence
<220>
   <223> Synthetic DNA
<400> 302
   agtctctaga agcatcagtg caatgaggat aa 32
<210> 303
   <211> 29
   <212> DNA
   <213> Artificial Sequence
<220>
   <223> synthetic DNA
<400> 303
   agtctctaga tccaaaacga tcctgttcc 29
<210> 304
   <211> 30
   <212> DNA
   <213> Artificial Sequence
<220>
   <223> Synthetic DNA
<400> 304
   agtctctaga ttccacacaa gagaccacca 30
<210> 305
   <211> 30
   <212> DNA
   <213> Artificial Sequence
<220>
   <223> Synthetic DNA
<400> 305
   agtctctaga tcgtgtttgc tttccttcca 30
<210> 306
   <211> 30
   <212> DNA
   <213> Artificial Sequence
<220>
   <223> synthetic DNA
<400> 306
   agtctctaga gacagtgcct tgtgttgatg 30
<210> 307
   <211> 30
   <212> DNA
   <213> Artificial Sequence
<220>
   <223> Synthetic DNA
<400> 307
   agtctctaga tcgaagcagc gcatgtaact 30
<210> 308
   <211> 31
   <212> DNA
   <213> Artificial Sequence
<220>
   <223> Synthetic DNA
<400> 308
   agtctctaga agagtcgcgt cctatagacc a 31
<210> 309
   <211> 30
   <212> DNA
   <213> Artificial Sequence
<220>
   <223> Synthetic DNA
<400> 309
   agcttctaga cgtgtgagtc ccctgaactt 30
<210> 310
   <211> 32
   <212> DNA
   <213> Artificial Sequence
<220>
   <223> Synthetic DNA
<400> 310
   agtctctaga ctctgtagtc agctggtcgt tg 32
<210> 311
   <211> 31
   <212> DNA
   <213> Artificial sequence
<220>
   <223> synthetic DNA
<400> 311
   agtctctaga cgagagagga agttcagcgt a 31
<210> 312
   <211> 31
   <212> DNA
   <213> Artificial Sequence
<220>
   <223> synthetic DNA
<400> 312
   atgctctaga agctccctta acgacttctg g 31
<210> 313
   <211> 32
   <212> DNA
   <213> Artificial Sequence
<220>
   <223> synthetic DNA
<400> 313
   agtctctaga agggtaacac actaggggaa ga 32
<210> 314
   <211> 31
   <212> DNA
   <213> Artificial Sequence
<220>
   <223> Synthetic DNA
<400> 314
   agtctctaga tccttcagac caacgctgat a 31
<210> 315
   <211> 30
   <212> DNA
   <213> Artificial Sequence
<220>
   <223> synthetic DNA
<400> 315
   agtctctaga tgacaggccg tttctatcaa 30
<210> 316
   <211> 29
   <212> DNA
   <213> Artificial Sequence
<220>
   <223> synthetic DNA
<400> 316
   agtctctaga gcctgcaatc aatccctgt 29
<210> 317
   <211> 29
   <212> DNA
   <213> Artificial Sequence
<220>
   <223> synthetic DNA
<400> 317
   agtctctaga ttctgcggaa ggaagctgt 29
<210> 318
   <211> 30
   <212> DNA
   <213> Artificial Sequence
<220>
   <223> Synthetic DNA
<400> 318
   agtctctaga agcggcagaa aatgagaaaa 30
<210> 319
   <211> 33
   <212> DNA
   <213> Artificial Sequence
<220>
   <223> synthetic DNA
<400> 319
   agtctctaga caaagcaatt agagtgaacg aca 33
<210> 320
   <211> 30
   <212> DNA
   <213> Artificial Sequence
<220>
   <223> Synthetic DNA
<400> 320
   agtctctaga agcacaggca caacgtttac 30
<210> 321
   <211> 30
   <212> DNA
   <213> Artificial Sequence
<220>
   <223> Synthetic DNA
<400> 321
   agtctctaga cggtaccaag ggccattttg 30
<210> 322
   <211> 30
   <212> DNA
   <213> Artificial sequence
<220>
   <223> synthetic DNA
<400> 322
   agtctctaga aagatgtcgg gaaagtgtcg 30
<210> 323
   <211> 30
   <212> DNA
   <213> Artificial Sequence
<220>
   <223> synthetic DNA
<400> 323
   agtctctaga ttggcttgcc ttatcattcg 30
<210> 324
   <211> 29
   <212> DNA
   <213> Artificial Sequence
<220>
   <223> Synthetic DNA
<400> 324
   agtctctaga gacgacttgc ttgctcact 29
<210> 325
   <211> 30
   <212> DNA
   <213> Artificial Sequence
<220>
   <223> Synthetic DNA
<400> 325
   agtctctaga tgctgcgaca cgaatttcta 30
<210> 326
   <211> 31
   <212> DNA
   <213> Artificial Sequence
<220>
   <223> Synthetic DNA
<400> 326
   agtctctaga gaggagggaa acaccgttaa t 31
<210> 327
   <211> 29
   <212> DNA
   <213> Artificial Sequence
<220>
   <223> Synthetic DNA
<400> 327
   agtctctaga cccttccaat tgaggaaaa 29
<210> 328
   <211> 30
   <212> DNA
   <213> Artificial Sequence
<220>
   <223> Synthetic DNA
<400> 328
   agtctctaga tgcaatgata tcgtcagcag 30
<210> 329
   <211> 30
   <212> DNA
   <213> Artificial Sequence
<220>
   <223> Synthetic DNA
<400> 329
   agtctctaga cacctggatt ttgccttcag 30
<210> 330
   <211> 29
   <212> DNA
   <213> Artificial Sequence
<220>
   <223> synthetic DNA
<400> 330
   agtctctaga cttccatggt aaaggagca 29
<210> 331
   <211> 30
   <212> DNA
   <213> Artificial Sequence
<220>
   <223> synthetic DNA
<400> 331
   agtctctaga ttctgcagca acaacagctt 30
<210> 332
   <211> 30
   <212> DNA
   <213> Artificial Sequence
<220>
   <223> Synthetic DNA
<400> 332
   agtctctaga agcttcttga ggcgctacag 30
<210> 333
   <211> 31
   <212> DNA
   <213> Artificial Sequence
<220>
   <223> Synthetic DNA
<400> 333
   agtctctaga tccagaggct gtggaaactg t 31
<210> 334
   <211> 29
   <212> DNA
   <213> Artificial Sequence
<220>
   <223> synthetic DNA
<400> 334
   agtctctaga agcatgaggc tgtatgagg 29
<210> 335
   <211> 32
   <212> DNA
   <213> Artificial Sequence
<220>
   <223> Synthetic DNA
<400> 335
   agtctctaga tgcttgtaga agagcagaat cg 32
<210> 336
   <211> 31
   <212> DNA
   <213> Artificial Sequence
<220>
   <223> Synthetic DNA
<400> 336
   agtctctaga cccagatgaa gtcactggaa c 31
<210> 337
   <211> 31
   <212> DNA
   <213> Artificial sequence
<220>
   <223> Synthetic DNA
<400> 337
   agtctctaga acggatttct tcctggtgtc t 31
<210> 338
   <211> 31
   <212> DNA
   <213> Artificial sequence
<220>
   <223> Synthetic DNA
<400> 338
   agtctctaga tgatatgctc ccattctttc g 31
<210> 339
   <211> 31
   <212> DNA
   <213> Artificial Sequence
<220>
   <223> Synthetic DNA
<400> 339
   agtctctaga tccttcgtat acgccagtac c 31
<210> 340
   <211> 30
   <212> DNA
   <213> Artificial sequence
<220>
   <223> Synthetic DNA
<400> 340
   agtctctaga gcatccacgt ttgttccatt 30
<210> 341
   <211> 30
   <212> DNA
   <213> Artificial Sequence
<220>
   <223> synthetic DNA
<400> 341
   agtctctaga agcattccct gtaccagacc 30
<210> 342
   <211> 30
   <212> DNA
   <213> Artificial Sequence
<220>
   <223> Synthetic DNA
<400> 342
   agtctctaga agccgaacga gctaagacat 30
<210> 343
   <211> 30
   <212> DNA
   <213> Artificial Sequence
<220>
   <223> synthetic DNA
<400> 343
   agtctctaga gaccaaagct ccctcttgaa 30
<210> 344
   <211> 30
   <212> DNA
   <213> Artificial Sequence
<220>
   <223> Synthetic DNA
<400> 344
   agtctctaga ggcatacgcc tgtaattgct 30
<210> 345
   <211> 27
   <212> DNA
   <213> Artificial Sequence
<220>
   <223> Synthetic DNA
<400> 345
   agtctctaga ttcttcaggg ccagcaa 27
<210> 346
   <211> 30
   <212> DNA
   <213> Artificial Sequence
<220>
   <223> Synthetic DNA
<400> 346
   agtctctaga aggaaagcct atcgcacaca 30
<210> 347
   <211> 31
   <212> DNA
   <213> Artificial Sequence
<220>
   <223> Synthetic DNA
<400> 347
   agtctctaga agaccaactc gtcccatttt c 31
<210> 348
   <211> 30
   <212> DNA
   <213> Artificial Sequence
<220>
   <223> Synthetic DNA
<400> 348
   agtctctaga gtgggctttg gttacatcgt 30
<210> 349
   <211> 30
   <212> DNA
   <213> Artificial Sequence
<220>
   <223> Synthetic DNA
<400> 349
   agtctctaga agttccttgc gtggatgttt 30
<210> 350
   <211> 31
   <212> DNA
   <213> Artificial Sequence
<220>
   <223> Synthetic DNA
<400> 350
   agtctctaga catccgatct gctttctctt g 31
<210> 351
   <211> 30
   <212> DNA
   <213> Artificial Sequence
<220>
   <223> synthetic DNA
<400> 351
   agtctctaga atgggagaga gcttcttgct 30
<210> 352
   <211> 31
   <212> DNA
   <213> Artificial Sequence
<220>
   <223> Synthetic DNA
<400> 352
   agtctctaga gatctccagc ttaagggttg c 31
<210> 353
   <211> 31
   <212> DNA
   <213> Artificial sequence
<220>
   <223> synthetic DNA
<400> 353
   agtctctaga agggttctcc tcaagttcat c 31
<210> 354
   <211> 30
   <212> DNA
   <213> Artificial Sequence
<220>
   <223> Synthetic DNA
<400> 354
   agtctctaga cgttgcagag ttcgttgttc 30
<210> 355
   <211> 30
   <212> DNA
   <213> Artificial Sequence
<220>
   <223> synthetic DNA
<400> 355
   agcttctaga cagaacaacg tctcctacgc 30
<210> 356
   <211> 30
   <212> DNA
   <213> Artificial Sequence
<220>
   <223> synthetic DNA
<400> 356
   agcttctaga agccaaacgc atagcttcat 30
<210> 357
   <211> 30
   <212> DNA
   <213> Artificial Sequence
<220>
   <223> Synthetic DNA
<400> 357
   agtctctaga tagaagttgc aggcgttcct 30
<210> 358
   <211> 32
   <212> DNA
   <213> Artificial Sequence
<220>
   <223> Synthetic DNA
<400> 358
   agtctctaga tccgtagttg ttacggaagg tt 32
<210> 359
   <211> 30
   <212> DNA
   <213> Artificial Sequence
<220>
   <223> Synthetic DNA
<400> 359
   agtctctaga tttcttggct ccctgcatag 30
<210> 360
   <211> 30
   <212> DNA
   <213> Artificial sequence
<220>
   <223> Synthetic DNA
<400> 360
   agtctctaga cgcagaatgg gataggacat 30
<210> 361
   <211> 30
   <212> DNA
   <213> Artificial Sequence
<220>
   <223> Synthetic DNA
<400> 361
   agtctctaga ttgcacacct tggacgtact 30
<210> 362
   <211> 34
   <212> DNA
   <213> Artificial Sequence
<220>
   <223> synthetic DNA
<400> 362
   agtctctaga tttcgttaat tctcccttag acca 34
<210> 363
   <211> 30
   <212> DNA
   <213> Artificial Sequence
<220>
   <223> Synthetic DNA
<400> 363
   agtctctaga aagtttctgt gtcgctacgg 30
<210> 364
   <211> 31
   <212> DNA
   <213> Artificial Sequence
<220>
   <223> Synthetic DNA
<400> 364
   agtctctaga agaaaaatcc atgggctgta a 31
<210> 365
   <211> 30
   <212> DNA
   <213> Artificial Sequence
<220>
   <223> Synthetic DNA
<400> 365
   agtctctaga ttcatcggct gttgctgtat 30
<210> 366
   <211> 30
   <212> DNA
   <213> Artificial Sequence
<220>
   <223> Synthetic DNA
<400> 366
   agtctctaga gaatacggaa agcgcaacat 30
<210> 367
   <211> 27
   <212> DNA
   <213> Artificial Sequence
<220>
   <223> Synthetic DNA
<400> 367
   atcgtaccat ggagcaaccc aattgtg 27
<210> 368
   <211> 38
   <212> DNA
   <213> Artificial Sequence
<220>
   <223> Synthetic DNA
<400> 368
   atcgtaccat ggaattaaat aaaacatccg agtctttg 38
<210> 369
   <211> 33
   <212> DNA
   <213> Artificial sequence
<220>
   <223> Synthetic DNA
<400> 369
   atcgtaccat ggttctcgct tcttgtcttc ttg 33
<210> 370
   <211> 35
   <212> DNA
   <213> Artificial Sequence
<220>
   <223> Synthetic DNA
<400> 370
   atcgtaccat ggatgaaggt attcaaaccg tttct 35
<210> 371
   <211> 33
   <212> DNA
   <213> Artificial Sequence
<220>
   <223> Synthetic DNA
<400> 371
   atcgtaccat ggggttctct tctccagcac ttc 33
<210> 372
   <211> 36
   <212> DNA
   <213> Artificial Sequence
<220>
   <223> SynthetiC DNA
<400> 372
   atcgtaccat ggagaacaaa acactaagcg ttttct 36
<210> 373
   <211> 31
   <212> DNA
   <213> Artificial Sequence
<220>
   <223> Synthetic DNA
<400> 373
   agtcggtacc agcttcctta acttcgctga g 31
<210> 374
   <211> 32
   <212> DNA
   <213> Artificial Sequence
<220>
   <223> synthetic DNA
<400> 374
   agtcggtacc ttggttttgt tcttgatctt gc 32
<210> 375
   <211> 37
   <212> DNA
   <213> Artificial Sequence
<220>
   <223> Synthetic DNA
<400> 375
   agtcggtacc atctaagaaa tcatcttctg taaggac 37
<210> 376
   <211> 33
   <212> DNA
   <213> Artificial Sequence
<220>
   <223> Synthetic DNA
<400> 376
   agtcggtacc gtctctagga actagcgttt cca 33
<210> 377
   <211> 35
   <212> DNA
   <213> Artificial Sequence
<220>
   <223> synthetic DNA
<400> 377
   agtcggtacc aatgtttgct atcaaactta caatt 35
<210> 378
   <211> 29
   <212> DNA
   <213> Artificial Sequence
<220>
   <223> Synthetic DNA
<400> 378
   agtcggtacc caatgaaaat agaggcgga 29